# EUROPÄISCHE PATENTSCHRIFT

(11) **EP 2 611 801 B1**
(45) Veröffentlichungstag und Bekanntmachung des Hinweises auf die Patenterteilung: **04.05.2016**
(21) Anmeldenummer: 11748685.2
(22) Anmeldetag: 29.08.2011
(51) Int. Cl.: C07D 471/04, C07D 495/04, C07D 498/04, C07D 513/04, C07D 231/40, C07D 271/08, C07D 277/56, C07D 333/38, A01N 43/90

(54) **HERBIZID WIRKSAME KETOSULTAME UND DIKETOPYRIDINE**
HERBICIDALLY EFFECTIVE KETOSULTAMS AND DIKETOPYRIDINES
CÉTOSULTAMES ET DICÉTOPYRIDINES À EFFICACITÉ HERBICIDE

(30) Priorität: 01.09.2010 EP 10174905
(43) Veröffentlichungstag der Anmeldung: 10.07.2013
(73) Patentinhaber: Bayer Intellectual Property GmbH, 40789 Monheim (DE)
(72) Erfinder: LEHR, Stefan, 65835 Liederbach (DE); WALDRAFF, Christian, 61118 Bad Vilbel (DE); GATZWEILER, Elmar, 61231 bad Nauheim (DE); HÄUSER-HAHN, Isolde, 51375 Leverkusen (DE); HEINEMANN, Ines, 65719 Hofheim (DE); ROSINGER, Christopher, Hugh, 65719 Hofheim am Taunus (DE)
(74) Vertreter: BIP Patents
(86) Internationale Anmeldenummer: PCT/EP2011/064825
(87) Internationale Veröffentlichungsnummer: WO 2012/028582

(56) Entgegenhaltungen:
- WO-A1-2008/009908
- WO-A1-2008/071918
- WO-A1-2009/063180
- WO-A1-2010/049269
- WO-A1-2011/051212
- WO-A2-2009/090401

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Speziell betrifft sie arylsubstituierte Ketosultam- und Diketopyridin-Derivate, Verfahren zu ihrer Herstellung und ihre Verwendung als Herbizide.

Verschiedene Schriften beschreiben herbizid wirksame Diketopyridine, die mit 6-gliedrigen Carbo- oder Heterocyclen ein kondensiertes Ringsystem bilden. In WO2008/009908 A1 und WO2008/071918 A1 werden Diketopyridine mit kondensiertem Pyrazin beschrieben. In WO2009/090401 A1 und WO2010/049269 A1 werden Diketopyridine genannt, die mit einem Pyridinring kondensiert sind. WO2009/063180 beschreibt Ketosultame, die mit Pyrazinringen kondensiert sind.

WO 2011/051212 beschreibt Ketopyridine, die mit 5-gliedrigen Heterocyclen ein kondensiertes Ringsystem bilden.

Die aus diesen Schriften bekannten Verbindungen zeigen jedoch häufig eine nicht ausreichende herbizide Wirksamkeit. Aufgabe der vorliegenden Erfindung ist daher die Bereitstellung alternativer herbizid wirksamer Verbindungen.

Es wurde gefunden, daß Ketosultame und Diketopyridine, die einen ankondensierten gesättigten oder ungesättigten heterocyclischen Fünfring tragen, als Herbizide besonders gut geeignet sind.

Ein Gegenstand der vorliegenden Erfindung sind Ketosultame und Diketopyridine der Formel (I) oder deren Salze 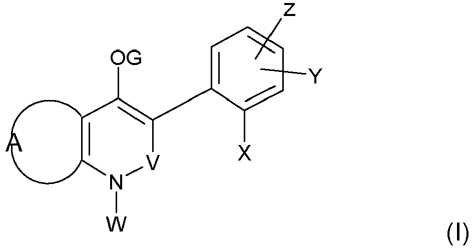 worin
A steht für einen ankondensierten gesättigten oder ungesättigten fünfgliedrigen Heterocyclus, der durch m Reste aus der Gruppe bestehend aus R¹⁰, R¹¹, R¹², R¹³ und R¹⁴ substituiert ist;
V bedeutet C(=O) oder S(O)₂;
m bedeutet 0, 1 oder 2;
n bedeutet 0, 1, 2 oder 3;
G bedeutet Wasserstoff, C(=O)R¹, C(=L)MR², SO₂R³, P(=L)R⁴R⁵, C(=L)NR⁶R⁷, E oder R⁸;
E bedeutet ein Metallionäquivalent oder ein Ammoniumion;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl, einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₄)-Alkyl und (C₁-C₄)-Alkoxy substituiertes (C₃-C₆)-Cycloalkyl, Phenyl, Phenyl-(C₁-C₄)-alkyl, Phenoxy-(C₁-C₄)-alkyl oder Heteroaryloxy-(C₁-C₄)-alkyl;
R² bedeutet jeweils durch n Halogenatome substituiertes (C₁-C₆)-Alkyl, (C₂-C₆)-Alkenyl, (C₁-C₄)-Alkoxy-(C₁-C₆)-alkyl oder Di-(C₁-C₄)-alkoxy-(C₁-C₆)-alkyl,
   oder durch jeweils n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl, (C₁-C₄)-Alkoxy, N-(C₁-C₆)-Alkylamino, N,N-Di-(C₁-C₆)-Alkylamino, (C₁-C₄)-Alkylthio, (C₂-C₄)-Alkenyl oder(C₃-C₆)-Cycloalkylthio,
   oder durch n Reste aus der Gruppe bestehend aus Halogen, (C₁-C₄)-Alkyl und (C₁-C₄)-Alkoxy substituiertes Phenyl, Benzyl, Phenoxy oder Phenylthio;
R⁶ und R⁷ bedeuten unabhängig voneinander jeweils Wasserstoff, durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₄)-Alkoxy-(C₁-C₆)-alkyl,
   durch jeweils n Reste aus der Gruppe bestehend aus Halogen, (C₁-C₄)-Alkyl und (C₁-C₄)-Alkoxy substituiertes Phenyl oder Benzyl,
   oder R⁶ und R⁷ bilden gemeinsam mit dem N-Atom, an das sie gebunden sind, einen 3- bis 6-gliedrigen Ring enthaltend 2 bis 5 Kohlenstoffatomen und 0 oder 1 Sauerstoff- oder Schwefelatome;
R⁸ bedeutet durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl,
   durch n Reste aus der Gruppe bestehend aus Halogen, (C₁-C₄)-Alkyl und (C₁-C₄)-Alkoxy substituiertes (C₃-C₆)-Cycloalkyl,
   einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₄)-Alkyl und (C₁-C₄)-Alkoxy substituiertes Phenyl, Phenyl-(C₁-C₄)-alkyl, Heteroaryl, Phenoxy-(C₁-C₄)-alkyl oder Heteroaryloxy-(C₁-C₄)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl-,
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl;
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl, und
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl.

Alkyl bedeutet gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methyl-propyl.

Halogenalkyl bedeutet geradkettige oder verzweigte Alkylgruppen mit 1 bis 8 Kohlenstoffatomen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit 2 bis 8 Kohlenstoffatomen und einer Doppelbindung in einer beliebigen Position, z. B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit 2 bis 8 Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy; Halogenalkoxy bedeutet geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2-difluorethoxy, 2,2-Dichlor-2-fluorethoxy, 2,2,2-Trichlorethoxy, Pentafluor-ethoxy und 1,1,1-Trifluorprop-2-oxy.

Alkylthio bedeutet gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
Halogenalkylthio bedeutet geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2-difluorethylthio, 2,2-Dichlor-2-fluorethylthio, 2,2,2-Trichlorethylthio, Pentafluorethylthio und 1,1,1-Trifluorprop-2-ylthio.

Heteroaryl bedeutet insbesondere 2-Furyl, 3-Furyl, 2-Thienyl, 3-Thienyl, 1-Pyrrolyl, 2-Pyrrolyl, 3-Pyrrolyl, 3-lsoxazolyl, 4-lsoxazolyl, 5-lsoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl. Dieses Heteroaryl ist - sofern nicht anders angegeben -jeweils unsubstituiert oder jeweils einfach oder mehrfach gleich oder verschieden substituiert durch Reste ausgewählt aus Fluor, Chlor, Brom, Iod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2,2,2-Trifluor-thoxy, 2,2-Dichlor-2-fluorethyl, 2,2-Difluor-2-chlorethyl, 2-Chlor-2-fluorethyl, 2,2,2-Trichlorethyl, 2,2,2-Trifluorethyl, 2-Fluorethyl, 2,2-Difluorethyl, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy.

Unter einem gesättigten oder ungesättigten fünfgliedrigen Heterocyclus ist ein fünfgliedriges Ringsystem zu verstehen, das außer Kohlenstoffatomen 1 bis 4 Heteroatome aus der Gruppe Sauerstoff, Schwefel und Stickstoff enthält. Beispiele für einen solchen Heterocyclus sind Furan, Thiophen, 1,2-Oxazol, 1,3-Oxazol, 1,2-Thiazol, 1,3-Thiaxazol, Imidazol, Pyrazol, 1,2-Diazol, 1,2,5-Oxadiazol und jeweils deren ungesättigten und teilweise gesättigten Analoga.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ein Metallionäquivalent bedeutet ein Metallion mit einer positiven Ladung wie Na⁺, K⁺, (Mg²⁺)_{1/2}, (Ca²⁺)_{1/2}, MgH⁺, CaH⁺, (Al³⁺)_{1/3} (Fe²⁺)_{1/2} oder (Fe³⁺)_{1/3}.

Halogen bedeutet Fluor, Chlor, Brom und Jod.

Ist eine Gruppe mehrfach durch Reste substituiert, so ist darunter zu verstehen, daß diese Gruppe durch ein oder mehrere gleiche oder verschiedene der genannten Reste substituiert ist.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.

Beispiele für anorganische Säuren sind Halogenwasserstoffsäuren wie Fluorwasserstoff, Chlorwasserstoff, Bromwasserstoff und lodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder - diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.
Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfaßt, jedoch nicht spezifisch definiert sind.

In allen nachfolgend genannten Formeln haben die Substituenten und Symbole, sofern nicht anders definiert, dieselbe Bedeutung wie unter Formel (I) beschrieben.

Bevorzugt sind Ketosultame und Diketopyridine der Formel (I), worin A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 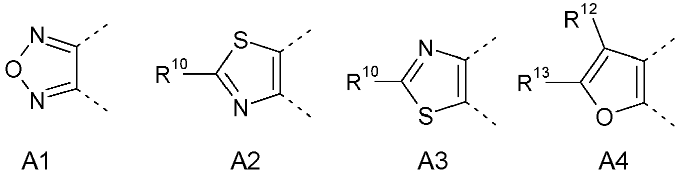 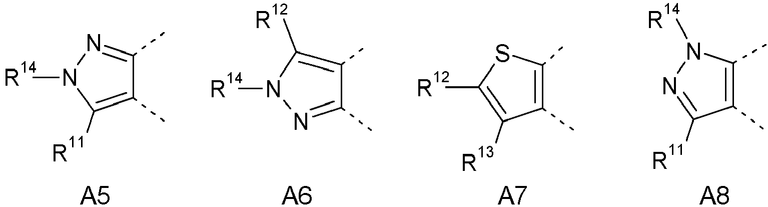 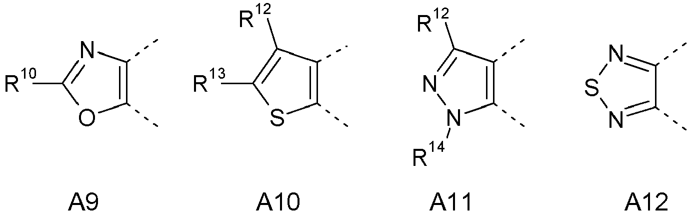
V bedeutet C(=O) oder S(O)₂;
n bedeutet 0, 1, 2 oder 3;
G bedeutet Wasserstoff, C(=O)R¹, C(=L)MR², SO₂R³, P(=L)R⁴R⁵, C(=L)NR⁶R⁷, E oder R⁸;
E bedeutet ein Metallionäquivalent oder ein Ammoniumion;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl, einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₄)-Alkyl und (C₁-C₄)-Alkoxy substituiertes (C₃-C₆)-Cycloalkyl, Phenyl, Phenyl-(C₁-C₄)-alkyl, Heteroaryl, Phenoxy-(C₁-C₄)-alkyl oder Heteroaryloxy-(C₁-C₄)-alkyl;
R² bedeutet jeweils durch n Halogenatome substituiertes (C₁-C₆)-Alkyl, (C₂-C₆)-Alkenyl, (C₁-C₄)-Alkoxy-(C₁-C₆)-alkyl oder Di-(C₁-C₄)-alkoxy-(C₁-C₆)-alkyl,
   oder durch jeweils n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl, (C₁-C₄)-Alkoxy, N-(C₁-C₆)-Alkylamino, N,N-Di-(C₁-C₆)-Alkylamino, (C₁-C₄)-Alkylthio, (C₂-C₄)-Alkenyl oder (C₃-C₆)-Cycloalkylthio,
   oder durch n Reste aus der Gruppe bestehend aus Halogen, (C₁-C₄)-Alkyl und (C₁-C₄)-Alkoxy substituiertes Phenyl, Benzyl, Phenoxy oder Phenylthio;
R⁶ und R⁷ bedeuten unabhängig voneinander jeweils Wasserstoff,
   durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₄)-Alkoxy-(C₁-C₆)-alkyl,
   durch jeweils n Reste aus der Gruppe bestehend aus Halogen, (C₁-C₄)-Alkyl und (C₁-C₄)-Alkoxy substituiertes Phenyl oder Benzyl;
   oder R⁶ und R⁷ bilden gemeinsam mit dem N-Atom, an das sie gebunden sind, einen 3- bis 6-gliedrigen Ring enthaltend 2 bis 5 Kohlenstoffatomen und 0 oder 1 Sauerstoff- oder Schwefelatome;
R⁸ bedeutet durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl,
   durch n Reste aus der Gruppe bestehend aus Halogen, (C₁-C₄)-Alkyl und (C₁-C₄)-Alkoxy substituiertes (C₃-C₆)-Cycloalkyl,
   einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₄)-Alkyl und (C₁-C₄)-Alkoxy substituiertes Phenyl, Phenyl-(C₁-C₄)-alkyl, Heteroaryl, Phenoxy-(C₁-C₄)-alkyl oder Heteroaryloxy-(C₁-C₄)-alkyl-,
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl-,
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

Besonders bevorzugt sind Ketosultame und Diketopyridine der Formel (I), worin A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 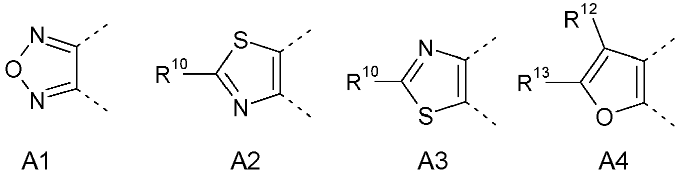 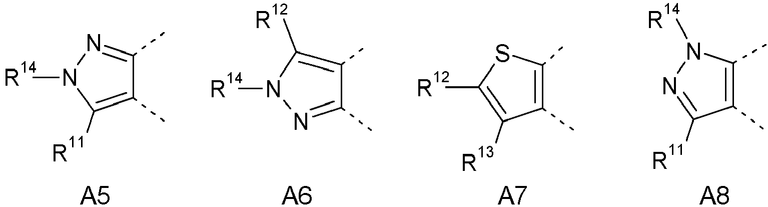 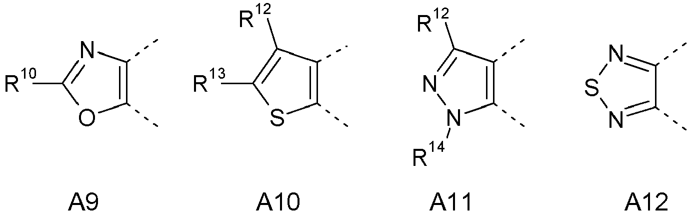
V bedeutet C(=O) oder S(O)₂;
n bedeutet 0, 1, 2 oder 3;
G bedeutet Wasserstoff;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl oder
   durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (Cᵢ₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆,)-alkyl**,** (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

Besonders bevorzugt sind auch Ketosultame und Diketopyridine der Formel (I), worin A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 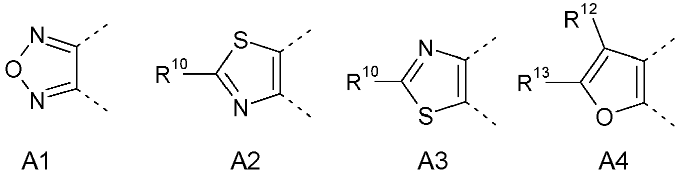 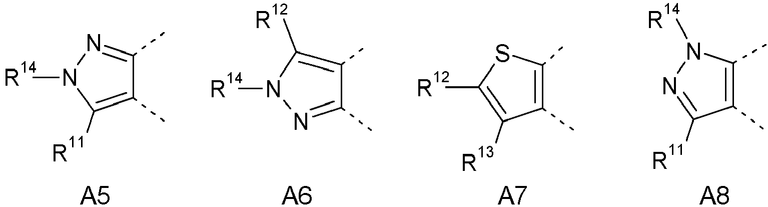 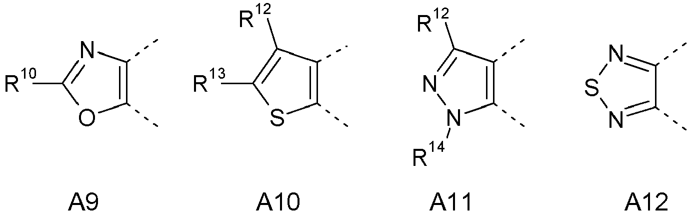
V bedeutet C(=O) oder S(O)₂;
n bedeutet 0, 1, 2 oder 3;
G bedeutet C(=O)R¹;
R¹ bedeutet jeweils durch n Halogenatome substituiertes (C₁-C₆)-Alkyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl-;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-( C₁-C₆)-alkyl, (C₁-C₄)-Alkylthio, (C₁-C₄)-Alkylsulfinyl oder (C₁-C₄)-Alkylsulfonyl oder
   durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

Besonders bevorzugt sind auch Ketosultame und Diketopyridine der Formel (I), worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 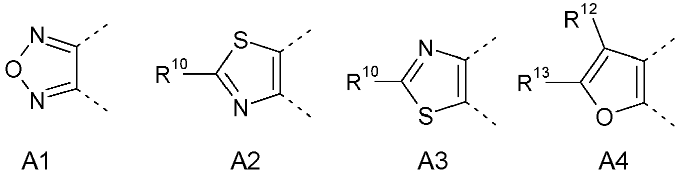 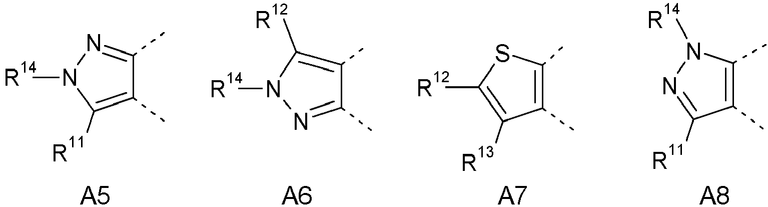 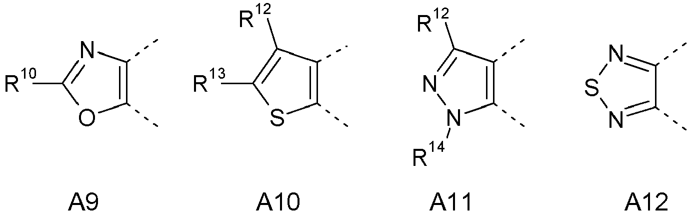
V bedeutet C(=O) oder S(O)₂;
n bedeutet 0, 1, 2 oder 3;
G bedeutet C(=L)MR²;
L bedeutet Sauerstoff;
M bedeutet Sauerstoff oder Schwefel;
R² bedeutet jeweils durch n Halogenatome substituiertes (C₁-C₆)-Alkyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl und
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl-;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl oder
   durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

Besonders bevorzugt sind auch Ketosultame und Diketopyridine der Formel (I), worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 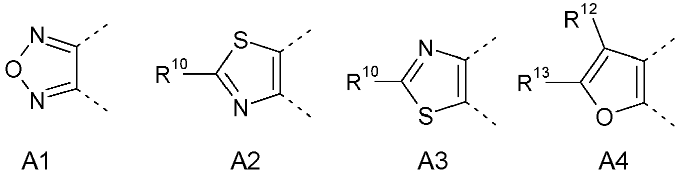 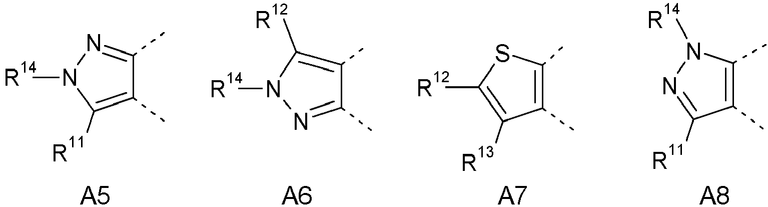 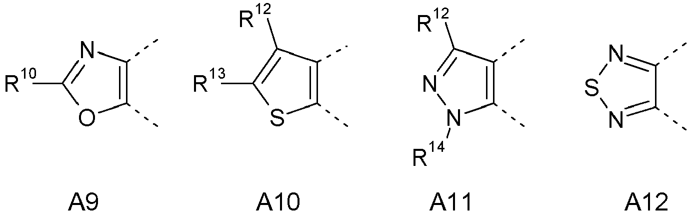
V bedeutet C(=O) oder S(O)₂;
n bedeutet 0, 1, 2 oder 3;
G bedeutet R⁸;
R⁸ bedeutet durch n Halogenatome substituiertes (C₁-C₆)-Alkyl, (C₂-C₆)-Alkenyl, (C₂-C₆)-Alkinyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-( C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl-;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
   durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

Ganz besonders bevorzugt sind die in den Tabellen 1 bis 124 angegebenen Verbindungen der Formel (I).

Die verwendeten Abkürzungen bedeuten:

| | | |
|---|---|---|
| Bz = Benzyl | c-Pr = cyclo-Propyl | Et = Ethyl |
| i-Bu = iso-Butyl | t-Bu = tertiär-Butyl | i-Pr = iso-Propyl |
| Me = Methyl | Ph = Phenyl | c = cyclo |

**Tabelle 1: Erfindungsgemäße Verbindungen der Formel (I), worin G für Wasserstoff steht, V für C=O steht, W für Difluorethyl steht, und A für A1 steht:**

| | | | |
|---|---|---|---|
| 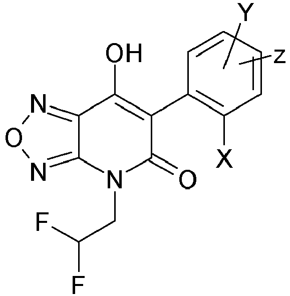 | | | |

| Nr. | X | Y | Z |
|---|---|---|---|
| 1 | F | H | H |
| 2 | Cl | H | H |
| 3 | Br | H | H |
| 4 | I | H | H |
| 5 | OMe | H | H |
| 6 | EtO | H | H |
| 7 | CF₃ | H | H |
| 8 | CN | H | H |
| 9 | NO₂ | H | H |
| 10 | OCF₃ | H | H |
| 11 | H | 3-CF₃ | H |
| 12 | H | 3-Me | H |
| 13 | H | 3-F | H |
| 14 | H | 3-Cl | H |
| 15 | H | 3-CN | H |
| 16 | H | 3-Br | H |
| 17 | H | 3-I | H |
| 18 | H | 3-NO₂ | H |
| 19 | H | 3-OCF₃ | H |
| 20 | H | 3-OMe | H |
| 21 | H | 3-EtO | H |
| 22 | H | 4-CF₃ | H |
| 23 | H | 4-Me | H |
| 24 | H | 4-F | H |
| 25 | H | 4-Cl | H |
| 26 | H | 4-CN | H |
| 27 | H | 4-Br | H |
| 28 | H | 4-I | H |
| 29 | H | 4-NO₂ | H |
| 30 | H | 4-OCF₃ | H |
| 31 | H | 4-OMe | H |
| 32 | H | 4-EtO | H |
| 33 | Cl | 4-Cl | H |
| 34 | H | 3-Cl | 4-Cl |
| 35 | Br | 4-Cl | H |
| 36 | Cl | H | 6-Cl |
| 37 | Cl | H | 6-F |
| 38 | F | H | 6-F |
| 39 | Me | 4-Cl | H |
| 40 | Me | 4-Br | H |
| 41 | Me | 4-I | H |
| 42 | Cl | 4-Cl | 6-Cl |
| 43 | Cl | 6-Me | 4-Br |
| 44 | Cl | 6-Me | 4-Cl |
| 45 | Br | 6-Me | 4-Cl |
| 46 | Br | 6-Me | 4-Br |
| 47 | OMe | 6-Me | 4-Cl |
| 48 | EtO | 6-Me | 4-Cl |
| 49 | Cl | 6-Me | 4-Br |
| 50 | Cl | 6-Et | 4-Cl |
| 51 | Br | 6-Et | 4-Cl |
| 52 | Br | 6-Et | 4-Br |
| 53 | OMe | 6-Et | 4-Cl |
| 54 | EtO | 6-Et | 4-Cl |
| 55 | Br | 4-Me | 6-Br |
| 56 | Cl | 4-Me | 6-Cl |
| 57 | OMe | 4-Me | 6-Me |
| 58 | EtO | 4-Me | 6-Me |
| 59 | OMe | 6-Et | 4-Me |
| 60 | EtO | 6-Et | 4-Me |
| 61 | Cl | 4-Me | 6-Et |
| 62 | Et | 6-Et | 4-Cl |
| 63 | Et | 6-Me | 4-Br |
| 64 | Et | 6-Et | 4-Br |
| 65 | Et | 6-Me | 4-Cl |
| 66 | Et | 6-Me | 4-Br |
| 67 | OMe | 4-Me | 6-Cl |
| 68 | EtO | 4-Me | 6-Cl |
| 69 | I | H | 4-Me |
| 70 | I | 6-Me | H |
| 71 | I | 6-Et | H |
| 72 | I | 4-Me | 6-Me |
| 73 | I | 6-Et | 4-Me |
| 74 | I | 6-Me | 4-Cl |
| 75 | I | 6-Et | 6-Cl |
| 76 | I | 6-Cl | 4-Me |
| 77 | Me | 4-I | H |
| 78 | Et | 4-I | H |
| 79 | Et | 4-I | 6-Me |
| 80 | Et | 4-I | 6-Et |
| 81 | Cl | 6-Me | 4-I |
| 82 | Cl | 6-Et | 4-I |
| 83 | c-Pr | H | H |
| 84 | c-Pr | 4-Me | H |
| 85 | c-Pr | H | 6-Me |
| 86 | c-Pr | 6-Et | H |
| 87 | c-Pr | 4-Me | 6-Me |
| 88 | c-Pr | 6-Et | 4-Me |
| 89 | c-Pr | 4-Me | 6-Cl |
| 90 | c-Pr | 6-Et | 4-Cl |
| 91 | c-Pr | 4-Cl | 6-Me |
| 92 | Me | 4-c-Pr | H |
| 93 | Et | 4-c-Pr | H |
| 94 | Me | 4-c-Pr | 6-Me |
| 95 | Et | 4-c-Pr | 6-Me |
| 96 | Et | 4-c-Pr | 6-Et |
| 97 | Cl | 6-Me | 4-c-Pr |
| 98 | Cl | 6-Et | 4-c-Pr |
| 99 | Et | 6-Et | 4-I |
| 100 | Cl | 6-F | 3-Me |
| 101 | F | 6-F | 3-F |
| 102 | EtO | 6-F | 3-F |
| 103 | F | 6-F | 3-EtO |
| 104 | F | H | 5-CI |
| 105 | H | 3-CF₃ | 5-CF₃ |
| 106 | Me | 4-OCF₃ | H |
| 107 | OCF₃ | 4-Me | H |
| 108 | OCF3 | 5-Me | H |
| 109 | OCF3 | 6-Me | H |
| 110 | OCF3 | 6-Et | H |
| 111 | Me | 5-OCF3 | H |
| 112 | Me | 3-OCF3 | 6-Me |
| 113 | Br | 4-OCF₃ | 6-CI |
| 114 | Br | 4-OCF₃ | 6-Br |
| 115 | OMe | 4-OCF₃ | 6-Br |
| 116 | OMe | 4-OCF₃ | 6-CI |
| 117 | Cl | 4-OCF₃ | 6-CI |
| 118 | OMe | 4-OCF₃ | 6-CI |
| 119 | OMe | 4-OCF₃ | 6-Br |
| 120 | Me | 4-OCF₃ | 6-Me |
| 121 | Cl | 4-OCF₃ | 6-Me |
| 122 | OCF₃ | 6-Cl | 4-Br |
| 123 | OCF₃ | 6-Me | 4-Me |
| 124 | OCF₃ | 6-OMe | 4-Cl |
| 125 | OCF₃ | 6-Cl | 4-Me |
| 126 | Cl | 5-OCF₃ | H |
| 127 | Br | 5-OCF₃ | H |
| 128 | OCF₃ | 6-Et | 4-Cl |
| 129 | Br | 4-Cl | 6-Br |
| 130 | Br | 4-Cl | 6-Cl |
| 131 | Br | 4-Me | 6-Cl |
| 132 | Cl | 3-Me | 6-Cl |
| 133 | Cl | 3-F | 6-F |
| 134 | F | 3-Me | 6-F |
| 135 | F | 4-OMe | 6-F |
| 136 | F | 3-OMe | 6-F |
| 137 | Cl | 3-Cl | 6-F |
| 138 | Cl | 4-Et | 6-Cl |
| 139 | Cl | 4-Et | 6-Br |
| 140 | Cl | 3-Br | 6-Cl |
| 141 | Cl | 4-CF₃ | 6-F |
| 142 | Cl | 4-CF₃ | 6-Cl |
| 143 | Cl | 3-Cl | 6-Cl |
| 144 | Cl | 3-CF₃ | 6-Cl |
| 145 | F | 3-F | 6-NO₂ |
| 146 | F | 4-NO₂ | 6-F |
| 147 | Cl | 4-CF₃ | 6-NO₂ |
| 148 | Br | 6-NO₂ | H |
| 149 | F | 4-CF₃ | 6-F |
| 150 | Br | 6-Br | H |
| 151 | Cl | 3-OMe | 6-F |
| 152 | F | 3-OMe | 6-Cl |
| 153 | F | 4-Cl | 6-F |
| 154 | F | 4-Br | 6-F |
| 155 | F | 4-Br | 6-Br |
| 156 | Cl | 4-Br | 6-Cl |
| 157 | F | 4-EtO | 6-F |
| 158 | F | 3-Cl | 6-F |
| 159 | Cl | 3-Cl | 6-Br |
| 160 | F | 3-F | 6-Cl |
| 161 | F | 3-F | 6-Br |
| 162 | F | 3-F | 6-I |
| 163 | Cl | 6-CF₃ | H |
| 164 | Cl | 3-Cl | 6-CF₃ |
| 165 | F | 3-Cl | 6-CF₃ |
| 166 | Cl | 3-CF₃ | 6-Cl |
| 167 | c-Pr | 4-Cl | 6-Cl |
| 168 | Me | 4-CF₂-CF₃ | 6-Me |
| 169 | Cl | 3-c-Pr | 6-Cl |
| 170 | Cl | 3-I | 6-Cl |
| 171 | Me | 4-c-Pr-(2'-c-Pr) | 6-Me |

Tabelle 2: Erfindungsgemäße Verbindungen der Formel (I), worin G für Wasserstoff steht, V für SO₂ steht, W für Difluorethyl steht, A für A1 steht, und X, Y und Z, die in Tabelle 1 angegebenen Bedeutungen haben:

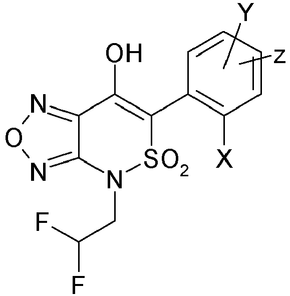

Tabelle 3: Erfindungsgemäße Verbindungen der Formel (I), worin G für Wasserstoff steht, V für C=O steht, W für Trifluorethyl steht, A für A1 steht, und X, Y und Z, die in Tabelle 1 angegebenen Bedeutungen haben:

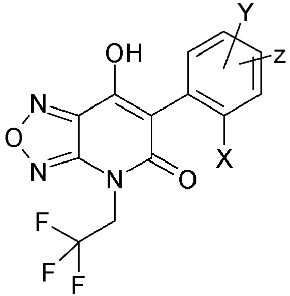

Tabelle 4: Erfindungsgemäße Verbindungen der Formel (I), worin G für Wasserstoff steht, V für SO₂ steht, W für Trifluorethyl steht, A für A1 steht, und X, Y und Z, die in Tabelle 1 angegebenen Bedeutungen haben:

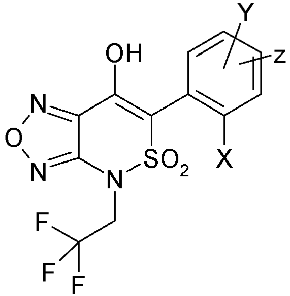

Tabelle 5: Erfindungsgemäße Verbindungen der Formel (I), worin G für Wasserstoff steht, V für C=O steht, W für Propinyl steht, A für A1 steht, und X, Y und Z, die in Tabelle 1 angegebenen Bedeutungen haben:

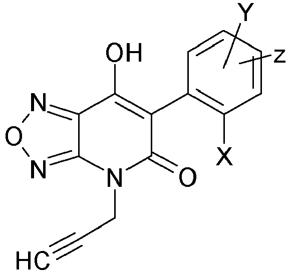

Tabelle 6: Erfindungsgemäße Verbindungen der Formel (I), worin G für Wasserstoff steht, V für SO₂ steht, W für Propinyl steht, A für A1 steht, und X, Y und Z, die in Tabelle 1 angegebenen Bedeutungen haben:

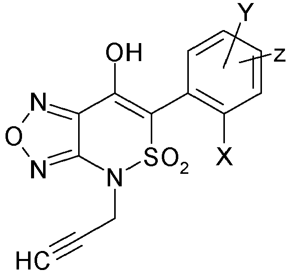

Tabelle 7: Erfindungsgemäße Verbindungen der Formel (I), worin G für Wasserstoff steht, V für C=O steht, W für Difluorethyl steht, A für A2 steht, R¹⁰ für Wasserstoff steht, und X, Y und Z, die in Tabelle 1 angegebenen Bedeutungen haben:

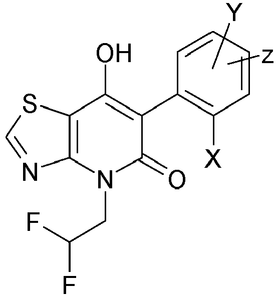

Tabelle 8: Erfindungsgemäße Verbindungen der Formel (I), worin G für Wasserstoff steht, V für SO₂ steht, W für Difluorethyl steht, A für A2 steht, R¹⁰ für Wasserstoff steht, und X, Y und Z, die in Tabelle 1 angegebenen Bedeutungen haben:

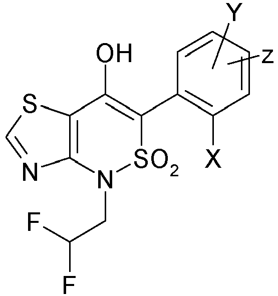

Tabelle 9: Erfindungsgemäße Verbindungen der Formel (I), worin G für Wasserstoff steht, V für C=O steht, W für Trifluorethyl steht, A für A2 steht, R¹⁰ für Wasserstoff steht, und X, Y und Z, die in Tabelle 1 angegebenen Bedeutungen haben:

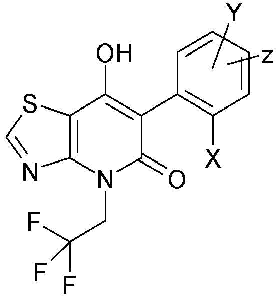

Tabelle 10: Erfindungsgemäße Verbindungen der Formel (I), worin G für Wasserstoff steht, V für SO₂ steht, W für Trifluorethyl steht, A für A2 steht, R¹⁰ für Wasserstoff steht, und X, Y und Z, die in Tabelle 1 angegebenen Bedeutungen haben:

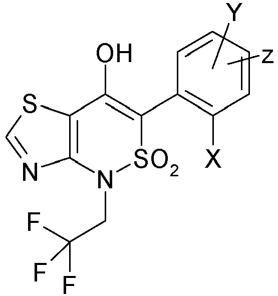

Tabelle 11: Erfindungsgemäße Verbindungen der Formel (I), worin G für Wasserstoff steht, V für C=O steht, W für Propinyl steht, A für A2 steht, R¹⁰ für Wasserstoff steht, und X, Y und Z, die in Tabelle 1 angegebenen Bedeutungen haben:

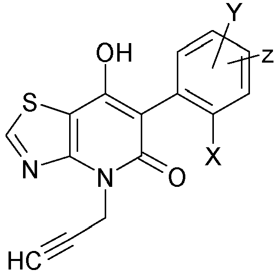

Tabelle 12: Erfindungsgemäße Verbindungen der Formel (I), worin G für Wasserstoff steht, V für SO₂ steht, W für Propinyl steht, R¹⁰ für Wasserstoff steht, A für A2 steht, und X, Y und Z, die in Tabelle 1 angegebenen Bedeutungen haben:

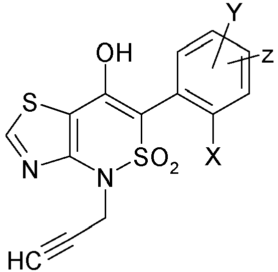

Tabelle 13: Erfindungsgemäße Verbindungen der Formel (I), worin G für Wasserstoff steht, V für C=O steht, W für Difluorethyl steht, A für A2 steht, R¹⁰ für Methylthio steht, und X, Y und Z, die in Tabelle 1 angegebenen Bedeutungen haben:

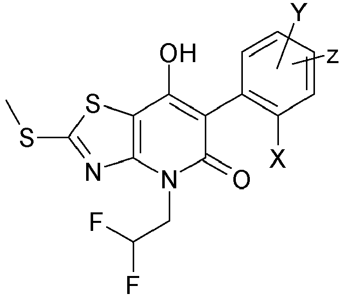

Tabelle 14: Erfindungsgemäße Verbindungen der Formel (I), worin G für Wasserstoff steht, V für SO₂ steht, W für Difluorethyl steht, A für A2 steht, R¹⁰ für Methylthio steht, und X, Y und Z, die in Tabelle 1 angegebenen Bedeutungen haben: 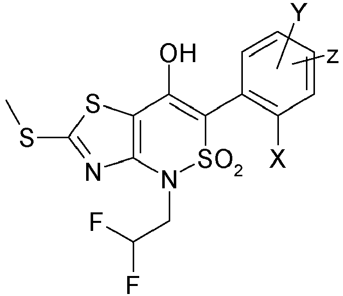 Tabelle 15: Erfindungsgemäße Verbindungen der Formel (I), worin G für Wasserstoff steht, V für C=O steht, W für Trifluorethyl steht, A für A2 steht, R¹⁰ für Methylthio steht, und X, Y und Z, die in Tabelle 1 angegebenen Bedeutungen haben:

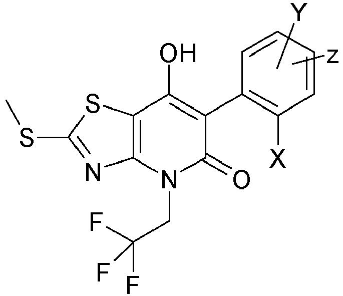

Tabelle 16: Erfindungsgemäße Verbindungen der Formel (I), worin G für Wasserstoff steht, V für SO₂ steht, W für Trifluorethyl steht, A für A2 steht, R¹⁰ für Methylthio steht, und X, Y und Z, die in Tabelle 1 angegebenen Bedeutungen haben:

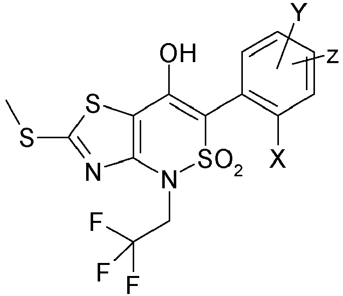

Tabelle 17: Erfindungsgemäße Verbindungen der Formel (I), worin G für Wasserstoff steht, V für C=O steht, W für Propinyl steht, A für A2 steht, R¹⁰ für Methylthio steht, und X, Y und Z, die in Tabelle 1 angegebenen Bedeutungen haben:

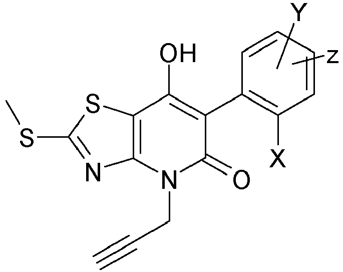

Tabelle 18: Erfindungsgemäße Verbindungen der Formel (I), worin G für Wasserstoff steht, V für SO₂ steht, W für Propinyl steht, A für A2 steht, R¹⁰ für Methylthio steht, und X, Y und Z, die in Tabelle 1 angegebenen Bedeutungen haben:

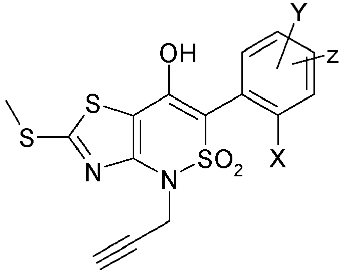

Tabelle 19: Erfindungsgemäße Verbindungen der Formel (I), worin G für Wasserstoff steht, V für C=O steht, W für Difluorethyl steht, A für A3 steht, R¹⁰ für Wasserstoff steht, und X, Y und Z, die in Tabelle 1 angegebenen Bedeutungen haben:

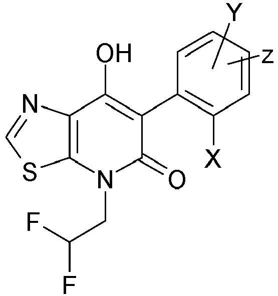

Tabelle 20: Erfindungsgemäße Verbindungen der Formel (I), worin G für Wasserstoff steht, V für SO₂ steht, W für Difluorethyl steht, A für A3 steht, R¹⁰ für Wasserstoff steht, und X, Y und Z, die in Tabelle 1 angegebenen Bedeutungen haben:

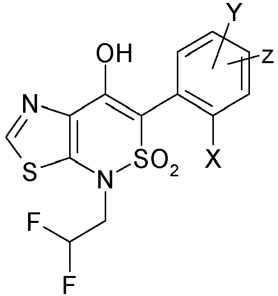

Tabelle 21: Erfindungsgemäße Verbindungen der Formel (I), worin G für Wasserstoff steht, V für C=O steht, W für Trifluorethyl steht, A für A3 steht, R¹⁰ für Wasserstoff steht, und X, Y und Z, die in Tabelle 1 angegebenen Bedeutungen haben:

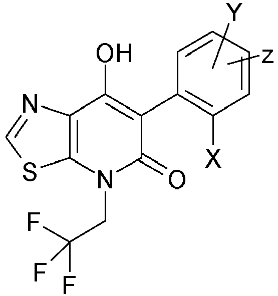

Tabelle 22: Erfindungsgemäße Verbindungen der Formel (I), worin G für Wasserstoff steht, V für SO₂ steht, W für Trifluorethyl steht, A für A3 steht, R¹⁰ für Wasserstoff steht, und X, Y und Z, die in Tabelle 1 angegebenen Bedeutungen haben:

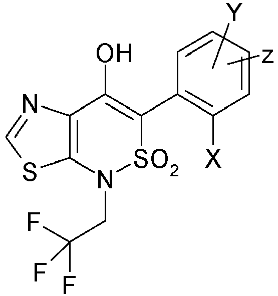

Tabelle 23: Erfindungsgemäße Verbindungen der Formel (I), worin G für Wasserstoff steht, V für C=O steht, W für Propinyl steht, A für A3 steht, R¹⁰ für Wasserstoff steht, und X, Y und Z, die in Tabelle 1 angegebenen Bedeutungen haben:

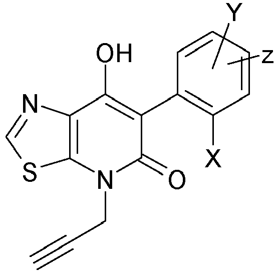

Tabelle 24: Erfindungsgemäße Verbindungen der Formel (I), worin G für Wasserstoff steht, V für SO₂ steht, W für Propinyl steht, A für A3 steht, R¹⁰ für Wasserstoff steht, und X, Y und Z, die in Tabelle 1 angegebenen Bedeutungen haben:

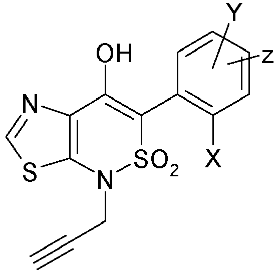

Tabelle 25: Erfindungsgemäße Verbindungen der Formel (I), worin G für Wasserstoff steht, V für C=O steht, W für Difluorethyl steht, A für A4 steht, R¹² und R¹³ für Wasserstoff stehen, und X, Y und Z, die in Tabelle 1 angegebenen Bedeutungen haben:

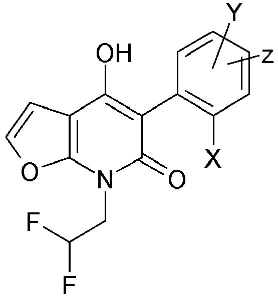

Tabelle 26: Erfindungsgemäße Verbindungen der Formel (I), worin G für Wasserstoff steht, V für SO₂ steht, W für Difluorethyl steht, A für A4 steht, R¹² und R¹³ für Wasserstoff stehen, und X, Y und Z, die in Tabelle 1 angegebenen Bedeutungen haben:

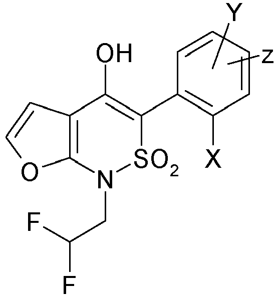

Tabelle 27: Erfindungsgemäße Verbindungen der Formel (I), worin G für Wasserstoff steht, V für C=O steht, W für Trifluorethyl steht, A für A4 steht, R¹² und R¹³ für Wasserstoff stehen, und X, Y und Z, die in Tabelle 1 angegebenen Bedeutungen haben:

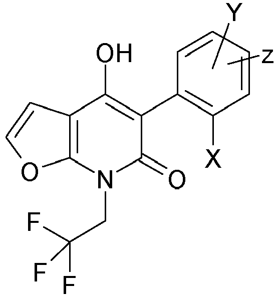

Tabelle 28: Erfindungsgemäße Verbindungen der Formel (I), worin G für Wasserstoff steht, V für SO₂ steht, W für Trifluorethyl steht, A für A4 steht, R¹² und R¹³ für Wasserstoff stehen, und X, Y und Z, die in Tabelle 1 angegebenen Bedeutungen haben:

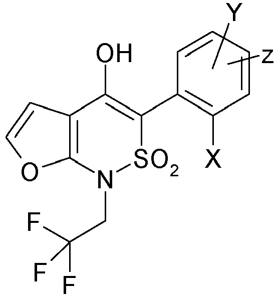

Tabelle 29: Erfindungsgemäße Verbindungen der Formel (I), worin G für Wasserstoff steht, V für C=O steht, W für Propinyl steht, A für A4 steht, R¹² und R¹³ für Wasserstoff stehen, und X, Y und Z, die in Tabelle 1 angegebenen Bedeutungen haben:

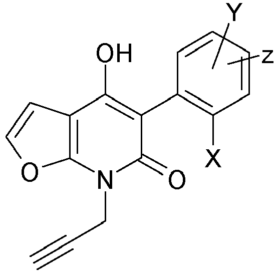

Tabelle 30: Erfindungsgemäße Verbindungen der Formel (I), worin G für Wasserstoff steht, V für SO₂ steht, W für Propinyl steht, A für A4 steht, R¹² und R¹³ für Wasserstoff stehen, und X, Y und Z, die in Tabelle 1 angegebenen Bedeutungen haben:

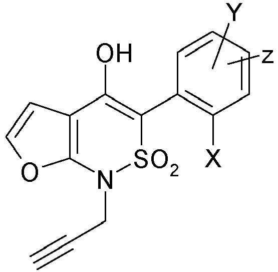

Tabelle 31: Erfindungsgemäße Verbindungen der Formel (I), worin G für Wasserstoff steht, V für C=O steht, W für Difluorethyl steht, A für A5 steht, R¹¹ für Wasserstoff steht, R¹⁴ für Methyl steht, und X, Y und Z, die in Tabelle 1 angegebenen Bedeutungen haben:

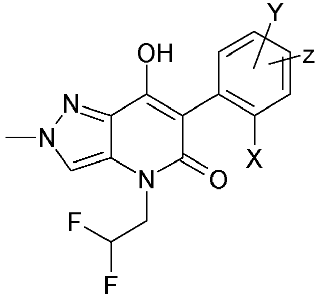

Tabelle 32: Erfindungsgemäße Verbindungen der Formel (I), worin G für Wasserstoff steht, V für SO₂ steht, W für Difluorethyl steht, A für A5 steht, R¹¹ für Wasserstoff steht, R¹⁴ für Methyl steht, und X, Y und Z, die in Tabelle 1 angegebenen Bedeutungen haben:

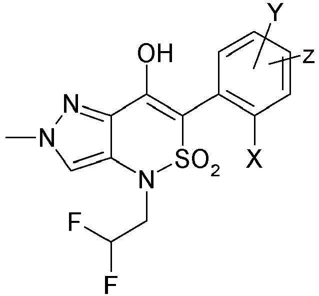

Tabelle 33: Erfindungsgemäße Verbindungen der Formel (I), worin G für Wasserstoff steht, V für C=O steht, W für Trifluorethyl steht, A für A5 steht, R¹¹ für Wasserstoff steht, R¹⁴ für Methyl steht, und X, Y und Z, die in Tabelle 1 angegebenen Bedeutungen haben:

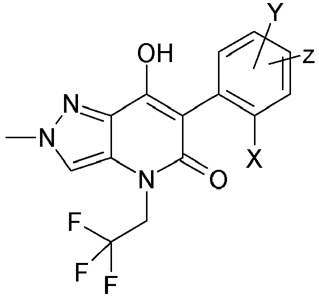

Tabelle 34: Erfindungsgemäße Verbindungen der Formel (I), worin G für Wasserstoff steht, V für SO2 steht, W für Trifluorethyl steht, A für A5 steht, R¹¹ für Wasserstoff steht, R¹⁴ für Methyl steht, und X, Y und Z, die in Tabelle 1 angegebenen Bedeutungen haben:

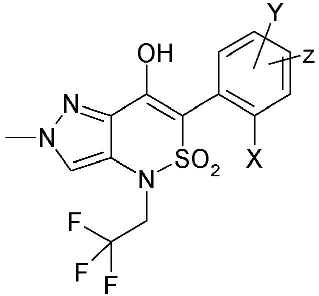

Tabelle 35: Erfindungsgemäße Verbindungen der Formel (I), worin G für Wasserstoff steht, V für C=O steht, W für Propinyl steht, A für A5 steht, R¹¹ für Wasserstoff steht, R¹⁴ für Methyl steht, und X, Y und Z, die in Tabelle 1 angegebenen Bedeutungen haben:

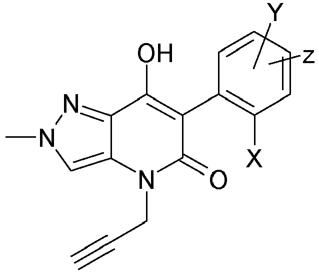

Tabelle 36: Erfindungsgemäße Verbindungen der Formel (I), worin G für Wasserstoff steht, V für C=O steht, W für Propinyl steht, A für A5 steht, R¹¹ für Wasserstoff steht, R¹⁴ für Methyl steht, und X, Y und Z, die in Tabelle 1 angegebenen Bedeutungen haben:

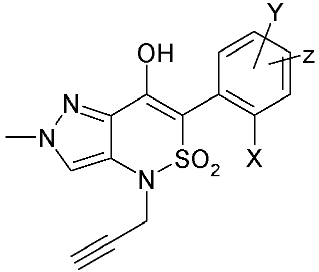

Tabelle 37: Erfindungsgemäße Verbindungen der Formel (I), worin G für Wasserstoff steht, V für C=O steht, W für Difluorethyl steht, A für A6 steht, R¹² für Wasserstoff steht, R¹⁴ für Methyl steht, und X, Y und Z, die in Tabelle 1 angegebenen Bedeutungen haben:

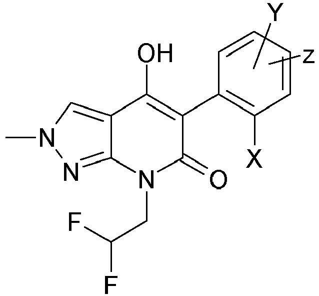

Tabelle 38: Erfindungsgemäße Verbindungen der Formel (I), worin G für Wasserstoff steht, V für SO2 steht, W für Difluorethyl steht, A für A6 steht, R¹² für Wasserstoff steht, R¹⁴ für Methyl steht, und X, Y und Z, die in Tabelle 1 angegebenen Bedeutungen haben:

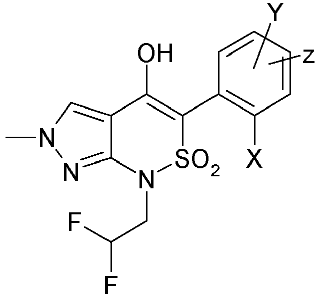

Tabelle 39: Erfindungsgemäße Verbindungen der Formel (I), worin G für Wasserstoff steht, V für C=O steht, W für Trifluorethyl steht, A für A6 steht, R¹² für Wasserstoff steht, R¹⁴ für Methyl steht, und X, Y und Z, die in Tabelle 1 angegebenen Bedeutungen haben:

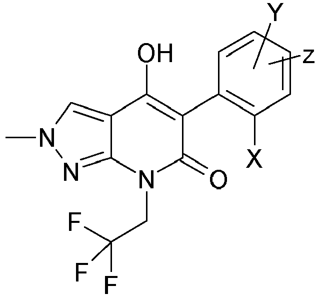

Tabelle 40: Erfindungsgemäße Verbindungen der Formel (I), worin G für Wasserstoff steht, V für SO₂ steht, W für Trifluorethyl steht, A für A6 steht, R¹² für Wasserstoff steht, R¹⁴ für Methyl steht, und X, Y und Z, die in Tabelle 1 angegebenen Bedeutungen haben:

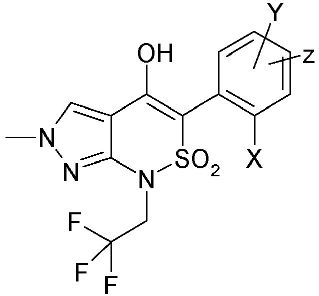

Tabelle 41: Erfindungsgemäße Verbindungen der Formel (I), worin G für Wasserstoff steht, V für C=O steht, W für Propinyl steht, A für A6 steht, R¹² für Wasserstoff steht, R¹⁴ für Methyl steht, und X, Y und Z, die in Tabelle 1 angegebenen Bedeutungen haben:

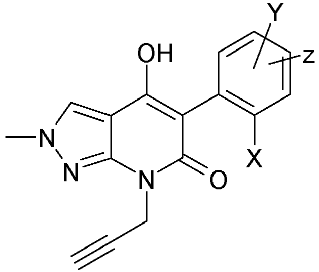

Tabelle 42: Erfindungsgemäße Verbindungen der Formel (I), worin G für Wasserstoff steht, V für C=O steht, W für Propinyl steht, A für A6 steht, R¹² für Wasserstoff steht, R¹⁴ für Methyl steht, und X, Y und Z, die in Tabelle 1 angegebenen Bedeutungen haben:

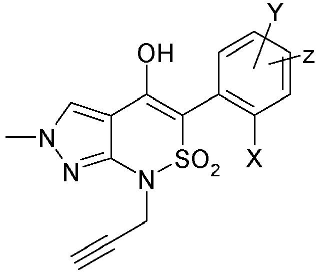

Tabelle 43: Erfindungsgemäße Verbindungen der Formel (I), worin G für Wasserstoff steht, V für C=O steht, W für Difluorethyl steht, A für A7 steht, R¹² und R¹³ für Wasserstoff stehen, und X, Y und Z, die in Tabelle 1 angegebenen Bedeutungen haben:

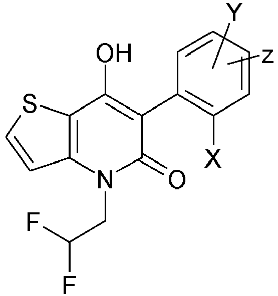

Tabelle 44: Erfindungsgemäße Verbindungen der Formel (I), worin G für Wasserstoff steht, V für SO₂ steht, W für Difluorethyl steht, A für A7 steht, R¹² und R¹³ für Wasserstoff stehen, und X, Y und Z, die in Tabelle 1 angegebenen Bedeutungen haben:

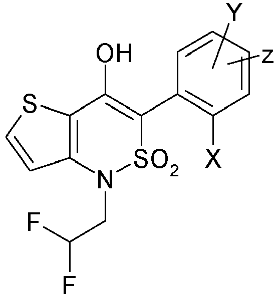

Tabelle 45: Erfindungsgemäße Verbindungen der Formel (I), worin G für Wasserstoff steht, V für C=O steht, W für Trifluorethyl steht, A für A7 steht, R¹² und R¹³ für Wasserstoff stehen, und X, Y und Z, die in Tabelle 1 angegebenen Bedeutungen haben:

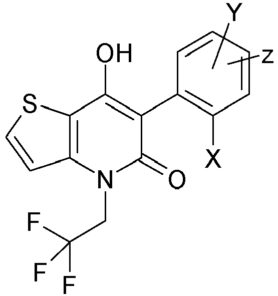

Tabelle 46: Erfindungsgemäße Verbindungen der Formel (I), worin G für Wasserstoff steht, V für SO₂ steht, W für Trifluorethyl steht, A für A7 steht, R¹² und R¹³ für Wasserstoff stehen, und X, Y und Z, die in Tabelle 1 angegebenen Bedeutungen haben:

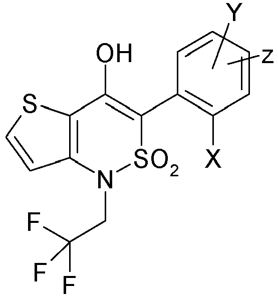

Tabelle 47: Erfindungsgemäße Verbindungen der Formel (I), worin G für Wasserstoff steht, V für C=O steht, W für Propinyl steht, A für A7 steht, R¹² und R¹³ für Wasserstoff stehen, und X, Y und Z, die in Tabelle 1 angegebenen Bedeutungen haben:

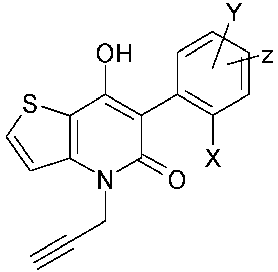

Tabelle 48: Erfindungsgemäße Verbindungen der Formel (I), worin G für Wasserstoff steht, V für SO₂ steht, W für Propinyl steht, A für A7 steht, R¹² und R¹³ für Wasserstoff stehen, und X, Y und Z, die in Tabelle 1 angegebenen Bedeutungen haben:

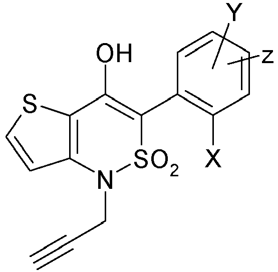

Tabelle 49: Erfindungsgemäße Verbindungen der Formel (I), worin G für Wasserstoff steht, V für C=O steht, W für Difluorethyl steht, A für A8 steht, R¹¹ für Wasserstoff steht, R¹⁴ für Methyl steht, und X, Y und Z, die in Tabelle 1 angegebenen Bedeutungen haben:

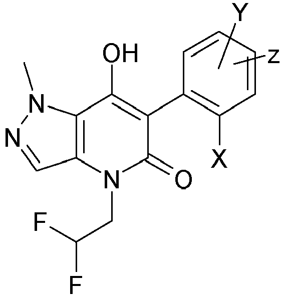

Tabelle 50: Erfindungsgemäße Verbindungen der Formel (I), worin G für Wasserstoff steht, V für SO₂ steht, W für Difluorethyl steht, A für A8 steht, R¹¹ für Wasserstoff steht, R¹⁴ für Methyl steht, und X, Y und Z, die in Tabelle 1 angegebenen Bedeutungen haben:

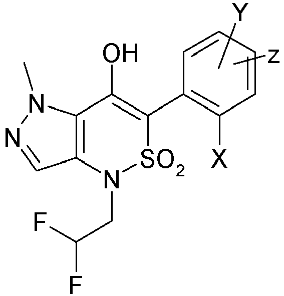

Tabelle 51: Erfindungsgemäße Verbindungen der Formel (I), worin G für Wasserstoff steht, V für C=O steht, W für Trifluorethyl steht steht, A für A8 steht, R¹¹ für Wasserstoff steht, R¹⁴ für Methyl steht, und X, Y und Z, die in Tabelle 1 angegebenen Bedeutungen haben:

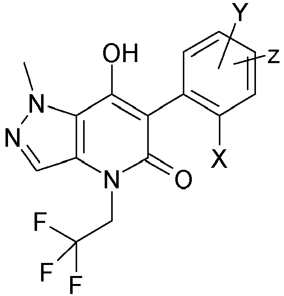

Tabelle 52: Erfindungsgemäße Verbindungen der Formel (I), worin G für Wasserstoff steht, V für SO₂ steht, W für Trifluorethyl steht, A für A8 steht, R¹¹ für Wasserstoff steht, R¹⁴ für Methyl steht, und X, Y und Z, die in Tabelle 1 angegebenen Bedeutungen haben:

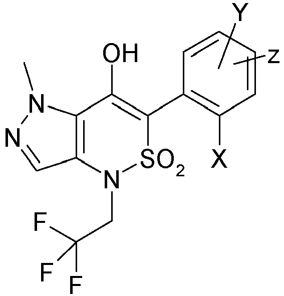

Tabelle 53: Erfindungsgemäße Verbindungen der Formel (I), worin G für Wasserstoff steht, V für C=O steht, W für Propinyl steht, A für A8 steht, R¹¹ für Wasserstoff steht, R¹⁴ für Methyl steht, und X, Y und Z, die in Tabelle 1 angegebenen Bedeutungen haben:

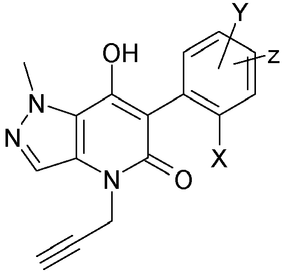

Tabelle 54: Erfindungsgemäße Verbindungen der Formel (I), worin G für Wasserstoff steht, V für SO₂ steht, W für Propinyl steht, A für A8 steht, R¹¹ für Wasserstoff steht, R¹⁴ für Methyl steht, und X, Y und Z, die in Tabelle 1 angegebenen Bedeutungen haben:

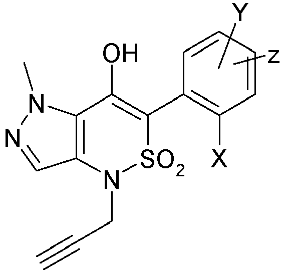

Tabelle 55: Erfindungsgemäße Verbindungen der Formel (I), worin G für Wasserstoff steht, V für C=O steht, W für Difluorethyl steht, A für A9 steht, R¹⁰ für Wasserstoff steht, und X, Y und Z, die in Tabelle 1 angegebenen Bedeutungen haben:

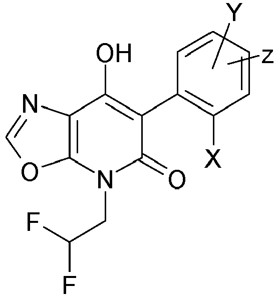

Tabelle 56: Erfindungsgemäße Verbindungen der Formel (I), worin G für Wasserstoff steht, V für SO₂ steht, W für Difluorethyl steht, A für A9 steht, R¹⁰ für Wasserstoff steht, und X, Y und Z, die in Tabelle 1 angegebenen Bedeutungen haben:

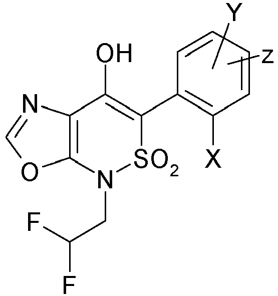

Tabelle 57: Erfindungsgemäße Verbindungen der Formel (I), worin G für Wasserstoff steht, V für C=O steht, W für Trifluorethyl steht, A für A9 steht, R¹⁰ für Wasserstoff steht, und X, Y und Z, die in Tabelle 1 angegebenen Bedeutungen haben:

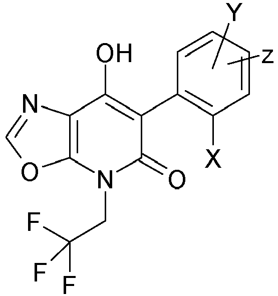

Tabelle 58: Erfindungsgemäße Verbindungen der Formel (I), worin G für Wasserstoff steht, V für SO₂ steht, W für Trifluorethyl steht, A für A9 steht, R¹⁰ für Wasserstoff steht, und X, Y und Z, die in Tabelle 1 angegebenen Bedeutungen haben:

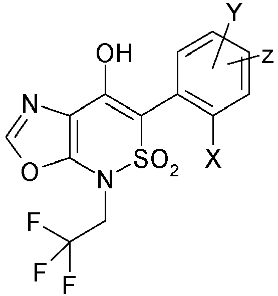

Tabelle 59: Erfindungsgemäße Verbindungen der Formel (I), worin G für Wasserstoff steht, V für C=O steht, W für Propinyl steht, A für A9 steht, R¹⁰ für Wasserstoff steht, und X, Y und Z, die in Tabelle 1 angegebenen Bedeutungen haben:

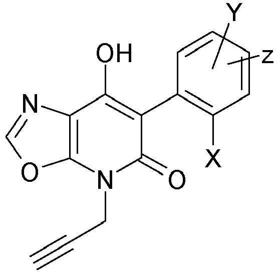

Tabelle 60: Erfindungsgemäße Verbindungen der Formel (I), worin G für Wasserstoff steht, V für SO₂ steht, W für Propinyl steht, A für A9 steht, R¹⁰ für Wasserstoff steht, und X, Y und Z, die in Tabelle 1 angegebenen Bedeutungen haben:

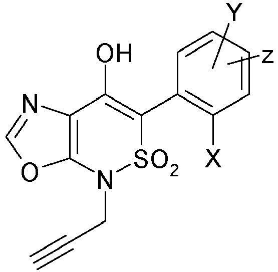

Tabelle 61: Erfindungsgemäße Verbindungen der Formel (I), worin G für Wasserstoff steht, V für C=O steht, W für Difluorethyl steht, A für A10 steht, R¹² und R¹³ für Wasserstoff stehen, und X, Y und Z, die in Tabelle 1 angegebenen Bedeutungen haben:

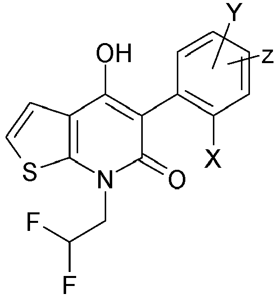

Tabelle 62: Erfindungsgemäße Verbindungen der Formel (I), worin G für Wasserstoff steht, V für SO₂ steht, W für Difluorethyl steht, A für A10 steht, R¹² und R¹³ für Wasserstoff stehen, und X, Y und Z, die in Tabelle 1 angegebenen Bedeutungen haben:

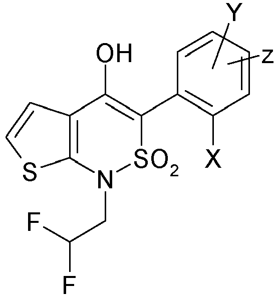

Tabelle 63: Erfindungsgemäße Verbindungen der Formel (I), worin G für Wasserstoff steht, V für C=O steht, W für Trifluorethyl steht, A für A10 steht, R¹² und R¹³ für Wasserstoff stehen, und X, Y und Z, die in Tabelle 1 angegebenen Bedeutungen haben:

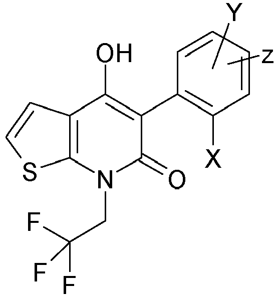

Tabelle 64: Erfindungsgemäße Verbindungen der Formel (I), worin G für Wasserstoff steht, V für SO₂ steht, W für Trifluorethyl steht, A für A10 steht, R¹² und R¹³ für Wasserstoff stehen, und X, Y und Z, die in Tabelle 1 angegebenen Bedeutungen haben:

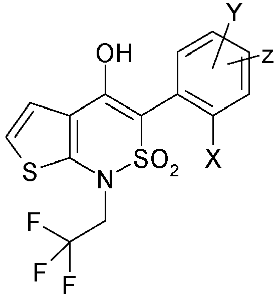

Tabelle 65: Erfindungsgemäße Verbindungen der Formel (I), worin G für Wasserstoff steht, V für C=O steht, W für Propinyl steht, A für A10 steht, R¹² und R¹³ für Wasserstoff stehen, und X, Y und Z, die in Tabelle 1 angegebenen Bedeutungen haben:

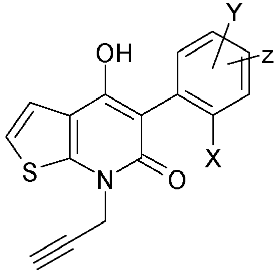

Tabelle 66: Erfindungsgemäße Verbindungen der Formel (I), worin G für Wasserstoff steht, V für SO₂ steht, W für Propinyl steht, A für A10 steht, R¹² und R¹³ für Wasserstoff stehen, und X, Y und Z, die in Tabelle 1 angegebenen Bedeutungen haben:

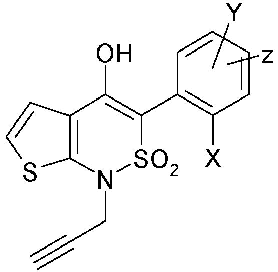

Tabelle 67: Erfindungsgemäße Verbindungen der Formel (I), worin G für Wasserstoff steht, V für C=O steht, W für Difluorethyl steht, A für A11 steht, R¹² für Wasserstoff steht, R¹⁴ für Methyl steht, und X, Y und Z, die in Tabelle 1 angegebenen Bedeutungen haben:

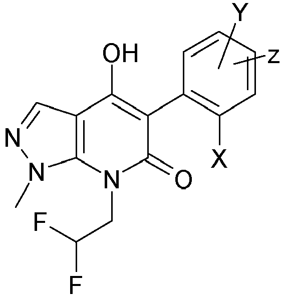

Tabelle 68: Erfindungsgemäße Verbindungen der Formel (I), worin G für Wasserstoff steht, V für SO₂ steht, W für Difluorethyl steht, A für A11 steht, R¹² für Wasserstoff steht, R¹⁴ für Methyl steht, und X, Y und Z, die in Tabelle 1 angegebenen Bedeutungen haben:

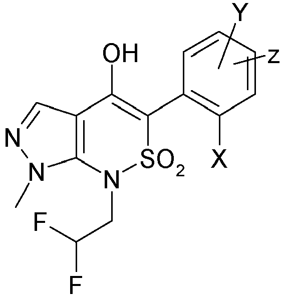

Tabelle 69: Erfindungsgemäße Verbindungen der Formel (I), worin G für Wasserstoff steht, V für C=O steht, W für Trifluorethyl steht, A für A11 steht, R¹² für Wasserstoff steht, R¹⁴ für Methyl steht, und X, Y und Z, die in Tabelle 1 angegebenen Bedeutungen haben:

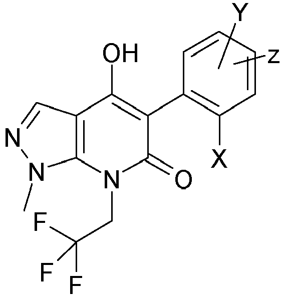

Tabelle 70: Erfindungsgemäße Verbindungen der Formel (I), worin G für Wasserstoff steht, V für SO₂ steht, W für Trifluorethyl steht, A für A11 steht, R¹² für Wasserstoff steht, R¹⁴ für Methyl steht, und X, Y und Z, die in Tabelle 1 angegebenen Bedeutungen haben:

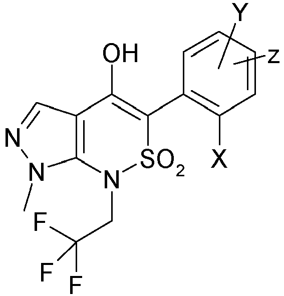

Tabelle 71: Erfindungsgemäße Verbindungen der Formel (I), worin G für Wasserstoff steht, V für C=O steht, W für Propinyl steht, A für A11 steht, R¹² für Wasserstoff steht, R¹⁴ für Methyl steht, und X, Y und Z, die in Tabelle 1 angegebenen Bedeutungen haben:

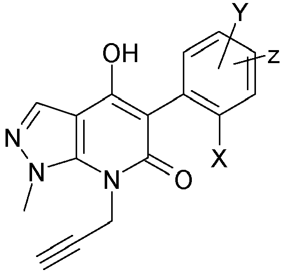

Tabelle 72: Erfindungsgemäße Verbindungen der Formel (I), worin G für Wasserstoff steht, V für SO₂ steht, W für Propinyl steht, A für A11 steht, R¹² für Wasserstoff steht, R¹⁴ für Methyl steht, und X, Y und Z, die in Tabelle 1 angegebenen Bedeutungen haben:

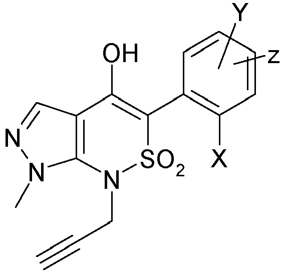

Tabelle 73: Erfindungsgemäße Verbindungen der Formel (I), worin G für Wasserstoff steht, V für SO₂ steht, W für Methyl steht, A für A1 steht, und X, Y und Z, die in Tabelle 1 angegebenen Bedeutungen haben:

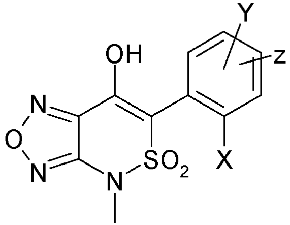

Tabelle 74: Erfindungsgemäße Verbindungen der Formel (I), worin G für Wasserstoff steht, V für C=O steht, W für Methyl steht, A für A1 steht, und X, Y und Z, die in Tabelle 1 angegebenen Bedeutungen haben:

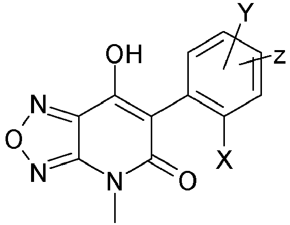

Tabelle 75: Erfindungsgemäße Verbindungen der Formel (I), worin G für Wasserstoff steht, V für SO₂ steht, W für Methyl steht, A für A2 steht, R¹⁰ für Wasserstoff steht, und X, Y und Z, die in Tabelle 1 angegebenen Bedeutungen haben:

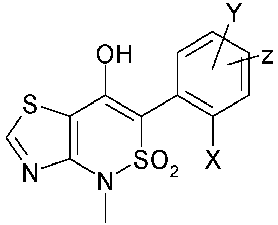

Tabelle 76: Erfindungsgemäße Verbindungen der Formel (I), worin G für Wasserstoff steht, V für C=O steht, W für Methyl steht, A für A2 steht, R¹⁰ für Wasserstoff steht, und X, Y und Z, die in Tabelle 1 angegebenen Bedeutungen haben:

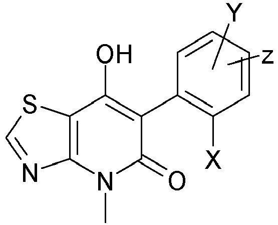

Tabelle 77: Erfindungsgemäße Verbindungen der Formel (I), worin G für Wasserstoff steht, V für SO₂ steht, W für Methyl steht, A für A2 steht, R¹⁰ für Methylthio steht, und X, Y und Z, die in Tabelle 1 angegebenen Bedeutungen haben:

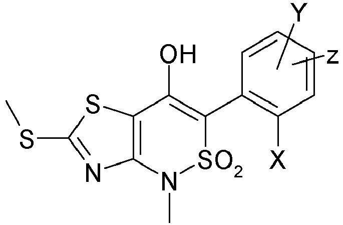

Tabelle 78: Erfindungsgemäße Verbindungen der Formel (I), worin G für Wasserstoff steht, V für C=O steht, W für Methyl steht, A für A2 steht, R¹⁰ für Methylthio steht, und X, Y und Z, die in Tabelle 1 angegebenen Bedeutungen haben:

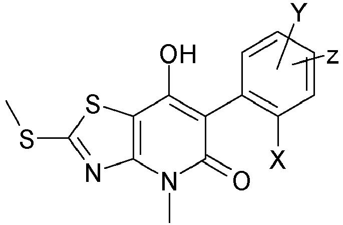

Tabelle 79: Erfindungsgemäße Verbindungen der Formel (I), worin G für Wasserstoff steht, V für SO₂ steht, W für Methyl steht, A für A3 steht, R¹⁰ für Wasserstoff steht, und X, Y und Z, die in Tabelle 1 angegebenen Bedeutungen haben:

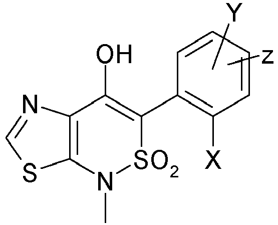

Tabelle 80: Erfindungsgemäße Verbindungen der Formel (I), worin G für Wasserstoff steht, V für C=O steht, W für Methyl steht, A für A3 steht, R¹⁰ für Wasserstoff steht, und X, Y und Z, die in Tabelle 1 angegebenen Bedeutungen haben:

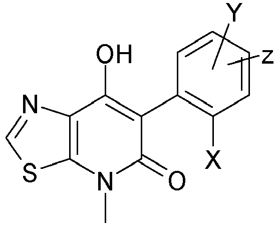

Tabelle 81: Erfindungsgemäße Verbindungen der Formel (I), +worin G für Wasserstoff steht, V für SO₂ steht, W für Methyl steht, A für A4 steht, R¹² und R¹³ für Wasserstoff stehen, und X, Y und Z, die in Tabelle 1 angegebenen Bedeutungen haben:

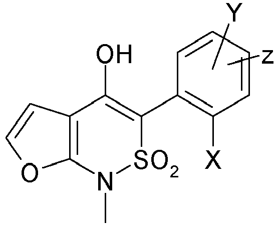

Tabelle 82: Erfindungsgemäße Verbindungen der Formel (I), worin G für Wasserstoff steht, V für C=O steht, W für Methyl steht, A für A4 steht, R¹² und R¹³ für Wasserstoff stehen, und X, Y und Z, die in Tabelle 1 angegebenen Bedeutungen haben:

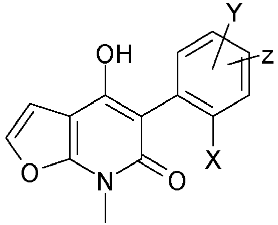

Tabelle 83: Erfindungsgemäße Verbindungen der Formel (I), worin G für Wasserstoff steht, V für SO₂ steht, W für Methyl steht, A für A5 steht, R¹¹ für Wasserstoff steht, R¹⁴ für Methyl steht, und X, Y und Z, die in Tabelle 1 angegebenen Bedeutungen haben:

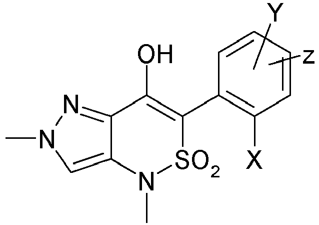

Tabelle 84: Erfindungsgemäße Verbindungen der Formel (I), worin G für Wasserstoff steht, V für C=O steht, W für Methyl steht, A für A5 steht, R¹¹ für Wasserstoff steht, R¹⁴ für Methyl steht, und X, Y und Z, die in Tabelle 1 angegebenen Bedeutungen haben:

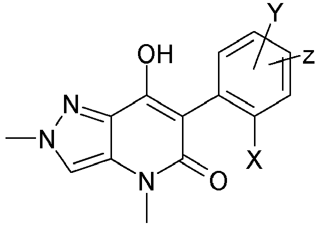

Tabelle 85: Erfindungsgemäße Verbindungen der Formel (I), worin G für Wasserstoff steht, V für SO₂ steht, W für Methyl steht, A für A6 steht, R¹² für Wasserstoff steht, R¹⁴ für Methyl steht, und X, Y und Z, die in Tabelle 1 angegebenen Bedeutungen haben:

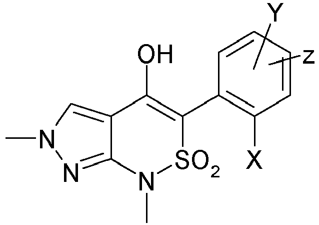

Tabelle 86: Erfindungsgemäße Verbindungen der Formel (I), worin G für Wasserstoff steht, V für C=O steht, W für Methyl steht, A für A6 steht, R¹² für Wasserstoff steht, R¹⁴ für Methyl steht, und X, Y und Z, die in Tabelle 1 angegebenen Bedeutungen haben:

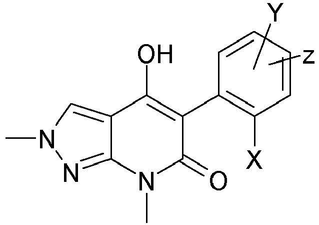

Tabelle 87: Erfindungsgemäße Verbindungen der Formel (I), worin G für Wasserstoff steht, V für SO₂ steht, W für Methyl steht, A für A7 steht, R¹² und R¹³ für Wasserstoff stehen, und X, Y und Z, die in Tabelle 1 angegebenen Bedeutungen haben:

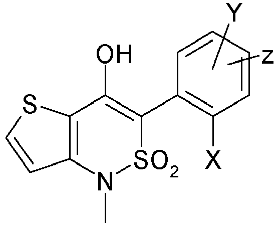

Tabelle 88: Erfindungsgemäße Verbindungen der Formel (I), worin G für Wasserstoff steht, V für C=O steht, W für Methyl steht, A für A7 steht, R¹² und R¹³ für Wasserstoff stehen, und X, Y und Z, die in Tabelle 1 angegebenen Bedeutungen haben:

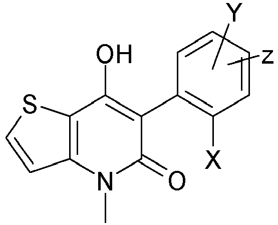

Tabelle 89: Erfindungsgemäße Verbindungen der Formel (I), worin G für Wasserstoff steht, V für SO₂ steht, W für Methyl steht, A für A8 steht, R¹¹ für Wasserstoff steht, R¹⁴ für Methyl steht, und X, Y und Z, die in Tabelle 1 angegebenen Bedeutungen haben:

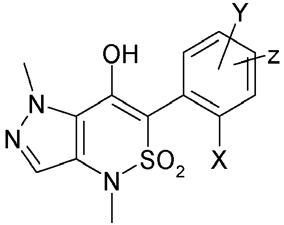

Tabelle 90: Erfindungsgemäße Verbindungen der Formel (I), worin G für Wasserstoff steht, V für C=O steht, W für Methyl steht, A für A8 steht, R¹¹ für Wasserstoff steht, R¹⁴ für Methyl steht, und X, Y und Z, die in Tabelle 1 angegebenen Bedeutungen haben:

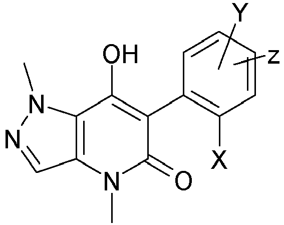

Tabelle 91: Erfindungsgemäße Verbindungen der Formel (I), worin G für Wasserstoff steht, V für SO₂ steht, W für Methyl steht, A für A9 steht, R¹⁰ für Wasserstoff steht, und X, Y und Z, die in Tabelle 1 angegebenen Bedeutungen haben:

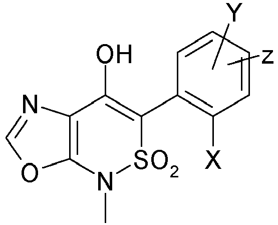

Tabelle 92: Erfindungsgemäße Verbindungen der Formel (I), worin G für Wasserstoff steht, V für C=O steht, W für Methyl steht, A für A9 steht, R¹⁰ für Wasserstoff steht, und X, Y und Z, die in Tabelle 1 angegebenen Bedeutungen haben:

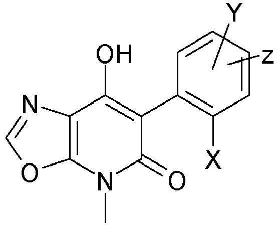

Tabelle 93: Erfindungsgemäße Verbindungen der Formel (I), worin G für Wasserstoff steht, V für SO₂ steht, A für A10 steht, R¹² und R¹³ für Wasserstoff stehen, und X, Y und Z, die in Tabelle 1 angegebenen Bedeutungen haben:

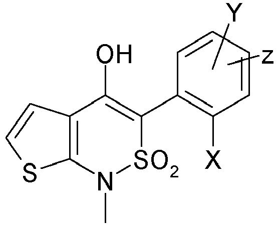

Tabelle 94: Erfindungsgemäße Verbindungen der Formel (I), worin G für Wasserstoff steht, V für C=O steht, W für Methyl steht, A für A10 steht, R¹² und R¹³ für Wasserstoff stehen, und X, Y und Z, die in Tabelle 1 angegebenen Bedeutungen haben:

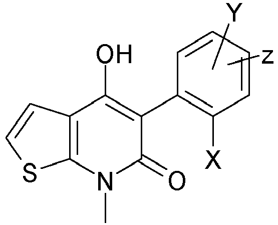

Tabelle 95: Erfindungsgemäße Verbindungen der Formel (I), worin G für Wasserstoff steht, V für SO₂ steht, W für Methyl steht, A für A11 steht, R¹² für Wasserstoff steht, R¹⁴ für Methyl steht, und X, Y und Z, die in Tabelle 1 angegebenen Bedeutungen haben:

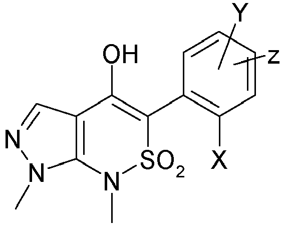

Tabelle 96: Erfindungsgemäße Verbindungen der Formel (I), worin G für Wasserstoff steht, V für C=O steht, W für Methyl steht, A für A11 steht, R¹² für Wasserstoff steht, R¹⁴ für Methyl steht, und X, Y und Z, die in Tabelle 1 angegebenen Bedeutungen haben:

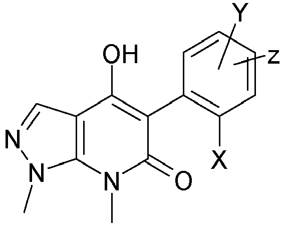

Tabelle 97: Erfindungsgemäße Verbindungen der Formel (I), worin G für Wasserstoff steht, V für SO₂ steht, W für Ethyl steht, A für A1 steht, und X, Y und Z, die in Tabelle 1 angegebenen Bedeutungen haben:

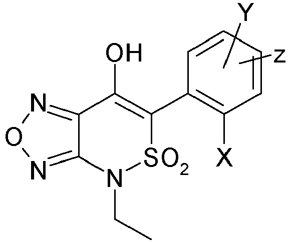

Tabelle 98: Erfindungsgemäße Verbindungen der Formel (I), worin G für Wasserstoff steht, V für C=O steht, W für Ethyl steht, A für A1 steht, und X, Y und Z, die in Tabelle 1 angegebenen Bedeutungen haben:

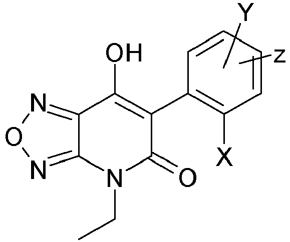

Tabelle 99: Erfindungsgemäße Verbindungen der Formel (I), worin G für Wasserstoff steht, V für SO₂ steht, W für Ethyl steht, A für A2 steht, R¹⁰ für Wasserstoff steht, und X, Y und Z, die in Tabelle 1 angegebenen Bedeutungen haben:

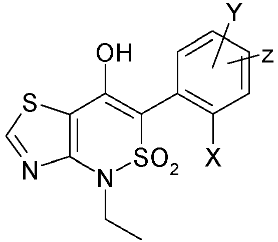

Tabelle 100: Erfindungsgemäße Verbindungen der Formel (I), worin G für Wasserstoff steht, V für C=O steht, W für Ethyl steht, A für A2 steht, R¹⁰ für Wasserstoff steht, und X, Y und Z, die in Tabelle 1 angegebenen Bedeutungen haben:

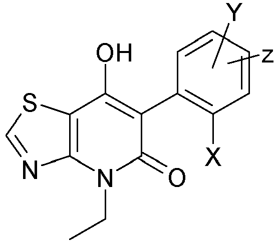

Tabelle 101: Erfindungsgemäße Verbindungen der Formel (I), worin G für Wasserstoff steht, V für SO₂ steht, W für Ethyl steht, A für A2 steht, R¹⁰ für Methylthio steht, und X, Y und Z, die in Tabelle 1 angegebenen Bedeutungen haben:

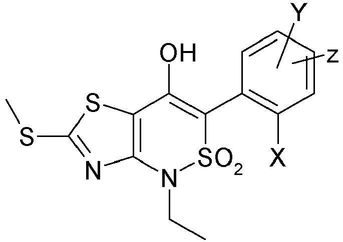

Tabelle 102: Erfindungsgemäße Verbindungen der Formel (I), worin G für Wasserstoff steht, V für C=O steht, W für Ethyl steht, A für A2 steht, R¹⁰ für Methylthio steht, und X, Y und Z, die in Tabelle 1 angegebenen Bedeutungen haben:

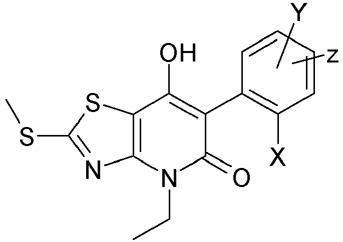

Tabelle 103: Erfindungsgemäße Verbindungen der Formel (I), worin G für Wasserstoff steht, V für SO₂ steht, W für Ethyl steht, A für A3 steht, R¹⁰ für Wasserstoff steht, und X, Y und Z, die in Tabelle 1 angegebenen Bedeutungen haben:

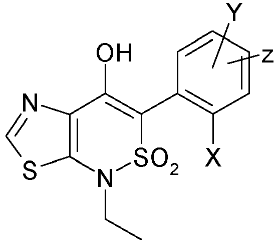

Tabelle 104: Erfindungsgemäße Verbindungen der Formel (I), worin G für Wasserstoff steht, V für C=O steht, W für Ethyl steht, A für A3 steht, R¹⁰ für Wasserstoff steht, und X, Y und Z, die in Tabelle 1 angegebenen Bedeutungen haben:

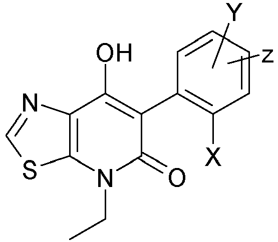

Tabelle 105: Erfindungsgemäße Verbindungen der Formel (I), worin G für Wasserstoff steht, V für SO₂ steht, W für Ethyl steht, A für A4 steht, R¹² und R¹³ für Wasserstoff stehen, und X, Y und Z, die in Tabelle 1 angegebenen Bedeutungen haben:

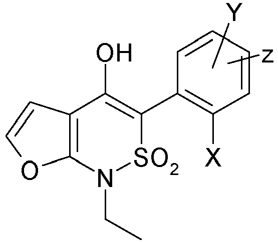

Tabelle 106: Erfindungsgemäße Verbindungen der Formel (I), worin G für Wasserstoff steht, V für C=O steht, W für Ethyl steht, A für A4 steht, R¹² und R¹³ für Wasserstoff stehen, und X, Y und Z, die in Tabelle 1 angegebenen Bedeutungen haben:

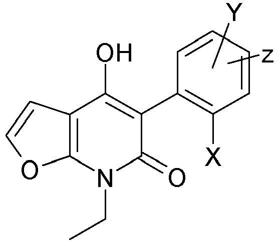

Tabelle 107: Erfindungsgemäße Verbindungen der Formel (I), worin G für Wasserstoff steht, V für SO2 steht, W für Ethyl steht, A für A5 steht, R¹¹ für Wasserstoff steht, R¹⁴ für Methyl steht, und X, Y und Z, die in Tabelle 1 angegebenen Bedeutungen haben:

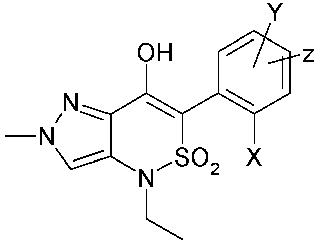

Tabelle 108: Erfindungsgemäße Verbindungen der Formel (I), worin G für Wasserstoff steht, V für C=O steht, W für Ethyl steht, A für A5 steht, R¹¹ für Wasserstoff steht, R¹⁴ für Methyl steht, und X, Y und Z, die in Tabelle 1 angegebenen Bedeutungen haben:

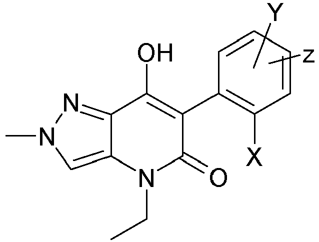

Tabelle 109: Erfindungsgemäße Verbindungen der Formel (I), worin G für Wasserstoff steht, V für SO₂ steht, W für Ethyl steht, A für A6 steht, R¹² für Wasserstoff steht, R¹⁴ für Methyl steht, und X, Y und Z, die in Tabelle 1 angegebenen Bedeutungen haben:

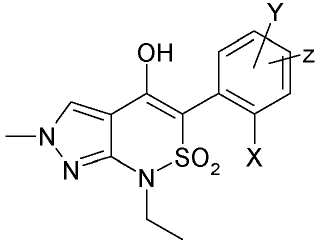

Tabelle 110: Erfindungsgemäße Verbindungen der Formel (I), worin G für Wasserstoff steht, V für C=O steht, W für Ethyl steht, A für A6 steht, R¹² für Wasserstoff steht, R¹⁴ für Methyl steht, und X, Y und Z, die in Tabelle 1 angegebenen Bedeutungen haben:

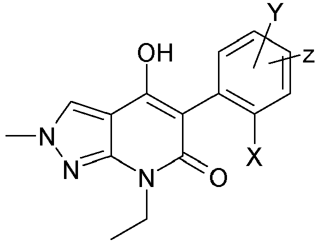

Tabelle 111: Erfindungsgemäße Verbindungen der Formel (I), worin G für Wasserstoff steht, V für SO₂ steht, W für Ethyl steht, A für A7 steht, R¹² und R¹³ für Wasserstoff stehen, und X, Y und Z, die in Tabelle 1 angegebenen Bedeutungen haben:

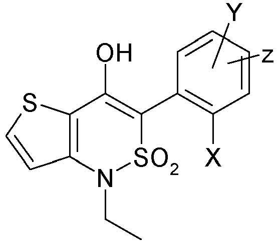

Tabelle 112: Erfindungsgemäße Verbindungen der Formel (I), worin G für Wasserstoff steht, V für C=O steht, W für Ethyl steht, A für A7 steht, R¹² und R¹³ für Wasserstoff stehen, und X, Y und Z, die in Tabelle 1 angegebenen Bedeutungen haben:

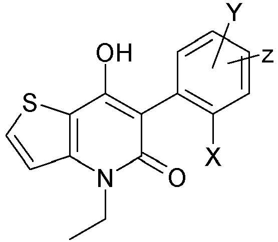

Tabelle 113: Erfindungsgemäße Verbindungen der Formel (I), worin G für Wasserstoff steht, V für SO₂ steht, W für Ethyl steht, A für A8 steht, R¹¹ für Wasserstoff steht, R¹⁴ für Methyl steht, und X, Y und Z, die in Tabelle 1 angegebenen Bedeutungen haben:

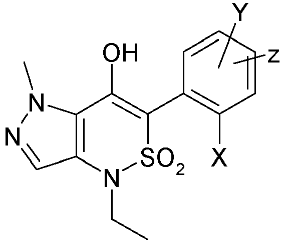

Tabelle 114: Erfindungsgemäße Verbindungen der Formel (I), worin G für Wasserstoff steht, V für C=O steht, W für Ethyl steht, A für A8 steht, R¹¹ für Wasserstoff steht, R¹⁴ für Methyl steht, und X, Y und Z, die in Tabelle 1 angegebenen Bedeutungen haben:

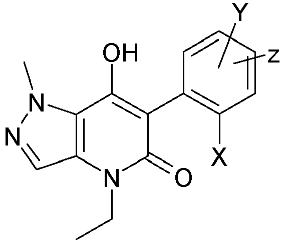

Tabelle 115: Erfindungsgemäße Verbindungen der Formel (I), worin G für Wasserstoff steht, V für SO₂ steht, W für Ethyl steht, A für A9 steht, R¹⁰ für Wasserstoff steht, und X, Y und Z, die in Tabelle 1 angegebenen Bedeutungen haben:

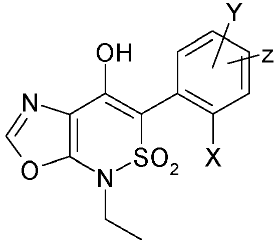

Tabelle 116: Erfindungsgemäße Verbindungen der Formel (I), worin G für Wasserstoff steht, V für C=O steht, W für Ethyl steht, A für A9 steht, R¹⁰ für Wasserstoff steht, und X, Y und Z, die in Tabelle 1 angegebenen Bedeutungen haben:

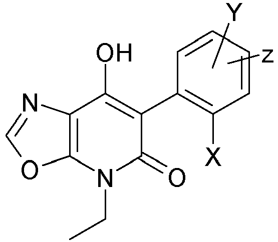

Tabelle 117: Erfindungsgemäße Verbindungen der Formel (I), worin G für Wasserstoff steht, V für SO₂ steht, W für Ethyl steht, A für A10 steht, R¹² und R¹³ für Wasserstoff stehen, und X, Y und Z, die in Tabelle 1 angegebenen Bedeutungen haben:

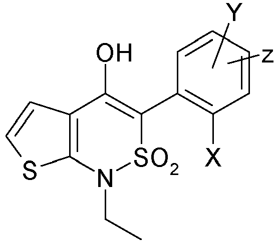

Tabelle 118: Erfindungsgemäße Verbindungen der Formel (I), worin G für Wasserstoff steht, V für C=O steht, W für Ethyl steht, A für A10 steht, R¹² und R¹³ für Wasserstoff stehen, und X, Y und Z, die in Tabelle 1 angegebenen Bedeutungen haben:

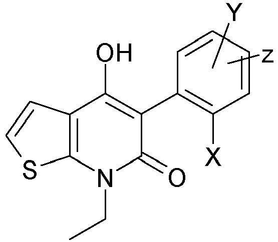

Tabelle 119: Erfindungsgemäße Verbindungen der Formel (I), worin G für Wasserstoff steht, V für SO₂ steht, W für Ethyl steht, A für A11 steht, R¹² für Wasserstoff steht, R¹⁴ für Methyl steht, und X, Y und Z, die in Tabelle 1 angegebenen Bedeutungen haben:

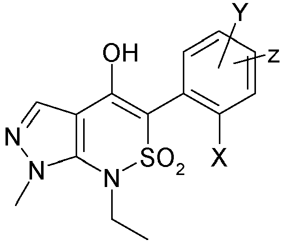

Tabelle 120: Erfindungsgemäße Verbindungen der Formel (I), worin G für Wasserstoff steht, V für C=O steht, W für Ethyl steht, A für A11 steht, R¹² für Wasserstoff steht, R¹⁴ für Methyl steht, und X, Y und Z, die in Tabelle 1 angegebenen Bedeutungen haben:

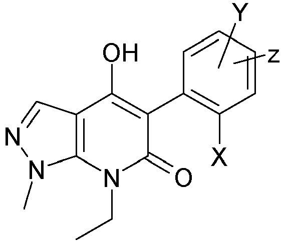

Tabelle 121: Erfindungsgemäße Verbindungen der Formel (I), worin G für Wasserstoff steht, V für SO₂ steht, W für Ethyl steht, A für A10 steht, R¹² für Ethyl und R¹³ für Methyl stehen, und X, Y und Z, die in Tabelle 1 angegebenen Bedeutungen haben:

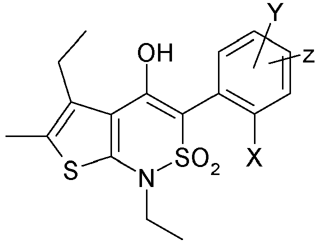

Tabelle 122: Erfindungsgemäße Verbindungen der Formel (I), worin G für Wasserstoff steht, V für C=O steht, W für Ethyl steht, A für A10 steht, R¹² für Ethyl und R¹³ für Methyl stehen, und X, Y und Z, die in Tabelle 1 angegebenen Bedeutungen haben:

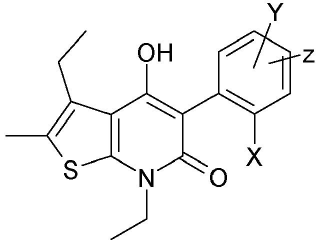

Tabelle 123: Erfindungsgemäße Verbindungen der Formel (I), worin G für Wasserstoff steht, V für SO₂ steht, W für Ethyl steht, A für A10 steht, R¹² und R¹³ für Methyl stehen, und X, Y und Z, die in Tabelle 1 angegebenen Bedeutungen haben:

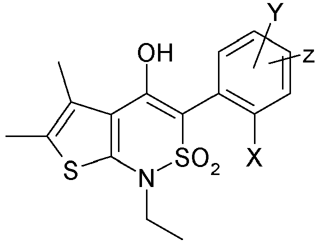

Tabelle 124: Erfindungsgemäße Verbindungen der Formel (I), worin G für Wasserstoff steht, V für C=O steht, W für Ethyl steht, A für A10 steht, R¹² und R¹³ für Methyl stehen, und X, Y und Z, die in Tabelle 1 angegebenen Bedeutungen haben:

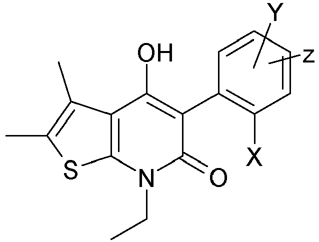

Tabelle 125: Erfindungsgemäße Verbindungen der Formel (I), worin G für Wasserstoff steht, V für SO₂ steht, W für Ethyl steht, A für A7 steht, R¹² für Phenyl steht und R¹³ für Wasserstoff steht, und X, Y und Z, die in Tabelle 1 angegebenen Bedeutungen haben:

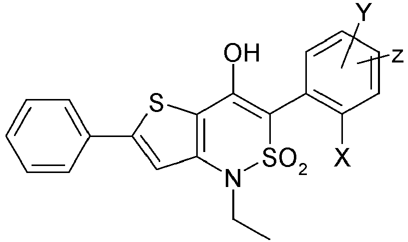

Tabelle 126: Erfindungsgemäße Verbindungen der Formel (I), worin G für Wasserstoff steht, V für C=O steht, W für Ethyl steht, A für A10 steht, R¹² für Phenyl steht und R¹³ für Wasserstoff steht, und X, Y und Z, die in Tabelle 1 angegebenen Bedeutungen haben:

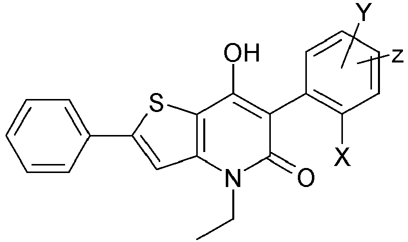

Tabelle 127: Erfindungsgemäße Verbindungen der Formel (I), worin G für Wasserstoff steht, V für SO₂ steht, W für Ethyl steht, A für A2 steht, R¹⁰ für Methyl steht, und X, Y und Z, die in Tabelle 1 angegebenen Bedeutungen haben:

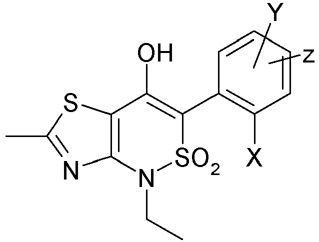

Tabelle 128: Erfindungsgemäße Verbindungen der Formel (I), worin G für Wasserstoff steht, V für C=O steht, W für Ethyl steht, A für A2 steht, R¹⁰ für Methyl steht, und X, Y und Z, die in Tabelle 1 angegebenen Bedeutungen haben:

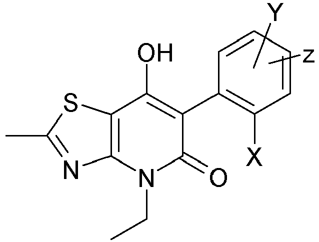

Tabelle 129: Erfindungsgemäße Verbindungen der Formel (I), worin G für Wasserstoff steht, V für SO₂ steht, W für Ethyl steht, A für A2 steht, R¹⁰ für Ethyl steht, und X, Y und Z, die in Tabelle 1 angegebenen Bedeutungen haben:

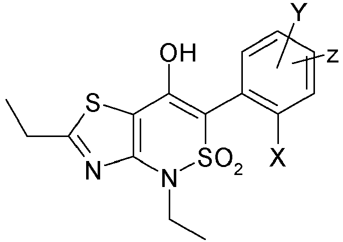

Tabelle 130: Erfindungsgemäße Verbindungen der Formel (I), worin G für Wasserstoff steht, V für C=O steht, W für Ethyl steht, A für A2 steht, R¹⁰ für Ethyl steht, und X, Y und Z, die in Tabelle 1 angegebenen Bedeutungen haben:

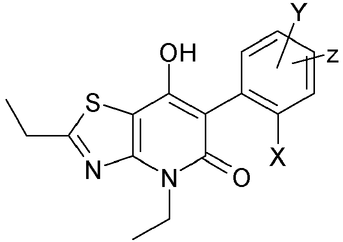

Tabelle 131: Erfindungsgemäße Verbindungen der Formel (I), worin G für Wasserstoff steht, V für SO₂ steht, W für Ethyl steht, A für A2 steht, R¹⁰ für n-Propyl steht, und X, Y und Z, die in Tabelle 1 angegebenen Bedeutungen haben:

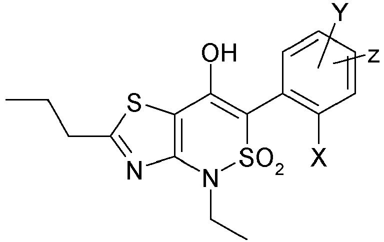

Tabelle 132: Erfindungsgemäße Verbindungen der Formel (I), worin G für Wasserstoff steht, V für C=O steht, W für Ethyl steht, A für A2 steht, R¹⁰ für n-Propyl steht, und X, Y und Z, die in Tabelle 1 angegebenen Bedeutungen haben:

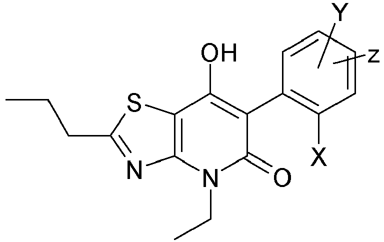

Tabelle 133: Erfindungsgemäße Verbindungen der Formel (I), worin G für Wasserstoff steht, V für SO₂ steht, W für Ethyl steht, A für A2 steht, R¹⁰ für i-Propyl steht, und X, Y und Z, die in Tabelle 1 angegebenen Bedeutungen haben:

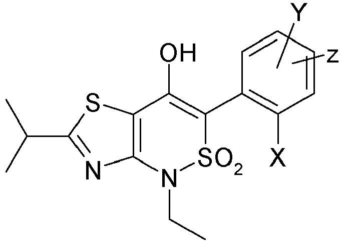

Tabelle 134: Erfindungsgemäße Verbindungen der Formel (I), worin G für Wasserstoff steht, V für C=O steht, W für Ethyl steht, A für A2 steht, R¹⁰ für i-Propyl steht, und X, Y und Z, die in Tabelle 1 angegebenen Bedeutungen haben:

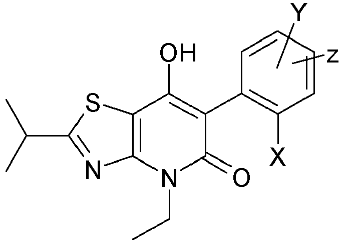

Tabelle 135: Erfindungsgemäße Verbindungen der Formel (I), worin G für Wasserstoff steht, V für SO₂ steht, W für Ethyl steht, A für A2 steht, R¹⁰ für c-Propyl steht, und X, Y und Z, die in Tabelle 1 angegebenen Bedeutungen haben:

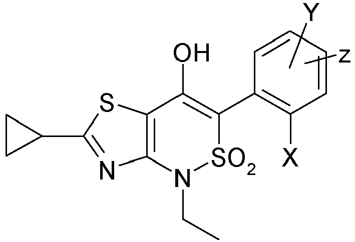

Tabelle 136: Erfindungsgemäße Verbindungen der Formel (I), worin G für Wasserstoff steht, V für C=O steht, W für Ethyl steht, A für A2 steht, R¹⁰ für c-Propyl steht, und X, Y und Z, die in Tabelle 1 angegebenen Bedeutungen haben:

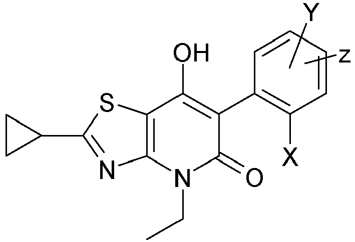

Tabelle 137: Erfindungsgemäße Verbindungen der Formel (I), worin G für Wasserstoff steht, V für SO₂ steht, W für Ethyl steht, A für A2 steht, R¹⁰ für Methoxy steht, und X, Y und Z, die in Tabelle 1 angegebenen Bedeutungen haben:

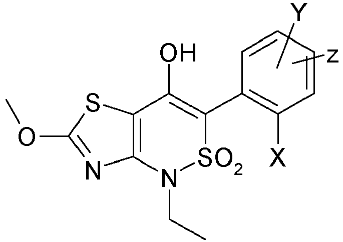

Tabelle 138: Erfindungsgemäße Verbindungen der Formel (I), worin G für Wasserstoff steht, V für C=O steht, W für Ethyl steht, A für A2 steht, R¹⁰ für Methoxy steht, und X, Y und Z, die in Tabelle 1 angegebenen Bedeutungen haben:

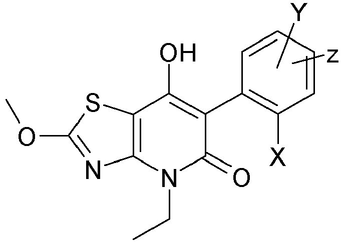

Tabelle 139: Erfindungsgemäße Verbindungen der Formel (I), worin G für Wasserstoff steht, V für SO₂ steht, W für Ethyl steht, A für A2 steht, R¹⁰ für Ethoxy steht, und X, Y und Z, die in Tabelle 1 angegebenen Bedeutungen haben:

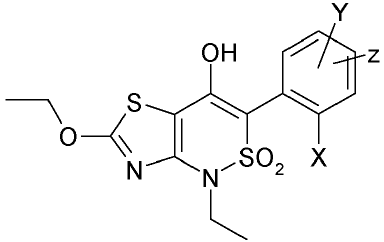

Tabelle 140: Erfindungsgemäße Verbindungen der Formel (I), worin G für Wasserstoff steht, V für C=O steht, W für Ethyl steht, A für A2 steht, R¹⁰ für Ethoxy steht, und X, Y und Z, die in Tabelle 1 angegebenen Bedeutungen haben:

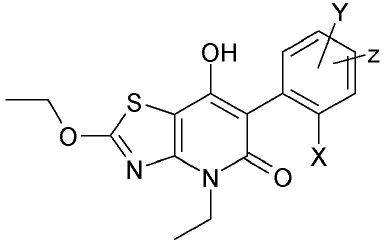

Tabelle 141: Erfindungsgemäße Verbindungen der Formel (I), worin G für Wasserstoff steht, V für SO₂ steht, W für Ethyl steht, A für A2 steht, R¹⁰ für Methoxyethoxy steht, und X, Y und Z, die in Tabelle 1 angegebenen Bedeutungen haben:

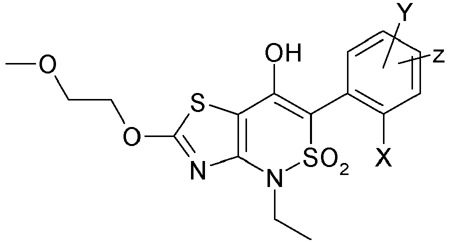

Tabelle 142: Erfindungsgemäße Verbindungen der Formel (I), worin G für Wasserstoff steht, V für C=O steht, W für Ethyl steht, A für A2 steht, R¹⁰ für Methoxyethoxy steht, und X, Y und Z, die in Tabelle 1 angegebenen Bedeutungen haben:

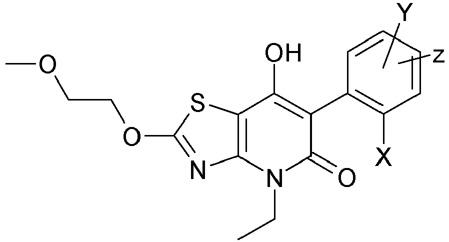

Tabelle 143: Erfindungsgemäße Verbindungen der Formel (I), worin G für Wasserstoff steht, V für SO₂ steht, W für Ethyl steht, A für A12 steht, und X, Y und Z, die in Tabelle 1 angegebenen Bedeutungen haben:

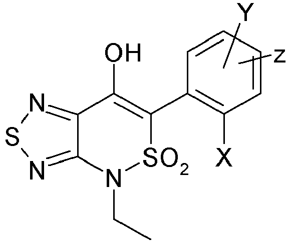

Tabelle 144: Erfindungsgemäße Verbindungen der Formel (I), worin G für Wasserstoff steht, V für C=O steht, W für Ethyl steht, A für A12 steht, und X, Y und Z, die in Tabelle 1 angegebenen Bedeutungen haben:

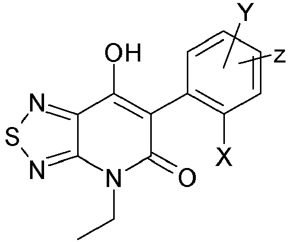

Tabelle 145: Erfindungsgemäße Verbindungen der Formel (I), worin G für Wasserstoff steht, V für SO₂ steht, W für Ethyl steht, A für A6 steht, R¹² für Wasserstoff steht, R¹⁴ für Ethyl steht, und X, Y und Z, die in Tabelle 1 angegebenen Bedeutungen haben:

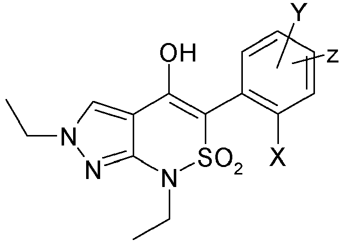

Tabelle 146: Erfindungsgemäße Verbindungen der Formel (I), worin G für Wasserstoff steht, V für C=O steht, W für Ethyl steht, A für A6 steht, R¹² für Wasserstoff steht, R¹⁴ für Ethyl steht, und X, Y und Z, die in Tabelle 1 angegebenen Bedeutungen haben:

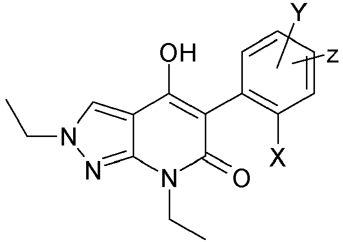

Tabelle 147: Erfindungsgemäße Verbindungen der Formel (I), worin G für Wasserstoff steht, V für SO₂ steht, W für 2,2-Difluorethyl steht, A für A10 steht, R¹² für Ethyl und R¹³ für Methyl stehen, und X, Y und Z, die in Tabelle 1 angegebenen Bedeutungen haben:

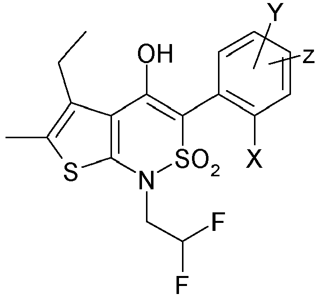

Tabelle 148: Erfindungsgemäße Verbindungen der Formel (I), worin G für Wasserstoff steht, V für C=O steht, W für 2,2-Difluorethyl steht, A für A10 steht, R¹² für Ethyl und R¹³ für Methyl stehen, und X, Y und Z, die in Tabelle 1 angegebenen Bedeutungen haben:

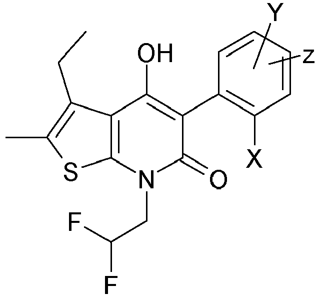

Tabelle 149: Erfindungsgemäße Verbindungen der Formel (I), worin G für Wasserstoff steht, V für SO₂ steht, W für 2,2-Difluorethyl steht, A für A10 steht, R¹² und R¹³ für Methyl stehen, und X, Y und Z, die in Tabelle 1 angegebenen Bedeutungen haben:

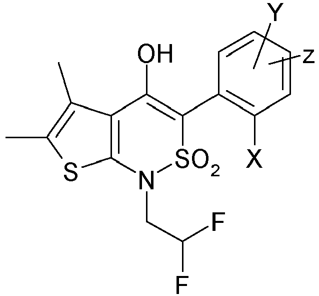

Tabelle 150: Erfindungsgemäße Verbindungen der Formel (I), worin G für Wasserstoff steht, V für C=O steht, W für 2,2-Difluorethyl steht, A für A10 steht, R¹² und R¹³ für Methyl stehen, und X, Y und Z, die in Tabelle 1 angegebenen Bedeutungen haben:

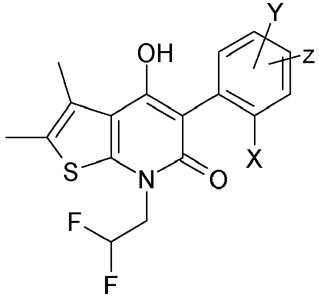

Tabelle 151: Erfindungsgemäße Verbindungen der Formel (I), worin G für Wasserstoff steht, V für SO₂ steht, W für 2,2-Difluorethyl steht, A für A7 steht, R¹² für Phenyl steht und R¹³ für Wasserstoff steht, und X, Y und Z, die in Tabelle 1 angegebenen Bedeutungen haben:

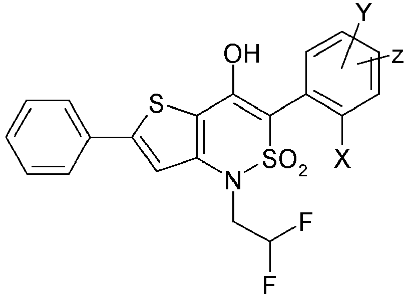

Tabelle 152: Erfindungsgemäße Verbindungen der Formel (I), worin G für Wasserstoff steht, V für C=O steht, W für 2,2-Difluorethyl steht, A für A10 steht, R¹² für Phenyl steht und R¹³ für Wasserstoff steht, und X, Y und Z, die in Tabelle 1 angegebenen Bedeutungen haben:

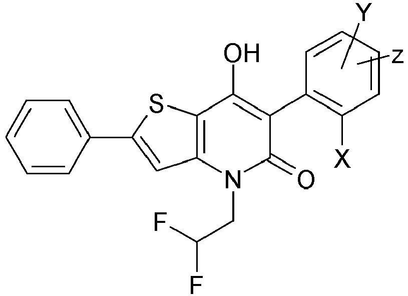

Tabelle 153: Erfindungsgemäße Verbindungen der Formel (I), worin G für Wasserstoff steht, V für SO₂ steht, W für 2,2-Difluorethyl steht, A für A2 steht, R¹⁰ für Methyl steht, und X, Y und Z, die in Tabelle 1 angegebenen Bedeutungen haben:

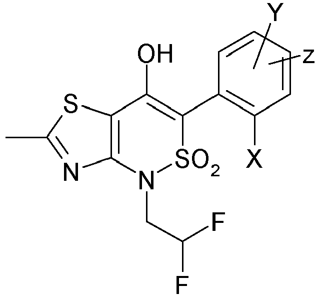

Tabelle 154: Erfindungsgemäße Verbindungen der Formel (I), worin G für Wasserstoff steht, V für C=O steht, W für 2,2-Difluorethyl steht, A für A2 steht, R¹⁰ für Methyl steht, und X, Y und Z, die in Tabelle 1 angegebenen Bedeutungen haben:

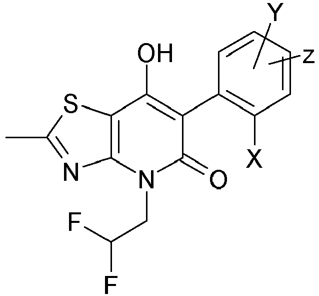

Tabelle 155: Erfindungsgemäße Verbindungen der Formel (I), worin G für Wasserstoff steht, V für SO₂ steht, W für 2,2-Difluorethyl steht, A für A2 steht, R¹⁰ für Ethyl steht, und X, Y und Z, die in Tabelle 1 angegebenen Bedeutungen haben:

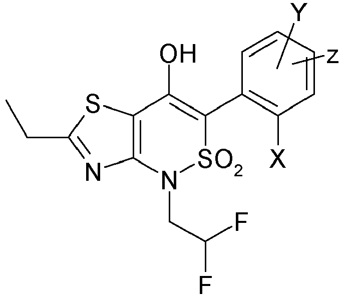

Tabelle 156: Erfindungsgemäße Verbindungen der Formel (I), worin G für Wasserstoff steht, V für C=O steht, W für 2,2-Difluorethyl steht, A für A2 steht, R¹⁰ für Ethyl steht, und X, Y und Z, die in Tabelle 1 angegebenen Bedeutungen haben:

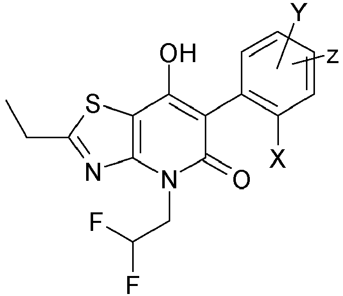

Tabelle 157: Erfindungsgemäße Verbindungen der Formel (I), worin G für Wasserstoff steht, V für SO₂ steht, W für 2,2-Difluorethyl steht, A für A2 steht, R¹⁰ für n-Propyl steht, und X, Y und Z, die in Tabelle 1 angegebenen Bedeutungen haben:

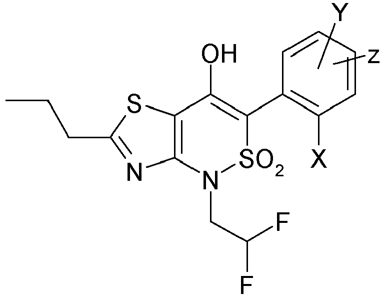

Tabelle 158: Erfindungsgemäße Verbindungen der Formel (I), worin G für Wasserstoff steht, V für C=O steht, W für 2,2-Difluorethyl steht, A für A2 steht, R¹⁰ für n-Propyl steht, und X, Y und Z, die in Tabelle 1 angegebenen Bedeutungen haben:

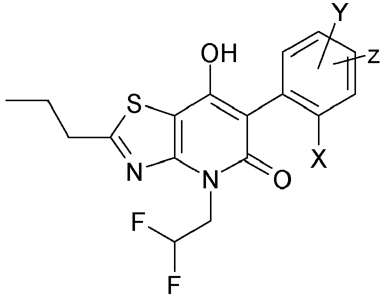

Tabelle 159: Erfindungsgemäße Verbindungen der Formel (I), worin G für Wasserstoff steht, V für SO₂ steht, W für 2,2-Difluorethyl steht, A für A2 steht, R¹⁰ für i-Propyl steht, und X, Y und Z, die in Tabelle 1 angegebenen Bedeutungen haben:

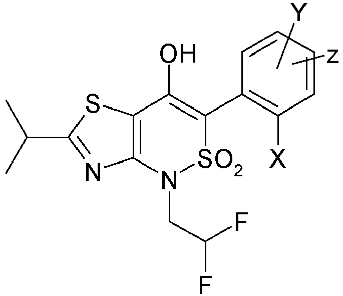

Tabelle 160: Erfindungsgemäße Verbindungen der Formel (I), worin G für Wasserstoff steht, V für C=O steht, W für 2,2-Difluorethyl steht, A für A2 steht, R¹⁰ für i-Propyl steht, und X, Y und Z, die in Tabelle 1 angegebenen Bedeutungen haben:

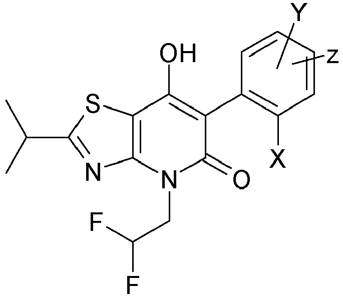

Tabelle 161: Erfindungsgemäße Verbindungen der Formel (I), worin G für Wasserstoff steht, V für SO₂ steht, W für 2,2-Difluorethyl steht, A für A2 steht, R¹⁰ für c-Propyl steht, und X, Y und Z, die in Tabelle 1 angegebenen Bedeutungen haben:

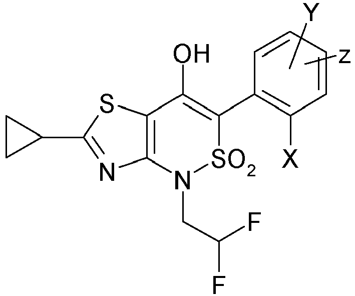

Tabelle 162: Erfindungsgemäße Verbindungen der Formel (I), worin G für Wasserstoff steht, V für C=O steht, W für 2,2-Difluorethyl steht, A für A2 steht, R¹⁰ für c-Propyl steht, und X, Y und Z, die in Tabelle 1 angegebenen Bedeutungen haben:

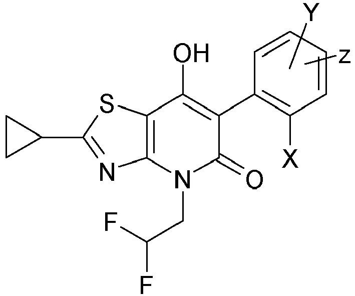

Tabelle 163: Erfindungsgemäße Verbindungen der Formel (I), worin G für Wasserstoff steht, V für SO₂ steht, W für 2,2-Difluorethyl steht, A für A2 steht, R¹⁰ für Methoxy steht, und X, Y und Z, die in Tabelle 1 angegebenen Bedeutungen haben:

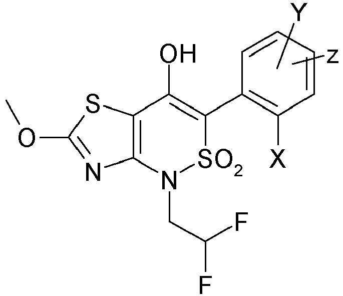

Tabelle 164: Erfindungsgemäße Verbindungen der Formel (I), worin G für Wasserstoff steht, V für C=O steht, W für 2,2-Difluorethyl steht, A für A2 steht, R¹⁰ für Methoxy steht, und X, Y und Z, die in Tabelle 1 angegebenen Bedeutungen haben:

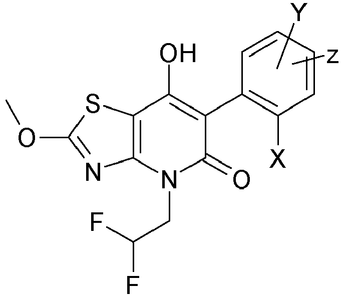

Tabelle 165: Erfindungsgemäße Verbindungen der Formel (I), worin G für Wasserstoff steht, V für SO₂ steht, W für 2,2-Difluorethyl steht, A für A2 steht, R¹⁰ für Ethoxy steht, und X, Y und Z, die in Tabelle 1 angegebenen Bedeutungen haben:

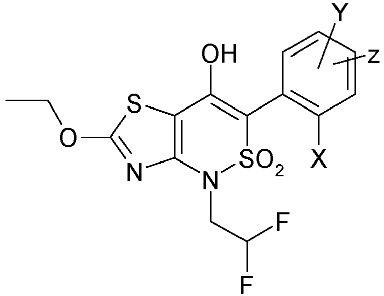

Tabelle 166: Erfindungsgemäße Verbindungen der Formel (I), worin G für Wasserstoff steht, V für C=O steht, W für 2,2-Difluorethyl steht, A für A2 steht, R¹⁰ für Ethoxy steht, und X, Y und Z, die in Tabelle 1 angegebenen Bedeutungen haben:

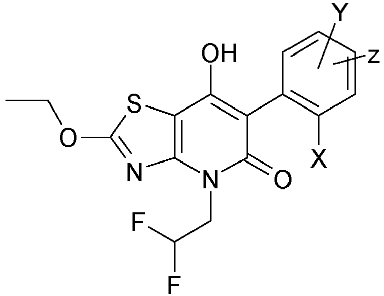

Tabelle 167: Erfindungsgemäße Verbindungen der Formel (I), worin G für Wasserstoff steht, V für SO₂ steht, W für 2,2-Difluorethyl steht, A für A2 steht, R¹⁰ für Methoxyethoxy steht, und X, Y und Z, die in Tabelle 1 angegebenen Bedeutungen haben:

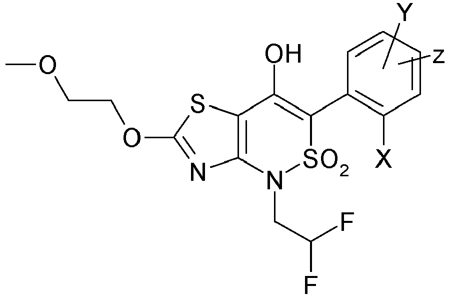

Tabelle 168: Erfindungsgemäße Verbindungen der Formel (I), worin G für Wasserstoff steht, V für C=O steht, W für 2,2-Difluorethyl steht, A für A2 steht, R¹⁰ für Methoxyethoxy steht, und X, Y und Z, die in Tabelle 1 angegebenen Bedeutungen haben:

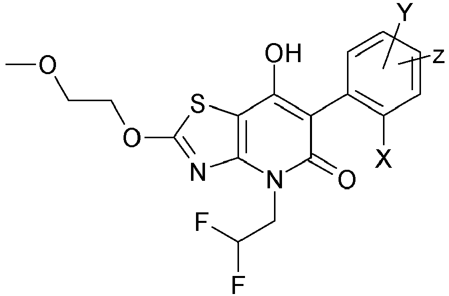

Tabelle 169: Erfindungsgemäße Verbindungen der Formel (I), worin G für Wasserstoff steht, V für SO₂ steht, W für 2,2-Difluorethyl steht, A für A12 steht, und X, Y und Z, die in Tabelle 1 angegebenen Bedeutungen haben:

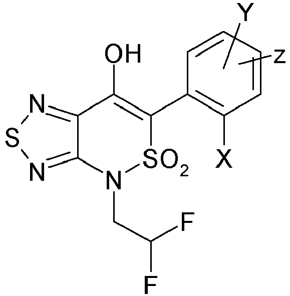

Tabelle 170: Erfindungsgemäße Verbindungen der Formel (I), worin G für Wasserstoff steht, V für C=O steht, W für 2,2-Difluorethyl steht, A für A12 steht, und X, Y und Z, die in Tabelle 1 angegebenen Bedeutungen haben:

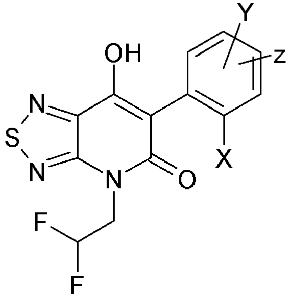

Tabelle 171: Erfindungsgemäße Verbindungen der Formel (I), worin G für Wasserstoff steht, V für SO₂ steht, W für 2,2-Difluorethyl steht, A für A6 steht, R¹² für Wasserstoff steht, R¹⁴ für Ethyl steht, und X, Y und Z, die in Tabelle 1 angegebenen Bedeutungen haben:

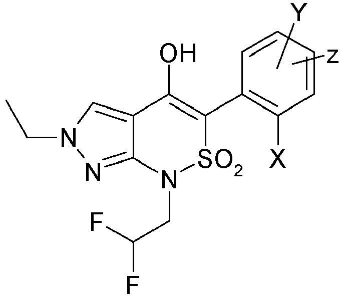

Tabelle 172: Erfindungsgemäße Verbindungen der Formel (I), worin G für Wasserstoff steht, V für C=O steht, W für 2,2-Difluorethyl steht, A für A6 steht, R¹² für Wasserstoff steht, R¹⁴ für Ethyl steht, und X, Y und Z, die in Tabelle 1 angegebenen Bedeutungen haben:

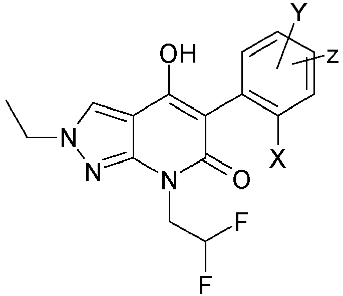

Tabelle 173: Erfindungsgemäße Verbindungen der Formel (I), worin G für Wasserstoff steht, V für SO₂ steht, W für Propinyl steht, A für A10 steht, R¹² für Ethyl und R¹³ für Methyl stehen, und X, Y und Z, die in Tabelle 1 angegebenen Bedeutungen haben:

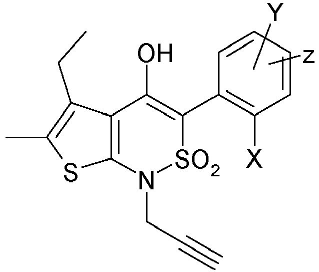

Tabelle 174: Erfindungsgemäße Verbindungen der Formel (I), worin G für Wasserstoff steht, V für C=O steht, W für Propinyl steht, A für A10 steht, R¹² für Ethyl und R¹³ für Methyl stehen, und X, Y und Z, die in Tabelle 1 angegebenen Bedeutungen haben:

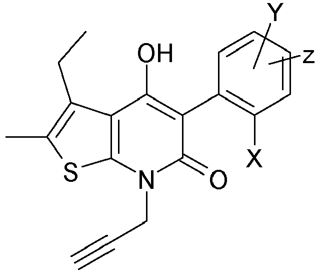

Tabelle 175: Erfindungsgemäße Verbindungen der Formel (I), worin G für Wasserstoff steht, V für SO₂ steht, W für Propinyl steht, A für A10 steht, R¹² und R¹³ für Methyl stehen, und X, Y und Z, die in Tabelle 1 angegebenen Bedeutungen haben:

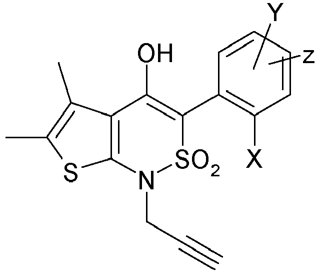

Tabelle 176: Erfindungsgemäße Verbindungen der Formel (I), worin G für Wasserstoff steht, V für C=O steht, W für Propinyl steht, A für A10 steht, R¹² und R¹³ für Methyl stehen, und X, Y und Z, die in Tabelle 1 angegebenen Bedeutungen haben:

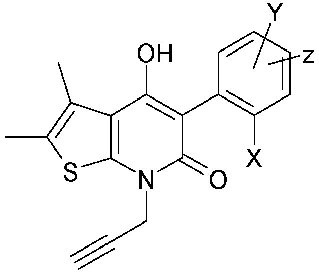

Tabelle 177: Erfindungsgemäße Verbindungen der Formel (I), worin G für Wasserstoff steht, V für SO₂ steht, W für Propinyl steht, A für A7 steht, R¹² für Phenyl steht und R¹³ für Wasserstoff steht, und X, Y und Z, die in Tabelle 1 angegebenen Bedeutungen haben:

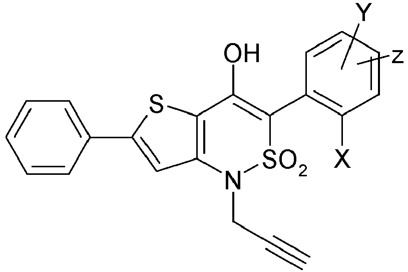

Tabelle 178: Erfindungsgemäße Verbindungen der Formel (I), worin G für Wasserstoff steht, V für C=O steht, W für Propinyl steht, A für A10 steht, R¹² für Phenyl steht und R¹³ für Wasserstoff steht, und X, Y und Z, die in Tabelle 1 angegebenen Bedeutungen haben:

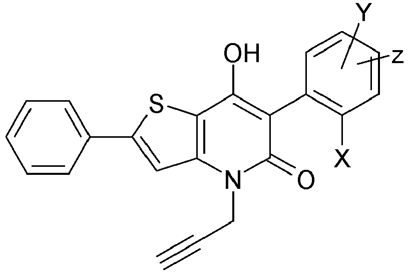

Tabelle 179: Erfindungsgemäße Verbindungen der Formel (I), worin G für Wasserstoff steht, V für SO₂ steht, W für Propinyl steht, A für A2 steht, R¹⁰ für Methyl steht, und X, Y und Z, die in Tabelle 1 angegebenen Bedeutungen haben:

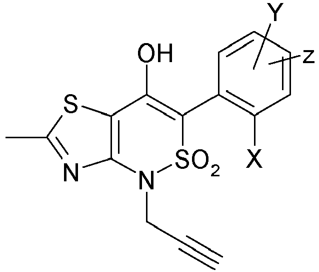

Tabelle 180: Erfindungsgemäße Verbindungen der Formel (I), worin G für Wasserstoff steht, V für C=O steht, W für Propinyl steht, A für A2 steht, R¹⁰ für Methyl steht, und X, Y und Z, die in Tabelle 1 angegebenen Bedeutungen haben:

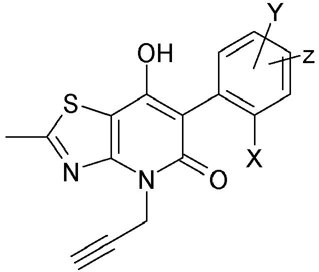

Tabelle 181: Erfindungsgemäße Verbindungen der Formel (I), worin G für Wasserstoff steht, V für SO₂ steht, W für Propinyl steht, A für A2 steht, R¹⁰ für Ethyl steht, und X, Y und Z, die in Tabelle 1 angegebenen Bedeutungen haben:

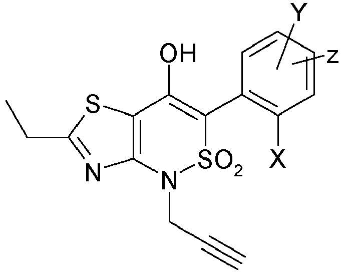

Tabelle 182: Erfindungsgemäße Verbindungen der Formel (I), worin G für Wasserstoff steht, V für C=O steht, W für Propinyl steht, A für A2 steht, R¹⁰ für Ethyl steht, und X, Y und Z, die in Tabelle 1 angegebenen Bedeutungen haben:

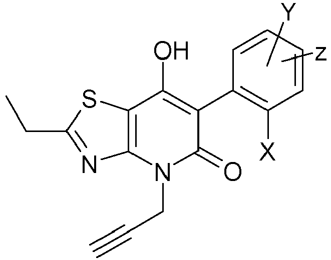

Tabelle 183: Erfindungsgemäße Verbindungen der Formel (I), worin G für Wasserstoff steht, V für SO₂ steht, W für Propinyl steht, A für A2 steht, R¹⁰ für n-Propyl steht, und X, Y und Z, die in Tabelle 1 angegebenen Bedeutungen haben:

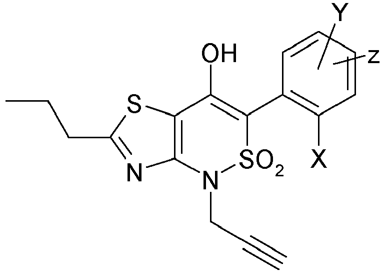

Tabelle 184: Erfindungsgemäße Verbindungen der Formel (I), worin G für Wasserstoff steht, V für C=O steht, W für Propinyl steht, A für A2 steht, R¹⁰ für n-Propyl steht, und X, Y und Z, die in Tabelle 1 angegebenen Bedeutungen haben:

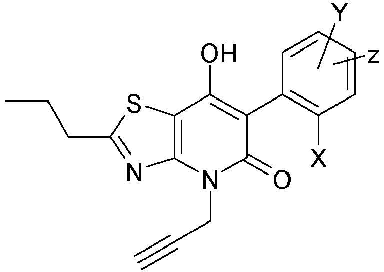

Tabelle 185: Erfindungsgemäße Verbindungen der Formel (I), worin G für Wasserstoff steht, V für SO₂ steht, W für Propinyl steht, A für A2 steht, R¹⁰ für i-Propyl steht, und X, Y und Z, die in Tabelle 1 angegebenen Bedeutungen haben:

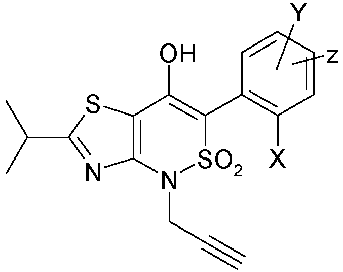

Tabelle 186: Erfindungsgemäße Verbindungen der Formel (I), worin G für Wasserstoff steht, V für C=O steht, W für Propinyl steht, A für A2 steht, R¹⁰ für i-Propyl steht, und X, Y und Z, die in Tabelle 1 angegebenen Bedeutungen haben:

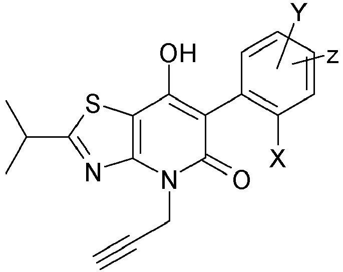

Tabelle 187: Erfindungsgemäße Verbindungen der Formel (I), worin G für Wasserstoff steht, V für SO₂ steht, W für Propinyl steht, A für A2 steht, R¹⁰ für c-Propyl steht, und X, Y und Z, die in Tabelle 1 angegebenen Bedeutungen haben:

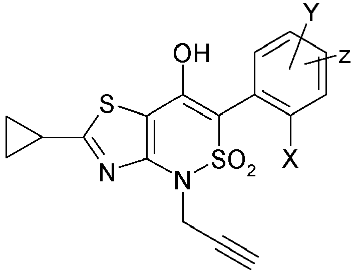

Tabelle 188: Erfindungsgemäße Verbindungen der Formel (I), worin G für Wasserstoff steht, V für C=O steht, W für Propinyl steht, A für A2 steht, R¹⁰ für c-Propyl steht, und X, Y und Z, die in Tabelle 1 angegebenen Bedeutungen haben:

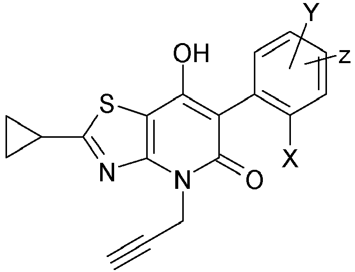

Tabelle 189: Erfindungsgemäße Verbindungen der Formel (I), worin G für Wasserstoff steht, V für SO₂ steht, W für Propinyl steht, A für A2 steht, R¹⁰ für Methoxy steht, und X, Y und Z, die in Tabelle 1 angegebenen Bedeutungen haben:

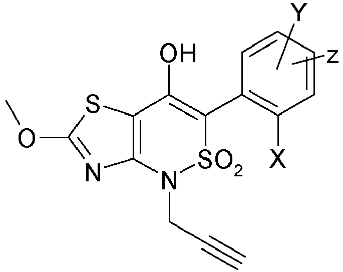

Tabelle 190: Erfindungsgemäße Verbindungen der Formel (I), worin G für Wasserstoff steht, V für C=O steht, W für Propinyl steht, A für A2 steht, R¹⁰ für Methoxy steht, und X, Y und Z, die in Tabelle 1 angegebenen Bedeutungen haben:

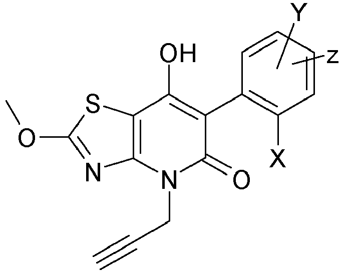

Tabelle 191: Erfindungsgemäße Verbindungen der Formel (I), worin G für Wasserstoff steht, V für SO₂ steht, W für Propinyl steht, A für A2 steht, R¹⁰ für Ethoxy steht, und X, Y und Z, die in Tabelle 1 angegebenen Bedeutungen haben:

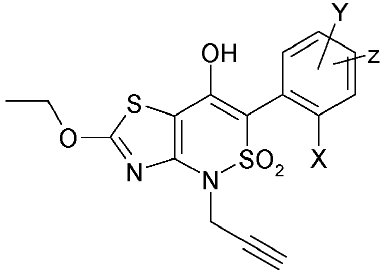

Tabelle 191: Erfindungsgemäße Verbindungen der Formel (I), worin G für Wasserstoff steht, V für C=O steht, W für Propinyl steht, A für A2 steht, R¹⁰ für Ethoxy steht, und X, Y und Z, die in Tabelle 1 angegebenen Bedeutungen haben:

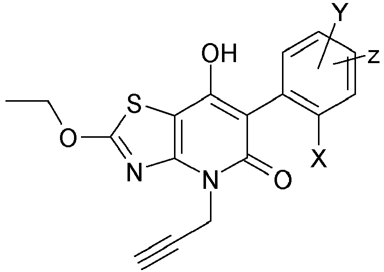

Tabelle 193: Erfindungsgemäße Verbindungen der Formel (I), worin G für Wasserstoff steht, V für SO₂ steht, W für Propinyl steht, A für A2 steht, R¹⁰ für Methoxyethoxy steht, und X, Y und Z, die in Tabelle 1 angegebenen Bedeutungen haben:

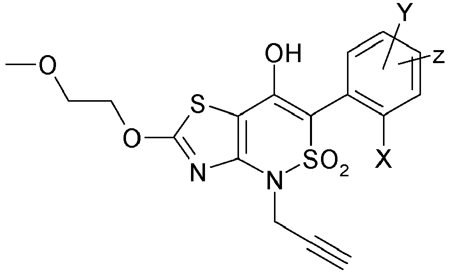

Tabelle 194: Erfindungsgemäße Verbindungen der Formel (I), worin G für Wasserstoff steht, V für C=O steht, W für Propinyl steht, A für A2 steht, R¹⁰ für Methoxyethoxy steht, und X, Y und Z, die in Tabelle 1 angegebenen Bedeutungen haben:

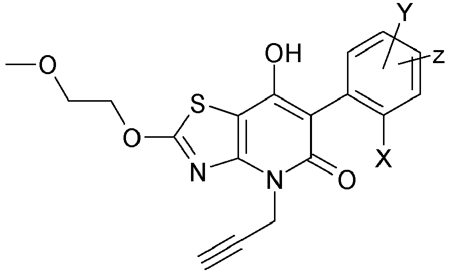

Tabelle 195: Erfindungsgemäße Verbindungen der Formel (I), worin G für Wasserstoff steht, V für SO₂ steht, W für Propinyl steht, A für A12 steht, und X, Y und Z, die in Tabelle 1 angegebenen Bedeutungen haben:

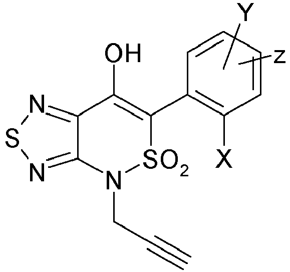

Tabelle 196: Erfindungsgemäße Verbindungen der Formel (I), worin G für Wasserstoff steht, V für C=O steht, W für Propinyl steht, A für A12 steht, und X, Y und Z, die in Tabelle 1 angegebenen Bedeutungen haben:

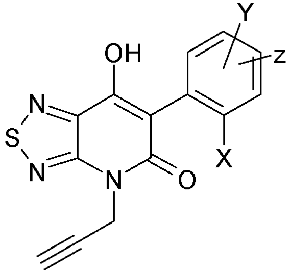

Tabelle 197: Erfindungsgemäße Verbindungen der Formel (I), worin G für Wasserstoff steht, V für SO₂ steht, W für Propinyl steht, A für A6 steht, R¹² für Wasserstoff steht, R¹⁴ für Ethyl steht, und X, Y und Z, die in Tabelle 1 angegebenen Bedeutungen haben:

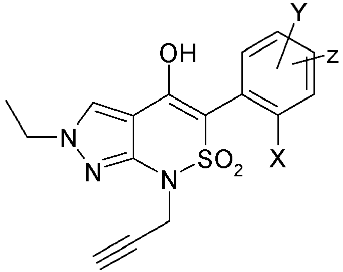

Tabelle 198: Erfindungsgemäße Verbindungen der Formel (I), worin G für Wasserstoff steht, V für C=O steht, W für Propinyl steht, A für A6 steht, R¹² für Wasserstoff steht, R¹⁴ für Ethyl steht, und X, Y und Z, die in Tabelle 1 angegebenen Bedeutungen haben:

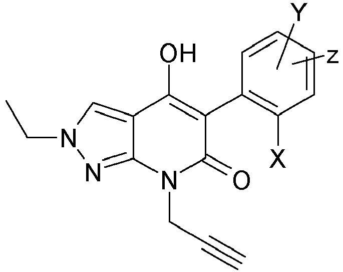

Tabelle 199: Erfindungsgemäße Verbindungen der Formel (I), worin G für Wasserstoff steht, V für SO₂ steht, W für Methyl steht, A für A10 steht, R¹² für Ethyl und R¹³ für Methyl stehen, und X, Y und Z, die in Tabelle 1 angegebenen Bedeutungen haben:

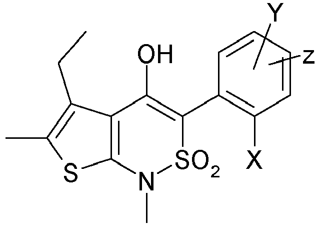

Tabelle 200: Erfindungsgemäße Verbindungen der Formel (I), worin G für Wasserstoff steht, V für C=O steht, W für Methyl steht, A für A10 steht, R¹² für Ethyl und R¹³ für Methyl stehen, und X, Y und Z, die in Tabelle 1 angegebenen Bedeutungen haben:

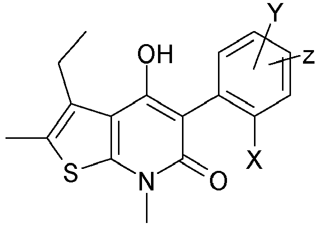

Tabelle 201: Erfindungsgemäße Verbindungen der Formel (I), worin G für Wasserstoff steht, V für SO₂ steht, W für Methyl steht, A für A10 steht, R'² und R¹³ für Methyl stehen, und X, Y und Z, die in Tabelle 1 angegebenen Bedeutungen haben:

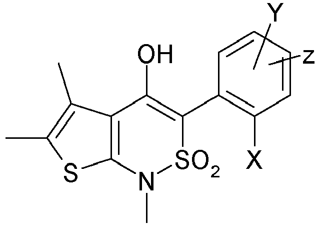

Tabelle 202: Erfindungsgemäße Verbindungen der Formel (I), worin G für Wasserstoff steht, V für C=O steht, W für Methyl steht, A für A10 steht, R¹² und R¹³ für Methyl stehen, und X, Y und Z, die in Tabelle 1 angegebenen Bedeutungen haben:

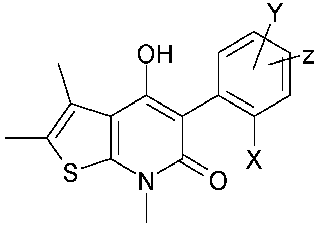

Tabelle 203: Erfindungsgemäße Verbindungen der Formel (I), worin G für Wasserstoff steht, V für SO₂ steht, W für Methyl steht, A für A7 steht, R¹² für Phenyl steht und R¹³ für Wasserstoff steht, und X, Y und Z, die in Tabelle 1 angegebenen Bedeutungen haben:

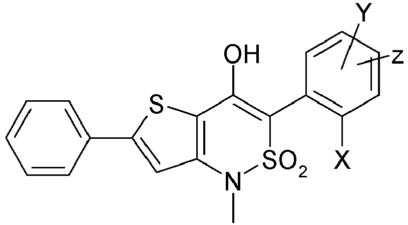

Tabelle 204: Erfindungsgemäße Verbindungen der Formel (I), worin G für Wasserstoff steht, V für C=O steht, W für Methyl steht, A für A10 steht, R¹² für Phenyl steht und R¹³ für Wasserstoff steht, und X, Y und Z, die in Tabelle 1 angegebenen Bedeutungen haben:

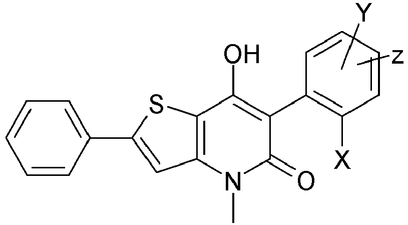

Tabelle 205: Erfindungsgemäße Verbindungen der Formel (I), worin G für Wasserstoff steht, V für SO₂ steht, W für Methyl steht, A für A2 steht, R¹⁰ für Methyl steht, und X, Y und Z, die in Tabelle 1 angegebenen Bedeutungen haben:

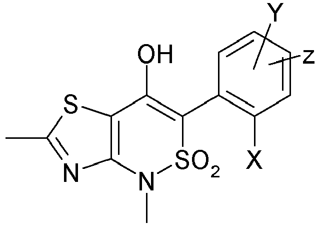

Tabelle 206: Erfindungsgemäße Verbindungen der Formel (I), worin G für Wasserstoff steht, V für C=O steht, W für Methyl steht, A für A2 steht, R¹⁰ für Methyl steht, und X, Y und Z, die in Tabelle 1 angegebenen Bedeutungen haben:

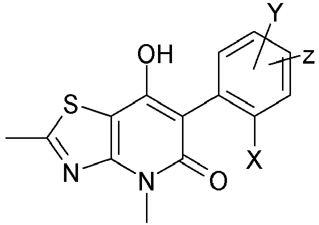

Tabelle 207: Erfindungsgemäße Verbindungen der Formel (I), worin G für Wasserstoff steht, V für SO₂ steht, W für Methyl steht, A für A2 steht, R¹⁰ für Ethyl steht, und X, Y und Z, die in Tabelle 1 angegebenen Bedeutungen haben:

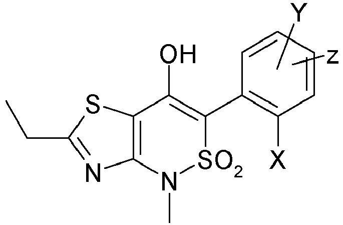

Tabelle 208: Erfindungsgemäße Verbindungen der Formel (I), worin G für Wasserstoff steht, V für C=O steht, W für Methyl steht, A für A2 steht, R¹⁰ für Ethyl steht, und X, Y und Z, die in Tabelle 1 angegebenen Bedeutungen haben:

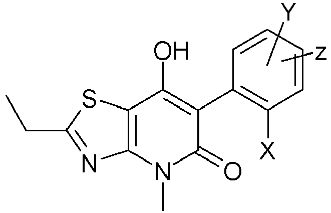

Tabelle 209: Erfindungsgemäße Verbindungen der Formel (I), worin G für Wasserstoff steht, V für SO₂ steht, W für Methyl steht, A für A2 steht, R¹⁰ für n-Propyl steht, und X, Y und Z, die in Tabelle 1 angegebenen Bedeutungen haben:

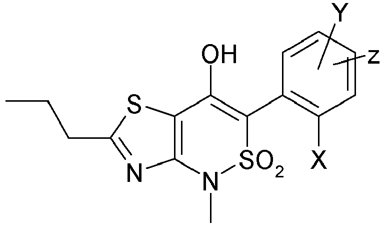

Tabelle 210: Erfindungsgemäße Verbindungen der Formel (I), worin G für Wasserstoff steht, V für C=O steht, W für Methyl steht, A für A2 steht, R¹⁰ für n-Propyl steht, und X, Y und Z, die in Tabelle 1 angegebenen Bedeutungen haben:

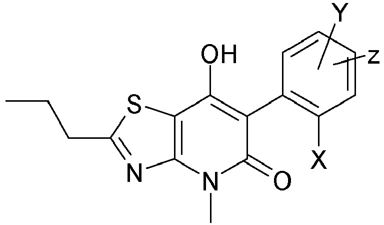

Tabelle 211: Erfindungsgemäße Verbindungen der Formel (I), worin G für Wasserstoff steht, V für SO₂ steht, W für Methyl steht, A für A2 steht, R¹⁰ für i-Propyl steht, und X, Y und Z, die in Tabelle 1 angegebenen Bedeutungen haben:

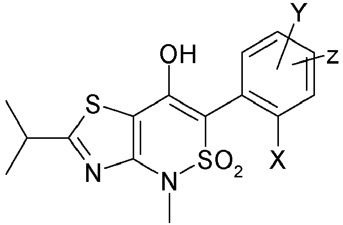

Tabelle 212: Erfindungsgemäße Verbindungen der Formel (I), worin G für Wasserstoff steht, V für C=O steht, W für Methyl steht, A für A2 steht, R¹⁰ für i-Propyl steht, und X, Y und Z, die in Tabelle 1 angegebenen Bedeutungen haben:

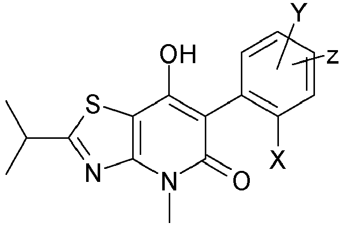

Tabelle 213: Erfindungsgemäße Verbindungen der Formel (I), worin G für Wasserstoff steht, V für SO₂ steht, W für Methyl steht, A für A2 steht, R¹⁰ für c-Propyl steht, und X, Y und Z, die in Tabelle 1 angegebenen Bedeutungen haben:

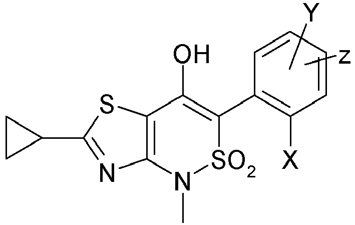

Tabelle 214: Erfindungsgemäße Verbindungen der Formel (I), worin G für Wasserstoff steht, V für C=O steht, W für Methyl steht, A für A2 steht, R¹⁰ für c-Propyl steht, und X, Y und Z, die in Tabelle 1 angegebenen Bedeutungen haben:

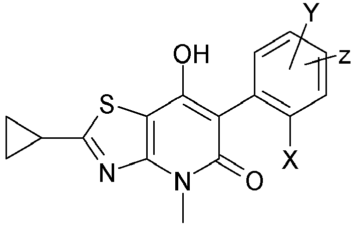

Tabelle 215: Erfindungsgemäße Verbindungen der Formel (I), worin G für Wasserstoff steht, V für SO₂ steht, W für Methyl steht, A für A2 steht, R¹⁰ für Methoxy steht, und X, Y und Z, die in Tabelle 1 angegebenen Bedeutungen haben:

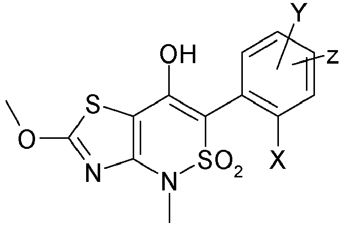

Tabelle 216: Erfindungsgemäße Verbindungen der Formel (I), worin G für Wasserstoff steht, V für C=O steht, W für Methyl steht, A für A2 steht, R¹⁰ für Methoxy steht, und X, Y und Z, die in Tabelle 1 angegebenen Bedeutungen haben:

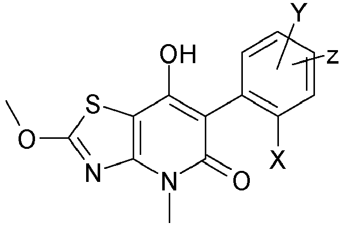

Tabelle 217: Erfindungsgemäße Verbindungen der Formel (I), worin G für Wasserstoff steht, V für SO₂ steht, W für Methyl steht, A für A2 steht, R¹⁰ für Ethoxy steht, und X, Y und Z, die in Tabelle 1 angegebenen Bedeutungen haben:

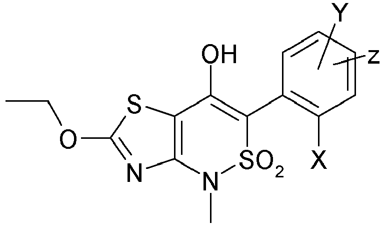

Tabelle 218: Erfindungsgemäße Verbindungen der Formel (I), worin G für Wasserstoff steht, V für C=O steht, W für Methyl steht, A für A2 steht, R¹⁰ für Ethoxy steht, und X, Y und Z, die in Tabelle 1 angegebenen Bedeutungen haben:

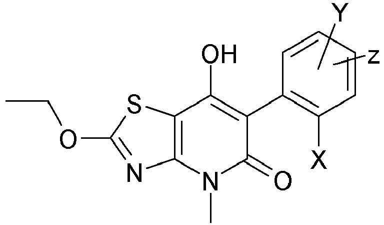

Tabelle 219: Erfindungsgemäße Verbindungen der Formel (I), worin G für Wasserstoff steht, V für SO₂ steht, W für Methyl steht, A für A2 steht, R¹⁰ für Methoxyethoxy steht, und X, Y und Z, die in Tabelle 1 angegebenen Bedeutungen haben:

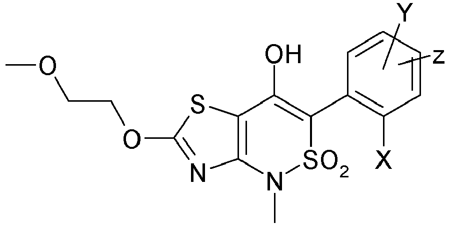

Tabelle 220: Erfindungsgemäße Verbindungen der Formel (I), worin G für Wasserstoff steht, V für C=O steht, W für Methyl steht, A für A2 steht, R¹⁰ für Methoxyethoxy steht, und X, Y und Z, die in Tabelle 1 angegebenen Bedeutungen haben:

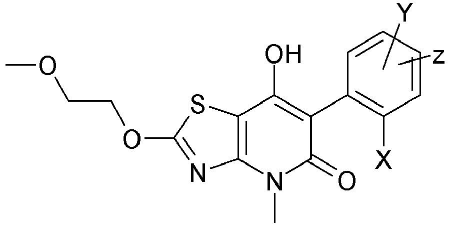

Tabelle 221: Erfindungsgemäße Verbindungen der Formel (I), worin G für Wasserstoff steht, V für SO₂ steht, W für Methyl steht, A für A12 steht, und X, Y und Z, die in Tabelle 1 angegebenen Bedeutungen haben:

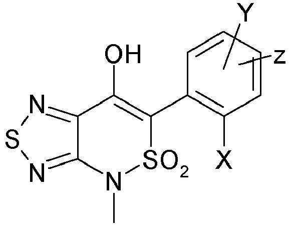

Tabelle 222: Erfindungsgemäße Verbindungen der Formel (I), worin G für Wasserstoff steht, V für C=O steht, W für Methyl steht, A für A12 steht, und X, Y und Z, die in Tabelle 1 angegebenen Bedeutungen haben:

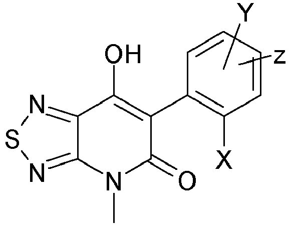

Tabelle 223: Erfindungsgemäße Verbindungen der Formel (I), worin G für Wasserstoff steht, V für SO₂ steht, W für Methyl steht, A für A6 steht, R¹² für Wasserstoff steht, R¹⁴ für Ethyl steht, und X, Y und Z, die in Tabelle 1 angegebenen Bedeutungen haben:

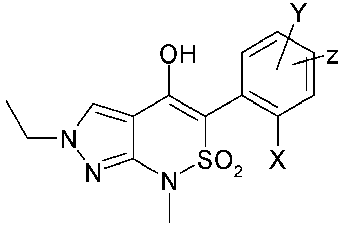

Tabelle 224: Erfindungsgemäße Verbindungen der Formel (I), worin G für Wasserstoff steht, V für C=O steht, W für Methyl steht, A für A6 steht, R¹² für Wasserstoff steht, R¹⁴ für Ethyl steht, und X, Y und Z, die in Tabelle 1 angegebenen Bedeutungen haben:

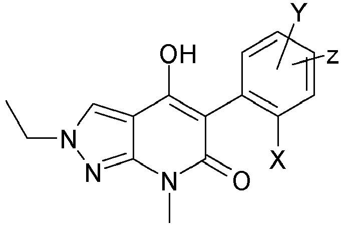

Tabelle 225: Erfindungsgemäße Verbindungen der Formel (I), worin G für Wasserstoff steht, V für SO₂ steht, W für Methoxyethyl steht, A für A2 steht, R¹⁰ für Wasserstoff steht, und X, Y und Z, die in Tabelle 1 angegebenen Bedeutungen haben:

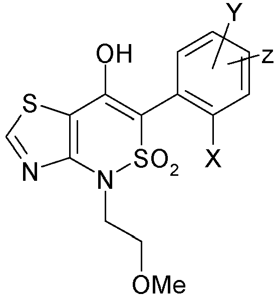

Tabelle 226: Erfindungsgemäße Verbindungen der Formel (I), worin G für Wasserstoff steht, V für C=O steht, W für Methoxyethyl steht, A für A2 steht, R¹⁰ für Wasserstoff steht, und X, Y und Z, die in Tabelle 1 angegebenen Bedeutungen haben:

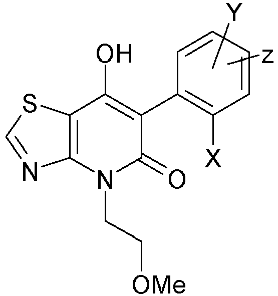

Tabelle 227: Erfindungsgemäße Verbindungen der Formel (I), worin G für Wasserstoff steht, V für SO₂ steht, W für Methylthioethyl steht, A für A2 steht, R¹⁰ für Wasserstoff steht, und X, Y und Z, die in Tabelle 1 angegebenen Bedeutungen haben:

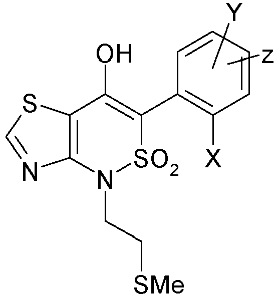

Tabelle 228: Erfindungsgemäße Verbindungen der Formel (I), worin G für Wasserstoff steht, V für C=O steht, W für Methylthioethyl steht, A für A2 steht, R¹⁰ für Wasserstoff steht, und X, Y und Z, die in Tabelle 1 angegebenen Bedeutungen haben:

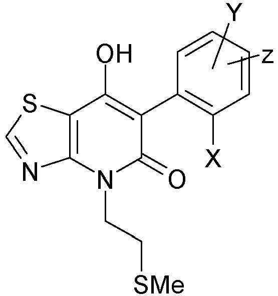

Erfindungsgemäße Verbindungen der Formel (I), worin G für Wasserstoff steht, V für C=O oder SO₂ steht, können beispielsweise gemäß der in Schema 1 angegebenen Methode durch baseninduzierte Kondensationsreaktion von Verbindungen der Formel (II) hergestellt werden. Darin steht R⁹ für (C₁-C₆)-Alkyl, insbesonders für Methyl oder Ethyl.

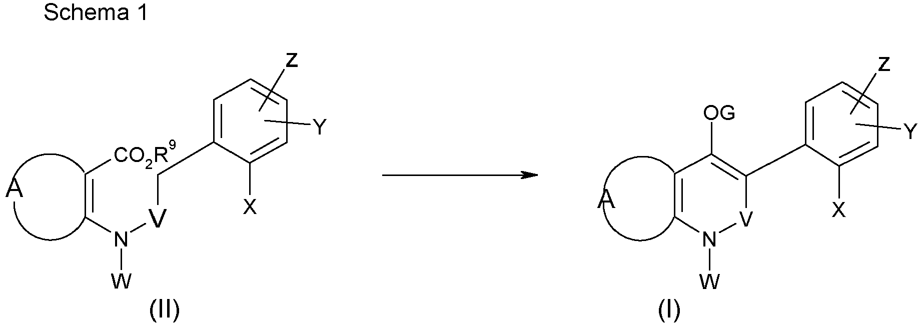

Verbindungen der Formel (II) lassen sich beispielsweise gemäß der in Schema 1a angegebenen Methoden durch Reaktion von Aminocarbonsäurederivaten mit Phenylessigsäurederivaten oder Benzylsulfonsäurederivaten herstellen. Darin steht U für eine durch Carbonsäureaktivierungsreagenzien, wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid, Phosgen oder Chlorameisensäureester eingeführte Abgangsgruppe. Solche Methoden sind auch aus WO2008/009908 A1 und WO2008/071918 A1 bzw. WO2009/063180 und dort zitierten Dokumenten dem Fachmann bekannt. Verbindungen der Formel (II) sind neu und ebenfalls ein Gegenstand der vorliegenden Erfindung.

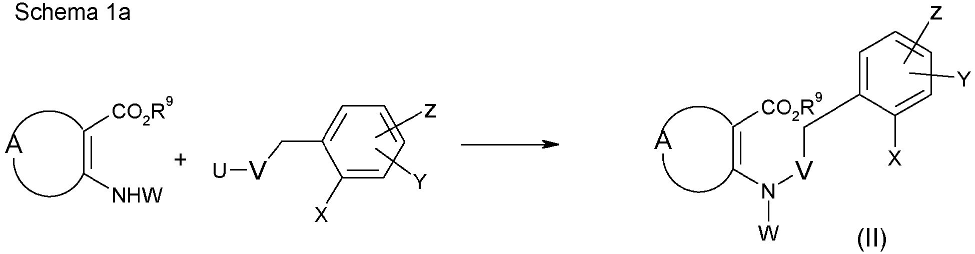

Die zur Herstellung der in Schema 1a genannten Phenylessigsäurederivaten notwendigen freien Phenylessigsäuren, d.h. solche worin U für Hydroxy und V für C=O steht, sind bekannt oder lassen sich nach an sich und beispielsweise aus WO 2005/075401, WO 2001/96277, WO 1996/35664 und WO 1996/25395 bekannten Verfahren herstellen. Für den Fall, dass W einen anderen Rest als Wasserstoff bedeuten soll, kann ein Rest W' nach literaturbekannten Methoden eingeführt werden beispielsweise über reduktive Aminierung eines entsprechenden Aminosäureesters mit einem Aldehyd gefolgt von einer Reduktion beispielsweise mit Natriumcyanoborhydrid. W' bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl, (C₃-C₆)-Cycloalkyl-(C₁-C₆)-alkyl.
Weiterhin ist bekannt, dass Umsetzungen von W'-Halogeniden oder auch entsprechenden Sulfonaten mit entsprechenden Aminosäureestern zu den gewünschten Vorstufen führen. Alternativ kann entsprechend auch nach erfolgter Kondensation des Aminosäureesters mit der entsprechenden Phenylessigsäure oder Benzylsulfonsäure die Alkylierung mit W'-Halogeniden oder Sulfonaten erfolgen (s. Schema 1 b), die dann ebenfalls zu den erfindungsgemäßen Zwischenprodukten II führt.

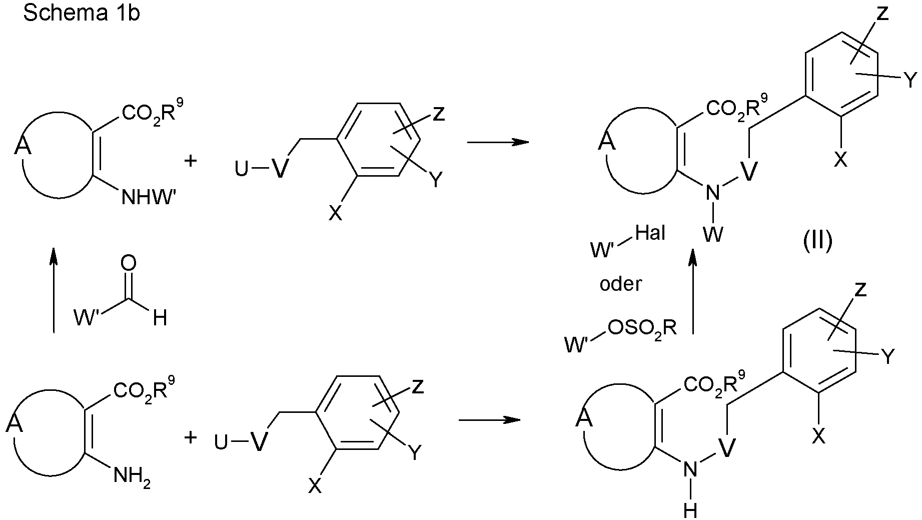

Bestimmte Phenylessigsäurederivate lassen sich auch unter Verwendung von Essigesterenolaten in Gegenwart von Palladiumkatalysatoren, z.B. gebildet aus einer Palladiumquelle (z.B. Pd₂(dba)₃ oder Pd(Oac)₂) und einem Liganden (z.B. (t-Bu)₃P, iMes*HCl oder 2'-(N,N-Dimethylamino)-2-(dicyclohexylphosphanyl)biphenyl) hergestellt (WO 2005/048710, J. Am. Chem. Soc 2002. 124,. 12557, J. Am. Chem. Soc 2003. 125, 11176 oder J. Am. Chem. Soc. 2001, 123, 799) herstellen. Darüber hinaus lassen sich bestimmte substituierte Arylhalogenide unter Kupferkatalyse in die korrespondierenden substituierten Malonester überführen (z.B. beschrieben in Org. Lett. 2002, 2, 269, WO 2004/108727), welche nach bekannten Methoden in Phenylessigsäuren überführt werden können.
Die zur Herstellung der in Schema 1a genannten Benzylsulfonsäurederivate notwendigen freien Benzylsulfonsäuren, d.h. solche worin U für Hydroxy und V für SO₂ steht, sind bekannt oder lassen sich nach an sich und beispielsweise aus WO2009/063180 bekannten Verfahren herstellen.

Erfindungsgemäße Verbindungen der Formel (I), worin G für Wasserstoff steht, können beispielsweise auch gemäß der in Schema 2 angegebenen Methode durch Reaktion von Verbindungen der Formel (I), worin G für Alkyl, bevorzugt für Methyl, steht, mit starken Mineralbasen, wie Natrium- oder Kaliumhydroxid, oder in konzentrierten Mineralsäuren, wie Bromwassserstoffsäure, hergestellt werden.

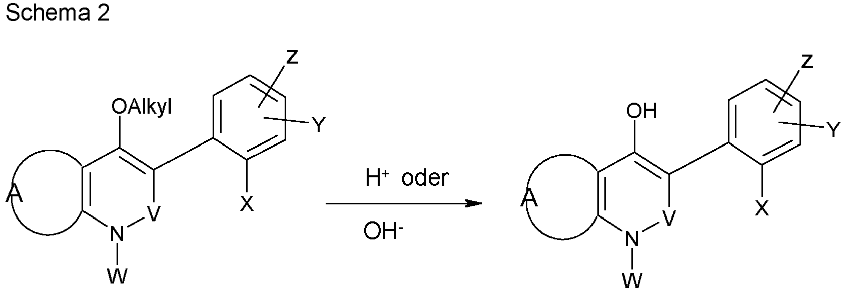

Erfindungsgemäße Verbindungen der Formel (I), worin G für C(=O)R¹ steht, können beispielsweise durch dem Fachmann bekannte Reaktionen von Verbindungen der Formel (I), worin G für Wasserstoff steht, mit Carbonsäurehalogeniden der Formel Hal-CO-R¹ oder mit Carbonsäureanhydriden der Formel R¹-CO-O-CO-R¹ hergestellt werden.
Erfindungsgemäße Verbindungen der Formel (I), worin G für C(=L)MR² steht, können beispielsweise durch dem Fachmann bekannte Reaktionen von Verbindungen der Formel (I), worin G für Wasserstoff steht, mit a) Chlorameisen-säureestern oder Chlorameisensäurethioestern der Formel R²-M-COOR¹ oder b) mit Chlorameisensäurehalogeniden oder Chlorameisensäurethiohalogeniden hergestellt werden.
Erfindungsgemäße Verbindungen der Formel (I), worin G für SO₂R³ steht, können beispielsweise durch dem Fachmann bekannte Reaktionen von Verbindungen der Formel (I), worin G für Wasserstoff steht, mit Sulfonsäurechloriden der Formel R³-SO₂-Cl hergestellt werden.
Erfindungsgemäße Verbindungen der Formel (I), worin G für P(=L)R⁴R⁵ steht, können beispielsweise durch dem Fachmann bekannte Reaktionen von Verbindungen der Formel (I), worin G für Wasserstoff steht, mit Phosphorsäurechloriden der Formel Hal-P(=L)R⁴R⁵ hergestellt werden. Erfindungsgemäße Verbindungen der Formel (I), worin G für E steht, können beispielsweise durch dem Fachmann bekannte Reaktionen von Verbindungen der Formel (I), worin G für Wasserstoff steht, mit Metallverbindungen der Formel Me(OR¹⁰)ₜ oder mit Aminen hergestellt werden. Darin bedeutet Me ein ein- oder zweiwertiges Metallion, bevorzugt ein Alkali- oder Erdalkalimetall wie Lithium, Natrium, Kalium, Magnesium oder Calcium. Der Index t steht für 1 oder 2. Ein Ammoniumion bedeutet die Gruppe NH₄⁺ oder R¹³R¹⁴R¹⁵R¹⁶N⁺, worin R¹³, R¹⁴, R¹⁵ und R¹⁶ unabhängig voneinander vorzugsweise (C₁-C₆)-Alkyl oder Benzyl bedeuten. Erfindungsgemäße Verbindungen der Formel (I), worin G für C(=L)NR⁶R⁷ steht, können beispielsweise durch dem Fachmann bekannte Reaktionen von Verbindungen der Formel (I), worin G für Wasserstoff steht, mit Isocyanaten oder Isothiocyanaten der Formel R⁶-N=C=L oder mit Carbamidsäurechloriden oder Thiocarbamidsäurechloriden der Formel R⁶R⁷N-C(=L)Cl hergestellt werden. Erfindungsgemäße Verbindungen der Formel (I), worin G für Alkyl, bevorzugt für Methyl, steht, können beispielsweise auch gemäß Schema 3 durch dem Fachmann bekannte Reaktionen von Verbindungen der Formel (III) mit Verbindungen der Formel (IV) hergestellt werden. Darin steht Z' für Brom oder lod, und Q bedeutet eine Trialkylzinngruppe, eine Magnesiumhalogenidgruppe oder bevorzugt eine Boronsäure oder deren Ester. Diese Reaktionen werden üblicherweise in Gegenwart eines Katalysators (z. B. Pd-Salze oder Pd-Komplexe) und in Gegenwart einer Base (z.B. Natriumcarbonat, Kaliumphosphat) durchgeführt.

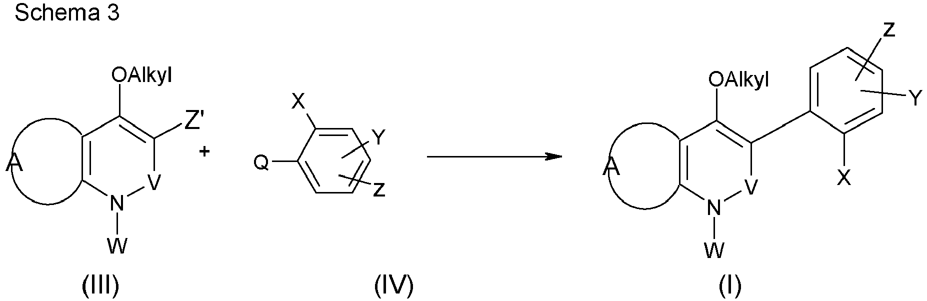

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Waiden, Essex, CB 11 3AZ, England oder MultiPROBE Automated Workstations der Firma Perkin Elmar, Waltham, Massachusetts 02451, USA. Für die parallelisierte Aufreinigung von Verbindungen der Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasenunterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono-oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen- Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2009 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequatchlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinateammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), Iodosulfuron, Iodosulfuronmethyl-natrium, Ioxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

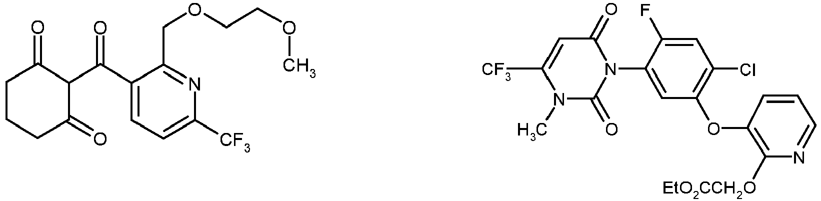

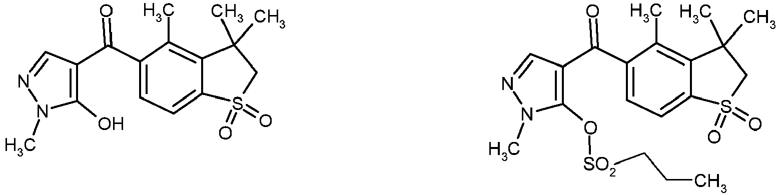

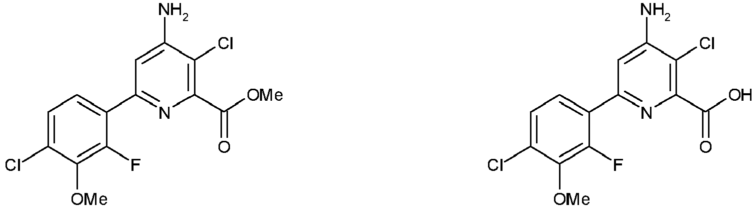

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung näher.

### A. Chemische Beispiele

### 1. Herstellung von 4-Allyl-7-hydroxy-6-mesityl[1,3]thiazolo[4,5-b]pyridin-5(4H)-on (Verbindung Nr. I-a-2):

1.8 g (5 mmol) Methyl-4-[allyl(mesitylacetyl)amino]-1,3-thiazol-5-carboxylat wurden in 5ml N, N-Dimethylformamid vorgelegt und auf 0 °C abgekühlt. Man gab 1.5 eq Natriumhydrid (60%ig) zu und ließ langsam auf Raumtemperatur (RT) erwärmen. Nach einstündigem Rühren bei RT wurde mit 5 ml Wasser versetzt und auf pH 1-2 angesäuert. Der entstandene Niederschlag wurde abgesaugt. Es wurden so 1.5 g an erfindungsgemäßer Verbindung I-a-2 erhalten.

### 2. Herstellung von 1-(2,2-Difluorethyl)-3-(2-iodphenyl)-1H-[1,3]thiazolo[4,5-c][1,2]thiazin-4-ol-2,2-dioxid (Verbindung Nr. I-a-14)

2.0 g (4 mmol) Methyl-4-{(2,2-difluorethyl)[(2-iodbenzyl)sulfonyl]amino}-1,3-thiazol-5-carboxylat wurden in 25 ml Dimethylformamid gelöst, auf 0°C abgekühlt und mit 143.3 mg (6 mmol) Natriumhydrid versetzt. Nach Zugabe wurde auf RT erwärmt und 12h gerührt. Danach wurde die Reaktionsmischung auf 100 ml Wasser gegossen und mit 2N HCl auf pH 4-5 gebracht. Der entstandene Niederschlag wurde abfiltriert und mit Wasser gewaschen. Man erhielt 1.7 g der Verbindung I-a-14.

In Analogie zu der Herstellung der Verbindungen Nr. I-a-2 und Nr. I-a-14 und gemäß den allgemeinen Angaben zur Herstellung erhält man folgende Verbindungen der Formel (I-a):

**Tabelle 229: Erfindungsgemäße Verbindungen der allgemeinen Formel (I), worin G für Wasserstoff steht:**

| | | | | | | | |
|---|---|---|---|---|---|---|---|
| 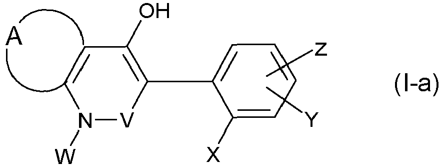 | | | | | | | |

| Nr. | A | V | W | X | Y | Z | Analytische Daten |
|---|---|---|---|---|---|---|---|
| I-a-1 | 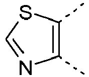 | SO₂ | CH₂CHF₂ | Cl | 4-Cl | H | ¹H-NMR (400 MHz, CDCl₃): 9.06 (s, 1H); 7.57 (d, 1H); 7.39 (d, 1H); 7.36 (dd, 1H); 6.14 (tt, 1H); 6.00 (s, 1H); 4.53 (m, 2H) |
| I-a-2 | 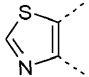 | C=O | CH₂-CH=CH₂ | Me | 4-Me | 6-Me | ¹H-NMR (400 MHz, d6-DMSO): 10.6 (bs, 1H), 9.31 (s, 1H), 6.90 (s, 2H), 5.96 (m, 1H), 5.07 (m, 1H), 4.90 (m, 3H), 2,27 (s, 3H), 1.97 (s, 6H) |
| I-a-3 | 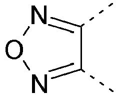 | SO₂ | CH₂CHF₂ | Cl | 6-CI | H | ¹H-NMR (400 MHz, CDCl₃): 7.48 (m, 2H); 7.38 (dd, 1H); 7.19 (s, 1H); 6.12 (tt, 1H); 3.94 (m, 2H) |
| I-a-4 | 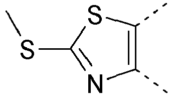 | C=O | CH₂CHF₂ | Br | 4-CI | 6-Et | ¹H-NMR (400 MHz, d6-DMSO): 11.34 (bs, 1H), 7.63 (d, 1H), 7.38 (d, 1H), 6.32 (m, 1H), 4.67 (m, 2H), 2.81 (s, 3H), 2,38 (m, 2H), 1.00 (t, 3H) |
| I-a-5 | 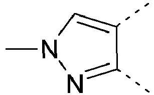 | SO₂ | CH₂CHF₂ | CF₃ | H | H | ¹H-NMR (400 MHz, CDCl₃): 7.95 (s, 1H); 7.80 (d, 1H); 7.60 (m, 2H); 7.26 (s, 1H); 6.16 (tt, 1H); 5.61 (s, 1H); 4.27 (m, 2H); 3.93 (s, 3H) |
| I-a-6 | 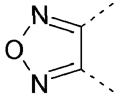 | SO₂ | CH₂CHF₂ | CF₃ | H | H | ¹H-NMR (400 MHz, CDCl₃): 7.84 (d, 1H); 7.66 (m, 3H); 7.24 (s, 1H); 6.11 (tt, 1H); 3.93 (m, 2H) |
| I-a-7 | 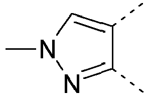 | SO₂ | CH₂CHF₂ | Cl | 4-Cl | H | ¹H-NMR (400 MHz, CDCl₃): 7.96 (s, 1H); 7.55 (d, 1H); 7.33 (m, 2H); 6.15 (tt, 1H); 5.84 (s, 1H); 4.25 (m, 2H); 3.93 (s, 3H) |
| I-a-8 | 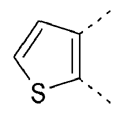 | C=O | CH₃ | Cl | 6-Cl | H | ¹H-NMR (400 MHz, d6-DMSO): 10.8 (bs, 1H), 7.53 (m, 3H), 7.38 (t, 1H), 7.28 (d, 1H), 3.53 (s, 3H) |
| I-a-9 | 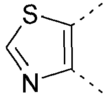 | SO₂ | CH₂CHF₂ | Cl | 6-CI | H | ¹H-NMR (400 MHz, CDCl₃): 9.05 (s, 1H); 7.50 (d, 1H); 7.43 (d, 1H); 7.37 (t, 1H); 6.24 (s, 1H); 6.20 (tt, 1H); 4.49 (m, 2H) |
| I-a-10 | 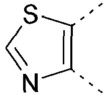 | SO₂ | CH₂CF₃ | CF₃ | H | H | ¹H-NMR (400 MHz, CDCl₃): 9.07 (s, 1H); 7.83 (d, 1H); 7.65 (m, 3H); 5.79 (s, 1H); 4.83 (m, 2H) |
| I-a-11 | 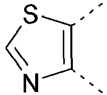 | SO₂ | CH₂CHF₂ | CF₃ | H | H | ¹H-NMR (400 MHz, CDCl₃): 9.06 (s, 1H); 7.83 (d, 1H); 7.64 (m, 3H); 6.15 (tt, 1H); 5.75 (s, 1H); 4.52 (m, 2H) |
| I-a-12 | 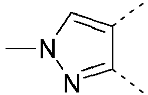 | SO₂ | CH₂CHF₂ | I | H | H | ¹H-NMR (400 MHz, CDCl₃): 7.97 (m, 2H); 7.39 (m, 2H); 7.14 (m, 1H); 6.15 (tt, 1H); 5.88 (s, 1H); 4.25 (m, 2H); 3.92 (s, 3H) |
| I-a-13 | 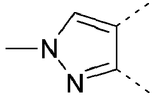 | SO₂ | CH₂CF₃ | I | H | H | ¹H-NMR (400 MHz, CDCl₃): 7.98 (m, 2H); 7.39 (m, 2H); 7.13 (m, 1H); 5.92 (s, 1H); 4.54 (q, 2H); 3.94 (s, 3H) |
| I-a-14 | 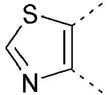 | SO₂ | CH₂CHF₂ | I | H | H | ¹H-NMR (400 MHz, CDCl₃): 9.06 (s, 1H); 7.99 (d, 1 H); 7.42 (m, 2H); 7.18 (m, 1H); 6.14 (tt, 1H); 6.04 (s, 1H); 4.53 (m, 2H) |
| I-a-15 | 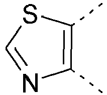 | SO₂ | CH₂CF₃ | I | H | H | ¹H-NMR (400 MHz, CDCl₃): 9.06 (s, 1H); 7.99 (d, 1H); 7.43 (m, 2H); 7.18 (m, 1H); 6.09 (s, 1H); 4.82 (m, 2H) |
| I-a-16 | 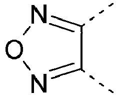 | C=O | CH₃ | Cl | 6-CI | H | ¹H-NMR (400 MHz, CDCl₃): 7.48 (m, 2H), 7.38 (t, 1H), 3.58 (s, 3H) |
| I-a-17 | 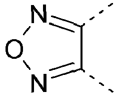 | SO₂ | CH₂CHF₂ | Cl | 4-CI | H | ¹H-NMR (400 MHz, CDCl₃): 12.00 (s, 1H); 7.80 (d, 1H); 7.55 (dd, 1H); 7.51 (d, 1H); 6.25 (tt, 1H); 3.88 (m, 2H) |
| I-a-18 | 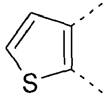 | C=O | CH₂-CH=CH₂ | Cl | 6-CI | H | ¹H-NMR (400 MHz, CDCl₃): 7.48 (m, 2H), 7.34 (m, 2H), 6.98 (d, 1H), 5.93 (m, 1H), 5.27 (m, 2H), 4.78 (m, 2H), |
| I-a-19 | 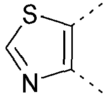 | C=O | CH₃ | Cl | 6-CI | H | ¹H-NMR (400 MHz, d6-DMSO): 11.3 (bs, 1H), 9.42 (s, 1H), 7.53 (m, 2H), 7.38 (t, 1H), 3.72 (s, 3H) |
| I-a-20 | 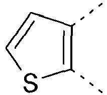 | C=O | CH₂-CH=CH₂ | Me | 4-Me | 6-Me | ¹H-NMR (400 MHz, d6-DMSO): 10.1 (bs, 1H), 7.45 (d, 1H), 7.24 (d, 1H), 6.88 (s, 2H), 5.89 (m, 1H), 5.19 (m, 1H), 5.05 (m, 1H), 4.68 (m, 2H), 2,26 (s, 3H), 1.96 (s, 6H) |
| I-a-21 | 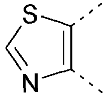 | C=O | CH₂CF₃ | Br | 4-CI | 6-Et | ¹H-NMR (400 MHz, d6-DMSO): 11.7 (bs, 1H), 9.38 (s, 1H), 7.63 (d, 1H), 7.38 (d, 1H), 5.12 (m, 2H), 2.39 (m, 2H), 1.02 (dt, 3H) |
| I-a-22 | 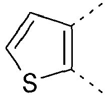 | C=O | CH₂-CH=CH₂ | Cl | 4-CI | H | ¹H-NMR (400 MHz, d6-DMSO): 10.7 (bs, 1H), 7.65 (m, 1H), 7.45 (m, 1H), 7.42 (m, 1H), 7.32 (d, 1H), 7.28 (d, 1H), 5.87 (m, 1H), 5.19 (m, 2H), 4.68 (m, 2H) |
| I-a-23 | 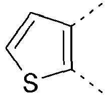 | C=O | CH₂-CH=CH₂ | Br | 4-CI | 6-Et | ¹H-NMR (400 MHz, d6-DMSO): 10.6 (bs, 1H), 7.62 (m, 1H), 7.47 (m, 1H), 7.38 (m, 1H), 7.27 (d, 1H), 5.87 (m, 1H), 5.19 (m, 1H), 5.05 (m, 1H), 4.68 (m, 2H), 2.39 (m, 2H), 1.01 (t, 3H) |
| I-a-24 | 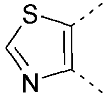 | C=O | CH₂-CH=CH₂ | Br | 4-CI | 6-Et | ¹H-NMR (400 MHz, d6-DMSO): 11.2 (bs, 1H), 9.36 (s, 1H), 7.64 (d, 1H), 7.40 (d, 1H), 5.95 (m, 1H), 5.08 (m, 1H), 4.94 (m, 3H), 2.38 (q, 2H), 1.01 (t, 3H) |
| I-a-25 | 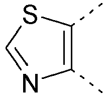 | C=O | CH₂-CH=CH₂ | Cl | 6-CI | H | ¹H-NMR (400 MHz, d6-DMSO): 11.4 (bs, 1H), 9.38 (s, 1H), 7.52 (m, 2H), 7.40 (m, 1H), 5.95 (m, 1H), 5.09 (m, 1H), 4.95 (m, 3H) |
| I-a-26 | 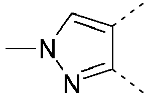 | SO₂ | CH₂CHF₂ | Cl | 6-CI | H | ¹H-NMR (400 MHz, CDCl₃): 7.99 (s, 1H); 7.48 (dd, 1H); 7.40 (dd, 1H); 7.33 (t, 1H); 6.22 (tt, 1H); 6.09 (s, 1H); 4.25 (m, 2H); 3.92 (s, 3H) |
| I-a-27 | 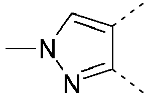 | C=O | CH₂-CH=CH₂ | Cl | 4-CI | H | ¹H-NMR (400 MHz, d6-DMSO): 8.18 (s, 1H), 7.62 (d, 1H), 7.38 (dd, 1H), 7.27 (d, 2H), 5.89 (m, 1H), 5.07 (m, 2H), 4.58 (m, 2H), 3.94 (s, 3H) |
| I-a-28 | 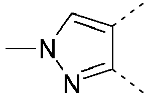 | C=O | CH₂-CH=CH₂ | Br | 4-CI | Et | ¹H-NMR (400 MHz, d6-DMSO): 8.17 (s, 1H), 7.58 (d, 1H), 7.34 (d, 1H), 5.89 (m, 1H), 5.05 (m, 1H), 4.95 (m, 1H), 4.58 (m, 2H), 3.94 (s, 3H), 2.38 (m, 2H), 0.99 (t, 3H) |
| I-a-29 | 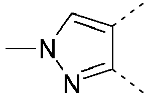 | C=O | CH₂-CH=CH₂ | Me | 4-Me | 6-Me | ¹H-NMR (400 MHz, d6-DMSO): 10.4 (bs, 1H), 8.13 (s, 1H), 6.84 (d, 2H), 5.89 (m, 1H), 5.05 (m, 1H), 4.94 (m, 1H), 4.60 (m, 2H), 3.93 (s, 3H) |
| I-a-30 | 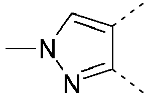 | C=O | CH₂-CH=CH₂ | Cl | 6-CI | H | ¹H-NMR (400 MHz, d6-DMSO): 11.1 (bs, 1H), 8.18 (s, 1H), 7.48 (dd, 2H), 7.34 (dd, 1H), 5.90 (m, 1H), 5.07 (m, 1H), 4.99 (m, 1H), 4.59 (m, 2H), 3.95 (s, 3H) |
| I-a-31 | 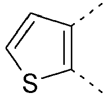 | C=O | CH=CH-OCH₃ | Cl | 6-CI | H | ¹H-NMR (400 MHz, CDCl₃): 7.44 (m, 2H), 7.28 (m, 2H), 6.93 (d, 1H), 6.27 (d, 1H), 5.84 (d, 1 H), 3.71 (s, 3H) |
| I-a-32 | 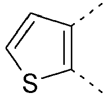 | C=O | CH=CH-F (Z-Isomer) | Cl | 6-CI | H | ¹H-NMR (400 MHz, CDCl₃): 7.85 (dd,1H), 7.47 (m, 2H), 7.33 (m, 2H), 6.93 (d, 1H), 6.27 (d, 1H) |
| I-a-33 | 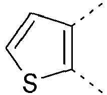 | C=O | CH=CH-F (E-Isomer) | Cl | 6-CI | H | ¹H-NMR (400 MHz, CDCl₃): 7.42 (m, 2H), 7.32 (m, 2H), 6.95 (d, 1H), 6.80 (dd, 1H), 6.32 (dd, 1H), |
| I-a-34 | 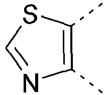 | C=O | CH₂CHF₂ | Cl | 4-CI | H | ¹H-NMR (400 MHz, d6-DMSO): 11.5 (bs, 1H), 9.41 (s, 1H), 7.67 (d, 1H), 7.46 (dd, 1H), 7.33 (d, 1H), 6.34 (m, 1H), 4.72 (m, 2H) |
| I-a-35 | 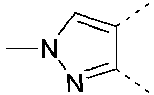 | C=O | CH₂CHF₂ | Cl | 6-Cl | H | ¹H-NMR (400 MHz, d6-DMSO): 11.3 (bs, 1H), 8.22 (s, 1H), 7.49 (dd, 2H), 7.36 (dd, 1H), 6.31 (m, 1H), 4.42 (m, 2H), 3.97 (s, 3H) |
| I-a-36 | 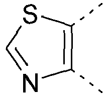 | C=O | CH₂CHF₂ | Br | 4-CI | 6-Et | ¹H-NMR (400 MHz, d6-DMSO): 11.5 (bs, 1H), 9.41 (s, 1H), 7.64 (d, 1H), 7.40 (d, 1H), 6.33 (m, 1H), 4.75 (m, 2H), 2.39 (m, 2H), 1.03 (t, 3H) |
| I-a-37 | 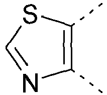 | C=O | CH₂CHF₂ | Me | 4-Me | 6-Me | ¹H-NMR (400 MHz, d6-DMSO): 10.8 (bs, 1H), 9.36 (s, 1H), 6.90 (s, 2H), 6.35 (m, 1H), 4.75 (m, 2H), 2.18 (s, 3H), 1.97 (s, 6H) |
| I-a-38 | 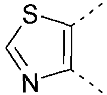 | C=O | CH₂CHF₂ | Cl | 6-Cl | H | ¹H-NMR (400 MHz, d6-DMSO): 11.7 (bs, 1H), 9.42 (s, 1H), 7.53 (d, 2H) 7.41 (t, 1H), 6.34 (m, 1H), 4.75 (m, 2H) |
| I-a-39 | 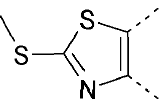 | C=O | CH₂CHF₂ | Cl | 6-CI | H | ¹H-NMR (400 MHz, d6-DMSO): 11.8 (bs, 1H), 7.51 (d, 2H) 7.41 (t, 1H), 6.33 (m, 1H), 4.67 (m, 2H), 2.81 (s, 3H) |
| I-a-40 | 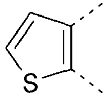 | C=O | CH₂CHF₂ | Cl | 6-CI | H | ¹H-NMR (400 MHz, CDCl₃): 7.49 (d, 2H) 7.33 (m, 2H), 7.00 (d, 1H), 6.23 (m, 1H), 4.44 (m, 2H) |
| I-a-41 | 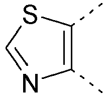 | C=O | CH₂CF₃ | Me | 4-Me | 6-Me | ¹H-NMR (400 MHz, d6-DMSO): 11.0 (bs, 1H), 9.37 (s, 1H), 6.91 (s, 2H), 5.13 (m, 2H), 2.27 (s, 3H), 1.98 (s, 6H) |
| I-a-42 | 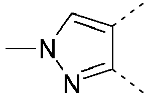 | C=O | CH₂CHF₂ | Me | 4-Me | 6-Me | ¹H-NMR (400 MHz, d6-DMSO): 10.7 (bs, 1H), 8.18 (s, 1H), 6.85 (s, 2H), 6.31 (m, 1H), 4.41 (m, 2H), 3.95 (s, 3H) |
| I-a-43 | 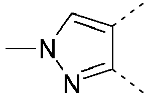 | C=O | CH₂CHF₂ | Br | 4-CI | 6-Et | ¹H-NMR (400 MHz, d6-DMSO): 11.1 (bs, 1H), 8.19 (s, 1H), 7.60 (d, 1H), 7.35 (d, 1H), 6.30 (m, 1H), 4.42 (m, 2H), 3.97 (s, 3H) |
| I-a-44 | 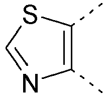 | C=O | CH₂C≡CH | Cl | 6-CI | H | ¹H-NMR (400 MHz, d6-DMSO): 11.6 (bs, 1H), 9.42 (s, 1H), 7.50 (d, 2H) 7.40 (t, 1H), 5.04 (d, 2H), 3.15 (t, 1H) |
| I-a-45 | 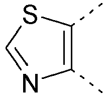 | C=O | CH₂CF₃ | Cl | 6-CI | H | ¹H-NMR (400 MHz, d6-DMSO): 11.8 (bs, 1H), 9.43 (s, 1H), 7.54 (d, 2H) 7.42 (t, 1H), 5.13 (m, 2H) |
| I-a-46 | 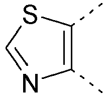 | C=O | CH₂C≡CH | Br | 4-CI | 6-Et | ¹H-NMR (400 MHz, d6-DMSO): 11.7 (bs, 1H), 9.42 (s, 1H), 7.64 (d, 1H), 7.40 (d, 1H), 5.04 (d, 2H), 3.13 (t, 1H), 2.39 (m, 2H), 1.01 (dt, 3H) |
| I-a-47 | 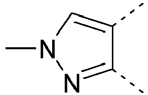 | C=O | CH₂CF₃ | Br | 4-CI | 6-Et | ¹H-NMR (400 MHz, d6-DMSO): 11.2 (bs, 1H), 8.20 (s, 1H), 7.60 (d, 1H), 7.36 (d, 1H), 4.80 (m, 2H), 3.96 (s, 3H), 2.38 (m, 2H), 0.99 (t, 3H) |
| I-a-48 | 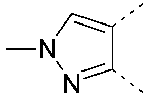 | C=O | CH₂CF₃ | Cl | 6-CI | H | ¹H-NMR (400 MHz, d6-DMSO): 11.4 (bs, 1H), 8.22 (s, 1H), 7.49 (d, 2H), 7.36 (t, 1H), 4.80 (m, 2H), 3.97 (s, 3H) |
| I-a-49 | 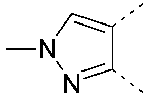 | C=O | CH₂CF₃ | Me | 4-Me | 6-Me | ¹H-NMR (400 MHz, d6-DMSO): 10.7 (bs, 1H), 8.16 (s, 1H), 6.86 (d, 2H), 4.80 (m, 2H), 3.95 (s, 3H), 2.18 (s, 3H), 1.95 (s, 6H) |
| I-a-50 | 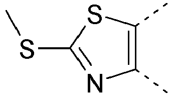 | C=O | CH₂C≡CH | Cl | 6-Cl | H | ¹H-NMR (400 MHz, d6-DMSO): 11.5 (bs, 1H), 7.51 (d, 2H), 7.39 (t, 1H), 4.97 (d, 2H), 3.16 (t, 1H), 2.85 (s, 3H) |
| I-a-51 | 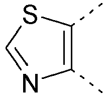 | C=O | CH₂CH₃ | Cl | 6-CI | H | ¹H-NMR (400 MHz, d6-DMSO): 11.3 (bs, 1H), 9.41 (s, 1H), 7.52 (d, 2H) 7.40 (t, 1H), 4.35 (m, 2H), 1.24 (m, 3H) |
| I-a-52 | 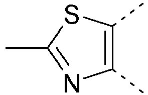 | C=O | CH₂C≡CH | Cl | 6-Cl | H | ¹H-NMR (400 MHz, d6-DMSO): 11.5 (bs, 1H), 7.52 (d, 2H), 7.40 (t, 1H), 4.98 (d, 2H), 3.14 (t, 1H), 2.83 (s, 3H) |
| I-a-53 | 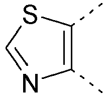 | C=O | CH₂-c-Pr | Cl | 6-Cl | H | ¹H-NMR (400 MHz, d6-CDCl₃): 8.91 (s, 1H), 7.41 (d, 2H) 7.23 (t, 1H), 4.35 (d, 2H), 1.43 (m, 1H), 0.47 (m, 4H) |
| I-a-54 | 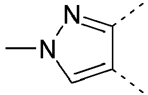 | C=O | CH₂CHF₂ | Cl | 6-Cl | H | ¹H-NMR (400 MHz, d6-DMSO): 11.6 (bs, 1H), 8.08 (s, 1H), 7.49 (d, 2H), 7.36 (t, 1H), 6.25 (m, 1H), 4.35 (m, 2H), 4.05 (s, 3H) |
| I-a-55 | 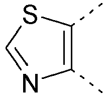 | C=O | CH₂C≡CH | Me | 3-Br | 6-Me | ¹H-NMR (400 MHz, d6-DMSO): 11.1 (bs, 1H), 9.40 (s, 1H), 7.48 (d, 1H), 7.06 (d, 1H), 5.04 (d, 2H), 3.12 (t, 1H), 2.22 (s, 3H), 1.98 (s, 3H) |
| I-a-56 | 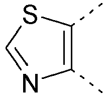 | C=O | CH₂CHF₂ | Cl | 4-Cl | 6-Et | ¹H-NMR (400 MHz, d6-DMSO): 11.6 (bs, 1H), 9.39 (s, 1H). 7.49 (d, 1H), 7.35 (d, 1H), 6.33 (m, 1H), 4.74 (m, 2H), 2.39 (m, 2H), 1.03 (m, 3H) |
| I-a-57 | 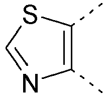 | C=O | CH₂CHF₂ | Me | 3-Br | Me | ¹H-NMR (400 MHz, d6-DMSO): 11.2 (bs, 1H), 9.39 (s, 1H). 7.48 (d, 1H), 7.06 (d, 1H), 6.35 (m, 1H), 4.74 (m, 2H), 2.10 (s, 3H), 1.98 (s, 3H) |
| I-a-58 | 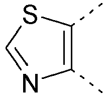 | C=O | CH₂CHF₂ | Cl | 4-Cl | 6-Me | ¹H-NMR (400 MHz, d6-DMSO): 11.6 (bs, 1H), 9.40 (s, 1H). 7.49 (d, 1H), 7.38 (d, 1H), 6.34 (m, 1H), 4.74 (m, 2H), 2.08 (s, 3H) |
| I-a-59 | 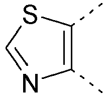 | C=O | CH₂C≡CH | Cl | 4-Cl | 6-Me | ¹H-NMR (400 MHz, d6-DMSO): 11.4 (bs, 1H), 9.42 (s, 1H). 7.49 (d, 1H), 7.38 (d, 1H), 5.04 (d, 2H), 3.14 (t, 1H), 2.08 (s, 3H) |
| I-a-60 | 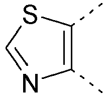 | C=O | CH₂CHF₂ | Et | 4-Cl | 6-OMe | ¹H-NMR (400 MHz, d6-DMSO): 10.9 (bs, 1H), 9.36 (s, 1H). 6.95 (d, 2H), 6.32 (m, 1H), 4.72 (m, 2H),3.66 (s, 3H), 2.31 (m, 2H), 0.86 (m, 3H) |
| I-a-61 | 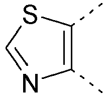 | C=O | CH₂CHF₂ | Cl | 4-Br | 6-Et | ¹H-NMR (400 MHz, d6-DMSO): 11.4 (bs, 1H), 9.41 (s, 1H). 7.62 (d, 1H), 7.50 (d, 1H), 6.33 (m, 1H), 4.74 (m, 2H), 2.38 (m, 2H), 1.01 (m, 3H) |
| I-a-62 | 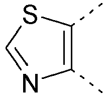 | C=O | CH₂CHF₂ | Me | 4-Cl | 6-CF₃ | ¹H-NMR (400 MHz, d6-DMSO): 11.4 (bs, 1H), 9.40 (s, 1H). 7.75 (d, 1H), 7.67 (d, 1H), 6.31 (m, 1H), 4.73 (m, 2H), 2.08 (s, 3H) |
| I-a-63 | 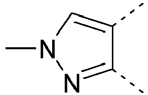 | C=O | CH₂CHF₂ | Cl | 4-Cl | 6-Et | ¹H-NMR (400 MHz, CDCl₃): 7.75 (s, 1H). 7.35 (d, 1H), 7.23 (d, 1H), 6.25 (m, 1H), 4.53 (m, 2H), 4.01 (s, 3H), 2.43 (m, 2H), 1.08 (m, 3H) |
| I-a-64 | 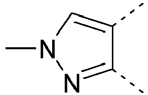 | C=O | CH₂CF₃ | Cl | 4-Cl | 6-Et | ¹H-NMR (400 MHz, CDCl₃): 7.78 (s, 1H). 7.31 (d, 1H), 7.20 (d, 1H), 4.82 (m, 2H), 3.98 (s, 3H), 2.40 (m, 2H), 1.06 (m, 3H) |
| I-a-65 | 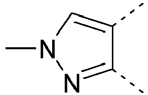 | C=O | CH₂CF₃ | Cl | 4-Cl | 6-Me | ¹H-NMR (400 MHz, d6-DMSO): 11.3 (bs, 1H), 8.22 (s, 1H). 7.46 (d, 1H), 7.36 (d, 1 H), 4.80 (m, 2H), 3.97 (s, 3H), 2.06 (s, 3H) |
| I-a-66 | 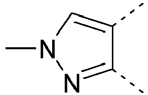 | C=O | CH₂CHF₂ | Cl | 4-Cl | 6-Me | ¹H-NMR (400 MHz, d6-DMSO): 11.2 (bs, 1H), 8.21 (s, 1H). 7.45 (d, 1H), 7.35 (d, 1H), 6.32 (m, 1H), 4.43 (m, 2H), 3.97 (s, 3H), 2.06 (s, 3H) |
| I-a-67 | 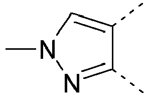 | C=O | CH₃ | Cl | 4-Cl | 6-Me | ¹H-NMR (400 MHz, d6-DMSO): 10.9 (bs, 1H), 8.17 (s, 1H). 7.44 (d, 1H), 7.34 (d, 1H), 3.97 (s, 3H), 3.41 (s, 3H), 2.06 (s, 3H) |
| I-a-68 | 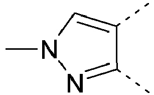 | C=O | CH₂CF₃ | Cl | 3-Br | 6-Cl | ¹H-NMR (400 MHz, d6-DMSO): 11.6 (bs, 1H), 8.23 (s, 1H). 7.78 (d, 1H), 7.48 (d, 1H), 4.80 (m, 2H), 3.97 (s, 3H) |
| I-a-69 | 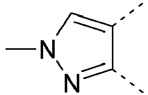 | C=O | CH₃ | Me | 4-CF₂-CF₃ | 6-Me | ¹H-NMR (400 MHz, d6-DMSO): 10.8 (bs, 1H), 8.18 (s, 1H). 7.37 (s, 2H), 3.97 (s, 3H), 3.42 (s, 3H), 2.09 (s, 6H) |
| I-a-70 | 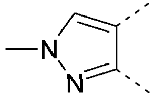 | C=O | CH₃ | Cl | 3-Br | 6-Cl | ¹H-NMR (400 MHz, d6-DMSO): 11.1 (bs, 1H), 8.18 (s, 1H). 7.75 (d, 1H), 7.45 (d, 1H), 3.97 (s, 3H), 3.41 (s, 3H) |
| I-a-71 | 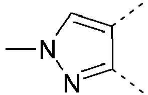 | C=O | CH₂CF₃ | Me | 4-CF₂-CF₃ | 6-Me | ¹H-NMR (400 MHz, d6-DMSO): 11.3 (bs, 1H), 8.22 (s, 1H). 7.38 (s, 2H), 4.82 (m, 2H), 3.97 (s, 3H), 2.09 (s, 6H) |
| I-a-72 | 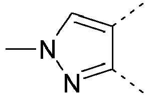 | C=O | CH₂CHF₂ | Me | 4-CF₂-CF₃ | 6-Me | ¹H-NMR (400 MHz, d6-DMSO): 11.1 (bs, 1H), 8.22 (s, 1H). 7.37 (s, 2H), 6.33(m, 1H), 4.42 (m, 2H), 3.98 (s, 3H), 2.09 (s, 6H) |
| I-a-73 | 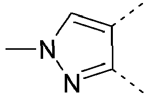 | C=O | CH₂CHF₂ | Cl | 3-Br | 6-Cl | ¹H-NMR (400 MHz, d6-DMSO): 11.4 (bs, 1H), 8.22 (s, 1H). 7.77 (d, 1H), 7.46 (d, 1H), 6.31 (m, 1H), 4.41 (m, 2H), 3.97 (s, 3H) |
| I-a-74 | 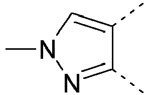 | C=O | CH₃ | Cl | 4-Cl | 6-Et | ¹H-NMR (400 MHz, d6-DMSO): 10.9 (bs, 1H), 8.16 (s, 1H). 7.45 (d, 1H), 7.32 (d, 1H), 3.97 (s, 3H), 3.41 (s, 3H), 2.35 (m, 2H), 1.00 (m, 3H) |
| I-a-75 | 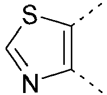 | C=O | CH₂CHF₂ | Cl | 3-Br | 6-Cl | ¹H-NMR (400 MHz, d6-DMSO): 11.8 (bs, 1H), 9.41 (s, 1H). 7.82 (d, 1H), 7.53 (d, 1H), 6.33 (m, 1H), 4.72 (m, 2H) |
| I-a-76 | 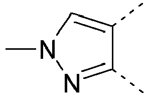 | C=O | CH₃ | F | 3-Me | 6-Cl | ¹H-NMR (400 MHz, d6-DMSO): 11.1 (bs, 1H), 8.17 (s, 1H). 7.25 (m, 2H), 3.97 (s, 3H), 3.40 (s, 3H), 2.22 (s, 3H) |
| I-a-77 | 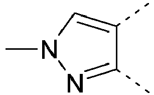 | C=O | CH₂CHF₂ | F | 3-Me | 6-Cl | ¹H-NMR (400 MHz, d6-DMSO): 11.5 (bs, 1H), 8.19 (s, 1H). 7.24 (m, 2H), 6.33 (m, 1H), 4.39 (m, 2H), 3.97 (s, 3H), 2.22 (s, 3H) |
| I-a-78 | 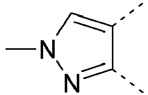 | C=O | CH₂CF₃ | F | 3-Me | 6-Cl | ¹H-NMR (400 MHz, d6-DMSO): 8.22 (s, 1H). 7.26 (m, 2H), 6.33 (m, 1H), 4.79 (m, 2H), 3.97 (s, 3H), 2.22 (s, 3H) |
| I-a-79 | 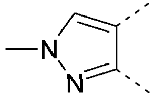 | C=O | CH₂C≡CH | Me | 4-CF₂-CF₃ | 6-Me | ¹H-NMR (400 MHz, CDCl₃): 7.77 (s, 1H). 7.34 (s, 2H), 4.94 (d, 2H), 4.02 (s, 3H), 2.18 (t, 1H), 2.16 (s, 6H) |
| I-a-80 | 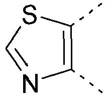 | C=O | CH₂CHF₂ | CF₃ | H | H | ¹H-NMR (400 MHz, d6-DMSO): 9.42 (s, 1H). 7.76 (d, 1H), 7.69 (t, 1H), 7.57 (t, 1H), 7.32 (d, 1H), 6.30 (m, 1H), 4.71 (m, 2H) |
| I-a-81 | 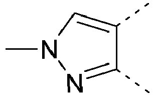 | C=O | CH₂C≡CH | F | 3-Me | 6-Cl | ¹H-NMR (400 MHz, d6-DMSO): 11.2 (bs, 1H), 8.20 (s, 1H). 7.25 (m, 2H), 4.73 (m, 2H), 3.99 (s, 3H), 3.10 (t, 1H), 2.22 (s, 3H) |
| I-a-82 | 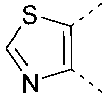 | C=O | CH₃ | I | H | H | ¹H-NMR (400 MHz, d6-DMSO): 11.0 (bs, 1H), 9.37 (s, 1H). 7.91 (d, 1H), 7.42 (t, 1H), 7.21 (d, 1H), 7.09 (t, 1H), 3.69 (s, 3H) |
| I-a-83 | 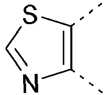 | C=O | CH₂CHF₂ | Cl | 3-c-Pr | 6-Cl | ¹H-NMR (400 MHz, d6-DMSO): 11.6 (bs, 1H), 9.38 (s, 1H). 7.40 (d, 1H), 7.05 (d, 1H), 6.33 (m, 1H), 4.73 (m, 2H), 2.13 (m, 1H), 0.74 (m, 2H), 0.65 (m, 2H) |
| I-a-84 | 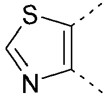 | C=O | CH₂CHF₂ | I | H | H | ¹H-NMR (400 MHz, d6-DMSO): 11.3 (bs, 1 H), 9.40 (s, 1 H). 7.92 (d, 1 H), 7.43 (t, 1 H), 7.24 (d, 1 H), 7.10 (t, 1 H), 6.33 (m, 1 H), 4.74 (m, 2H), |
| I-a-85 | 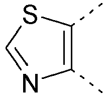 | C=O | CH₃ | CF₃ | H | H | ¹H-NMR (400 MHz, d6-DMSO): 11.0 (bs, 1H), 9.37 (s, 1H). 7.78 (d, 1H), 7.68 (t, 1H), 7.58 (t, 1H), 7.30 (d, 1H), 3.68 (s, 3H) |
| I-a-86 | 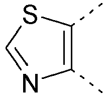 | C=O | CH₂CF₃ | Cl | 3-Me | 6-Cl | ¹H-NMR (400 MHz, d6-DMSO): 11.7 (bs, 1H), 9.42 (s, 1H). 7.40 (m, 2H), 5.12 (m, 2H), 2.37 (s, 3H) |
| I-a-87 | 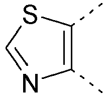 | C=O | CH₂CHF₂ | Br | 4-Br | 6-OCF₃ | ¹H-NMR (400 MHz, d6-DMSO): 11.8 (bs, 1H), 9.44 (s, 1H). 8.06 (d, 1H), 7.73 (d, 1H), 6.31 (m, 1H), 4.74 (m, 2H) |
| I-a-88 | 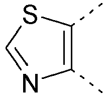 | C=O | CH₂CHF₂ | Cl | 3-I | 6-Cl | ¹H-NMR (400 MHz, d6-DMSO): 11.7 (bs, 1H), 9.42 (s, 1H). 7.98 (d, 1H), 7.31 (d, 1H), 6.33 (m, 1H), 4.73 (m, 2H) |
| I-a-89 | 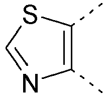 | C=O | CH₂CHF₂ | Cl | 3-Me | 6-Cl | ¹H-NMR (400 MHz, d6-DMSO): 9.41 (s, 1H). 7.40 (m, 2H), 6.33 (m, 1H), 4.73 (m, 2H), 2.33 (s, 3H) |
| I-a-90 | 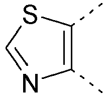 | C=O | CH₃ | Cl | 3-Me | 6-Cl | ¹H-NMR (400 MHz, d6-DMSO): 11.2 (bs, 1H), 9.39 (s, 1H). 7.40 (m, 2H), 3.70 (s, 3H), 2.36 (s, 3H) |
| I-a-91 | 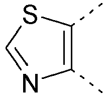 | C=O | CH₂CF₃ | Me | 3-Br | 6-Cl | ¹H-NMR (400 MHz, d6-DMSO): 9.43 (s, 1H). 7.65 (d, 1H), 7.34 (d, 1H), 5.13 (m, 2H), 2.16 (s, 3H) |
| I-a-92 | 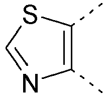 | C=O | CH₃ | Cl | 3-I | 6-Cl | ¹H-NMR (400 MHz, d6-DMSO): 11.5 (bs, 1H), 9.41 (s, 1H). 7.96 8d, 1H), 7.31 (d, 1H), 3.70 (s, 3H) |
| I-a-93 | 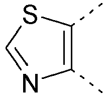 | C=O | CH₃ | Me | 3-Br | 6-Cl | ¹H-NMR (400 MHz, d6-DMSO): 9.38 (s, 1H). 7.63 (d, 1H), 7.31 (d, 1H), 3.70 (s, 3H), 2.15 (s, 3H) |
| I-a-94 | 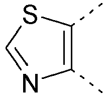 | C=O | CH₂CHF₂ | Me | 3-Br | 6-Cl | ¹H-NMR (400 MHz, d6-DMSO): 11.5 (bs, 1H), 9.41 (s, 1H). 7.63 (d, 1H), 7.32 (d, 1H), 6.34 (m, 1H), 4.74 (m, 2H), 2.16 (s, 3H) |
| I-a-95 | 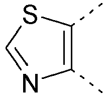 | C=O | CH₂CF₃ | Cl | 3-I | 6-Cl | ¹H-NMR (400 MHz, CDCl₃): 8.96 (s, 1H). 7.82 (d, 1H), 7.14 (t, 1H), 5.13 (m, 2H) |
| I-a-96 | 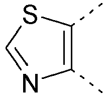 | C=O | CH₂CHF₂ | Me | 4-Br | 6-Et | ¹H-NMR (400 MHz, d6-DMSO): 9.39 (s, 1H). 7.33 (d, 1H), 7.30 (d, 1H), 6.34 (m, 1H), 4.74 (m, 2H), 2.31 (m, 2H), 2.10 (s, 3H), 0.99 (m, 3H) |
| I-a-97 | 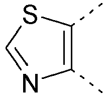 | C=O | CH₂CHF₂ | Me | 4-Br | 6-Me | ¹H-NMR (400 MHz, d6-DMSO): 11.2 (bs, 1H), 9.39 (s, 1H). 7.32 (d, 2H), 6.35 (m, 1H), 4.74 (m, 2H), 2.01 (s, 6H) |
| I-a-98 | 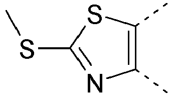 | C=O | CH₃ | Cl | 6-Cl | H | ¹H-NMR (400 MHz, d6-DMSO): 11.3 (bs, 1H), 7.49 (d, 2H), 7.39 (t, 1H), 3.68 (s, 3H), 2.86 (s, 3H) |
| I-a-99 | 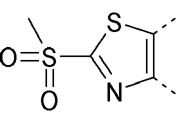 | C=O | CH₂CHF₂ | Cl | 6-Cl | H | ¹H-NMR (400 MHz, CDCl₃): 7.48 (d, 2H), 7.37 (t, 1H), 6.22 (m, 1H), 4.81 (m, 2H); 3.41 (s, 3H) |
| I-a-100 | 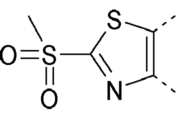 | C=O | CH₃ | Cl | 6-Cl | H | ¹H-NMR (400 MHz, d6-DMSO): 7.36 (d, 2H), 7.19 (t, 1H), 3.50 (s, 3H), 3.36 (s, 3H) |
| I-a-101 | 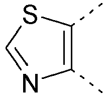 | C=O | CH₃ | Me | 4-O-CH₂-CF₃ | 6-Et | ¹H-NMR (400 MHz, CDCl₃): 8.90 (s, 1H), 6.80 (d, 1H), 6.76 (d, 1H), 4.38 (m, 2H); 3.90 (s, 3H), 2.44 (m, 2H), 1.09 (m, 3H) |
| I-a-102 | 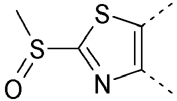 | C=O | CH₂CHF₂ | Cl | 6-Cl | H | ¹H-NMR (400 MHz, d6-DMSO): 12.0 (bs, 1H), 7.54 (d, 2H), 7.43 (t, 1H), 6.36 (m, 1H), 4.70 (m, 2H); 3.12 (s, 3H) |
| I-a-103 | 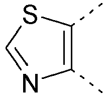 | C=O | CH₃ | Me | 4-Br | 6-Et | ¹H-NMR (400 MHz, d6-DMSO): 10.8 (bs, 1H), 9.36 (s, 1H). 7.32 (d, 1H), 7.29 (d, 1H), 3.70 (s, 3H), 2.31 (m, 2H), 1.98 (s, 3H), 0.98 (t, 3H) |
| I-a-104 | 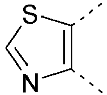 | C=O | CH₂CHF₂ | Me | 4-CF₂-CF₃ | 6-Me | ¹H-NMR (400 MHz, d6-DMSO): 11.3 (bs, 1H), 9.40 (s, 1H). 7.41 (s, 2H), 6.36 (m, 1H), 4.75 (m, 2H); 2.11 (s, 6H) |
| I-a-105 | 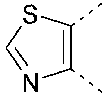 | C=O | CH₂C≡CH | Me | 4-CF₂-CF₃ | 6-Me | ¹H-NMR (400 MHz, d6-DMSO): 11.3 (bs, 1H), 9.42 (s, 1H). 7.41 (s, 2H), 5.05 (d, 2H), 3.14 (t, 1H); 2.11 (s, 6H) |
| I-a-106 | 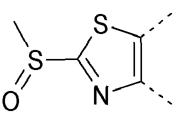 | C=O | CH₃ | Cl | 6-Cl | H | ¹H-NMR (400 MHz, d6-DMSO): 11.7 (bs, 1H), 7.53 (d, 2H), 7.42 (t, 1H), 3.67 (s, 3H), 3.12 (s, 3H) |
| I-a-107 | 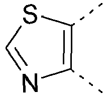 | C=O | CH₃ | Me | 4-O-CH₂-CF₃ | 6-Me | ¹H-NMR (400 MHz, CDCl₃): 8.91 (s, 1H), 6.78 (s, 2H), 4.37 (m, 2H); 3.90 (s, 3H), 2.13 (s, 6H) |
| I-a-108 | 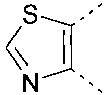 | C=O | CH₃ | Me | 4-CF₂-CF₃ | 6-Me | ¹H-NMR (400 MHz, d6-DMSO): 11.0 (bs, 1H), 9.38 (s, 1H). 7.40 (s, 2H), 3.71 (s, 3H); 2.10 (s, 6H) |
| I-a-109 | 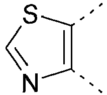 | C=O | CH₂C≡CH | I | 4-Cl | 6-Et | ¹H-NMR (400 MHz, d6-DMSO): 11.3 (bs, 1H), 9.42 (s, 1H). 7.81 (d, 1H), 7.39 (d, 1H), 5.04 (d, 2H), 3.12 (t, 1H); 2.37 (m, 2H), 0.99 (m, 3H) |
| I-a-110 | 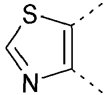 | C=O | CH₂CHF₂ | I | 4-Cl | 6-Et | ¹H-NMR (400 MHz, d6-DMSO): 11.3 (bs, 1H), 9.41 (s, 1H). 7.81 (d, 1H), 7.40 (d, 1H), 6.32 (m, 1H), 4.75 (m, 2H), 2.36 (m, 2H), 0.97 (m, 3H) |
| I-a-111 | 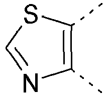 | C=O | CH₃ | I | 4-Cl | 6-Et | ¹H-NMR (400 MHz, d6-DMSO): 11.1 (bs, 1H), 9.38 (s, 1H). 7.80 (d, 1H), 7.39 (d, 1H), 3.70 (s, 3H), 2.36 (m, 2H), 0.99 (m, 3H) |
| I-a-112 | 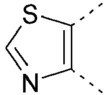 | C=O | CH₂CHF₂ | F | 4-Me | 6-Me | ¹H-NMR (400 MHz, d6-DMSO): 11.3 (bs, 1H), 9.39 (s, 1H). 6.94 (d, 1H), 6.86 (dd, 1H), 6.35 (m, 1H), 4.73 (m, 2H), 2.32 (s, 3H), 2.06 (s, 3H) |
| I-a-113 | 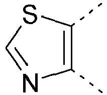 | C=O | CH₂CHF₂ | Br | 4-Br | 6-Me | ¹H-NMR (400 MHz, d6-DMSO): 11.4 (bs, 1H), 9.40 (s, 1H). 7.74 (d, 1H), 7.55 (d, 1H), 6.34 (m, 1H), 4.74 (m, 2H), 2.09 (s, 3H) |
| I-a-114 | 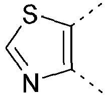 | C=O | CH₃ | F | 4-Me | 6-Me | ¹H-NMR (400 MHz, d6-DMSO): 10.9 (bs, 1H), 9.36 (s, 1H). 6.93 (d, 1H), 6.84 (dd, 1H), 3.69 (s, 3H), 2.32 (s, 3H), 2.04 (s, 3H) |
| I-a-115 | 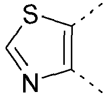 | C=O | CH₂CHF₂ | Br | 4-Br | 6-Et | ¹H-NMR (400 MHz, d6-DMSO): 11.4 (bs, 1H), 9.40 (s, 1H). 7.75 (d, 1H), 7.52 (d, 1H), 6.33 (m, 1H), 4.74 (m, 2H), 2.39 (m, 2H), 0.98 (m, 3H) |
| I-a-116 | 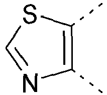 | C=O | CH₂C≡CH | Br | 4-Br | 6-Et | ¹H-NMR (400 MHz, d6-DMSO): 11.3 (bs, 1H), 9.42 (s, 1H). 7.76 (d, 1H), 7.52 (d, 1H), 5.04 (d, 2H), 3.13 (t, 1H), 2.36 (m, 2H), 1.00 (m, 3H) |
| I-a-117 | 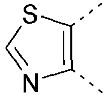 | C=O | CH₂C≡CH | Br | 4-Br | 6-Me | ¹H-NMR (400 MHz, d6-DMSO): 11.3 (bs, 1H), 9.42 (s, 1H). 7.75 (d, 1H), 7.55 (d, 1 H), 5.04 (d, 2H), 3.13 (t, 1H), 2.08 (s, 3H) |
| I-a-118 | 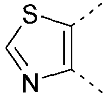 | C=O | CH₃ | Br | 4-Br | 6-Me | ¹H-NMR (400 MHz, d6-DMSO): 11.1 (bs, 1H), 9.38 (s, 1H). 7.73 (d, 1H), 7.54 (d, 1H), 3.70 (s, 3H), 2.08 (s, 3H) |
| I-a-119 | 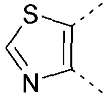 | C=O | CH₃ | Br | 4-Br | 6-Et | ¹H-NMR (400 MHz, d6-DMSO): 11.1 (bs, 1H), 9.38 (s, 1H). 7.74 (d, 1H), 7.51 (d, 1H), 3.70 (s, 3H), 2.37 (m, 2H), 1.00 (t, 3H) |
| I-a-120 | 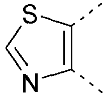 | C=O | CH₂C≡CH | F | 4-Me | 6-Me | ¹H-NMR (400 MHz, d6-DMSO): 11.3 (bs, 1H), 9.41 (s, 1H). 6.94 (d, 1H), 6.86 (dd, 1H), 5.04 (d, 2H), 3.17 (t, 1H), 2.32 (s, 3H), 2.04 (s, 3H) |
| I-a-121 | 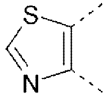 | C=O | CH₂CHF₂ | Me | 4-(2'-c-Pr)-c-Pr | Me | ¹H-NMR (400 MHz, CDCl₃): 8.91 (s, 1H). 6.84 (d, 2H), 6.28 (m, 1H), 4.86 (m, 2H), 2.08 (s, 6H), 1.60 (m, 1H), 1.10 (m, 1H) 0.90 (m, 1H), 0.75 (m, 2H), 0.40 (m, 2H), 0.15 (m, 2H) |
| I-a-122 | 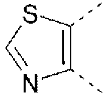 | C=O | CH₃ | Me | 4-(2'-c-Pr)-c-Pr | Me | ¹H-NMR (400 MHz, d6-DMSO): 10.6 (bs, 1H), 9.33 (s, 1H), 6.74 (d, 2H), 3.69 (s, 3H), 1.94 (s, 6H), 1.60 (m, 1H), 1.10 (m, 1H) 0.90 (m, 1H), 0.75 (m, 2H), 0.40 (m, 2H), 0.15 (m, 2H) |
| I-a-123 | 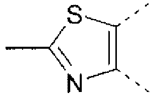 | C=O | CH₃ | Cl | 6-Cl | H | ¹H-NMR (400 MHz, d6-DMSO): 11.2 (bs, 1H), 7.52 (d, 2H), 7.39 (t, 1H), 3.65 (s, 3H), 2.81 (s, 3H) |
| I-a-124 | 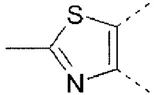 | C=O | CH₂CHF₂ | Cl | 6-Cl | H | ¹H-NMR (400 MHz, d6-DMSO): 11.5 (bs, 1H), 7.52 (d, 2H), 7.40 (t, 1H), 6.33 (m, 1H), 4.69 (m, 2H), 2.82 (s, 3H) |
| I-a-125 | 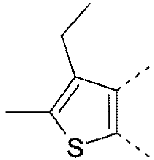 | C=O | CH₂CHF₂ | Cl | 4-Br | 6-Et | ¹H-NMR (400 MHz, CDCl₃): 7.58 (d, 1H); 7.45 (d, 1H); 6.21 (tt, 1H); 5.39 (s, 1H); 4.38 (m, 2H); 2.82 (m, 2H); 2.47 (m, 2H); 2.39 (s, 3H); 1.15 (t, 3H); 1.11 (t, 3H) |
| I-a-126 | 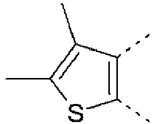 | C=O | CH₂CHF₂ | Cl | 4-Br | 6-Et | ¹H-NMR (400 MHz, CDCl₃): 7.37 (d, 1H); 7.23 (d, 1H); 6.05 (tt, 1H); 4.33 (m, 2H); 4.18 (m, 1H); 3.73 (s, 2H); 3.69 (m, 1H); 2,54 (q, 2H); 2.37 (s, 3H); 2.30 (s, 3H); 1.36 (t, 3H); 1.17 (t, 3H) |
| I-a-128 | 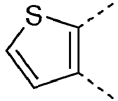 | C=O | CH₂CHF₂ | Cl | 4-BrI | 6-Et | ¹H-NMR (400 MHz, CDCl₃): 7.47 (d, 1H); 7.40 (d, 1H); 7.33 (d, 1H); 6.92 (d, 1H); 6.12 (tt, 1H); 4.15 (m, 2H); 2.55 (m, 2H); 1.04 (t, 3H) |
| I-a-129 | 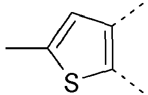 | C=O | CH₂CHF₂ | Br | 4-Cl | 6-Et | ¹H-NMR (400 MHz, CDCl₃): 7.59 (d, 1H); 7.32 (d, 1H); 6.94 (q, 1H); 6.20 (tt, 1H); 5.54 (s, 1H); 4.37 (m, 2H); 2.52 (d, 3H); 2.47 (m, 2H); 1.10 (t, 3H) |
| I-a-130 | 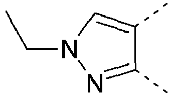 | C=O | CH₂CHF₂ | Br | 4-Cl | 6-Et | ¹H-NMR (400 MHz, d₆-DMSO): 11.11 (s, 1H); 8.24 (s, 1H); 7.60 (d, 1H); 7.36 (d, 1H); 6.31 (tt, 1H); 4.42 (m, 2H); 4.26 (q, 2H); 2.38 (m, 2H); 1.44 (t, 3H); 0.99 (t, 3H) |
| I-a-131 | 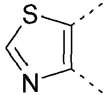 | C=O | CH₃ | Br | 4-Br | 6-OCF₃ | ¹H-NMR (400 MHz, d6-DMSO): 11.5 (bs, 1H), 9.41 (s, 1H). 8.05 (d, 1H), 7.71 (d, 1H), 3.69 (s, 3H) |
| I-a-132 | 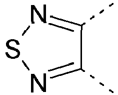 | C=O | CH₂CHF₂ | Cl | 6-Cl | H | ¹H-NMR (400 MHz, d6-DMSO): 12.3 (bs, 1H), 7.59 (m, 2H), 7.45 (m, 1H), 6.38 (m, 1 H), 4.65 (m, 2H) |
| I-a-133 | 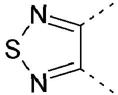 | C=O | CH₃ | Cl | 6-Cl | H | ¹H-NMR (400 MHz, d6-DMSO): 12.3 (bs, 1H), 7.55 (d, 2H), 7.44 (t, 1H), 3.61 (s, 3H) |
| I-a-134 | 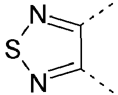 | C=O | CH₃ | Br | 4-Cl | 6-Et | ¹H-NMR (400 MHz, d6-DMSO): 12.0 (bs, 1H), 7.66 (d, 1H), 7.42 (d, 1H), 3.60 (s, 3H), 2.40 (m, 2H), 1.02 (t, 3H) |
| I-a-135 | 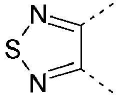 | C=O | CH₂CHF₂ | Br | 4-Cl | 6-Et | ¹H-NMR (400 MHz, CDCl₃): 7.58 (d, 1H), 7.31 (m, 1H), 6.22 (m, 1H), 4.70 (m, 2H), 2.47 (m, 2H), 1.12 (m, 3H) |
| I-a-136 | 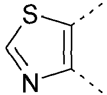 | C=O | CH₂CHF₂ | Cl | 4-Br | 6-OCF₃ | ¹H-NMR (400 MHz, d6-DMSO): 11.9 (bs, 1H), 9.44 (s, 1H). 7.94 (d, 1H), 7.70 (d, 1H), 6.31 (m, 1H), 4.74 (m, 2H) |
| I-a-137 | 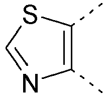 | C=O | CH₃ | Cl | 4-Br | 6-OCF₃ | ¹H-NMR (400 MHz, d6-DMSO): 11.5 (bs, 1H), 9.42 (s, 1H). 7.93 (d, 1H), 7.69 (d, 1H), 3.69 (s, 3H) |
| I-a-138 | 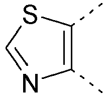 | C=O | CH₂C≡CH | Br | 4-Br | 6-OCF₃ | ¹H-NMR (400 MHz, d6-DMSO): 11.7 (bs, 1H), 9.45 (s, 1H). 8.06 (d, 1H), 7.72 (d, 1H), 5.03 (d, 2H), 3.13 (t, 1H) |
| I-a-139 | 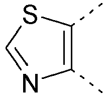 | C=O | CH₂C≡CH | Cl | 4-Br | 6-OCF₃ | ¹H-NMR (400 MHz, d6-DMSO): 11.8 (bs, 1H), 9.45 (s, 1H). 7.93 (d, 1H), 7.69 (d, 1H), 5.03 (d, 2H), 3.14 (t, 1H) |
| I-a-140 | 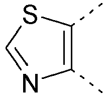 | C=O | CH₂CHF₂ | Br | 4-Cl | 6-OCF₃ | ¹H-NMR (400 MHz, d6-DMSO): 11.9 (bs, 1H), 9.44 (s, 1H). 7.94 (d, 1H), 7.63 (d, 1H), 6.31 (m, 1H), 4.74 (m, 2H) |
| I-a-141 | 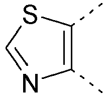 | C=O | CH₃ | Br | 4-Cl | 6-OCF₃ | ¹H-NMR (400 MHz, d6-DMSO): 11.5 (bs, 1H), 9.41 (s, 1H). 7.94 (d, 1H), 7.62 (d, 1H), 3.69 (s, 3H) |
| I-a-142 | 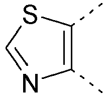 | C=O | CH₂CHF₂ | CF₃ | 6-F | H | ¹H-NMR (400 MHz, d6-DMSO): 11.7 (bs, 1H), 9.43 (s, 1H). 7.66 (m, 2H), 7.60 (m, 1H), 6.31 (m, 1H), 4.73 (m, 2H) |
| I-a-143 | 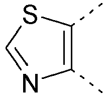 | C=O | CH₂C≡CH | Br | 4-Cl | 6-OCF₃ | ¹H-NMR (400 MHz, d6-DMSO): 11.7 (bs, 1H), 9.45 (s, 1H). 7.95 (d, 1H), 7.63 (d, 1H), 5.04 (d, 2H), 3.13 (t, 1H) |
| I-a-144 | 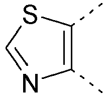 | C=O | CH₃ | CF₃ | 6-F | H | ¹H-NMR (400 MHz, d6-DMSO): 11.4 (bs, 1H), 9.41 (s, 1H). 7.65 (m, 2H), 7.59 (m, 1H), 3.69 (s, 3H) |
| I-a-145 | 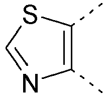 | C=O | CH₂CHF₂ | CF₃ | 6-Cl | H | ¹H-NMR (400 MHz, d6-DMSO): 11.8 (bs, 1H), 9.42 (s, 1H). 7.86 (dd, 1H), 7.80 (dd, 1H), 7.63 (t, 1H), 6.30 (m, 1H), 4.73 (m, 2H) |
| I-a-146 | 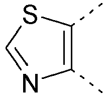 | C=O | CH₂C≡CH | CF₃ | 6-F | H | ¹H-NMR (400 MHz, CDCl₃): 8.96 (s, 1H). 7.57 (dd, 1H), 7.49 (m, 1H), 7.34 (m, 1H), 5.17 (d, 2H), 2.21 (d, 1H) |
| I-a-147 | 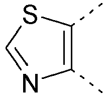 | C=O | CH₃ | CF₃ | 6-Cl | H | ¹H-NMR (400 MHz, d6-DMSO): 11.3 (bs, 1H), 9.40 (s, 1H). 7.87 (dd, 1H), 7.78 (dd, 1H), 7.62 (m, 1H), 3.68 (s, 3H) |
| I-a-148 | 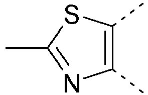 | C=O | CH₂CHF₂ | Cl | 4-Cl | 6-Et | ¹H-NMR (400 MHz, d6-DMSO): 11.3 (bs, 1H), 7.49 (d, 1H), 7.35 (d, 1H), 6.33 (m, 1H), 4.69 (m, 2H), 2.81 (s, 3H), 2.37 (m, 2H), 1.00 (t, 3H) |
| I-a-149 | 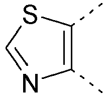 | C=O | CH₂C≡CH | CF₃ | 6-Cl | H | ¹H-NMR (400 MHz, CDCl₃): 8.98 (s, 1H). 7.66 (m, 2H), 7.43 (m, 1H), 7.34 (m, 1H), 5.16 (d, 2H), 2.21 (d, 1H) |
| I-a-150 | 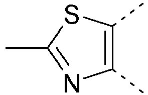 | C=O | CH₂C≡CH | Cl | 4-Cl | 6-Et | ¹H-NMR (400 MHz, CDCl₃): 7.36 (d, 1H), 7.25 (d, 1H), 5.17 (dd, 2H), 2.82 (s, 3H), 2.50 (m, 2H), 2,16 (t, 1H), 1.09 (t, 3H) |
| I-a-151 | 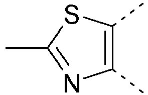 | C=O | CH₃ | Cl | 4-Cl | 6-Et | ¹H-NMR (400 MHz, CDCl₃): 7.40 (d, 1H), 7.26 (s, 1H), 3.82 (s, 3H), 2.82 (s, 3H), 2.44 (m, 2H), 1.09 (t, 3H) |
| I-a-152 | 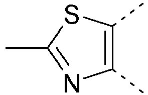 | C=O | CH₂CHF₂ | Br | 4-Cl | 6-Et | ¹H-NMR (400 MHz, d6-DMSO): 11.3 (bs, 1H), 7.63 (d, 1H), 7.39 (d, 1H), 6.32 (m, 1H), 4.69 (m, 2H), 2.81 (s, 3H), 2.40 (m, 2H), 1.00 (t, 3H) |
| I-a-153 | 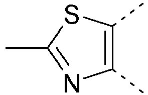 | C=O | CH₂C≡CH | Br | 4-Cl | 6-Et | ¹H-NMR (400 MHz, d6-DMSO): 11.2 (bs, 1 H), 7.63 (d, 1H), 7.39 (d, 1H), 4.98 (d, 2H), 2.82 (s, 3H), 3.13 (t, 1H), 2.36 (dq, 2H), 1.00 (dt, 3H) |
| I-a-154 | 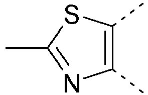 | C=O | CH₃ | Br | 4-Cl | 6-Et | ¹H-NMR (400 MHz, d6-DMSO): 10.9 (bs, 1H), 7.62 (d, 1H), 7.38 (s, 1H), 3.65 (s, 3H), 2.80 (s, 3H), 2.36 (m, 2H), 1.00 (m, 3H) |
| I-a-155 | 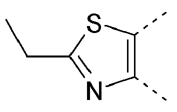 | C=O | CH₂CHF₂ | Cl | 6-Cl | H | ¹H-NMR (400 MHz, d6-DMSO): 11.4 (bs, 1H), 7.51 (d, 2H), 7.40 (t, 1H), 6.33 (m, 1H), 4.69 (m, 2H), 3.12 (q, 2H), 1.37 (t, 3H) |
| Nr. | A | V | W | X | Y | Z | Analytische Daten |
| I-a-156 | 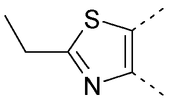 | C=O | CH₂C=CH | Cl | 6-CI | H | ¹H-NMR (400 MHz, d6-DMSO): 11.4 (bs, 1H), 7.52 (d, 2H), 7.40 (t, 1H), 5.00 (d, 2H), 3.14 (m, 2H+1 H), 1.38 (t, 3H) |
| I-a-157 | 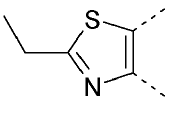 | C=O | CH₂CHF₂ | Cl | 4-CI | 6-Et | ¹H-NMR (400 MHz, d6-DMSO): 11.4 (bs, 1H), 7.49 (d, 1H), 7.35 (d, 1H), 6.33 (m, 1H), 4.70 (m, 2H), 3.12 (q, 2H), 2.36 (m, 2H), 1.37 (t, 3H), 0.99 (t, 3H) |
| I-a-158 | 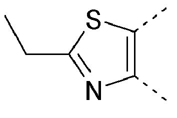 | C=O | CH₃ | Cl | 6-CI | H | ¹H-NMR (400 MHz, d6-DMSO): 11.3 (bs, 1H), 7.51 (d, 2H), 7.40 (t, 1H), 3.66 (s, 3H), 3.11 (q, 2H), 1.35 (t, 3H) |
| I-a-159 | 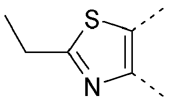 | C=O | CH₃ | Cl | 4-CI | 6-Et | ¹H-NMR (400 MHz, d6-DMSO): 10.9 (bs, 1H), 7.49 (d, 1H), 7.36 (d, 1H), 3.67 (s, 3H), 3.12 (q, 2H), 2.38 8m, 2H), 1.35 (t, 3H), 0.99 (t, 3H) |
| I-a-160 | 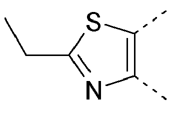 | C=O | CH₂CHF₂ | Br | 4-CI | 6-Et | ¹H-NMR (400 MHz, d6-DMSO): 11.3 (bs, 1H), 7.63 (d, 1H), 7.39 (d, 1H), 6.32 (m, 1H), 4.69 (m, 2H), 3.12 (q, 2H), 2.38 (m, 2H), 1.37 (t, 3H), 0.99 (t, 3H) |
| I-a-161 | 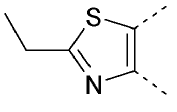 | C=O | CH₃ | Br | 4-CI | 6-Et | ¹H-NMR (400 MHz, d6-DMSO): 10.9 (bs, 1H), 7.63 (d, 1H), 7.39 (d, 1H), ^{∧}3.64 (s, 3H), 3.12 (q, 2H), 2.38 (m, 2H), 1.37 (t, 3H), 1.01 (t, 3H) |
| I-a-162 | 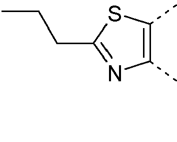 | C=O | CH₂CHF₂ | Cl | 6-Cl | H | ¹H-NMR (400 MHz, d6-DMSO): 11.6 (bs, 1H), 7.52 (d, 2H), 7.40 (t, 1H), 6.33 (m, 1H), 4.69 (m, 2H), 3.08 (t, 2H), 1.77 (q, 2H), 0.97 (t, 3H) |
| I-a-163 | 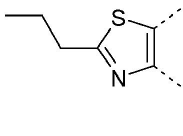 | C=O | CH₃ | Cl | 6-CI | H | ¹H-NMR (400 MHz, d6-DMSO): 11.2 (bs, 1 H), 7.51 (d, 2H), 7.40 (t, 1 H), 3.66 (s, 3H), 3.07 (t, 2H), 1.78 (q, 2H), 0.99 (t, 3H) |
| I-a-164 | 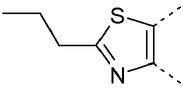 | C=O | CH₂CHF₂ | Cl | 4-CI | 6-Et | ¹H-NMR (400 MHz, d6-DMSO): 11.4 (bs, 1H), 7.48 (d, 1H), 7.35 (d, 1H), 6.32 (m, 1H), 4.69 (m, 2H), 3.07 (t, 2H), 2.37 (m, 2H), 1.80 (m, 2H), 0.99 (m, 6H) |
| I-a-165 | 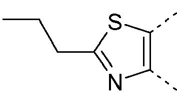 | C=O | CH₃ | Cl | 4-CI | 6-Et | ¹H-NMR (400 MHz, d6-DMSO): 11.0 (bs, 1H), 7.48 (d, 1H), 7.34 (d, 1H), 3.66 (s, 3H), 3.07 (t, 2H), 2.37 (m, 2H), 1.79 (m, 2H), 1.00 (m, 6H) |
| I-a-166 | 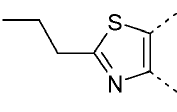 | C=O | CH₂CHF₂ | Br | 4-CI | 6-Et | ¹H-NMR (400 MHz, d6-DMSO): 11.3 (bs, 1H), 7.63 (d, 1H), 7.39 (d, 1H), 6.32 (m, 1H), 4.70 (m, 2H), 3.07 (t, 2H), 2.38 (m, 2H), 1.79 (m, 2H), 0.98 (m, 6H) |
| I-a-167 | 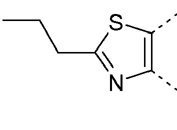 | C=O | CH₃ | Br | 4-CI | 6-Et | ¹H-NMR (400 MHz, d6-DMSO): 11.0 (bs, 1H), 7.62 (d, 1H), 7.38 (d, 1H), 3.70 (s, 3H), 3.07 (t, 2H), 2.37 (m, 2H), 1.79 (m, 2H), 1.00 (m, 6H) |
| I-a-168 | 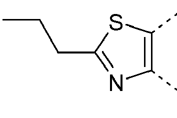 | C=O | CH₂CΞCH | Cl | 6-CI | H | ¹H-NMR (400 MHz, CDCl₃): 7.33 (d, 1H), 7.14 (m, 1H), 5.17 (dd, 2H), 3.01 (m, 2H), 2,14 (t, 1H), 1.86 (m, 2H), 1.09 (t, 3H) |
| I-a-169 | 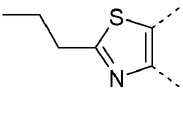 | C=O | CH₂CΞCH | Cl | 4-CI | 6-Et | ¹H-NMR (400 MHz, CDCl₃): 7.39 (d, 1H), 7.26 (d, 1H), 5.18 (dd, 2H), 3.08 (s, 3H), 2.48 (m, 2H), 2,17 (t, 1H), 1.90 (m, 2H), 1.09 (m, 3H) |
| I-a-170 | 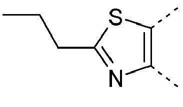 | C=O | CH₂CΞCH | Br | 4-CI | 6-Et | ¹H-NMR (400 MHz, CDCl₃): 7.58 (d, 1H), 7.31 (d, 1H), 5.18 (dd, 2H), 3.09 (s, 3H), 2.48 (m, 2H), 2,17 (t, 1H), 1.92 (m, 2H), 1.10 (m, 3H) |
| I-a-171 | 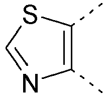 | C=O | CH₂ CH₂-OCH₃ | Cl | 6-CI | H | ¹H-NMR (400 MHz, d6-DMSO): 11.4 (bs, 1H), 9.40 (s, 1H), 7.53 (d, 2H), 7.41 (t, 1H), 4.50 (t, 2H), 3.64 (t, 2H), 3.25 (s, 3H) |
| I-a-172 | 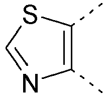 | C=O | CH₂ CH₂-SCH₃ | Cl | 6-Cl | H | ¹H-NMR (400 MHz, d6-DMSO): 11.4 (bs, 1H), 9.42 (s, 1H), 7.53 (d, 2H), 7.42 (t, 1H), 4.51 (m, 2H), 2.82 (m, 2H), 2.13 (s, 3H) |
| I-a-173 | 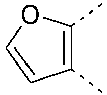 | C=O | CH₂CHF₂ | Cl | 6-CI | H | ¹H-NMR (400 MHz, d6-DMSO): 11.7 (bs, 1H), 8.12 (d, 1H), 7.52 (d, 2H), 7.38 (t, 1H), 7.12 (d, 1H), 6.32 (m, 1H), 4.51 (m, 2H), |
| I-a-174 | 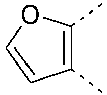 | C=O | CH₂CHF₂ | Br | 4-CI | Et | ¹H-NMR (400 MHz, d6-DMSO): 11.6 (bs, 1H), 8.12 (d, 1H), 7.62 (d, 1H), 7.37 (d, 1H), 7.12 (d, 1H), 6.32 (m, 1H), 4.51 (m, 2H), 2.38 (m 2H), 1.00 (t, 3H) |
| I-a-175 | 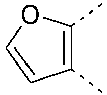 | C=O | CH₂CΞCH | Cl | 6-CI | H | ¹H-NMR (400 MHz, d6-DMSO): 11.7 (bs, 1H), 8.12 (d, 1H), 7.52 (d, 2H), 7.38 (t, 1H), 7.07 (d, 1H), 4.91 (d, 2H), 3.29 (t, 1H), |
| I-a-176 | 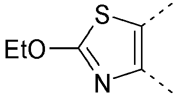 | C=O | CH₂CHF₂ | Cl | 6-CI | H | ¹H-NMR (400 MHz, d6-DMSO): 11.4 (bs, 1H), 7.52 (d, 2H), 7.39 (t, 1H), 6.32 (m, 1H),4.60 (m, 4H), 1.42 (t, 3H) |

### 3. Herstellung von 7-Allyl-5-mesityl-6-oxo-6,7-dihydrothieno[2,3-b]pyridin-4-yl-2-methylpropanoat (Verbindung Nr. I-b-8):

1.1 g (3.4 mmol) erfindungsgemäßer Verbindung I-a-20 wurden in 10ml Dichlormethan vorgelegt. Man gab 1.1 eq 2-Methylpropionylchlorid und 1.3 eq Triethylamin zu und ließ 1 h bei RT rühren. Anschließend wurde auf Wasser gegeben und die Phasen mittels einer Extraktionskartusche getrennt. Die so erhaltene organische Phase wurde eingeengt und säulenchromatographisch an Kieselgel getrennt (Gradient n-Heptan / EtOAc 100 : 0 nach 50: 50). Man erhielt so 1.1 g an erfindungsgemäßer Verbindung I-b-8.

### 4. Herstellung von 3-(2-Iodphenyl)-2,2-dioxido-1-(2,2,2-trifluorethyl)-1H-[1,3]thiazolo[4,5-c][1,2]thiazin-4-yl-2-methylpropanoat (Verbindung Nr. I-b-32):

150 mg (0.31 mmol) 3-(2-Iodphenyl)-1-(2,2,2-trifluorethyl)-1H-[1,3]thiazolo[4,5-c][1,2]thiazin-4-ol-2,2-dioxid (Verbindung I-a-15) wurden in 5 ml Dichlormethan gelöst und bei RT mit 0.04 ml (0.46 mmol) Pyridin versetzt. Die Reaktionsmischung wurde 5 min bei RT gerührt, und anschließend wurden 0.04 ml (0.4 mmol) 2-Methylpropionylchlorid zugegeben und weitere 4h bei Raumtemperatur gerührt. Danach wurde das Lösungsmittel unter Vakuum entfernt und der Rückstand mittels präparativer HPLC (C₁₈-SiO₂, Gradient Acetonitril/Wasser 20:80 nach 100:0) gereinigt. Man erhielt 47 mg der Verbindung I-b-32.

In Analogie zu den genannten Beispielen (I-b-8 und I-b-32) und gemäß den allgemeinen Angaben zur Herstellung erhält man folgende Verbindungen der Formel (I-b):

**Tabelle 148: Erfindungsgemäße Verbindungen der allgemeinen Formel (I), worin G für C(=O)R¹ steht:**

| | | | | | | | | |
|---|---|---|---|---|---|---|---|---|
| 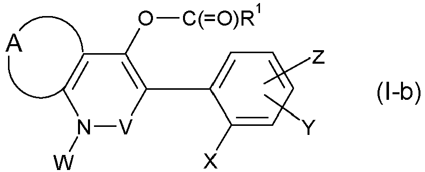 | | | | | | | | |

| Nr. | A | V | W | X | Y | Z | R¹ | Analytische Daten |
|---|---|---|---|---|---|---|---|---|
| I-b-1 | 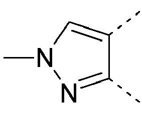 | SO₂ | CH₂CHF₂ | Cl | 6-Cl | H | i-Pr | ¹H-NMR (400 MHz, CDCl₃): 7.47 (s, 1H); 7.41 (d, 2H); 7.30 (dd, 1H); 6.25 (tt, 1H); 4.30 (td, 2H); 3.93 (s, 3H); 2.57 (m, 1H); 0.98 (d, 6H) |
| I-b-2 | 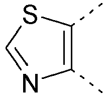 | SO₂ | CH₂CHF₂ | Cl | 4-Cl | H | i-Bu | ¹H-NMR (400 MHz, d6-DMSO): 10.6 (bs, 1H), 9.31 (s, 1H), 6.90 (s, 2H), 5.96 (m, 1H), 5.07 (m, 1H), 4.90 (m, 3H), 2,27 (s, 3H), 1.97 (s, 6H) |
| I-b-3 | 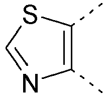 | SO₂ | CH₂CHF₂ | Cl | 6-Cl | H | i-Pr | ¹H-NMR (400 MHz, CDCl₃): 8.88 (s, 1H); 7.45 (m, 2H); 7.34 (m, 1H); 6.24 (tt, 1H); 4.55 (m, 2H); 2.59 (m, 1H); 1.00 (d, 6H) |
| I-b-4 | 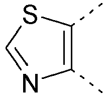 | SO₂ | CH₂CHF₂ | I | H | H | i-Pr | ¹H-NMR (400 MHz, CDCl₃): 8.86 (s, 1H); 7.95 (d, 1H); 7.51 (dd, 1H); 7.44 (td, 1H); 7.14 (td, 1H); 6.27 (m, 1H); 4.66 (m, 1H); 4.51 (m, 1H); 2.55 (m, 1H); 0.99 (d, 3H); 0.89 (d, 3H) |
| I-b-5 | 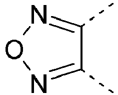 | SO₂ | CH₂CHF₂ | CF₃ | H | H | i-Pr | ¹H-NMR (400 MHz, CDCl₃): 7.86 (d, 1H); 7.69 (m, 3H); 6.24 (tt, 1H); 4.04 (m, 2H); 2.76 (m, 1H); 1.28 (d, 6H) |
| I-b-6 | 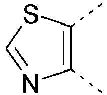 | SO₂ | CH₂CHF₂ | I | H | H | i-Pr | ¹H-NMR (400 MHz, CDCl₃): 8.86 (s, 1H); 7.95 (d, 1H); 7.51 (dd, 1H); 7.44 (td, 1H); 7.14 (td, 1H); 6.27 (m, 1H); 4.66 (m, 1H); 4.51 (m, 1H); 2.55 (m, 1H); 0.99 (d, 3H); 0.89 (d, 3H) |
| I-b-7 | 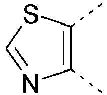 | C=O | CH₂-C-Pr | Cl | 6-Cl | H | Et | ¹H-NMR (400 MHz, CDCl₃): 8.93 (s, 1H), 7.41 (d, 2H), 7.26 (m, 1H), 4.40 (d, 2H), 2.38 (q, 2H), 1.45 (m, 1H); 1.02 (t, 3H), 0.50 (m, 4H) |
| I-b-8 | 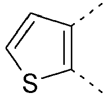 | C=O | CH₂-CH=CH₂ | Me | 4-Me | 6-Me | i-Bu | ¹H-NMR (400 MHz, CDCl₃): 6.95 (m, 2H), 6.87 (s, 2H), 5.96 (m, 1 H), 5.30 (m, 2H), 4.83 (m, 2H), 2.50 (m, 1 H), 2,26 (s, 3H), 2.07 (s, 6H), 0.92 (d, 6H) |
| I-b-9 | 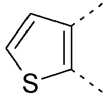 | C=O | CH₂-CH=CH₂ | Br | 4-CI | 6-Et | i-Bu | ¹H-NMR (400 MHz, CDCl₃): 7.51 (d, 1H), 7.25 (s, 1H), 7.00 (d, 1H), 6.93 (d, 1H), 5.94 (m, 1H), 5.33 (m, 2H), 4.84 (m, 2H), 2.60 (m, 1H), 2,48 (m, 2H), 1.12 (t, 3H), 0.99 (d, 6H) |
| I-b-10 | 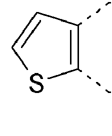 | C=O | CH₂-CH=CH₂ | Cl | 6-Cl | H | i-Bu | ¹H-NMR (400 MHz, CDCl₃): 7.39 (d, 2H), 7.22 (m, 1H), 7.00 (d, 1H), 6.97 (d, 1H), 5.96 (m, 1H), 5.33 (m, 2H), 4.84 (m, 2H), 2.63 (m, 1H), 0.99 (d, 6H) |
| I-b-11 | 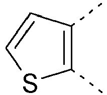 | C=O | CH₂-CH=CH₂ | Cl | 4-Cl | H | i-Bu | ¹H-NMR (400 MHz, CDCl₃): 7.39 (d, 2H), 7.22 (m, 1H), 7.00 (d, 1H), 6.97 (d, 1H), 5.96 (m, 1H), 5.33 (m, 2H), 4.84 (m, 2H), 2.63 (m, 1H), 0.99 (d, 6H) |
| I-b-12 | 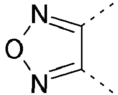 | SO₂ | CH₂CHF₂ | Cl | 4-Cl | H | i-Pr | ¹H-NMR (400 MHz, CDCl₃): 7.60 (d, 1H); 7.59 (d, 1H); 7.40 (dd, 1H); 6.23 (tt, 1H); 4.03 (m, 2H); 2.78 (m, 1H); 1.28 (d, 6H) |
| I-b-13 | 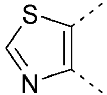 | C=O | CH₂-CH=CH₂ | Cl | 6-Cl | H | i-Bu | ¹H-NMR (400 MHz, CDCl₃): 8.94 (s, 1H), 7.40 (d, 2H), 7.26 (m, 1H), 6.07 (m, 1H), 5.28 (m, 1H), 5,23 (m, 1H), 5,15 (m, 2H), 2.62 (m, 1H), 1.03 (d, 6H) |
| I-b-14 | 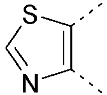 | C=O | CH₂-CH=CH₂ | Br | 4-Cl | 6-Et | i-Bu | ¹H-NMR (400 MHz, CDCl₃): 8.93 (s, 1H), 7.52 (d, 1H), 7.26 (d, 1H), 6.05 (m, 1H), 5.28 (m, 1H), 5,22 (m, 1H), 5,14 (m, 2H), 2.59 (m, 1H), 2,46 (m, 2H), 1.12 (t, 3H), 1.00 (d, 6H) |
| I-b-15 | 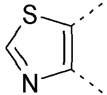 | C=O | CH₂-CH=CH₂ | Cl | 4-Cl | H | i-Bu | ¹H-NMR (400 MHz, CDCl₃): 8.92 (s, 1 H), 7.50 (d, 1H), 7.34 (dd, 1H), 7.22 (d, 1 H), 6.06 (m, 1 H), 5.30 (m, 1 H), 5,24 (m, 1H), 5,10 (m, 2H), 2.61 (m, 1 H), 1.07 (dd, 6H) |
| I-b-16 | 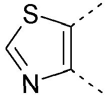 | C=O | CH₂-CH=CH₂ | Me | 4-Me | 6-Me | i-Bu | ¹H-NMR (400 MHz, CDCl₃): 8.88 (s, 1H), 6.87 (d, 2H), 6.07 (m, 1H), 5,24 (m, 2H), 5,13 (m, 2H), 2.50 (m, 1H), 2.27 (s,3H), 2.07 (s, 6H), 0.92 (d, 6H) |
| I-b-17 | 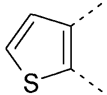 | C=O | CH₂CHF₂ | Me | 4-Me | 6-Me | i-Bu | ¹H-NMR (400 MHz, CDCl₃): 7.02 (d, 1H), 6.98 (d, 1H), 6.88 (d, 2H), 6.26 (m, 1H), 4.48 (m, 2H), 2.51 (m, 1H), 2.27 (s, 3H), 2.06 (s, 3H), 0.92 (d, 6H) |
| I-b-18 | 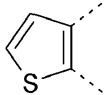 | C=O | CH₂CF₃ | Me | 4-Me | 6-Me | i-Bu | ¹H-NMR (400 MHz, CDCl₃): 7.02 (d, 1H), 6.95 (d, 1H), 6.87 (d, 2H), 4.85 (m, 2H), 2.52 (m, 1H), 2.26 (s, 3H), 2.06 (s, 3H), 0.92 (d, 6H) |
| I-b-19 | 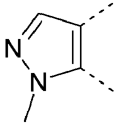 | C=O | CH₂-CH=CH₂ | Cl | 4-Cl | H | t-Bu | ¹H-NMR (400 MHz, CDCl₃): 7.49 (d, 1H), 7.47 (s, 1H), 7.26 (dd, 1H), 7.19 (d, 1H), 6.07 (m, 1H), 5,30 (m, 1H), 5,04 (m, 3H), 4.18 (s, 3H), 1.12 (s, 9H) |
| I-b-20 | 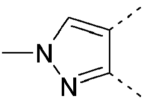 | C=O | CH₂-CH=CH₂ | Cl | 6-Cl | H | Et | ¹H-NMR (400 MHz, CDCl₃): 7.52 (s, 1H), 7.38 (d, 2H), 7.23 (m, 1H), 6.04 (m, 1H), 5,21 (m, 2H), 4.85 (m, 2H), 3.99 (s, 3H), 2.37 (q, 2H), 1.02 (t, 3H) |
| I-b-21 | 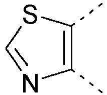 | C=O | CH=CH(CH₃) | Cl | 6-Cl | H | t-Bu | ¹H-NMR (400 MHz, CDCl₃): 8.94 (s, 1H), 7.39 (d, 2H), 7.26 (m, 1H), 6.64 (m, 1H), 6.10 (m, 1H), 1.62 (dd, 3H), 1.09 (d, 9H) |
| I-b-22 | 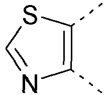 | C=O | CH₂-CH=CH₂ | Br | 4-Cl | Et | t-Bu | ¹H-NMR (400 MHz, CDCl₃): 8.93 (s, 1H), 7.51 (d, 1H), 7.26 (d, 1H), 6.05 (m, 1H), 5,22 (m, 2H), 5,14 (m, 2H), 2,47 (m, 2H), 1.12 (t, 3H), 0.88 (d, 9H) |
| I-b-23 | 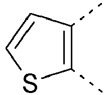 | C=O | CH₂-CH=CH₂ | Cl | 4-Cl | H | t-Bu | ¹H-NMR (400 MHz, CDCl₃): 7.49 (d, 1H), 7.27 (m, 1H), 7.24 (m, 1H), 7.00 (d, 1H), 6.93 (d, 1H), 5.98 (m, 1H), 5.35 (m, 2H), 4.82 (m, 2H), 1.11 (d, 9H) |
| I-b-24 | 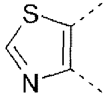 | C=O | CH₂-CH=CH₂ | Cl | 6-Cl | H | t-Bu | ¹H-NMR (400 MHz, CDCl₃): 8.94 (s, 1H), 7.39 (d, 2H), 7.25 (m, 1H), 6.08 (m, 1H), 5.24 (m, 2H), 5.15 (d, 2H), 1.09 (d, 9H) |
| I-b-25 | 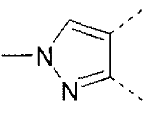 | C=O | CH₂CHF₂ | Me | 4-Me | 6-Me | Et | ¹H-NMR (400 MHz, CDCl₃): 7.49 (s, 1H), 6.87 (s, 2H), 6.30 (m, 1H), 4.58 (m, 2H), 3.99 (s, 3H), 2.27 (s, 3H), 2.25 (q, 2H), 2.05 (s, 6H), 0.90 (t, 3H) |
| I-b-26 | 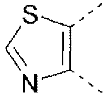 | C=O | CH₂CHF₂ | Cl | 4-Cl | H | Et | ¹H-NMR (400 MHz, CDCl₃): 8.97 (s, 1H), 7.52 (d, 1H), 7.32 (d, 1H), 7.23 (dd, 1 H), 6.30 (m, 1H), 4.91 (m, 2H), 2.40 (m, 2H), 1.06 (t, 3H) |
| I-b-27 | 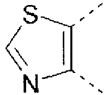 | C=O | CH₂-CH=CH₂ | Cl | 6-Cl | H | Et | ¹H-NMR (400 MHz, CDCl₃): 8.97 (s, 1H), 7.37 (d, 2H), 7.25 (t, 1H), 6.06 (m, 1H), 5.24 (m, 2H), 5.17 (m, 2H), 2.37 (m, 2H), 1.01 (t, 3H) |
| I-b-28 | 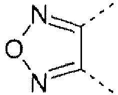 | SO₂ | CH₂CHF₂ | Cl | 6-Cl | H | i-Pr | ¹H-NMR (400 MHz, CDCl₃): 7.48 (m, 2H); 7.40 (dd, 1H); 6.24 (tt, 1H); 4.05 (m, 2H); 2.78 (m, 1H); 1.28 (d, 6H) |
| I-b-30 | 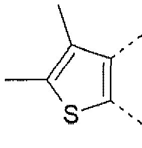 | C=O | CH₂CHF₂ | Cl | 4-Br | 6-Et | i-Pr | ¹H-NMR (400 MHz, CDCl₃): 7.48 (d, 1H); 7.36 (d, 1H); 6.22 (tt, 1H); 4.43 (m, 2H); 2.49 (m, 3H); 2.37 (s, 3H); 2.19 (s, 3H); 1.14 (t, 3H); 0.91 (s, 3H); 0.87 (d, 3H) |

### 5. Herstellung von 5-(2,6-Dichlorphenyl)-7-(2,2-difluorethyl)-6-oxo-6,7-dihydrothieno[2,3-b]pyridin-4-yl-ethylcarbonat (Verbindung Nr. I-c-27):

0.134 g (0.35 mmol) erfindungsgemäßer Verbindung I-a-40 wurden in 5ml Dichlormethan vorgelegt. Man gab 1.3 eq Triethylamin und anschließend 1.1 eq Chlorameisensäureethylester zu und ließ 1 h bei RT rühren. Anschließend wurde auf Wasser gegeben und die Phasen mittels einer Extraktionskartusche getrennt. Die organische Phase wurde eingeengt und säulenchromatographisch an Kieselgel getrennt (Gradient n-Heptan / EtOAc 100 : 0 nach 50: 50). Man erhielt 0.1 g an erfindungsgemäßer Verbindung I-c-27.

### 6. Herstellung von O-[1-(2,2-Difluorethyl)-3-(2-iodphenyl)-2,2-dioxido-1H-[1,3]thiazolo[4,5-c][1,2]thiazin-4-yl]-S-methylthiocarbonat (Verbindung I-c-16):

150 mg (0.3 mmol) 1-(2,2-Difluorethyl)-3-(2-iodphenyl)-1H-[1,3]thiazolo[4,5-c][1,2]thiazin-4-ol-2,2-dioxid (Verbindung I-a-14) wurden in 5 ml Dichlormethan gelöst und bei RT mit 0.04 ml (0.48 mmol) Pyridin versetzt. Die Reaktionsmischung wurde 5 min bei RT gerührt, und anschließend wurden 0.03 ml (0.38 mmol) Methylchlorthiolformiat zugegeben und weitere 4h bei RT gerührt. Nach Entfernen des Lösemittels unter Vakuum wurde der Rückstand mittels Säulenchromatographie (SiO₂, Gradient Ethylacetat/n-Heptan 10:90 nach 75:25) gereinigt. Man erhielt 144 mg der Verbindung I-c-16.

In Analogie zu den genannten Beispielen (I-c-16 und I-c-27) und gemäß den allgemeinen Angaben zur Herstellung erhält man folgende Verbindungen der Formel (I-c).

**Tabelle 149: Erfindungsgemäße Verbindungen der allgemeinen Formel (I), worin G für C(=L)MR² steht:**

| | | | | | | | | | | |
|---|---|---|---|---|---|---|---|---|---|---|
| 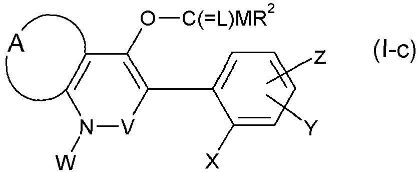 | | | | | | | | | | |

| Nr. | A | V | W | X | Y | Z | L | M | R² | Analytische Daten |
|---|---|---|---|---|---|---|---|---|---|---|
| I-c-1 | 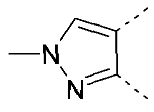 | SO₂ | CH₂CHF₂ | Cl | 6-Cl | H | O | S | Et | ¹H-NMR (400 MHz, CDCl₃): 7.56 (s, 1H); 7.43 (d, 2H); 7.31 (dd, 1H); 6.24 (tt, 1H); 4.28 (td, 2H); 3.94 (s, 3H); 2.81 (q, 2H); 1.22 (t, 3H) |
| I-c-2 | 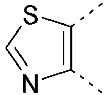 | SO₂ | CH₂CHF₂ | Cl | 4-Cl | H | O | S | Me | ¹H-NMR (400 MHz, CDCl₃): 8.89 (s, 1H); 7.55 (d, 1H); 7.45 (d, 1H); 7.35 (dd, 1H); 6.17 (tt, 1H); 4.56 (m, 2H); 2.30 (s, 3H) |
| 1-c-3 | 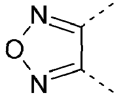 | SO₂ | CH₂CHF₂ | Cl | 6-Cl | H | O | S | Me | ¹H-NMR (400 MHz, CDCl₃): 7.49 (m, 2H); 7.41 (dd, 1H); 6.21 (tt, 1H); 4.03 (m, 2H); 2.42 (s, 3H) |
| I-c-4 | 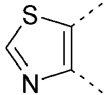 | SO₂ | CH₂CHF₂ | Cl | 4-Cl | H | O | S | Et | ¹H-NMR (400 MHz, CDCl₃): 8.89 (s, 1H); 7.54 (d, 1H); 7.45 (d, 1H); 7.35 (dd, 1H); 6.16 (tt, 1H); 4.54 (m, 2H); 2.79 (m, 2H); 1.20 (t, 3H) |
| I-c-5 | 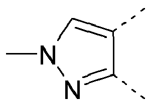 | SO₂ | CH₂CHF₂ | Cl | 4-Cl | H | O | S | Me | ¹H-NMR (400 MHz, CDCl₃): 7.53 (m, 2H); 7.44 (d, 1H); 7.32 (dd, 1H); 6.20 (tt, 1H); 4.30 (m, 2H); 3.93 (s, 3H); 2.30 (s, 3H) |
| I-c-6 | 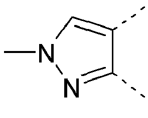 | SO₂ | CH₂CHF₂ | Cl | 4-Cl | H | O | S | Et | ¹H-NMR (400 MHz, CDCl₃): 7.53 (m, 2H); 7.44 (d, 1H); 7.32 (dd, 1H); 6.21 (tt, 1H); 4.30 (m, 2H); 3.93 (s, 3H); 2.79 (q, 2H); 1.22 (t, 3H) |
| I-c-7 | 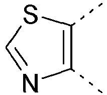 | SO₂ | CH₂CHF₂ | Cl | 6-Cl | H | O | S | Me | ¹H-NMR (400 MHz, CDCl₃): 8.91 (s, 1H); 7.46 (dd, 2H); 7.35 (dd, 1H); 6.23 (tt, 1H); 4.55 (m, 2H); 2.31 (s, 3H) |
| I-c-8 | 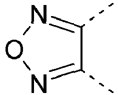 | SO₂ | CH₂CHF₂ | CF3 | H | H | O | S | Me | ¹H-NMR (400 MHz, CDCl₃): 7.87 (d, 1H); 7.69 (m, 3H); 6.21 (tt, 1H); 4.02 (m, 2H); 2.42 (s, 3H) |
| I-c-9 | 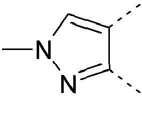 | SO₂ | CH₂CHF₂ | CF3 | H | H | O | O | Et | ¹H-NMR (400 MHz, CDCl₃): 7.78 (d, 1H); 7.63 (m, 3H); 7.53 (s, 1H); 6.20 (m, 1H); 4.37 (m, 1H); 4.20 (m, 1H); 4.12 (m, 2H); 3.93 (s, 3H); 1.17 (t, 3H) |
| I-c-10 | 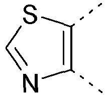 | SO₂ | CH₂CF₃ | CF3 | H | H | O | S | Et | ¹H-NMR (400 MHz, CDCl₃): 8.89 (s, 1H); 7.80 (d, 1H); 7.64 (m, 3H); 4.78 (m, 2H); 2.76 (m, 2H); 1.16 (t, 3H) |
| I-c-11 | 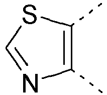 | SO₂ | CH₂CHF₂ | CF3 | H | H | O | S | Me | ¹H-NMR (400 MHz, CDCl₃): 8.88 (s, 1H); 7.80 (d, 1H); 7.64 (m, 3H); 6.17 (m, 1H); 4.62 (m, 1H); 4.44 (m, 1H); 2.25 (s, 3H) |
| I-c-12 | 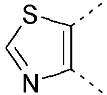 | SO₂ | CH₂CHF₂ | CF3 | H | H | O | S | Et | ¹H-NMR (400 MHz, CDCl₃): 8.87 (s, 1H); 7.81 (m, 1H); 7.65 (m, 3H); 6.17 (m, 1H); 4.63 (m, 1H); 4.44 (m, 1H); 2.75 (m, 2H); 1.16 (t, 3H) |
| I-c-13 | 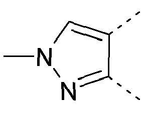 | SO₂ | CH₂CHF₂ | CF3 | H | H | O | S | Et | ¹H-NMR (400 MHz, CDCl₃): 7.93 (dd, 1H); 7.48 (dd, 1H); 7.45 (s, 1H); 7.41 (td, 1H); 7.11 (td, 1H); 6.27 (tt, 1H); 4.39 (m, 1H); 4.26 (m, 1H); 3.92 (s, 3H); 2.53 (m, 1H); 0.97 (d, 3H); 9.88 (d, 3H) |
| I-c-14 | 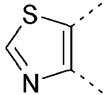 | C=O | CH₂-CH=CH₂ | Cl | 4-Cl | H | O | O | Et | ¹H-NMR (400 MHz, CDCl₃): 8.93 (s, 1H), 7.52 (d, 1H), 7.26 (d, 1H), 6.05 (m, 1H), 5.28 (m, 1H), 5,22 (m, 1H), 5,14 (m, 2H), 2.59 (m, 1H), 2,46 (m, 2H), 1.12 (t, 3H), 1.00 (d, 6H) |
| I-c-15 | 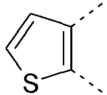 | C=O | CH₂-CH=CH₂ | Cl | 6-Cl | H | O | O | Et | ¹H-NMR (400 MHz, CDCl₃): 8.92 (s, 1H), 7.50 (d, 1H), 7.34 (dd, 1H), 7.22 (d, 1H), 6.06 (m, 1H), 5.30 (m, 1H), 5,24 (m, 1H), 5,10 (m, 2H), 2.61 (m, 1H), 1.07 (dd, 6H) |
| I-c-16 | 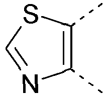 | SO₂ | CH₂CHF₂ | I | H | H | O | S | Me | ¹H-NMR (400 MHz, CDCl₃): 8.88 (s, 1H); 7.96 (d, 1H); 7.52 (dd, 1H); 7.46 (td, 1H); 7.17 (td, 1H); 6.27 (m, 1H); 4.66 (m, 1H); 4.49 (m, 1H); 2.27 (s, 3H) |
| I-c-17 | 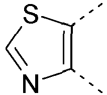 | SO₂ | CH₂CHF₂ | I | H | H | O | S | Et | ¹H-NMR (400 MHz, CDCl₃): 8.88 (s, 1H); 7.96 (d, 1H); 7.53 (dd, 1H); 7.45 (td, 1H); 7.16 (td, 1H); 6.26 (m, 1H); 4.67 (m, 1H); 4.49 (m, 1H); 2.77 (m, 2H); 1.17 (t, 3H) |
| I-c-18 | 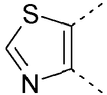 | SO₂ | CH₂CF₃ | I | H | H | O | S | Me | ¹H-NMR (400 MHz, CDCl₃): 8.90 (s, 1H); 7.97 (d, 1H); 7.47 (m, 2H); 7.16 (m, 1H); 4.80 (m, 2H); 2.77 (s, 3H) |
| I-c-19 | 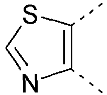 | SO₂ | CH₂CF₃ | I | H | H | O | S | Et | ¹H-NMR (400 MHz, CDCl₃): 8.89 (s, 1H); 7.95 (d, 1H); 7.49 (dd, 1H); 7.45 (t, 1H); 7.15 (td, 1H); 4.80 (m, 2H); 2.77 (m, 2H); 1.17 (t, 3H) |
| I-c-20 | 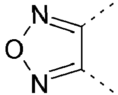 | SO₂ | CH₂CHF₂ | CF₃ | H | H | O | S | Et | ¹H-NMR (400 MHz, CDCl₃): 7.87 (d, 1H); 7.69 (m, 3H); 6.21 (tt, 1H); 4.01 (m, 2H); 2.96 (q, 2H); 1.38 (t, 3H) |
| I-c-21 | 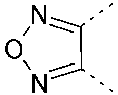 | C=O | CH₃ | Cl | 6-Cl | H | O | O | Et | ¹H-NMR (400 MHz, CDCl₃): 7.42 (m, 2H), 7.33 (m, 1H), 7.10 (m, 2H), 4.29 (q, 2H), 3.72 (s, 3H), 1.32 (m, 3H) |
| I-c-22 | 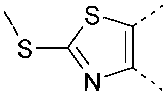 | C=O | CH₃ | Me | 4-Me | 6-Me | O | O | Et | ¹H-NMR (400 MHz, CDCl₃): 6.89 (s, 2H), 4.06 (q, 2H), 3.88 (s, 3H), 2.78 (s, 3H), 2.28 (s, 3H), 2.08 (s, 6H), 1.11 (t, 3H) |
| I-c-23 | 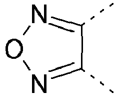 | SO₂ | CH₂CHF₂ | Cl | 4-Cl | H | O | S | Me | ¹H-NMR (400 MHz, CDCl₃): 7.60 (m, 2H); 7.40 (dd, 1H); 6.20 (tt, 1H); 4.00 (m, 2H); 2.42 (s, 3H) |
| I-c-24 | 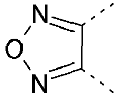 | SO₂ | CH₂CHF₂ | Cl | 4-Cl | H | O | S | Et | ¹H-NMR (400 MHz, CDCl₃): 7.59 (m, 2H); 7.40 (dd, 1H); 6.20 (tt, 1H); 4.00 (m, 2H); 2.95 (q, 2H); 1.38 (t, 3H) |
| I-c-25 | 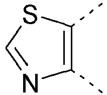 | C=O | CH₂CHF₂ | Cl | 6-Cl | H | O | O | Et | ¹H-NMR (400 MHz, CDCl₃): 8.99 (s, 1H), 7.42 (dd, 2H), 7.32 (m, 1H), 6.30 (m (1H), 4.93 (m, 2H), 4.24 (m, 2H), 1.26 (m, 3H) |
| I-c-26 | 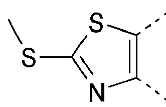 | C=O | CH₂CHF₂ | Me | 4-Me | 6-Me | O | O | Et | ¹H-NMR (400 MHz, CDCl₃): 6.90 (s, 2H), 6.30 (m, 1H), 4.84 (m, 2H), 4.06 (m, 2H), 2.78 (s, 3H), 2.28 (s, 3H), 2.07 (s, 6H). 1.11 (t, 3H) |
| I-c-27 | 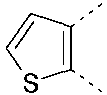 | C=O | CH₂CHF₂ | Cl | 6-Cl | H | O | O | Et | ¹H-NMR (400 MHz, CDCl₃): 7.42 (m, 2H), 7.31 (m, 1H), 7.10 (m, 2H), 6.28 (m, 1H), 4.49 (m, 2H), 4.19 (q, 2H), 1.22 (m, 3H) |
| I-c-28 | 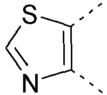 | C=O | CH₂CF₃ | Cl | 6-Cl | H | O | O | Et | ¹H-NMR (400 MHz, CDCl₃): 8.98 (s, 1H), 7.42 (dd, 2H), 7.30 (m, 1H), 5.21 (m, 2H), 4.22 (m, 2H), 1.25 (m, 3H) |
| I-c-29 | 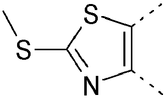 | C=O | CH₂CHF₂ | Br | 4-Cl | 6-Et | O | O | Et | ¹H-NMR (400 MHz, CDCl₃): 7.53 (d, 1H), 7.26 (d, 1H), 6.28 (m, 1H), 4.84 (m, 2H), 4.19 (m, 2H), 2.79 (s, 3H), 2.46 (m, 2H), 1.23 (m, 3H), 1.12 (t, 3H) |
| I-c-30 | 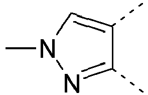 | C=O | CH₂CHF₂ | Br | 4-Cl | 6-Et | O | O | Et | ¹H-NMR (400 MHz, CDCl₃): 7.59 (s, 1H), 7.51 (d, 1H), 7.26 (d, 1H), 6.28 (m, 1H), 4.58 (m, 2H), 4.22 (m, 2H), 2.45 (m, 2H), 1.26 (m, 3H), 1.10 (t, 3H) |
| I-c-31 | 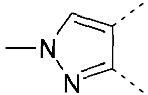 | C=O | CH₂CHF₂ | Me | 4-Me | 6-Me | O | O | Et | ¹H-NMR (400 MHz, CDCl₃): 7.59 (s, 1H), 6.89 (s, 2H), 6.29 (m, 1H), 4.58 (m, 2H), 4.08 (m, 2H), 2.28 (s, 3H), 2.07 (s, 6H), 1.15 (t, 3H) |
| I-c-32 | 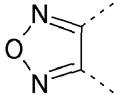 | SO₂ | CH₂CHF₂ | Cl | 6-Cl | H | O | S | Me | ¹H-NMR (400 MHz, CDCl₃): 7.49 (m, 2H); 7.41 (dd, 1H); 6.21 (tt, 1H); 4.03 (m, 2H); 2.42 (s, 3H) |
| I-c-33 | 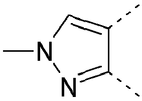 | SO₂ | CH₂CHF₂ | Cl | 6-Cl | H | O | S | Me | ¹H-NMR (400 MHz, CDCl₃): 7.56 (s, 1 H); 7.43 (m, 2H); 7.32 (dd, 1H); 6.24 (tt, 1H); 4.29 (td, 2H); 3.94 (s, 3H); 2.31 (s, 3H) |
| I-c-34 | 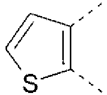 | C=O | CH₂CHF₂ | Br | 4-Cl | 6-Et | O | O | Et | ¹H-NMR (400 MHz, CDCl₃): 7.52 (d, 1H), 7.28 (d, 1H), 7.10 (m, 2H), 6.25 (m, 1H), 4.50 (m, 2H), 4.18 (q, 2H), 2.48 (q, 2H), 1.23 (m, 3H), 1.14 (t, 3H) |
| I-c-35 | 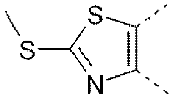 | C=O | CH₂CF₃ | Cl | 6-Cl | H | O | O | Et | ¹H-NMR (400 MHz, CDCl₃): 7.41 (d, 2H), 7.27 (t, 1H), 5.14 (m, 2H), 4.21 (m, 2H), 2.77 (s, 3H), 1.23 (m, 3H) |
| I-c-36 | 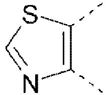 | C=O | CH₂CΞCH | Br | 4-Cl | 6-Et | O | O | Et | ¹H-NMR (400 MHz, CDCl₃): 9.01 (s, 1H), 7.52 (d, 1H), 7.28 (d, 1H), 5.28 (m, 2H), 4.21 (m, 2H), 2.47 (m, 2H), 2.24 (t, 1H), 1.23 (m, 3H), 1.12 (t, 3H) |
| I-c-37 | 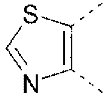 | C=O | CH₂CH₃ | Cl | 6-Cl | H | O | O | Et | ¹H-NMR (400 MHz, CDCl₃): 8.97 (s, 1H), 7.41 (dd, 2H), 7.27 (m, 1H), 4.59 (m, 2H), 4.21 (m, 2H), 1.42 (t, 3H), 1.25 (m, 3H) |
| I-c-38 | 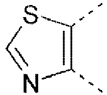 | C=O | CH₃ | Me | 4-Br | 6-Et | O | O | Et | ¹H-NMR (400 MHz, CDCl₃): 8.95 (s, 1H), 7.29 (d, 1H), 7.27 (d, 1H), 4.12 (q, 2H), 3.95 (s, 3H), 2.40 (m, 2H), 2.10 (s, 3H), 1.18 (m, 3H), 1.11 (t, 3H) |
| I-c-39 | 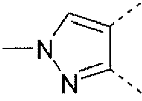 | SO₂ | CH₂CHF₂ | I | H | H | O | S | Me | ¹H-NMR (400 MHz, CDCl₃): 7.94 (m, 1H); 7.55 (s, 1H); 7.50 (dd, 1H); 7.43 (td, 1H); 7.14 (td, 1H); 6.27 (tdd, 1H); 4.40 (m, 1H); 4.25 (m, 1H); 3.93 (s, 3H); 2.27 (s, 3H |
| I-c-40 | 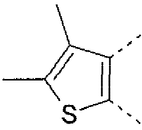 | C=O | CH₂CHF₂ | Cl | 4-Br | 6-Et | O | S | Me | ¹H-NMR (400 MHz, CDCl₃): 7.48 (br s, 1H); 7.37 (br s, 1H); 6.22 (tt, 1H); 4.42 (m, 2H); 2.45 (m, 2H); 2.38 (s, 3H); 2.24 (s, 6H); 1.14 (t, 3H) |

### 6. Herstellung 4-(2,2-Difluorethyl)-6-mesityl-5-oxo-4,5-dihydro[1,3]thiazolo[4,5-b]pyridin-7-ylmethansulfonat (I-d-1)

0.2 g (0.57 mmol) von erfindungsgemäßer Verbindung 1-a-37 wurden in 5ml Dichlormethan gelöst zunächst 0.075g (1.3 eq, 0.1 ml) Triethylamin und anschließend 0.05 g (1.1 eq, 0.5 ml) Methansulfonsäurechlorid hinzugegeben. Man ließ eine Stunde bei Raumtemperatur rühren, versetzte mit 10 ml Wasser, trennte die organische Phase ab und engte diese unter vermindertem Druck ein. Man erhielt so 0.221 g als Feststoff von Verbindung I-d-1.

In Analogie zu dem genannten Beispiel und gemäß den allgemeinen Angaben zur Herstellung erhält man folgende Verbindungen der Formel (I-d).

**Tabelle 150: Erfindungsgemäße Verbindungen der allgemeinen Formel (I), worin G für SO₂R³ steht:**

| | | | | | | | | |
|---|---|---|---|---|---|---|---|---|
| 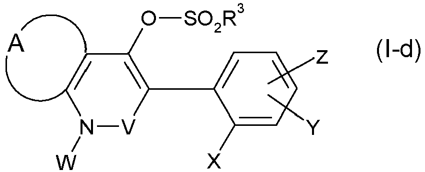 | | | | | | | | |

| Nr. | A | V | W | X | Y | Z | R³ | Analytische Daten |
|---|---|---|---|---|---|---|---|---|
| I-d-1 | 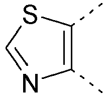 | C=O | CH₂CHF₂ | Me | 4-Me | 6-Me | Me | ¹H-NMR (400 MHz, CDCl₃): 8.97 (s, 1H), 6.88 (s, 2H), 6.30 (m, 1H), 4.91 (m, 2H), 2.43 (s, 3H), 2.30 (s, 3H), 2.14 (s, 6H) |
| I-d-2 | 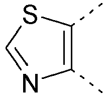 | C=O | CH₂C≡CH | Me | 3-Br | 6-Me | 4-Me-Phe -nyl | ¹H-NMR (400 MHz, CDCl₃): 9.04 (s, 1H), 7.36 (d, 1H), 7.21 (d, 2H), 7.13 (d, 2H), 5.27 (d, 2H), 2.44 (s, 3H), 2.24 (t, 1H), 2.04 (s, 3H), 2.02 (s, 3H) |
| I-d-3 | 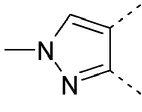 | C=O | CH₂-CH=CH₂ | Br | 4-Cl | 6-Et | Me | ¹H-NMR (400 MHz, CDCl₃): 7.87 (s, 1H), 7.54 (d, 1H), 7.29 (d, 1H), 6.01 (m, 1H), 5.21 (m, 2H), 4.82 (m, 2H), 4.02 (s, 3H), 2.81 (s, 3H), 2.49 (m, 2H), 1.14 (m, 3H), |
| I-d-4 | 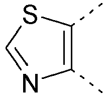 | C=O | CH₂CHF₂ | Br | 4-Cl | 6-Et | Me | ¹H-NMR (400 MHz, CDCl₃): 9.02 (s, 1H), 7.58 (d, 1H), 7.34 (d, 1H), 6.28 (m, 1H), 4.92 (m, 2H), 2.78 (s, 3H), 2.52 (m, 2H), 1.12 (m, 3H) |
| I-d-5 | 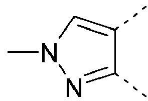 | C=O | CH₂CF₃ | Br | 4-Cl | 6-Et | Me | ¹H-NMR (400 MHz, CDCl₃): 7.89 (s, 1H), 7.54 (d, 1H), 7.30 (d, 1H), 4.87 (m, 2H), 4.02 (s, 3H), 2.81 (s, 3H), 2.48 (m, 2H), 1.15 (m, 3H), |
| I-d-6 | 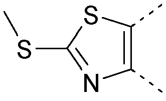 | C=O | CH₂C≡CH | Cl | 6-Cl | H | 4-Me-Phe -nyl | ¹H-NMR (400 MHz, CDCl₃): 7.42 (d, 1H), 7.18 (d, 2H), 7.12 (m, 3H), 5.21 (d, 2H), 2.81 (s, 3H), 2.43 (s, 3H), 2.23 (t, 1H), |
| I-d-7 | 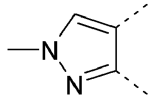 | C=O | CH₂CF₃ | Cl | 6-Cl | H | 4-Me-Phe -nyl | ¹H-NMR (400 MHz, CDCl₃): 8.00 (s, 1H), 7.42 (d, 2H), 7.19 (d, 2H), 7.12 (m ,3H), 4.87 (m, 2H), 4.04 (s, 3H), 2.41 (s, 3H) |

### 7.Herstellung von Natrium-6-(2,6-dichlorphenyl)-4-(2,2-difluorethyl)-5-oxo-4,5-dihydro[1,3]thiazolo[4,5-b]pyridin-7-olat (Verbindung I-g-1):

9,14 mg Natrium (0.39 mmol) wurden in 2 ml MeOH gelöst, und eine Lösung von 0.15 g (1.0 eq) an erfindungsgemäßer Verbindung I-a-38 in 5ml MeOH hinzugegeben, Man ließ 10 min. rühren und engte unter vermindertem Druck zur Trockne ein. Man erhielt so Verbindung I-g-1

In Analogie zu dem genannten Beispiel und gemäß den allgemeinen Angaben zur Herstellung erhält man folgende Verbindungen der Formel (I-g).

**Tabelle 151: Erfindungsgemäße Verbindungen der allgemeinen Formel (I), worin G für E steht:**

| | | | | | | | | |
|---|---|---|---|---|---|---|---|---|
| 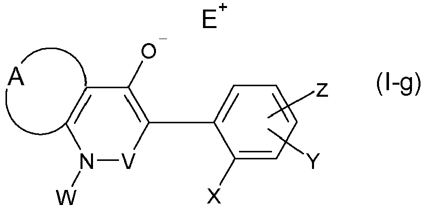 | | | | | | | | |

| Nr. | A | V | W | X | Y | Z | E | Analytische Daten |
|---|---|---|---|---|---|---|---|---|
| I-g-1 | 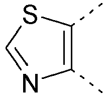 | C=O | CH₂CHF₂ | Cl | 6-Cl | H | Na⁺ | ¹H-NMR (400 MHz, d6-DMSO): 7.35 (d, 2H) 7.18 (t, 1H), 6.22 (m, 1H), 4.53 (m, 2H) |
| I-g-2 | 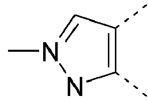 | C=O | CH₂CH=CH₂ | Cl | 4-Cl | H | Na⁺ | ¹H-NMR (400 MHz, d6-DMSO): 7.67 (s, 1H), 7.33 (d, 1H), 7.21 (d, 2H), 7.16 (dd, 1H), 5.86 (m, 1H), 4.99 (m, 2H), 4.44 (m, 2H), 3.78 (s, 3H) |
| I-g-3 | 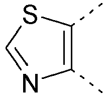 | C=O | CH₂CH₃ | Cl | 6-Cl | H | K⁺ | ¹H-NMR (400 MHz, d6-DMSO): 8.97 (s, 1H), 7.33 (d, 2H) 7.13 (t, 1H), 4.16 (q, 2H), 1.12 (t, 3H) |
| I-g-4 | 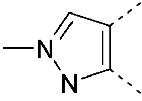 | C=O | CH₂CH=CH₂ | Cl | 6-Cl | H | N(Me)₄⁺ | ¹H-NMR (400 MHz, d6-DMSO): 7.63 (s, 1H), 7.28 (d, 1H), 7.09 (d, 2H), 5.85 (m, 1H), 4.97 (m, 2H), 4.43 (m, 2H), 3.78 (s, 3H), 3.19 (s, 12H) |
| I-g-5 | 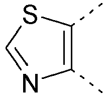 | C=O | CH₂CHF₂ | Me | 4-Me | 6-Me | N(Me)₄⁺ | ¹H-NMR (400 MHz, d6-DMSO): 6.72 (s, 2H), 4.53 (m, 2H), 3.17 (s, 12H), 2.21 (s, 3H), 1.98 (s, 6H) |
| I-g-6 | 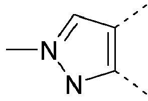 | C=O | CH₂CHF₂ | Br | 4-Cl | 6-Et | K⁺ | ¹H-NMR (400 MHz, d6-DMSO): 7.70 (s, 1H), 7.39 (s, 1H), 7.14 (s, 1H), 6.21 (m, 1H), 4.22 (m, 2H), 3.80 (s, 3H), 2.42 (q, 2H), 0.95 (t, 3H) |
| I-g-7 | 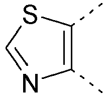 | C=O | CH₂CHF₂ | OMe | 4-Cl | 6-Et | Na⁺ | ¹H-NMR (400 MHz, d6-DMSO): 6.77 (d, 1H) 6.73 (d, 1H), 6.21 (m, 1H), 4.52 (m, 2H), 3.56 (s, 3H), 2.35 (q, 2H), 0.96 (t, 3H) |

### 8. Herstellung von 1-(2,2-Difluorethyl)-3-(2-iodphenyl)-4-(prop-2-in-1-yloxy)-1H-[1,3]thiazolo[4,5-c][1,2]thiazin-2,2-dioxid (Verbindung Nr. I-h-2)

150 mg (0.3 mmol) 1-(2,2-Difluorethyl)-3-(2-iodphenyl)-1 H-[1,3]thiazolo[4,5-c][1,2]thiazin-4-ol-2,2-dioxid (Verbindung I-a-14) wurden in 3 ml DMF gelöst und bei RT mit 85 mg Kaliumcarbonat versetzt. Die Reaktionsmischung wurde 5 min bei RT gerührt, und anschließend wurden 0.035 ml (0.46 mmol) Propargylbromid zugetropft. Das Reaktionsgemisch wurde 4h bei 90°C gerührt, danach auf Wasser gegossen und mit Ethylacetat mehrmals extrahiert. Die vereinigten organischen Phasen wurden getrocknet (Natriumsulfat) und ins Trockne eingedampft. Der Rückstand wurde mittels präparativer HPLC (C₁₈-SiO₂, Gradient Acetonitril/Wasser 20:80 nach 100:0) gereinigt. Man erhielt 51 mg der Verbindung I-h-3.

In Analogie zu Beispiel (I-h-2) und gemäß den allgemeinen Angaben zur Herstellung erhält man folgende Verbindungen der Formel (I-h):

**Tabelle 152: Erfindungsgemäße Verbindungen der allgemeinen Formel (I), worin G für R⁸ steht:**

| | | | | | | | | |
|---|---|---|---|---|---|---|---|---|
| 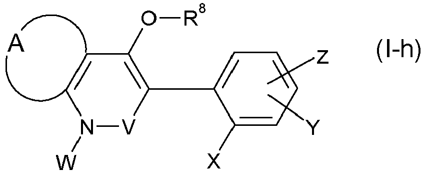 | | | | | | | | |

| Nr. | A | V | W | X | Y | Z | R⁸ | Analytische Daten |
|---|---|---|---|---|---|---|---|---|
| I-h-1 | 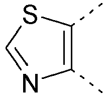 | SO₂ | CH₂CF₃ | I | H | H | CH₂-C≡CH | ¹H-NMR (400 MHz, CDCl₃): 8.88 (s, 1H); 7.97 (dd, 1H); 7.67 (dd, 1H); 7.48 (td, 1H); 7.18 (td, 1H); 4.74 (m, 2H); 4.40 (dd, 1H); 4.22 (dd, 1H); 2.56 (t, 1H) |
| I-h-2 | 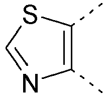 | SO₂ | CH₂CHF₂ | I | H | H | CH₂-C≡CH | ¹H-NMR (400 MHz, CDCl₃): 8.87 (s, 1H); 7.96 (dd, 1H); 7.72 (dd, 1H); 7.49 (td, 1H); 7.18 (td, 1H); 6.21 (m, 1H); 4.62 (m, 1H); 4.42 (m, 1H); 4.38 (dd, 1H); 4.22 (dd, 1H); 2.56 (t, 1H) |
| I-h-3 | 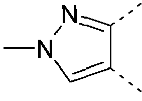 | SO₂ | CH₂CHF₂ | I | H | H | CH₂-C≡CH | ¹H-NMR (400 MHz, CDCl₃): 7.94 (dd, 1H); 7.77 (s, 1H); 7.66 (dd, 1H); 7.44 (td, 1H); 7.14 (td, 1H); 6.22 (tt, 1H); 4.32 (m, 1H); 4.33 (m, 1H); 4.19 (m, 1H); 3.94 (s, 3H); 2.55 (t, 1H) |
| I-h-4 | 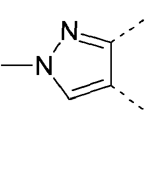 | SO₂ | CH₂CF₃ | I | H | H | CH₂-C≡CH | ¹H-NMR (400 MHz, CDCl₃): 7.94 (dd, 1H); 7.78 (s, 1H); 7.64 (dd, 1H); 7.44 (td, 1H); 7.13 (td, 1H); 4.56 (m, 1H); 4.40 (m, 1H); 4.38 (dd, 1H); 4.28 (dd, 1H); 3.95 (s, 3H); 2.55 (t, 1H) |
| I-h-5 | 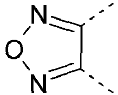 | C=O | CH₂-CH=CH₂ | Cl | 6-Cl | H | CH₂-CH=CH₂ | ¹H-NMR (400 MHz, CDCl₃): 7.42 (d, 2H), 7.30 (m, 1H), 5.94 (m, 2H), 5.27 (m, 6H), 4.76 (m, 2H) |
| I-h-6 | 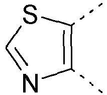 | C=O | CH₂CHF₂ | Cl | 4-Cl | H | CH₂CHF₂ | ¹H-NMR (400 MHz, CDCl₃): 8.97 (s, 1H), 7.56 (d, 1H), 7.37 (dd, 1H), 7.26 (d, 1H), 6.27 (m, 1H), 5.84 (m, 1H), 4.86 (m, 2H), 3.90 (m, 2H) |
| I-h-7 | 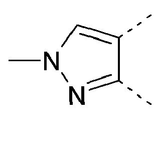 | SO₂ | CH₂CHF₂ | Cl | 6-Cl | H | CH₂-C≡CH | ¹H-NMR (400 MHz, CDCl₃): 7.79 (s, 1H); 7.44 (d, 2H); 7.33, (dd, 1H); 6.17 (tt, 1H); 4.41 (d, 2H); 4.21 (td, 2H); 3.95 (s, 3H); 2.53 (t, 1 H) |
| I-h-8 | 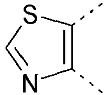 | C=O | CH₂CHF₂ | CF₃ | H | H | CH₂CHF₂ | ¹H-NMR (400 MHz, CDCl₃): 8.95 (s, 1H), 7.82 (d, 1H), 7.67 (t, 1H), 7.58 (t, 1H), 7.41 (d, 1H), 6.25 (m, 1H), 5.77 (m,1H), 4.85 (m, 2H), 3.88 (m, 2H) |
| I-h-9 | 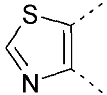 | C=O | CH₃ | I | H | H | CH₃ | ¹H-NMR (400 MHz, CDCl₃): 8.91 (s, 1H), 7.94 (d, 1H), 7.41 (t, 1H), 7.32 (d, 1H), 7.07 (t, 1H), 3.89 (s, 3H), 3.69 (s, 3H), |
| I-h-10 | 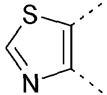 | C=O | CH₃ | CF₃ | H | H | CH₃ | ¹H-NMR (400 MHz, CDCl₃): 8.91 (s, 1H), 7.78 (d, 1H), 7.62 (t, 1H), 7.51 (t, 1H), 7.36 (d, 1H), 3.88 (s, 3H), 3.71 (s, 3H) |
| I-h-11 | 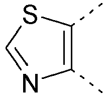 | C=O | CH₂C≡CH | CF₃ | H | H | CH₂C≡CH | ¹H-NMR (400 MHz, CDCl₃): 8.98 (s, 1H), 7.78 (d, 1H), 7.65 (t, 1H), 7.54 (t, 1H), 7.46 (d, 1H), 5.23 (m, 2H), 4.43 (m,2H), 2.51 (m, 1H), 2.22 (m, 1H) |
| I-h-12 | 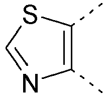 | C=O | CH₂CF₃ | CF₃ | H | H | CH₂CF₃ | ¹H-NMR (400 MHz, CDCl₃): 8.96 (s, 1H), 7.83 (d, 1H), 7.69 (t, 1H), 7.60 (t, 1H), 7.41 (d, 1H), 5.13 (m, 2H), 3.93 (m, 2H) |
| I-h-13 | 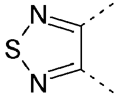 | C=O | CH₃ | Cl | 6-Cl | H | CH₃ | ¹H-NMR (400 MHz, CDCl₃): 7.42 (d, 2H), 7.29 (t, 1H), 4.27 (s, 3H), 3.78 (s, 3H) |
| I-h-14 | 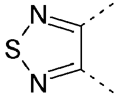 | C=O | CH₂CHF₂ | Cl | 6-Cl | H | CH₂CHF₂ | ¹H-NMR (400 MHz, CDCl₃): 7.44 (d, 2H), 7.32 (t, 1H), 6.23 (m, 1H), 5.95 (m,1H), 4.94 (m, 2H), 4.73 (m, 2H) |
| I-h-15 | 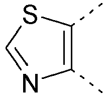 | C=O | CH₂-CH₂-SCH₃ | Cl | 6-Cl | H | CH₂-CH₂-SCH₃ | ¹H-NMR (400 MHz, CDCl₃): 8.92 (s, 1H), 7.42 (d, 2H), 7.27 (4, 1H), 4.69 (t, 2H), 4.04 (t, 2H), 2.93 (t, 2H), 2.67 (t, 2H), 2.21 (s, 3H), 2.01 (s, 3H) |

### 8. Herstellung von Verbindung Nr. II-1:

0.5 g (1.45 mmol) Methyl-4-{[(2,6-dichlorphenyl)acetyl]amino}-1,3-thiazol-5-carboxylat wurden in 10 ml Acetonitril gelöst und mit 0.46 g (2.5 eq) Dimethylsulfat sowie 0.44 g Kaliumcarbonat versetzt. Man erhitzte 2,5 h unter Rückfluss zum Sieden, befreite unter vermindertem Druck vom Lösungsmittel und nahm den Rückstand in 10 ml Wasser auf. Nach Zugabe von 10 ml Dichlormethan wurden die Phasen über eine Extraktionskartusche separiert und die organische Phase eingeengt. Chromatographische Reinigung an Kieselgel (Gradient EtOAc : n-Heptan 1:9 nach 1:1) ergab 0.40 g erfindungsgemäßer Verbindung II-1

### 9. Herstellung von Verbindung Nr. II-25:

0.75 g (2.17 mmol) Methyl-2-{[(2,6-dichlorphenyl)acetyl]amino}thiophen-3-carboxylat wurden in 7 ml THF gelöst und mit 0.1 g Natriumhydrid (60%ig) versetzt. Man ließ 10 min nachrühren und tropfte dann innerhalb von 10 min 0.7 g (1.5 eq) 2,2 Difluormethantrifluormethylsulfonat in 3 ml THFzu. Anschließend erhitzte man für 2 h zum Sieden und befreite vom Lösungsmittel. Der entstandene Rückstand wurde zwischen 10 ml Wasser und 10 ml Dichlormethan verteilt und die beiden Phasen über eine Extraktionskartusche separiert. Die organische Phase wurde eingeengt. Chromatographische Reinigung an Kieselgel (Gradient EtOAc : n-Heptan 1:9 nach 1:1) ergab 0.74 g erfindungsgemäßer Verbindung II-25.

### 10. Herstellung von Verbindung Nr. II-42:

2.0 g (9.16 mmol) Methyl-4-amino-2-methylthio-1,3-thiazol-5-carboxylat wurden in 40 ml Dioxan vorgelegt und bei 70 °C eine Lösung von 1.98 g (1.1eq) Mesitylacetylchlorid in 10 ml Dioxan zugetropft. Man erhitzte unter Rückfluss zum Sieden bis keine Gasentwicklung mehr zu beobachten war. Nach Entfernen des Lösungsmittels unter vermindertem Druck, wurde in 50ml Dichlormethan aufgenommen und mit 5%iger NaHCO₃-Lösung gewaschen, die organische Phase abgetrennt und mit Natriumsulfat getrocknet. Nach Entfernen des Lösungsmittels nahm man in Ethylacetat /n-Heptan 1:10 auf und filtrierte den entstandenen Niederschlag ab. So erhielt man 2.7 g an erfindungsgemäßer Verbindung II-42.

In Analogie zu den obengenannten Beispielen und gemäß den allgemeinen Angaben zur Herstellung erhält man folgende Verbindungen der Formel (II):

**Tabelle 153: Erfindungsgemäße Verbindungen der allgemeinen Formel (II)**

| | | | | | | | | |
|---|---|---|---|---|---|---|---|---|
| 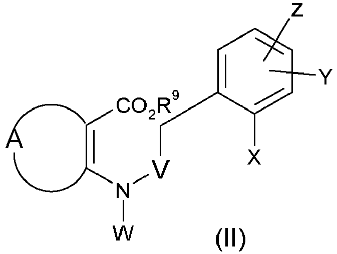 | | | | | | | | |

| Nr. | A | V | W | X | Y | Z | R⁹ | Analytische Daten |
|---|---|---|---|---|---|---|---|---|
| II-1 | 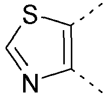 | C=O | Me | Cl | 6-Cl | H | Me | ¹H-NMR (400 MHz, CDCl₃): 8.87 (s, 1H), 7.32 (m, 2H), 7.10 (m, 1H), 3.93 (s, 3H), 3.82 (bs, 2H), 3.31 (bs, 3H) |
| II-2 | 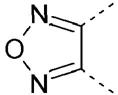 | C=O | Me | Cl | 6-Cl | H | Me | Fp: 121 - 124 °C |
| II-3 | 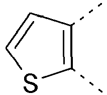 | C=O | CH₂-CH=CH₂ | Cl | 6-Cl | H | Me | ¹H-NMR (400 MHz, CDCl₃): 7.47 (d, 1H), 7.22 (d, 3H), 7.10 (m, 1H), 5.87 (m, 1H), 5.12 (m, 2H), 4.60 (m, 1H), 4.05 (m, 1H), 3.89 (s, 3H), 3.87 (dd, 2H) |
| II-4 | 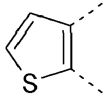 | C=O | CH₂-CH=CH₂ | Me | 4-Me | 6-Me | Me | ¹H-NMR (400 MHz, CDCl₃): 7.43 (d, 1H), 7.22 (d, 1H), 6.82 (s, 2H), 5.87 (m, 1H), 5.09 (m, 2H), 4.60 (m, 1H), 4.03 (m, 1H), 3.87 (s, 3H), 3.48 (dd, 2H) |
| II-5 | 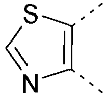 | C=O | CH₂-CH=CH₂ | Cl | 6-Cl | H | Me | ¹H-NMR (400 MHz, CDCl₃): 8.87 (s, 1H), 7.27 (m, 2H), 7.10 (m, 1H), 5.87 (m, 1H), 5.07 (m, 2H), 4.43 (m, 2H), 3.94 (s, 3H), 3.81 (bs, 2H) |
| II-6 | 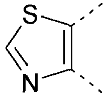 | C=O | CH₂-CH=CH₂ | Br | 4-Cl | 6-Et | Me | ¹H-NMR (400 MHz, CDCl₃): 8.89 (s, 1H), 7.38 (d, 1H), 7.12 (d, 1H), 5.85 (m, 1H), 5.07 (m, 2H), 4.43 (m, 2H), 3.94 (s, 3H), 3.65 (bs, 2H) |
| II7 | 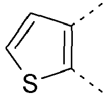 | C=O | CH₂-CH=CH₂ | Br | 4-Cl | 6-Et | Me | ¹H-NMR (400 MHz, CDCl₃): 7.45 (d, 1H), 7.40 (d, 1H), 7.23 (d, 1H), 7.12 (m, 1H), 5.86 (m, 1H), 5.13 (m, 2H), 4.58 (m, 1H), 4.04 (m, 1H), 3.89 (s, 3H), 3.71 (d, 2H) |
| II-8 | 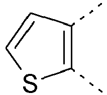 | C=O | CH₂-CH=CH₂ | Cl | 4-Cl | H | Me | ¹H-NMR (400 MHz, CDCl₃): 7.40 (d, 1H), 7.32 (d, 1H), 7.18 (m, 3H), 5.84 (m, 1H), 5.10 (m, 2H), 4.59 (m, 1H), 4.03 (m, 1H), 3.83 (s, 3H), 3.63 (dd, 2H) |
| II-9 | 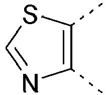 | C=O | CH₂-CH=CH₂ | Me | 4-Me | 6-Me | Me | ¹H-NMR (400 MHz, CDCl₃): 8.86 (s, 1H), 6.79 (s, 2H), 5.85 (m, 1H), 5.07 (m, 2H), 4.41 (m, 2H), 3.92 (s, 3H), 3.42 (bs, 2H) |
| II-10 | 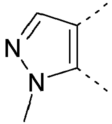 | C=O | CH₂-CH=CH₂ | Cl | 4-Cl | H | Et | ¹H-NMR (400 MHz, CDCl₃): 7.96 (s, 1H), 7.35 (d, 1H), 7.19 (dd, 1H), 7.14 (d, 1H), 5.82 (m, 1H), 5.11 (m, 2H), 4.39 (m, 1H), 4.28 (m, 2H), 4.12 (m, 1H), 3.67 (s, 3H), 3.47 (dd, 2H), 1.33 (m, 3H) |
| II-11 | 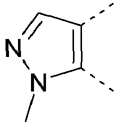 | C=O | CH₂-CH=CH₂ | Cl | 6-Cl | H | Me | ¹H-NMR (400 MHz, CDCl₃): 8.01 (s, 1H), 7.29 (d, 2H), 7.15 (t, 1H), 5.83 (m, 1H), 5.13 (m, 2H), 4.44 (m, 1H), 4.15 (m, 1H), 3.87 (s, 3H), 3.87 (d, 1H), 3.49 (d, 1H) |
| II-12 | 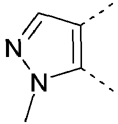 | C=O | CH₂-CH=CH₂ | Br | 4-Cl | 6-Et | Et | ¹H-NMR (400 MHz, CDCl₃): 8.01 (s, 1H), 7.41 (d, 1H), 7.15 (dd, 1H), 7.14 (d, 1H), 5.85 (m, 1H), 5.11 (m, 2H), 4.44 (m, 1H), 4.38 (m, 1H), 4.27 (m, 1H), 4.12 (m, 1H), 3.88 (s, 3H), 3.64 (d, 1H), 3.40 (d, 1H), 2.59 (m, 2H), 1.36 (t, 3H), 1.15 (t, 3H) |
| II-13 | 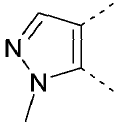 | C=O | CH₂-CH=CH₂ | Me | 4-Me | 6-Me | Et | ¹H-NMR (400 MHz, CDCl₃): 8.00 (s, 1H), 6.82 (s, 2H), 5.84 (m, 1H), 5.10 (m, 2H), 4.54 (m, 1H), 4.38 (m, 1H), 4.27 (m, 1H), 4.02 (m, 1H), 3.72 (s, 3H), 3.42 (d, 1H), 3.17 (d, 1H), 2.24 (s, 3H), 2.14 (s, 6H) |
| II-14 | 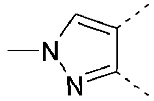 | C=O | CH₂-CH=CH₂ | Cl | 4-Cl | H | Et | ¹H-NMR (400 MHz, CDCl₃): 7.86 (s, 1H), 7.28 (d, 1H), 7.20 (dd, 1H), 7.14 (d, 1H), 5.85 (m, 1H), 5.09 (m, 2H), 4.31 (m, 2H), 4.22 (m, 2H), 3.89 (s, 3H), 3.62 (s, 2H), 1.31 (t, 3H) |
| II-15 | 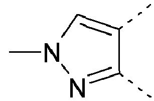 | C=O | CH₂-CH=CH₂ | Cl | 6-Cl | H | Et | ¹H-NMR (400 MHz, CDCl₃): 7.93 (s, 1H), 7.26 (d, 2H), 7.09 (dd, 1H), 5.87 (m, 1H), 5.11 (m, 2H), 4.31 (m, 4H), 3.93 (s, 3H), 3.81 (s, 2H), 1.35 (t, 3H) |
| II-16 | 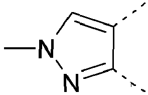 | C=O | CH₂-CH=CH₂ | Br | 4-Cl | 6-Et | Et | ¹H-NMR (400 MHz, CDCl₃): 7.93 (s, 1H), 7.38 (d, 1H), 7.10 (d, 1H), 5.86 (m, 1H), 5.11 (m, 2H), 4.29 (m, 4H), 3.93 (s, 3H), 3.66 (s, 2H), 2.60 (m, 2H), 1.34 (dt, 3H), 1.14 (t, 3H) |
| II-17 | 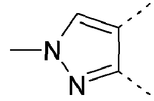 | C=O | CH₂-CH=CH₂ | Me | 4-Me | 6-Me | Et | ¹H-NMR (400 MHz, CDCl₃): 7.90 (s, 1H), 6.78 (s, 2H), 5.87 (m, 1H), 5.10 (m, 2H), 4.29 (m, 4H), 3.92 (s, 3H), 3.43 (s, 2H), 2.21 (s, 3H), 2.16 (s, 6H), 1.34 (dt, 3H) |
| II-18 | 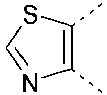 | C=O | CH₂-CHF₂ | Cl | 6-Cl | H | Me | ¹H-NMR (400 MHz, CDCl₃): 8.86 (s, 1H), 7.27 (d, 2H), 7.11 (t, 1H), 6.10 (m, 1H), 4.11 (m, 2H), 3.95 (s, 3H), 3.86 (s, 2H) |
| II-19 | 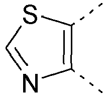 | C=O | CH₂-CHF₂ | Me | 4-Me | 6-Me | Me | ¹H-NMR (400 MHz, CDCl₃): 8.87 (s, 1H), 6.80 (s, 2H), 6.09 (m, 1H), 4.09 (m, 2H), 3.93 (s, 3H), 3.46 (s, 2H), 2.22 (s, 3H), 2.16 (s, 6H) |
| II-20 | 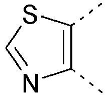 | C=O | CH₂-CHF₂ | Cl | 4-Cl | H | Me | ¹H-NMR (400 MHz, CDCl₃): 8.85 (s, 1H), 7.31 (d, 1H), 7.17 (d, 2H), 6.08 (m, 1H), 4.09 (m, 2H), 3.88 (s, 3H), 3.64 (s, 2H) |
| II-21 | 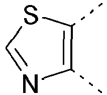 | C=O | CH₂-CHF₂ | Br | 4-Cl | 6-Et | Me | ¹H-NMR (400 MHz, CDCl₃): 8.90 (s, 1H), 7.39 (d, 1H), 7.13 (d, 2H), 6.08 (m, 1H), 4.11 (m, 2H), 3.94 (s, 3H), 3.68 (s, 2H), 2.60 (m, 2H), 1.16 (t, 3H) |
| II-22 | 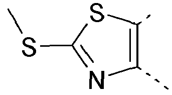 | C=O | CH₂-CHF₂ | Cl | 6-Cl | H | Et | ¹H-NMR (400 MHz, CDCl₃): 7.27 (d, 2H), 7.11 (t, 1H), 6.08 (m, 1H), 4.36 (m, 2H), 4.07 (m, 2H), 3.94 (s, 2H), 2.68 (s, 3H), 1.36 (t, 3H) |
| II-23 | 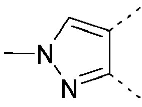 | C=O | CH₂-CHF₂ | Cl | 6-Cl | H | Et | ¹H-NMR (400 MHz, CDCl₃): 7.94 (s, 1H), 7.26 (d, 2H), 7.11 (dd, 1H), 6.08 (m, 1H), 4.31 (m, 2H), 4.05 (m, 2H), 3.94 (s, 3H), 3.85 (s, 2H), 1.34 (t, 3H) |
| II-24 | 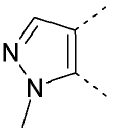 | C=O | CH₂-CHF₂ | Cl | 6-Cl | H | Et | ¹H-NMR (400 MHz, d6-DMSO): 8.04 (s, 1H), 7.48 (d, 2H), 7.32 (t, 1H), 6.23 (m, 1H), 5.13 (m, 2H), 4.23 (m, 4H), 3.89 (s, 3H), 3.87 (d, 1H), 3.54 (d, 1H), 1.28 (t, 3H) |
| II-25 | 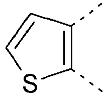 | C=O | CH₂-CHF₂ | Cl | 6-Cl | H | Me | ¹H-NMR (400 MHz, CDCl₃): 7.45 (d, 1H), 7.28 (m, 3H), 7.14 (m, 1H), 6.11 (m, 1H), 4.23 (m, 2H), 3.90 (s, 3H), 3.87 (m, 2H) |
| II-26 | 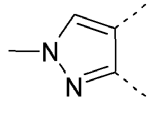 | C=O | CH₂-CHF₂ | Me | 4-Me | 6-Me | Et | ¹H-NMR (400 MHz, CDCl₃): 7.91 (s, 1H), 6.75 (s, 2H), 6.07 (m, 1H), 4.29 (m, 2H), 4.01 (breit, 3H), 3.94 (s, 3H), 3.46 (s, 2H), 1.34 (t, 3H) |
| II-27 | 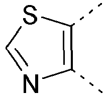 | C=O | CH₂-C≡CH | Cl | 6-Cl | H | Me | ¹H-NMR (400 MHz, CDCl₃): 8.91 (s, 1H), 7.27 (d, 2H), 7.11 (t, 1H), 4.67 (d, 2H), 3.95 (s, 3H), 3.84 (s, 2H), 2.14 (t, 1H) |
| II-28 | 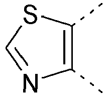 | C=O | CH₂-CF₃ | Cl | 6-Cl | H | Me | ¹H-NMR (400 MHz, CDCl₃): 8.91 (s, 1H), 7.28 (d, 2H), 7.13 (t, 1H), 4.51 (q, 2H), 3.96 (s, 3H), 3.86 (s, 2H) |
| II-29 | 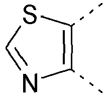 | C=O | CH₂-CF₃ | Me | 4-Me | 6-Me | Me | ¹H-NMR (400 MHz, CDCl₃): 8.91 (s, 1H), 6.80 (s, 2H), 4.48 (q, 2H), 3.94 (s, 3H), 3.47 (s, 2H), 2.26 (s, 3H), 2.14 (s, 6H) |
| II-30 | 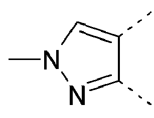 | C=O | CH₂-CHF₂ | Br | 4-Cl | 6-Et | Et | ¹H-NMR (400 MHz, CDCl₃): 7.94 (s, 1H), 7.39 (d, 1H), 7.12 (d, 1H), 6.06 (m, 1H), 4.31 (m, 2H), 4.05 (m, 2H), 3.96 (s, 3H), 3.69 (s, 2H), 2.58 (q, 2H), 1.35 (t, 3H), 1.14 (t, 3H) |
| II-31 | 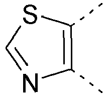 | C=O | CH₂-CF₃ | Br | 4-Cl | 6-Et | Me | ¹H-NMR (400 MHz, CDCl₃): 8.94 (s, 1H), 7.39 (d, 1H), 7.13 (d, 1H), 4.51 (q, 2H), 3.95 (s, 3H), 3.65 (s, 2H), 2.60 (m, 2H), 1.20 (m, 3H) |
| II-32 | 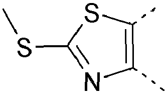 | C=O | CH₂-CHF₂ | Br | 4-Cl | 6-Et | Et | ¹H-NMR (400 MHz, CDCl₃): 7.39 (d, 1H), 7.12 (d, 1H), 6.06 (m, 1H), 4.35 (m, 2H), 4.07 (m, 2H), 3.77 (s, 2H), 2.70 (s, 3H), 2.61 (m, 2H), 1.37 (t, 3H), 1.16 (t, 3H) |
| II-33 | 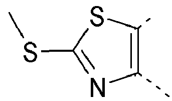 | C=O | CH₂-CHF₂ | Me | 4-Me | 6-Me | Et | ¹H-NMR (400 MHz, CDCl₃): 6.79 (s, 2H), 6.07 (m, 1H), 4.35 (m, 2H), 4.05 (m, 2H), 3.54 (s, 2H), 2.70 (s, 3H), 2.27 (s, 3H), 2.17 (s, 6H), 1.39 (m, 3H) |
| II-34 | 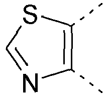 | C=O | CH₂-C≡CH | Br | 4-Cl | 6-Et | Me | ¹H-NMR (400 MHz, CDCl₃): 8.93 (s, 1H), 7.38 (d, 1H), 7.12 (d, 1H), 4.68 (s, 2H), 3.94 (s, 3H), 3.67 (s, 2H), 2.61 (m, 2H), 2.14 (s, 1H), 1.16 (m, 3H) |
| II-35 | 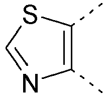 | C=O | CH₂-C≡CH | Me | 4-Me | 6-Me | Me | ¹H-NMR (400 MHz, CDCl₃): 8.90 (s, 1H), 6.79 (s, 2H), 4.64 (s, 2H), 3.92 (s, 3H), 3.46 (s, 2H), 2.22 (s, 3H), 2.16 (s, 1H), 2.13 (s, 6H) |
| II-36 | 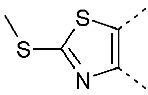 | C=O | CH₂-CF₃ | Cl | 6-Cl | H | Et | ¹H-NMR (400 MHz, CDCl₃): 7.27 (d, 2H), 7.11 (t, 1H), 4.46 (q, 2H), 4.37 (m, 2H), 3.95 (s, 2H), 2.71 (s, 3H), 1.37 (t, 3H) |
| II-37 | 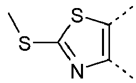 | C=O | CH₂-CF₃ | Br | 4-Cl | 6-Et | Et | ¹H-NMR (400 MHz, CDCl₃): 7.39 (d, 1H), 7.12 (d, 1H), 4.45 (q, 2H), 4.36 (m, 2H), 3.78 (s, 2H), 2.71 (s, 3H), 2.61 (m, 2H), 1.37 (t, 3H), 1.16 (t, 3H) |
| II-38 | 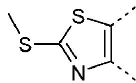 | C=O | CH₂-CF₃ | Me | 4-Me | 6-Me | Et | ¹H-NMR (400 MHz, CDCl₃): 6.79 (s, 2H), 4.43 (q, 2H), 4.35 (m, 2H), 3.58 (s, 2H), 2.71 (s, 3H), 2.22 (s, 3H), 2.17 (s, 6H), 1.36 (t, 3H) |
| II-39 | 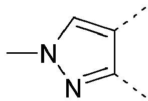 | C=O | CH₂-CF₃ | Cl | 6-Cl | H | Et | ¹H-NMR (400 MHz, CDCl₃): 7.96 (s, 1H), 7.27 (d, 2H), 7.12 (dd, 1H), 4.42 (q, 2H), 4.31 (m, 2H), 3.96 (s, 3H), 3.86 (s, 2H), 1.34 (t, 3H) |
| II-40 | 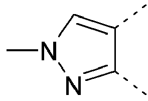 | C=O | CH₂-CF₃ | Br | 4-Cl | 6-Et | Et | ¹H-NMR (400 MHz, CDCl₃): 7.96 (s, 1H), 7.38 (d, 1H), 7.12 (d, 1H), 4.41 (q, 2H), 4.30 (m, 2H), 3.96 (s, 3H), 3.70 (s, 2H), 2.58 (q, 2H), 1.34 (t, 3H), 1.15 (t, 3H) |
| II-41 | 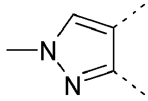 | C=O | CH₂-CF₃ | Me | 4-Me | 6-Me | Et | ¹H-NMR (400 MHz, CDCl₃): 7.93 (s, 1H), 6.79 (s, 2H), 4.40 (m, 2H), 4.29 (m, 2H), 3.96 (s, 3H), 3.46 (s, 2H), 2.22 (s, 3H), 2.14 (s, 6H), 1.33 (t, 3H) |
| II-42 | 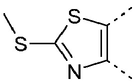 | C=O | CH₂-C≡CH | Cl | 6-Cl | H | Et | ¹H-NMR (400 MHz, CDCl₃): 7.26 (m, 2H); 7.10 (dd, 1H); 4.63 (d, 2H); 4.35 (q, 2H); 3.92 (br s, 2H); 2.70 (s, 3H); 2.14 (br s, 1H); 1.37 (t, 3H) |
| II-43 | 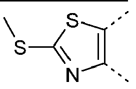 | C=O | CH₂-C≡CH | Br | 4-Cl | 6-Et | Et | ¹H-NMR (400 MHz, CDCl₃): 7.38 (d, 1H); 7.12 (d, 1H); 4.61 (d, 2H); 4.35 (q, 2H); 3.76 (br s, 2H); 2.71 (s, 3H); 2.62 (q, 2H); 2.14 (br s, 1H); 1.37 (t, 3H); 1.16 (t, 3H) |
| II-44 | 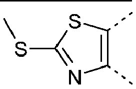 | C=O | CH₂-C≡CH | Me | 4-Me | 6-Me | Et | ¹H-NMR (400 MHz, CDCl₃): 6.78 (s, 2H); 4.60 (d, 2H); 4.34 (q, 2H); 3.54 (br s, 2H); 2.71 (s, 3H); 2.22 (s, 3H); 2.17 (s, 6H); 2.13 (br s, 1H); 1.36 (t, 3H) |
| II-45 | 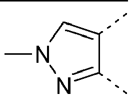 | C=O | CH₂-C≡CH | Cl | 6-Cl | H | Et | ¹H-NMR (400 MHz, CDCl₃): 7.95 (s, 1H); 7.25 (m, 2H); 7,14 (t, 1H); 4.59 (d, 2H); 4.29 (q, 2H); 3.96 (s, 3H); 3.84 (s, 2H); 2.15 (t, 1H); 1.34 (t, 3H) |
| II-46 | 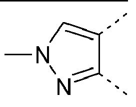 | C=O | CH₂-C≡CH | Br | 4-Cl | 6-Et | Et | ¹H-NMR (400 MHz, CDCl₃): 7.96 (s, 1H); 7.37 (d, 1H); 7.11 (d, 1H); 4.59 (d, 2H); 4.29 (q, 2H); 3.97 (s, 3H); 3.69 (s, 2H); 2.60 (q, 2H); 2.15 (t, 1H); 1.34 (t, 3H); 1.14 (t, 3H) |
| II-47 | 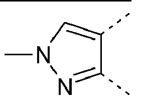 | C=O | CH₂-C≡CH | Me | 4-Me | 6-Me | Et | ¹H-NMR (400 MHz, CDCl₃): 7.92 (s, 1H); 6.77 (s, 2H); 4.57 (br s, 2H); 4.28 (q, 2H); 3.95 (s, 3H); 3.46 (s, 2H); 2.21 (s, 3H); 2.15 (s, 6H); 2.14 (t, 1H); 1.33 (t, 3H) |
| II-48 | 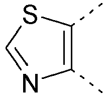 | C=O | CH₂-c-Pr | Cl | 6-Cl | H | Me | ¹H-NMR (400 MHz, CDCl₃): 8.88 (s, 1H); 7.26 (m, 2H); 7.10 (t, 1H); 3.94 (s, 3H); 3.81 (s, 2H); 3.71 (d, 2H); 0.98 (m, 1H); 0.37 (m, 2H); 0.03 (m, 2H) |
| II-49 | 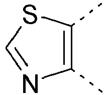 | C=O | CH₂-C≡CH | Cl | 4-Cl | 6-Me | Me | ¹H-NMR (400 MHz, CDCl₃): 8.91 (s, 1H); 7.18 (s, 1H); 7.06 (d, 1H); 4.65 (d, 2H); 3.93 (s, 3H); 3.64 (s, 2H); 2.27 (s, 3H); 2.15 (br s, 1H) |
| II-50 | 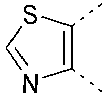 | C=O | CH₂-C≡CH | Me | 3-Br | 6-Me | Me | ¹H-NMR (400 MHz, CDCl₃): 8.92 (s, 1H); 7.32 (d, 1H); 6.83 (d, 1H); 4.64 (s, 2H); 3.94 (s, 3H); 3.56 (s, 2H); 2.30 (s, 3H); 2.16 (s, 4H) |
| II-51 | 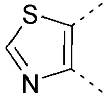 | C=O | CH₂-CHF₂ | Cl | 4-Cl | 6-Et | Me | ¹H-NMR (400 MHz, CDCl₃): 8.90 (s, 1H); 7.20 (br s, 1H); 7.09 (d, 1H); 6.07 (tt, 1H); 4.10 (td, 2H); 3.94 (s, 3H); 3.65 (s, 2H); 2.59 (q, 2H); 1.16 (t, 3H) |
| II-52 | 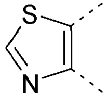 | C=O | CH₂-CHF₂ | Cl | 4-Cl | 6-Me | Me | ¹H-NMR (400 MHz, CDCl₃): 8.88 (s, 1H); 7.19 (s, 1H); 7.07 (s, 1H); 6.08 (tt, 1H); 4.10 (m, 2H); 3.94 (s, 3H); 3.65 (s, 2H); 2.27 (s, 3H) |
| II-53 | 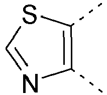 | C=O | CH₂-CHF₂ | Me | 3-Br | 6-Me | Me | ¹H-NMR (400 MHz, CDCl₃): 8.90 (s, 1H); 7.33 (d, 1H); 6.84 (d, 1H); 6.08 (tt, 1H); 4.09 (td, 2H); 3.95 (s, 3H); 3.57 (s, 2H); 2.29 (s, 3H); 2.15 (s, 3H) |
| II-54 | 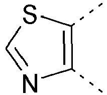 | C=O | CH₂-C≡CH | Cl | 4-Cl | 6-Et | Me | ¹H-NMR (400 MHz, CDCl₃): 8.93 (s, 1H); 7.20 (d, 1H); 7.08 (d, 1H); 4.66 (d, 2H); 3.94 (s, 3H); 3.63 (s, 2H); 2.60 (q, 2H); 2.14 (br s, 1H); 1.16 (t, 3H) |
| II-55 | 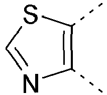 | C=O | CH₂-C≡CH | Me | 4-Cl | 6-CF₃ | Me | ¹H-NMR (400 MHz, CDCl₃): 8.96 (s, 1H); 7.46 (s, 1H); 7.36 (s, 1H); 4.64 (d, 2H); 3.95 (s, 3H); 3.58 (s, 2H); 2.31 (s, 3H); 2.14 (br s, 1H) |
| II-56 | 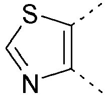 | C=O | CH₂-CHF₂ | Me | 4-Cl | 6-CF₃ | Me | ¹H-NMR (400 MHz, CDCl₃): 8.94 (s, 1H); 7.47 (d, 1H); 7.37 (s, 1H); 6.05 (tt, 1H); 4.10 (td, 2H); 3.95 (s, 3H); 3.60 (s, 2H); 2.30 (s, 3H) |
| II-57 | 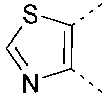 | C=O | CH₂-C≡CH | Et | 4-Cl | 6-OMe | Me | ¹H-NMR (400 MHz, CDCl₃): 8.82 (s, 1H); 6.77 (d, 1H); 6.60 (d, 1H); 4.63 (d, 2H); 3.92 (s, 3H); 3.70 (s, 3H); 3.52 (s, 2H); 2.54 (q, 2H); 2.12 (br s, 1H); 1.14 (t, 3H) |
| II-58 | 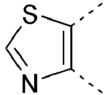 | C=O | CH₂-CHF₂ | Et | 4-Cl | 6-OMe | Me | ¹H-NMR (400 MHz, CDCl₃): 8.78 (s, 1H); 6.77 (d, 1H); 6.60 (d, 1H); 6.08 (tt, 1H); 4.07 (td, 2H); 3.93 (s, 3H); 3.70 (s, 3H); 3.53 (s, 2H); 2.53 (q, 2H); 1.14 (t, 3H) |
| II-59 | 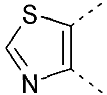 | C=O | CH₂-C≡CH | Cl | 4-Br | 6-Et | Me | ¹H-NMR (400 MHz, CDCl₃): 8.92 (s, 1H); 7.35 (d, 1H); 7.23 (d, 1H); 4.65 (d, 2H); 3.93 (s, 3H); 3.63 (s, 2H); 2.60 (q, 2H); 2.14 (br s, 1H); 1.16 (t, 3H) |
| II-60 | 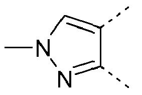 | C=O | CH₂-C≡CH | Cl | 4-Cl | 6-Et | Et | ¹H-NMR (400 MHz, CDCl₃): 7.96 (s, 1H); 7.19 (d, 1H); 7.06 (d, 1H); 4.58 (s, 2H); 4.29 (q, 2H); 3.97 (s, 3H); 3.64 (s, 2H); 2.58 (q, 2H); 2.16 (t, 1H); 1.34 (t, 3H); 1.15 (t, 3H) |
| II-61 | 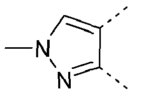 | C=O | CH₂-CHF₂ | Cl | 4-Cl | 6-Et | Et | ¹H-NMR (400 MHz, CDCl₃): 7.94 (s, 1H); 7.21 (d, 1H); 7.07 (d, 1H); 6.06 (tt, 1H); 4.31 (q, 2H); 4.02 (td, 2H); 3.95 (s, 3H); 3.65 (s, 2H); 2.57 (q, 2H); 1.34 (t, 3H); 1.15 (t, 3H) |
| II-62 | 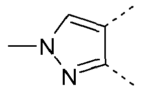 | C=O | CH₃ | Cl | 4-Cl | 6-Me | Et | ¹H-NMR (400 MHz, CDCl₃): 7.92 (s, 1H); 7.17 (d, 1H); 7.04 (s, 1H); 4.28 (q, 2H); 3.93 (s, 3H); 3.62 (s, 2H); 3.24 (s, 3H); 2.25 (s, 3H); 1.33 (t, 3H) |
| II-63 | 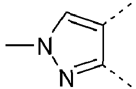 | C=O | CH₂-CF₃ | Cl | 4-Cl | 6-Me | Et | ¹H-NMR (400 MHz, CDCl₃): 7.93 (s, 1H); 7.19 (s, 1H); 7.05 (s, 1H); 6.06 (tt, 1H); 4.29 (q, 2H); 4.02 (br s, 2H); 3.95 (s, 3H); 3.66 (s, 2H); 2.24 (s, 3H); 1.34 (t, 3H) |
| II-64 | 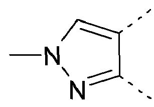 | C=O | CH₃ | Cl | 4-Cl | 6-Et | Et | ¹H-NMR (400 MHz, CDCl₃): 7.95 (s, 1H); 7.19 (d, 1H); 7.06 (d, 1H); 4.40 (brs, 1H); 4.30 (q, 2H); 3.96 (s, 3H); 3.67 (s, 2H); 2.24 (s, 3H); 1.34 (t, 3H) |
| II-65 | 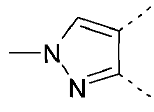 | C=O | CH₂-C≡CH | Cl | 4-Cl | 6-Me | Et | ¹H-NMR (400 MHz, CDCl₃): 7.94 (s, 1H); 7.18 (d, 1H); 7.04 (s, 1H); 4.57 (br s, 2H); 4.28 (q, 2H); 3.96 (s, 3H); 3.65 (s, 2H); 2.25 (s, 3H); 2.16 (t, 1H); 1.33 (t, 3H) |
| II-66 | 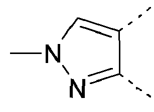 | C=O | CH₂-CHF₂ | Cl | 4-Cl | 6-Me | Et | ¹H-NMR (400 MHz, CDCl₃): 7.94 (s, 1H); 7.19 (d, 1H); 7.06 (d, 1H); 4.29 (q, 2H); 3.94 (s, 3H); 3.62 (s, 2H); 3.24 (s, 3H); 2.58 (q, 2H); 1.34 (t, 3H); 1.15 (t, 3H) |
| II-67 | 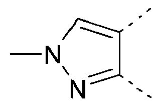 | C=O | CH₂-CHF₂ | Me | 4-CF₂-CF₃ | 6-Me | Et | ¹H-NMR (400 MHz, CDCl₃): 7.92 (s, 1H); 7.19 (s, 2H); 6.07 (tt, 1H); 4.31 (q, 2H); 4.02 (br s, 1H); 3.95 (s, 3H); 3.56 (s, 2H); 2.26 (s, 6H); 1.35 (t, 3H) |
| II-68 | 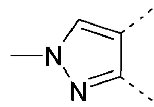 | C=O | CH₂-CHF₂ | Cl | 3-Br | 6-Cl | Et | ¹H-NMR (400 MHz, CDCl₃): 7.95 (s, 1H); 7.47 (d, 1H); 7.16 (d, 1H); 6.08 (tt, 1H); 4.31 (q, 2H); 4.03 (br t, 2H); 3.96 (s, 3H); 3.92 (s, 2H); 3.41 (brt, 1H); 1.35 (t, 3H) |
| II-69 | 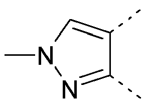 | C=O | CH₂-CF₃ | Me | 4-CF₂-CF₃ | 6-Me | Et | ¹H-NMR (400 MHz, CDCl₃): 7.94 (s, 1H); 7.19 (s, 2H); 4.40 (br s, 2H); 4.31 (q, 2H); 3.96 (s, 3H); 3.57 (s, 2H); 2.25 (s, 6H); 1.34 (t, 3H) |
| II-70 | 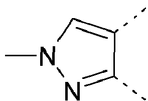 | C=O | CH₃ | Me | 4-CF₂-CF₃ | 6-Me | Et | ¹H-NMR (400 MHz, CDCl₃): 7.91 (s, 1H); 7.17 (s, 2H); 4.30 (q, 2H); 3.93 (s, 3H); 3.52 (s, 2H); 3.24 (s, 3H); 2.26 (s, 6H); 1.34 (t, 3H) |
| II-71 | 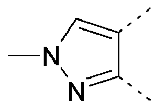 | C=O | CH₂-CF₃ | Cl | 3-Br | 6-Cl | Et | ¹H-NMR (400 MHz, CDCl₃): 7.97 (s, 1H); 7.47 (d, 1H); 7.16 (d, 1H); 4.42 (br s, 2H); 4.31 (q, 2H); 3.97 (s, 3H); 3.93 (s, 2H); 1.35 (t, 3H) |
| II-72 | 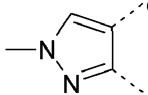 | C=O | CH₃ | Cl | 3-Br | 6-Cl | Et | ¹H-NMR (400 MHz, CDCl₃): 7.95 (s, 1H); 7.44 (d, 1H); 7.14 (d, 1H); 4.30 (q, 2H); 3.94 (s, 3H); 3.88 (s, 2H); 3.25 (s, 3H); 1.34 (t, 3H) |
| II-73 | 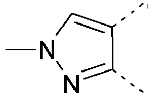 | C=O | CH₃-C≡CH | Cl | 3-Br | 6-Cl | Et | ¹H-NMR (400 MHz, CDCl₃): 7.96 (s, 1H); 7.45 (d, 1H); 7.14 (d, 1H); 4.59 (br s, 2H); 4.30 (q, 2H); 3.97 (s, 3H); 3.91 (s, 2H); 2.17 (t, 1H); 1.34 (t, 3H) |
| II-74 | 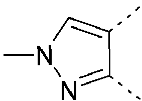 | C=O | CH₃ | F | 3-Me | 6-Cl | Et | ¹H-NMR (400 MHz, CDCl₃): 7.90 (s, 1H); 6.98 (m, 2H); 4.29 (q, 2H); 3.92 (s, 3H); 3.64 (s, 2H); 3.24 (s, 3H); 2.20 (s, 3H); 1.33 (t, 3H) |
| II-75 | 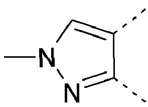 | C=O | CH₂-C≡CH | F | 3-Me | 6-CI | Et | ¹H-NMR (400 MHz, CDCl₃): 7.92 (s, 1H); 6.99 (m, 2H); 4.57 (d, 2H); 4.28 (q, 2H); 3.95 (s, 3H); 3.68 (s, 2H); 2.20 (d, 3H); 2.15 (t, 1H); 1.33 (t, 3H) |
| II-76 | 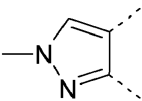 | C=O | CH₂-CF₃ | F | 3-Me | 6-Cl | Et | ¹H-NMR (400 MHz, CDCl₃): 7.93 (s, 1H); 7.00 (m, 2H); 4.41 (br q, 2H); 4.30 (q, 2H); 3.95 (s, 3H); 3.70 (s, 2H); 2.20 (d, 3H); 1.34 (t, 3H) |
| II-77 | 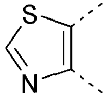 | C=O | CH₃ | I | H | H | Me | ¹H-NMR (400 MHz, CDCl₃): 8.82 (s, 1H); 7.74 (d, 1H); 7.28 (m, 2H); 6.88 (t, 1H); 3.85 (s, 3H); 3.68 (br s, 2H); 3.32 (s, 3H) |
| II-78 | 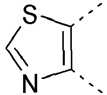 | C=O | CH₂-CHF₂ | Cl | 3-c-Pr | 6-Cl | Me | ¹H-NMR (400 MHz, CDCl₃): 8.86 (s, 1H); 7.16 (d, 1H); 6.80 (d, 1H); 6.11 (tt, 1H); 4.12 (td, 2H); 3.95 (s, 3H); 3.89 (s, 2H); 2.10 (m, 1H); 0.99 (m, 2H); 0.63 (m, 2H) |
| II-79 | 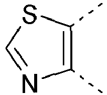 | C=O | CH₂-CHF₂ | Cl | 3-Br | 6-Cl | Me | ¹H-NMR (400 MHz, CDCl₃): 8.88 (s, 1H); 7.47 (d, 1H); 7.16 (d, 1H); 6.09 (tt, 1H); 4.11 (td, 2H); 3.95 (s, 3H); 3.91 (s, 2H) |
| II-80 | 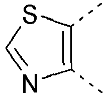 | C=O | CH₂-C≡CH | CF₃ | H | H | Me | ¹H-NMR (400 MHz, CDCl₃): 8.89 (s, 1H); 7.58 (d, 1H); 7.49 (t, 1H); 7.41 (d, 1H); 7.33 (t, 1H); 4.65 (d, 2H); 3.90 (s, 3H); 3.68 (br s, 2H); 2.16 (br s, 1H) |
| II-81 | 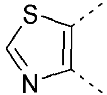 | C=O | CH₃ | Cl | 3-Me | 6-Cl | Me | ¹H-NMR (400 MHz, CDCl₃): 8.86 (s, 1H); 7.16 (d, 1H); 7.05 (d, 1H); 3.94 (s, 3H); 3.82 (s, 2H); 3.32 (s, 3H); 2.32 (s, 3H) |
| II-82 | 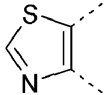 | C=O | CH₂-CHF₂ | Cl | 3-Me | 6-Cl | Me | ¹H-NMR (400 MHz, CDCl₃): 8.86 (s, 1H); 7.17 (d, 1H); 7.06 (d, 1H); 6.10 (tt, 1H); 4.11 (td, 2H); 3.95 (s, 3H); 3.87 (s, 2H); 2.32 (s, 3H) |
| II-83 | 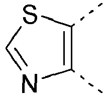 | C=O | CH₂-CHF₂ | Cl | 3-I | 6-Cl | Me | ¹H-NMR (400 MHz, CDCl₃): 8.87 (s, 1H); 7.69 (d, 1H); 7.01 (d, 1H); 6.09 (tt, 1H); 4.11 (td, 2H); 3.95 (s, 5H) |
| II-84 | 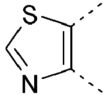 | C=O | CH₂-CF₃ | Me | 3-Br | 6-Cl | Me | ¹H-NMR (400 MHz, CDCl₃): 8.92 (s, 1H); 7.38 (d, 1H); 7.05 (d, 1H); 4.49 (q, 2H); 3.95 (s, 3H); 3.79 (s, 2H); 2.38 (s, 3H) |
| II-85 | 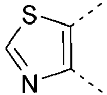 | C=O | CH₂-CHF₂ | Me | 3-Br | 6-Cl | Me | ¹H-NMR (400 MHz, CDCl₃): 8.88 (s, 1H); 7.38 (d, 1H); 7.04 (d, 1H); 6.08 (tt, 1H); 4.10 (td, 2H); 3.94 (s, 3H); 3.78 (s, 2H); 2.38 (s, 3H) |
| II-86 | 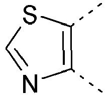 | C=O | CH₂-CHF₂ | Me | 4-Br | 6-Et | Me | ¹H-NMR (400 MHz, CDCl₃): 8.91 (s, 1H); 7.14 (s, 2H); 6.08 (tt, 1H); 4.08 (td, 2H); 3.95 (s, 3H); 3.45 (s, 2H); 2.48 (q, 2H; 2.17 (s, 3H); 1.10 (t, 3H) |
| II-87 | 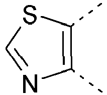 | C=O | CH₃ | Me | 4-Br | 6-Me | Me | ¹H-NMR (400 MHz, CDCl₃): 8.89 (s, 1H); 7.12 (s, 2H); 3.93 (s, 3H); 3.40 (s, 2H); 3.29 (s, 3H); 2.17 (s, 6H) |
| II-88 | 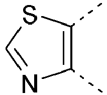 | C=O | CH₃ | Me | 4-Br | 6-Et | Me | ¹H-NMR (400 MHz, CDCl₃): 8.89 (s, 1H); 7.13 (s, 2H); 3.94 (s, 3H); 3.41 (s, 2H); 3.29 (s, 3H); 2.49 (m, 2H); 2.18 (s, 3H); 1.11 (t, 3H) |
| II-89 | 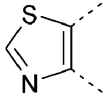 | C=O | CH₂-CHF₂ | Me | 4-Br | 6-Me | Me | ¹H-NMR (400 MHz, CDCl₃): 8.90 (s, 1H); 7.13 (s, 2H); 6.08 (tt, 1H); 4.08 (td, 2H); 3.94 (s, 3H); 3.45 (s, 2H); 2.16 (s, 6H) |
| II-90 | 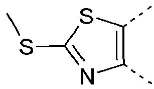 | C=O | CH₃ | Cl | 6-Cl | H | Et | ¹H-NMR (400 MHz, CDCl₃): 7.26 (m, 2H); 7.10 (t, 1H); 4.35 (q, 2H); 3.88 (s, 2H); 3.27 (s, 3H); 2.70 (s, 3H); 1.36 (t, 3H) |
| II-91 | 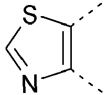 | C=O | CH₂-CHF₂ | Me | 4-CF₂-CF₃ | 6-Me | Me | ¹H-NMR (400 MHz, CDCl₃): 8.89 (s, 1H); 7.19 (s, 2H); 6.08 (tt, 1H); 4.09 (td, 2H); 3.94 (s, 3H); 3.56 (s, 2H); 2.25 (s, 6H) |
| II-92 | 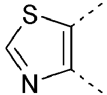 | C=O | CH₃ | I | 4-Cl | 6-Et | Me | ¹H-NMR (400 MHz, CDCl₃): 8.90 (s, 1H); 7.65 (d, 1H); 7.15 (d, 1H); 3.94 (s, 3H); 3.68 (s, 2H); 3.32 (s, 3H); 2.61 (q, 2H); 1.15 (t, 3H) |
| II-93 | 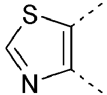 | C=O | CH₂-CHF₂ | I | 4-Cl | 6-Et | Me | ¹H-NMR (400 MHz, CDCl₃): 8.92 (s, 1H); 7.65 (d, 1H); 7.16 (d, 1H); 6.08 (tt, 1H); 4.12 (td, 2H); 3.95 (s, 3H); 3.72 (s, 2H); 2.61 (q, 2H); 1.15 (t, 3H) |
| II-94 | 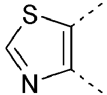 | C=O | CH₂-C≡CH | Me | 4-CF₂-CF₃ | 6-Me | Me | ¹H-NMR (400 MHz, CDCl₃): 8.91 (s, 1H); 7.19 (s, 2H); 4.65 (d, 2H); 3.93 (s, 3H); 3.55 (s, 2H); 2.27 (s, 6H); 2.15 (br s, 1H) |
| II-95 | 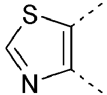 | C=O | CH₃ | Me | 4-CF₂-CF₃ | 6-Me | Me | ¹H-NMR (400 MHz, CDCl₃): 8.87 (s, 1H); 7.18 (s, 2H); 3.93 (s, 3H); 3.52 (s, 2H); 3.31 (s, 3H); 2.27 (s, 6H) |
| II-96 | 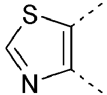 | C=O | CH₂-C≡CH | I | 4-Cl | 6-Et | Me | ¹H-NMR (400 MHz, CDCl₃): 8.94 (s, 1H); 7.65 (d, 1H); 7.15 (d, 1H); 4.67 (d, 2H); 3.94 (s, 3H); 3.71 (s, 2H); 2.62 (q, 2H); 2.14 (br s, 1H); 1.15 (t, 3H) |
| II-97 | 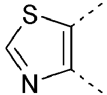 | C=O | CH₃ | F | 4-Me | 6-Me | Me | ¹H-NMR (400 MHz, CDCl₃): 8.81 (s, 1H); 6.73 (s, 1H); 6.59 (d, 1H); 3.89 (s, 3H); 3.51 (s, 2H); 3.28 (s, 3H); 2.24 (s, 3H); 2.20 (s, 3H) |
| II-98 | 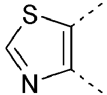 | C=O | CH₂-CHF₂ | F | 4-Me | 6-Me | Me | ¹H-NMR (400 MHz, CDCl₃): 8.81 (s, 1H); 6.74 (s, 1H); 6.60 (d, 1H); 6.09 (tt, 1H); 4.07 (td, 2H); 3.91 (s, 3H); 3.54 (s, 2H); 2.24 (s, 3H); 2.20 (s, 3H) |
| II-99 | 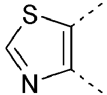 | C=O | CH₂-CHF₂ | Br | 4-Br | 6-Me | Me | ¹H-NMR (400 MHz, CDCl₃): 8.89 (s, 1H); 7.54 (d, 1H); 7.26 (m, 1H); 6.08 (tt, 1H); 4.10 (td, 2H); 3.94 (s, 3H); 3.68 (s, 2H); 2.28 (s, 3H) |
| II-100 | 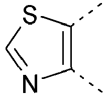 | C=O | CH₃ | Br | 4-Br | 6-Me | Me | ¹H-NMR (400 MHz, CDCl₃): 8.88 (s, 1H); 7.52 (br s, 1H); 7.25 (d, 1H); 3.93 (s, 3H); 3.64 (s, 2H); 3.30 (s, 3H); 2.29 (s, 3H) |
| II-101 | 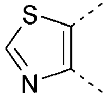 | C=O | CH₃ | Br | 4-Br | 6-Et | Me | ¹H-NMR (400 MHz, CDCl₃): 8.89 (s, 1H); 7.53 (m, 1H); 7.26 (m, 1H); 3.93 (s, 3H); 3.64 (s, 2H); 3.31 (s, 3H); 2.61 (q, 2H); 1.16 (s, 3H) |
| II-102 | 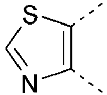 | C=O | CH₂-CHF₂ | Br | 4-Br | 6-Et | Me | ¹H-NMR (400 MHz, CDCl₃): 8.91 (s, 1H); 7.53 (d, 1H); 7.27 (d, 1H); 6.08 (tt, 1H); 4.11 (td, 2H); 3.94 (s, 3H); 3.67 (s, 2H); 2.60 (q, 2H); 1.16 (t, 3H) |
| II-103 | 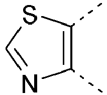 | C=O | CH₂-C≡CH | Br | 4-Br | 6-Et | Me | ¹H-NMR (400 MHz, CDCl₃): 8.93 (s, 1H); 7.53 (d, 1H); 7.27 (d, 1H); 4.66 (d, 2H); 3.94 (s, 3H); 3.66 (s, 2H); 2.61 (q, 2H); 2.14 (br s, 1H); 1.16 (t, 3H) |
| II-104 | 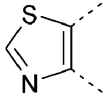 | C=O | CH₂-C≡CH | Br | 4-Br | 6-Me | Me | ¹H-NMR (400 MHz, CDCl₃): 8.92 (s, 1H); 7.51 (m, 1H); 7.25 (m, 1H); 4.65 (d, 2H); 3.93 (s, 3H); 3.66 (s, 2H); 2.29 (s, 3H); 2.15 (br s, 1H) |
| II-105 | 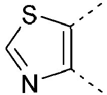 | C=O | CH₂-CHF₂ | Me | 4-(2'-c-Pr)-c-Pr | 6-Me | Me | ¹H-NMR (400 MHz, CDCl₃): 8.88 (s, 1H); 6.64 (br s, 2H); 6.09 (br t, 1H); 4.07 (br t, 2H); 3.93 (s, 3H); 3.44 (s, 2H); 2.14 (s, 6H); 1.53 (m, 1H); 1.07 (m, 1H); 0.89 (m, 1H); 0.73 (m, 1H); 0.67 (m, 1H); 0.38 (m, 2H); 0.12 (m, 2H) |
| II-106 | 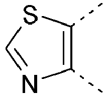 | C=O | CH₃ | Me | 4-(2'-c-Pr)-c-Pr | 6-Me | Me | ¹H-NMR (400 MHz, CDCl₃): 8.86 (s, 1H); 6.63 (br s, 2H); 3.92 (s, 3H); 3.41 (br s, 2H); 3.28 (s, 3H); 2.15 (s, 6H); 1.53 (m, 1H); 1.07 (m, 1H); 0.89 (m, 1H); 0.73 (m, 1H); 0.66 (m, 1H); 0.38 (m, 2H); 0.12 (m, 2H) |
| II-107 | 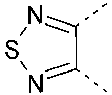 | C=O | CH₃ | Cl | 6-Cl | H | Me | ¹H-NMR (400 MHz, CDCl₃): 7.30 (m, 2H); 7.16 (m, 1H); 4.29-3.27 (m, 8H) |
| II-108 | 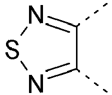 | C=O | CH₃ | Br | 4-Cl | 6-Et | Me | ¹H-NMR (400 MHz, CDCl₃): 7.42 (br s, 1H); 7.15 (br s, 1H); 4.11 - 3.26 (m, 8H); 2.63 (m, 2H); 1.21 (m, 3H) |
| II-109 | 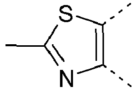 | C=O | CH₂-CHF₂ | Cl | 6-Cl | H | Me | ¹H-NMR (400 MHz, CDCl₃): 7.26 (d, 2H); 7.11 (dd, 1H); 6.09 (tt, 1H); 4.07 (td, 2H); 3.91 (s, 3H); 3.90 (s, 2H); 2.70 (s, 3H) |
| II-110 | 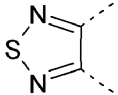 | C=O | CH₂-CHF₂ | Br | 4-Cl | 6-Et | Me | ¹H-NMR (400 MHz, CDCl₃): 7.40 (br s, 1H); 7.14 (br s, 1H); 6.07 (br t, 1H);4.12 (m, 2H); 4.04 (s, 3H); 3.60 (s, 2H); 2.60 (q, 2H); 1.17 (t, 3H) |
| II-111 | 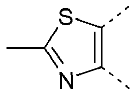 | C=O | CH₃ | Cl | 6-Cl | H | Me | ¹H-NMR (400 MHz, CDCl₂): 7.26 (d, 2H); 7.10 (t, 1H); 3.90 (s, 3H); 3.84 (s, 2H); 3.28 (s, 3H); 2.71 (s, 3H) |
| II-112 | 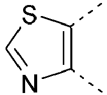 | C=O | CH₃ | Br | 4-Br | 6-OCF₃ | Me | ¹H-NMR (400 MHz, CDCl₃): 8.87 (s, 1H); 7.65 (d, 1H); 7.35 (br s, 1H); 3.93 (s, 3H); 3.65 (s, 2H); 3.31 (s, 3H) |
| II-113 | 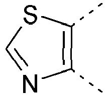 | C=O | CH₂-CHF₂ | Br | 4-Br | 6-OCF₃ | Me | ¹H-NMR (400 MHz, CDCl₃): 8.87 (s, 1H); 7.66 (d, 1H); 7.35 (m, 1H); 6.07 (tt, 1H); 4.10 (td, 2H); 3.94 (s, 3H); 3.70 (s, 2H) |
| II-114 | 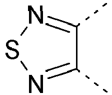 | C=O | CH₂-C≡CH | Br | 4-Cl | 6-Et | Me | ¹H-NMR (400 MHz, CDCl₃): 7.41 (br s, 1H); 7.15 (br s, 1H); 4.70 (br s, 2H); 3.99 (br s, 3H); 3.61 (br s, 2H); 2.63 (m, 2H); 2.17 (br s, 1H); 1.19 (br s, 3H) |
| II-115 | 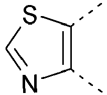 | C=O | CH₃ | Cl | 4-Br | 6-OCF₃ | Me | ¹H-NMR (400 MHz, CDCl₃): 8.87 (s, 1H); 7.48 (d, 1H); 7.34 (d, 1H); 3.93 (s, 3H); 3.62 (s, 2H); 3.30 (s, 3H) |
| II-116 | 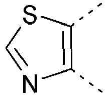 | C=O | CH₂-CHF₂ | Cl | 4-Br | 6-OCF₃ | Me | ¹H-NMR (400 MHz, CDCl₃): 8.87 (s, 1H); 7.48 (d, 1H); 7.31 (m, 1H); 6.07 (tt, 1H); 4.09 (td, 2H); 3.94 (s, 3H); 3.67 (s, 2H) |
| II-117 | 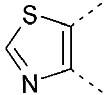 | C=O | CH₂-CHF₂ | Br | 4-Cl | 6-OCF₃ | Me | ¹H-NMR (400 MHz, CDCl₃): 8.88 (s, 1H); 7.51 (d, 1H); 7.22 (m, 1H); 6.08 (tt, 1H); 4.10 (td, 2H); 3.94 (s, 3H); 3.71 (s, 2H) |
| II-118 | 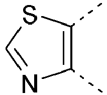 | C=O | CH₃ | Br | 4-Cl | 6-OCF₃ | Me | ¹H-NMR (400 MHz, CDCl₃): 8.88 (s, 1H); 7.51 (d, 1H); 7.21 (br s, 1H); 3.93 (s, 3H); 3.66 (s, 2H); 3.31 (s, 3H) |
| II-119 | 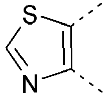 | C=O | CH₂-CHF₂ | F | 6-CF₃ | H | Me | ¹H-NMR (400 MHz, CDCl₃): 8.93 (s, 1H); 7.44 (d, 1H); 7.37 (q, 1H); 7.26 (m, 1H); 6.07 (tt, 1H); 4.11 (td, 2H); 3.96 (s, 3H); 3.68 (s, 2H) |
| II-120 | 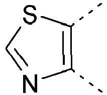 | C=O | CH₂-C≡CH | F | 6-CF₃ | H | Me | ¹H-NMR (400 MHz, CDCl₃): 8.95 (s, 1H); 7.43 (d, 1H); 7.35 (q, 1H); 7.25 (t, 1H); 4.65 (d, 2H); 3.95 (s, 3H); 3.65 (s, 2H); 2.14 (br s, 1H) |
| II-121 | 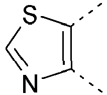 | C=O | CH₃ | F | 6-CF₃ | H | Me | ¹H-NMR (400 MHz, CDCl₃): 8.91 (s, 1H); 7.43 (d, 1H); 7.35 (q, 1H); 7.25 (t, 1H); 3.95 (s, 3H); 3.63 (s, 2H); 3.31 (s, 3H) |
| II-122 | 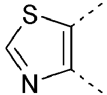 | C=O | CH₂-CHF₂ | Cl | 6-CF₃ | H | Me | ¹H-NMR (400 MHz, CDCl₃): 8.95 (s, 1H); 7.58 (dd, 2H); 7.33 (t, 1H); 6.07 (tt, 1H); 4.12 (td, 2H); 3.96 (s, 3H); 3.80 (s, 2H) |
| II-123 | 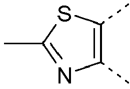 | C=O | CH₃ | Cl | 4-Cl | 6-Et | Me | ¹H-NMR (400 MHz, CDCl₃): 7.19 (d, 1H); 7.07 (d, 1H); 3.89 (s, 3H); 3.65 (s, 2H); 3.26 (s, 3H); 2.72 (s, 3H); 2.60 (q, 2H); 1.16 (t, 3H) |
| II-124 | 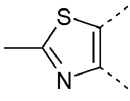 | C=O | CH₂-C≡CH | Cl | 4-Cl | 6-Et | Me | ¹H-NMR (400 MHz, CDCl₃): 7.19 (d, 1H); 7.08 (d, 1H); 4.61 (d, 1H); 3.89 (s, 3H); 3.68 (s, 2H); 2.75 (s, 3H); 2.60 (q, 2H); 2.14 (br s, 1H); 1.16 (t, 3H) |
| II-125 | 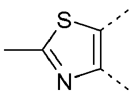 | C=O | CH₂-CHF₂ | Cl | 4-Cl | 6-Et | Me | ¹H-NMR (400 MHz, CDCl₃): 7.20 (d, 1H); 7.08 (d, 1H); 6.06 (tt, 1H); 4.06 (td, 2H); 3.90 (s, 3H); 3.69 (s, 2H); 2.73 (s, 3H); 2.58 (q, 2H); 1.16 (t, 3H) |
| II-126 | 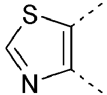 | C=O | CH₃ | Cl | 6-CF₃ | H | Me | ¹H-NMR (400 MHz, CDCl₃): 8.93 (s, 1H); 7.57 (dd, 1H); 7.31 (t, 1H); 3.96 (s, 3H); 3.76 (s, 2H); 3.32 (s, 3H) |
| II-127 | 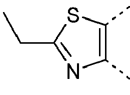 | C=O | CH₃ | Cl | 6-Cl | H | Me | ¹H-NMR (400 MHz, CDCl₃): 7.26 (m, 2H); 7.09 (t, 1H); 3.90 (s, 3H); 3.84 (s, 2H); 3.29 (s, 3H); 3.01 (q, 2H); 1.41 (t, 3H) |
| II-128 | 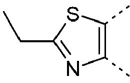 | C=O | CH₂-C≡CH | Cl | 6-Cl | H | Me | ¹H-NMR (400 MHz, CDCl₃): 7.26 (m, 2H); 7.10 (dd, 1H); 4.64 (d, 2H); 3.90 (s, 3H); 3.87 (s, 2H); 3.04 (q, 2H); 2.13 (br s, 1H); 1.42 (t, 3H) |
| II-129 | 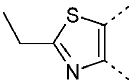 | C=O | CH₂-CHF₂ | Cl | 4-Cl | 6-Et | Me | ¹H-NMR (400 MHz, CDCl₃): 7.20 (d, 1H); 7.08 (d, 1H); 6.06 (tt, 1H); 4.07 (td, 2H); 3.90 (s, 3H); 3.69 (s, 2H); 3.03 (q, 2H); 2.59 (q, 2H); 1.42 (t, 3H); 1.16 (t, 3H) |
| II-130 | 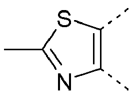 | C=O | CH₂-C≡CH | Br | 4-Cl | 6-Et | Me | ¹H-NMR (400 MHz, CDCl₃): 7.38 (d, 1H); 7.12 (d, 1H); 4.62 (d, 2H); 3.89 (s, 3H); 3.72 (s, 2H); 2.75 (s, 3H); 2.61 (q, 2H); 2.14 (br s, 1H); 1.16 (t, 3H) |
| II-131 | 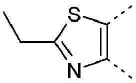 | C=O | CH₂-CHF₂ | Cl | 6-Cl | H | Me | ¹H-NMR (400 MHz, CDCl₃): 7.26 (m, 2H); 7.11 (dd, 1H); 6.09 (tt, 1H); 4.08 (td, 2H); 3.91 (s, 3H); 3.89 (s, 2H); 3.00 (q, 2H); 1.40 (t, 3H) |
| II-132 | 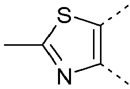 | C=O | CH₂-CHF₂ | Br | 4-Cl | 6-Et | Me | ¹H-NMR (400 MHz, CDCl₃): 7.38 (d, 1H); 7.12 (d, 1H); 6.06 (tt, 1H); 4.07 (td, 2H); 3.90 (s, 3H); 3.73 (s, 2H); 2.74 (s, 3H); 2.60 (q, 2H); 1.16 (t, 3H) |
| II-133 | 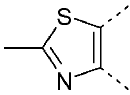 | C=O | CH₃ | Br | 4-Cl | 6-Et | Me | ¹H-NMR (400 MHz, CDCl₃): 7.38 (d, 1H); 7.11 (d, 1H); 3.89 (s, 3H); 3.69 (s, 2H); 3.27 (s, 3H); 2.73 (s, 3H); 2.61 (q, 2H); 1.16 (t, 3H) |
| II-134 | 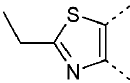 | C=O | CH₃ | Cl | 4-Cl | 6-Et | Me | ¹H-NMR (400 MHz, CDCl₃): 7.19 (d, 1H); 7.07 (d, 1H); 3.89 (s, 3H); 3.65 (s, 2H); 3.27 (s, 3H); 3.02 (q, 2H); 2.60 (q, 2H); 1.42 (t, 3H); 1.16 (t, 3H) |
| II-135 | 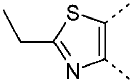 | C=O | CH₂-C≡CH | Br | 4-Cl | 6-Et | Me | ¹H-NMR (400 MHz, CDCl₃): 7.38 (d, 1H); 7.12 (d, 1H); 4.63 (d, 2H); 3.90 (s, 3H); 3.71 (s, 2H); 3.05 (q, 2H); 2.62 (q, 2H); 2.13 (br s, 1H); 1.43 (t, 3H); 1.16 (t, 3H) |
| II-136 | 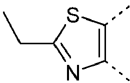 | C=O | CH₃ | Br | 4-Cl | 6-Et | Me | ¹H-NMR (400 MHz, CDCl₃): 7.38 (d, 1H); 7.11 (d, 1H); 3.90 (s, 3H); 3.68 (s, 2H); 3.28 (s, 3H); 3.03 (q, 2H); 2.61 (q, 2H); 1.42 (t, 3H); 1.16 (t, 3H) |
| II-137 | 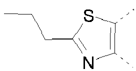 | C=O | CH₂-CHF₂ | Cl | 6-Cl | H | Me | ¹H-NMR (400 MHz, CDCl₃): 7.26 (m, 2H); 7.11 (dd, 1H); 6.09 (tt, 1H); 4.09 (td, 2H); 3.91 (s, 3H); 3.88 (s, 2H); 2.95 (t, 2H); 1.83 (sxt, 2H); 1.04 (t, 3H) |
| II-138 | 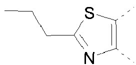 | C=O | CH₃ | Cl | 6-Cl | H | Me | ¹H-NMR (400 MHz, CDCl₃): 7.26 (m, 2H); 7.10 (dd, 1H); 3.90 (s, 3H); 3.83 (s, 2H); 3.29 (s, 3H); 2.96 (t, 2H); 1.85 (sxt, 2H); 1.05 (t, 3H) |
| II-139 | 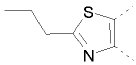 | C=O | CH₂-CHF₂ | Cl | 4-Cl | 6-Et | Me | ¹H-NMR (400 MHz, CDCl₃a): 7.20 (d, 1H); 7.08 (d, 1H); 6.06 (tt, 1H); 4.07 (td, 2H); 3.90 (s, 3H); 3.68 (s, 2H); 2.97 (t, 2H); 2.59 (q, 2H); 1.85 (sxt, 2H); 1.16 (t, 3H); 1.04 (t, 3H) |
| II-140 | 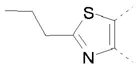 | C=O | CH₂-C≡CH | Cl | 6-Cl | H | Me | ¹H-NMR (400 MHz, CDCl₃): 7.26 (m, 2H); 7.10 (dd, 1H); 4.64 (d, 2H); 3.90 (s, 3H); 3.86 (s, 2H); 2.98 (t, 2H); 2.12 (br s, 1H); 1.85 (sxt, 2H); 1.04 (t, 3H) |
| II-141 | 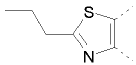 | C=O | CH₂-C≡CH | Cl | 4-Cl | 6-Et | Me | ¹H-NMR (400 MHz, CDCl₃): 7.19 (d, 1H); 7.07 (d, 1H); 4.63 (d, 2H); 3.89 (s, 3H); 3.67 (s, 2H); 2.99 (t, 2H); 2.60 (q, 2H); 2.12 (br s, 1H); 1.86 (sxt, 2H); 1.16 (t, 3H); 1.04 (t, 3H) |
| II-142 | 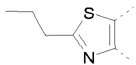 | C=O | CH₃ | BrI | 4-Cl | 6-Et | Me | ¹H-NMR (400 MHz, CDCl₃): 7.38 (d, 1H); 7.11 (d, 1H); 3.90 (s, 3H); 3.67 (s, 2H); 3.28 (s, 3H); 2.97 (t, 2H); 2.61 (q, 2H); 1.85 (sxt, 2H); 1.16 (t, 3H); 1.05 (t, 3H) |
| II-143 | 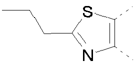 | C=O | CH₃ | Cl | 4-Cl | 6-Et | Me | ¹H-NMR (400 MHz, CDCl₃): 7.19 (d, 1H); 7.07 (d, 1H); 3.89 (s, 3H); 3.64 (s, 2H); 3.27 (s, 3H); 2.96 (t, 2H); 2.60 (q, 2H); 1.85 (sxt, 2H); 1.16 (t, 3H); 1.05 (t, 3H) |
| II-144 | 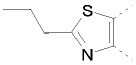 | C=O | CH₂-CHF₂ | Br | 4-Cl | 6-Et | Me | ¹H-NMR (400 MHz, CDCl₃): 7.39 (d, 1H); 7.13 (d, 1H); 6.06 (tt, 1H); 4.08 (td, 2H); 3.90 (s, 3H); 3.72 (s, 2H); 2.98 (t, 2H); 2.60 (q, 2H); 1.85 (sxt, 2H); 1.16 (t, 3H); 1.05 (t, 3H) |
| II-145 | 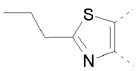 | C=O | CH₂-C≡CH | Br | 4-Cl | 6-Et | Me | ¹H-NMR (400 MHz, CDCl₃): 7.38 (d, 1H); 7.12 (d, 1H); 4.64 (d, 2H); 3.90 (s, 3H); 3.71 (s, 2H); 2.99 (t, 2H); 2.61 (q, 2H); 2.12 (br s, 1H); 1.84 (sxt, 2H); 1.16 (t, 3H); 1.04 (t, 3H) |
| II-146 | 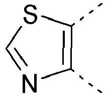 | C=O | CH₂-CH₂-SCH₃ | Cl | 6-Cl | H | Me | ¹H-NMR (400 MHz, CDCl₃): 8.87 (s, 1H), 7.26 (d, 2H), 7.11 (t, 1H), 3.95 (m, 5H), 3,79 (s, 2H), 2.76 (t, 2H), 2.11 (s, 3H) |
| II-147 | 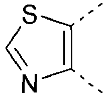 | C=O | CH₂-CH₂-OCH₃ | Cl | 6-Cl | H | Me | ¹H-NMR (400 MHz, CDCl₃): 8.85 (s, 1H), 7.26 (d, 2H), 7.10 (t, 1H), 4.00 (t, 2H),3.97 (s, 3H), 3,82 (s, 2H), 3.60 (t, 2H), 3.21 (s, 3H) |
| II-148 | 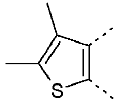 | C=O | CH₂-CHF₂ | Cl | 4-Br | 6-Et | Et | ¹H-NMR (400 MHz, CDCl₃): 7.37 (d, 1H); 7.23 (d, 1H); 6.05 (tt, 1H); 4.33 (m, 2H); 4.18 (m, 1H); 3.73 (s, 2H); 3.69 (m, 1H); 2.54 (q, 2H); 2.37 (s, 3H); 2.30 (s, 3H); 1.36 (t, 3H); 1.17 (t, 3H) |
| II-150 | 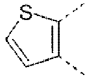 | C=O | CH₂-CHF₂ | Cl | 4-Br | 6-Et | Et | ¹H-NMR (400 MHz, CDCl₃): 7.62 (d, 1H); 7.36 (d, 1H); 7.24 (d, 1H); 7.11 (d, 1H); 6.09 (br tt, 1H); 4.25 (m, 1H); 3.92 (s, 3H); 3.63 (m, 1H); 3.55 (d, 2H); 2.55 (q, 2H); 1.16 (t, 3H) |
| II-151 | 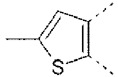 | C=O | CH₂-CHF₂ | Br | 4-Cl | 6-Et | Me | ¹H-NMR (400 MHz, CDCl₃): 7.41 (d, 1H); 7.13 (d, 1H); 7.09 (q, 1H); 6.07 (tt, 1H); 4.15 (m, 1H); 3.87 (s, 3H); 3.76 (m, 3H); 2.56 (q, 2H); 2.49 (d, 3H); 1.16 (t, 3H) |
| II-152 | 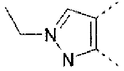 | C=O | CH₂-CHF₂ | Br | 4-Cl | 6-Et | Et | ¹H-NMR (400 MHz, CDCl₃): 7.98 (s, 1H); 7.39 (d, 1H); 7.12 (d, 1H); 6.06 (tt, 1H); 4.30 (q, 2H); 4.20 (q, 2H); 4.03 (m, 2H); 3.68 (m, 2H); 2.58 (q, 2H); 1.55 (t, 3H); 1.35 (t, 3H); 1.14 (t, 3H) |

### Beispielhafte Herstellung der Vorprodukte:

### 11. Herstellung von Methyl-4-{(2,2-difluorethyl)[(2-iodbenzyl)sulfonyl]amino}-1,3-thiazol-5-carboxylat:

2.1 g (4.8 mmol) Methyl-4-{[(2-iodbenzyl)sulfonyl]amino}-1,3-thiazol-5-carboxylat wurden in 20 ml Acetonitril gelöst und bei RT mit 0.9 ml (5.3 mmol) N,N-Diisopropylethylamin versetzt. Die Reaktionsmischung wurde 5 min bei RT gerührt und anschließend wurde eine Lösung von 1.54 g (7.2 mmol) Difluorethyltrifluormethansulfonat in 5 ml Acetonitril innerhalb 10 min zugetropft. Die Reaktionsmischung wurde 12h bei RT gerührt und anschließend ins Trockne eingedampft. Der Rückstand wurde mittels präparativer HPLC (C₁₈-SiO₂, Gradient Acetonitril/Wasser 20:80 nach 100:0) gereinigt. Man erhielt 2.09 g Methyl-4-{(2,2-difluorethyl)[(2-iodbenzyl)-sulfonyl]amino}-1,3-thiazol-5-carboxylat.
¹H-NMR (400 MHz, CDCl₃): 8.82 (s, 1H); 7.90 (br d, 1H); 7.63 (dd, 1H); 7.35 (td, 1H); 7.04 (td, 1 H); 5.96 (tt, 1 H); 4.81 (s, 2H); 4.14 (td, 1 H); 3.92 (s, 3H).

### 12. Herstellung von Methyl-4-{[(2-iodbenzyl)sulfonyl]amino}-1,3-thiazol-5-carboxylat:

2 g (12.64 mmol) Methyl-4-amino-1,3-thiazol-5-carboxylat wurden zusammen mit 5.1 ml NEt₃ (63.2 mmol) in 20 ml Dioxan gelöst und bei RT mit 4.4 g (13.9 mmol) (2-Iodphenyl)methansulfonylchlorid versetzt. Die Reaktionsmischung wurde 5h bei RT gerührt. Danach wurden 40 ml Wasser zugegeben und mehrmals mit Ethylacetat extrahiert. Die vereinigten organischen Phasen wurden mit 2N HCL gewaschen, getrocknet und eingedampft. Der Rückstand wurde mittels Säulenchromatographie (SiO₂, Gradient Ethylacetat/n-Heptan 10:90 nach 75:25) gereinigt. Man erhielt 4.3 g Methyl-4-{[(2-iod-benzyl)sulfonyl]amino}-1,3-thiazol-5-carboxylat.
¹H-NMR (400 MHz, CDCl₃): 9.01 (br s, 1H); 8.81 (s, 1H); 7.87 (d, 1H);7.51 (d, 1H); 7.36 (t, 1H); 7.04 (td, 1H); 5.06 (s, 2H); 3.88 (s, 3H).

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man 75 Gew.-Teile einer Verbindung der Formel (I),

| | |
|---|---|
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der Formel (I),

| | |
|---|---|
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen und Verträglichkeit gegenüber Kulturpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. Ia-26, I-c-4, I-a-9, I-a-8, I-a-66, I-g-5, I-a-90, I-a-96 und I-b-13 bei einer Aufwandmenge von 1280 Gramm pro Hektar jeweils eine mindestens 80%-ige Wirkung gegen Veronica persica. Die Verbindungen der Nr. I-a-13, I-a-65, I-a-74 und I-b-14 zeigen bei einer Aufwandmenge von 1280 Gramm pro Hektar jeweils eine mindestens 80%-ige Wirkung gegen Echinoa crus galli, Lolium multiflorum und Setaria viridis. Die Verbindungen der Nr. I-a-75, I-a-89, I-a-94, I-a-97 und I-c-38 zeigen bei einer Aufwandmenge von 320 Gramm pro Hektar jeweils eine mindestens 80%-ige Wirkung gegen Stellaria media und Veronica persica und dabei keinerlei Schäden in Mais. Die Verbindungen der Nr. I-a-2, I-a-28 und I-a-43 zeigen bei einer Aufwandmenge von 320 Gramm pro Hektar jeweils eine mindestens 80%-ige Wirkung gegen Setaria viridis und dabei keinerlei Schäden in Raps. Die Verbindungen der Nr. I-a-36, I-b-22 und I-a-44 zeigen bei einer Aufwandmenge von 320 Gramm pro Hektar jeweils eine mindestens 80%-ige Wirkung gegen Lolium multiflorum und dabei keinerlei Schäden in Mais.

### 2. Herbizide Wirkung gegen Schadpflanzen und Verträglichkeit gegenüber Kulturpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen durch optische Bonitur ermittelt. So zeigen beispielsweise die Verbindungen der Nr. Ia-26, I-a-38 und I-b-1 bei einer Aufwandmenge von 1280 Gramm pro Hektar jeweils eine mindestens 80%-ige Wirkung gegen Amaranthus retroflexus, Lolium multiflorum und Stellaria media.

Die Verbindungen der Nr. I-a-26, I-a-9, I-a-75, I-a-88, I-a-89, I-a-94, I-a-8 und I-b-13 zeigen bei einer Aufwandmenge von 1280 Gramm pro Hektar jeweils eine mindestens 80%-ige Wirkung gegen Matricaria inodora und Fallopia convolvulus. Die Verbindungen der Nr. I-g-5, I-h-11, I-a-83, I-a-86 und I-a-93 zeigen bei einer Aufwandmenge von 320 Gramm pro Hektar jeweils eine mindestens 80%-ige Wirkung gegen Stellaria media und Veronica persica und dabei keinerlei Schäden in Mais. Die Verbindungen der Nr. I-a-36, I-a-37 und I-a-44 zeigen bei einer Aufwandmenge von 320 Gramm pro Hektar jeweils eine mindestens 80%-ige Wirkung gegen Matricaria inodora, Fallopia convolvulus, Stellaria media und Veronica persica und dabei keinerlei Schäden in Mais.

## Patentansprüche

1. Ketosultame und Diketopyridine der Formel (I) oder deren Salze 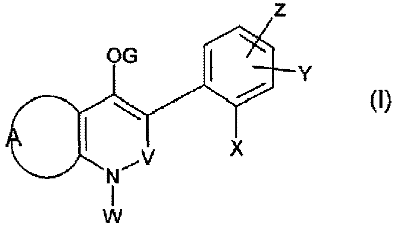 worin
A steht für einen ankondensierten gesättigten oder ungesättigten fünfgliedrigen Heterocyclus, der durch m Reste aus der Gruppe bestehend aus R¹⁰, R¹¹, R¹², R¹³ und R¹⁴ substituiert ist;
V bedeutet C(=O) oder S(O)₂;
m bedeutet 0,1 oder 2;
n bedeutet 0,1, 2 oder 3;
G bedeutet Wasserstoff, C(=O)R¹, C(=L)MR², SO₂R³, P(=L)R⁴R⁵, C(=L)NR⁶R⁷, E oder R⁸;
E bedeutet ein Metallionäquivalent oder ein Ammoniumion;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl,
einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₄)-Alkyl und (C₁-C₄)-Alkoxy substituiertes (C₃-C₆)-Cycloalkyl, Phenyl, Phenyl-(C₁-C₄)-alkyl, Phenoxy-(C₁-C₄)-alkyl oder Heteroaryloxy-(C₁-C₄)-alkyl;
R² bedeutet jeweils durch n Halogenatome substituiertes (C₁-C₅)-Alkyl, (C₂-C₆)-Alkenyl, (C₁-C₄)-Alkoxy-(C₁-C₆)-alkyl oder Di-(C₁-C₄)-alkoxy-(C₁-C₆)-alkyl,
oder durch jeweils n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl, (C₁-C₄)-Alkoxy, N-(C₁-C₆)-Alkylamino, N,N-Di-(C₁-C₆)-Alkylamino, (C₁-C₄)-Alkylthio, (C₂-C₄)-Alkenyl oder (C₃-C₆)-Cycloalkylthio,
oder durch n Reste aus der Gruppe bestehend aus Halogen, (C₁-C₄)-Alkyl und (C₁-C₄)-Alkoxy substituiertes Phenyl, Benzyl, Phenoxy oder Phenylthio;
R⁶ und R⁷ bedeuten unabhängig voneinander jeweils Wasserstoff,
durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₄)-Alkoxy-(C₁-C₆)-alkyl,
durch jeweils n Reste aus der Gruppe bestehend aus Halogen, (C₁-C₄)-Alkyl und (C₁-C₄)-Alkoxy substituiertes Phenyl oder Benzyl,
oder R⁶ und R⁷ bilden gemeinsam mit dem N-Atom, an das sie gebunden sind, einen 3- bis 6-gliedrigen Ring enthaltend 2 bis 5 Kohlenstoffatomen und 0 oder 1 Sauerstoff oder Schwefelatome;
R⁸ bedeutet durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl,
durch n Reste aus der Gruppe bestehend aus Halogen, (C₁-C₄)-Alkyl und (C₁-C₄)-Alkoxy substituiertes (C₃-C₆)-Cycloalkyl,
einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₄)-Alkyl und (C₁-C₄)-Alkoxy substituiertes Phenyl, Phenyl-(C₁-C₄)-alkyl, Heteroaryl, Phenoxy-(C₁-C₄)-alkyl oder Heteroaryloxy-(C₁-C₄)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁ -C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl;
R¹⁴ bedeutet Wasserstoff, durch n Halogenatome substituiertes (C₁-C₆)-Alkyl, (C₂-C₆)-Alkeny), (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylearbonyl, (C₃-C₆)-Cycloalkoxycarbonyl, (C₃-C₆)-Cyctoalkylsulfonyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl, und
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl.

2. Ketosultame und Diketopyridine nach Anspruch 1, worin A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 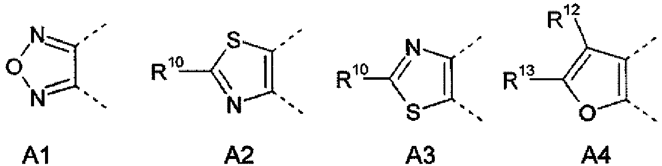 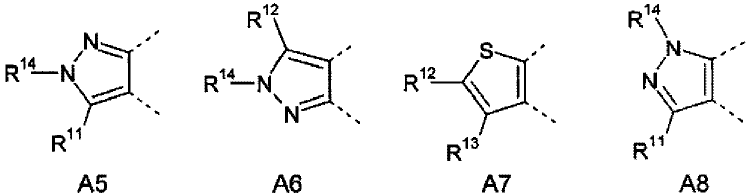 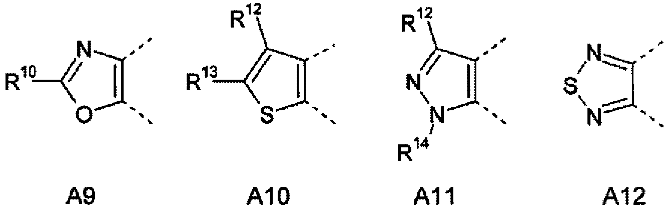
V bedeutet C(=O) oder S(O)₂;
n bedeutet 0, 1, 2 oder 3;
G bedeutet Wasserstoff, C(=O)R¹, C(=L)MR², SO₂R³, P(=L)R⁴R⁵, C(=L)NR⁶R⁷, E oder R⁸;
E bedeutet ein Metallionäquivalent oder ein Ammoniumion;
L bedeutet Sauerstoff oder Schwefel;
M bedeutet Sauerstoff oder Schwefel;
R¹ bedeutet jeweils durch n Halogenatome substituiertes (C₁-C₆)-Alkyl, (C₂-C₆)-Alkenyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl,
einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₄)-Alkyl und (C₁-C₄)-Alkoxy substituiertes (C₃-C₆)-Cycloalkyl, Phenyl, Phenyl-(C₁-C₄)-alkyl, Heteroaryl, Phenoxy-(C₁-C₄)-alkyl oder Heteroaryloxy-(C₁-C₄)-alkyl;
R² bedeutet jeweils durch n Halogenatome substituiertes (C₁-C₆)-Alkyl, (C₂-C₆)-Alkenyl, (C₁-C₄)-Alkoxy-(C₁-C₆)-alkyl oder Di-(C₁-C₄)-alkoxy-(C₁-C₆)-alkyl,
oder durch jeweils n Reste aus der Gruppe bestehend aus Halogen, (C₁-C₄)-Alkyl und (C₁-C₄)-Alkoxy substituiertes (C₃-C₆)-Cycloalkyl, Phenyl oder Benzyl;
R³, R⁴ und R⁵ bedeuten unabhängig voneinander jeweils durch n Halogenatome substituiertes (C₁-C₆)-Alkyl, (C₁-C₄)-Alkoxy, N-(C₁-C₆)-Alkylamino, N,N-Di-(C₁-C₆)-Alkylamino, (C₁-C₄)-Alkylthio, (C₂-C₄)-Alkenyl oder (C₃-C₆)-Cycloalkylthio,
oder durch n Reste aus der Gruppe bestehend aus Halogen, (C₁-C₄)-Alkyl und (C₁-C₄)-Alkoxy substituiertes Phenyl, Benzyl, Phenoxy oder Phenylthio;
R⁶ und R⁷ bedeuten unabhängig voneinander jeweils Wasserstoff,
durch n Halogenatome substituiertes (C₁-C₆)-Alkyl, (C₃-C₆)-Gycloalkyl, (C₂-C₆)-Alkenyl, (C₁-C₆)-Alkoxy oder (C₁-C₄)-Alkoxy-(C₁-C₆)-alkyl,
durch jeweils n Reste aus der Gruppe bestehend aus Halogen, (C₁-C₄)-Alkyl und (C₁-C₄)-Alkoxy substituiertes Phenyl oder Benzyl;
oder R⁶ und R⁷ bilden gemeinsam mit dem N-Atom, an das sie gebunden sind, einen 3- bis 6-gliedrigen Ring enthaltend 2 bis 5 Kohlenstoffatomen und 0 oder 1 Sauerstoff- oder Schwefelatome;
R⁸ bedeutet durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl,
durch n Reste aus der Gruppe bestehend aus Halogen, (C₁-C₄)-Alkyl und (C₁-C₄)-Alkoxy substituiertes (C₃-C₆)-Cycloalkyl,
einen durch n Reste aus der Gruppe bestehend aus Halogen, (C₁-C₄)-Alkyl und (C₁-C₄)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₄)-Alkyl und (C₁-C₄)-Alkoxy substituiertes Phenyl, Phenyl-(C₁-C₄)-alkyl, Heteroaryl, Phenoxy-(C₁-C₄)-alkyl oder Heteroaryloxy-(C₁-C₄)-alkyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₈)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-Ce)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl. (C₃-C₆)-Cycloalkylcarbonyl, (C₃-C₆)-Cycloalkoxycarbonyl, (C₃-C₆)-Cycloalkylsulfonyl.

3. Ketosultame und Diketopyridine nach Anspruch 1 oder 2, worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 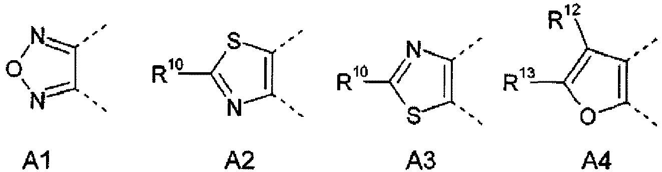 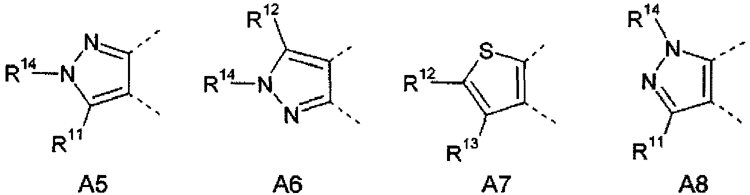 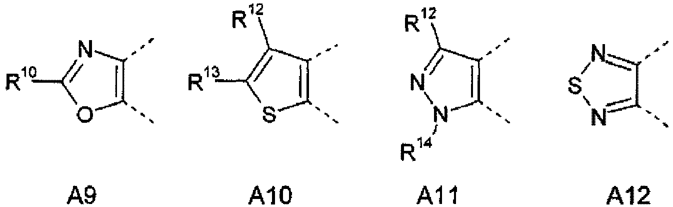
V bedeutet C(=O) oder S(O)₂;
n bedeutet 0, 1, 2 oder 3;
G bedeutet Wasserstoff;
W bedeutet jeweils durch n Halogenatome substituiertes (C₁-C₆)-Alkyl, (C₂-C₈)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₁-C₆)-Cycloalkyl-(C₁-C₆)-alkyl;
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsutfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl oder
durch n Reste aus der Gruppe bestehend aus Halogen, (C₁-C₄)-Alkyl und (C₁**-**C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

4. Ketosultame und Diketopyridine nach Anspruch 1 oder 2, worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 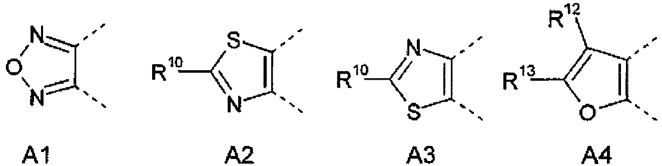 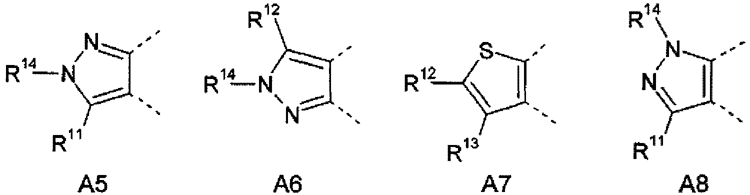 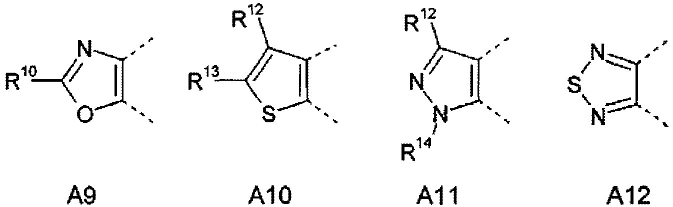
V bedeutet C(=O) oder S(O)₂;
n bedeutet 0, 1, 2 oder 3;
G bedeutet C(=O)R¹;
R¹ bedeutet jeweils durch n Halogenatome substituiertes (C₁-C₆)-Alkyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkytsulfinyl oder (C₁-C₄)-Alkylsulfonyl;
R¹¹ R¹² R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl. (C₁-C₄)-Alkylthio-(C₁-C4)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl oder durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkytsulfonyl.

5. Ketosultame und Diketopyridine nach Anspruch 1 oder 2, worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 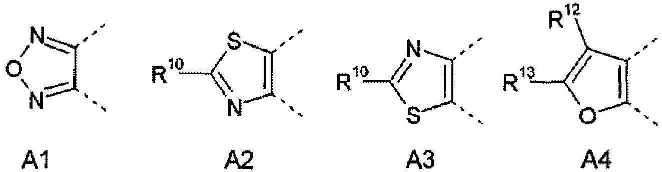 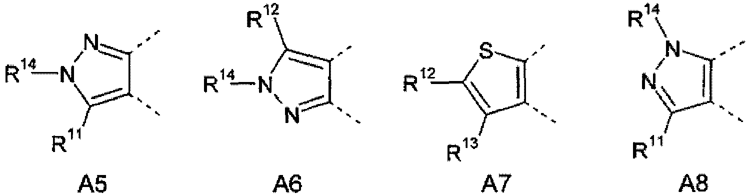 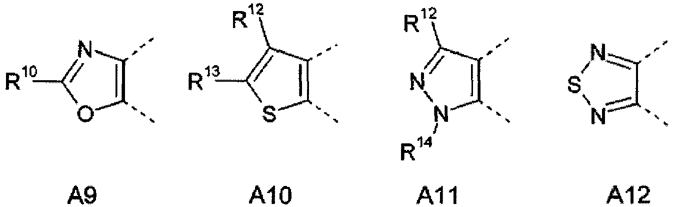
V bedeutet C(=O) oder S(O)₂;
n bedeutet 0, 1, 2 oder 3;
G bedeutet C(=L)MR²;
L bedeutet Sauerstoff;
M bedeutet Sauerstoff oder Schwefel;
R² bedeutet jeweils durch n Halogenatome substituiertes (C₁-C₆)-Alkyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl und
X, Y und Z bedeuten jeweils unabhängig voneinander Wasserstoff, (C₁-C₆)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkytsulfinyl oder (C₁-C₄)-Alkylsulfonyl oder
durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkylcarbonyl, (C₃-C₆)-Cycloalkoxycarbonyl, (C₃-C₆)-Cycloalkylsulfonyl.

6. Ketosultame und Diketopyridine nach Anspruch 1 oder 2, worin
A bedeutet einen der nachfolgend genannten fünfgliedrigen Heterocyclen A1 bis A12, worin die gestrichelten Linien die Verknüpfung zum benachbarten Pyridin- oder Ketosultam-Ring bedeuten, 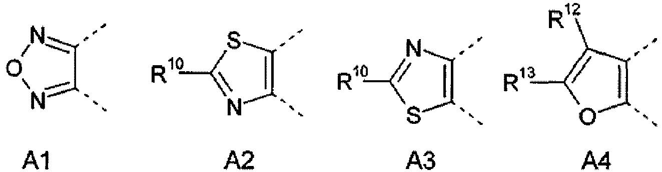 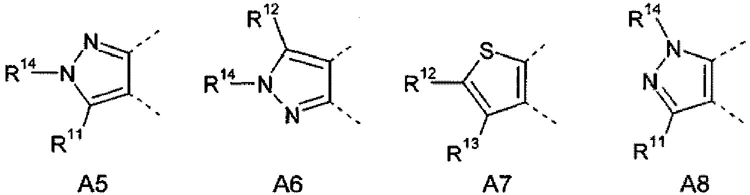 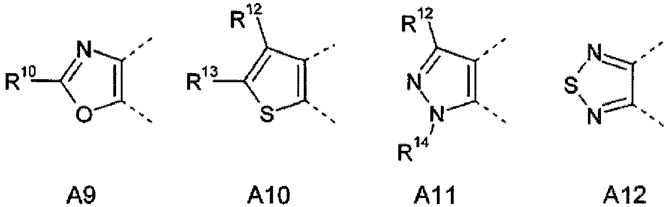
V bedeutet C(=O) oder S(O)₂;
n bedeutet 0, 1, 2 oder 3;
G bedeutet R⁸;
R⁸ bedeutet durch n Halogenatome substituiertes (C₁-C₆)-Alkyl, (C₂-C₆)-Alkenyl, (C₂-C₆)-Alkinyl;
W bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl, (C₁-C₄)-Alkylthio-(C₁-C₆)-alkyl, (C₁-C₄)-Alkylsulfinyl-(C₁-C₆)-alkyl, (C₁-C₄)-Alkylsulfonyl-(C₁-C₆)-alkyl oder (C₃-C₆)-Cycloalkyl-(C₁-C₆)-alkyl;
X, Y und Z bedeuten jeweils unabhängig voneinander Wasserstoff, (C₁-C₅)-Alkyl, (C₃-C₆)-Cycloalkyl, Halogen, Cyano, Nitro, Halogen-(C₁-C₆)-alkyl, Halogen-(C₁-C₆)-alkoxy, (C₃-C₆)-Cycloalkyl, (C₁-C₆)-Alkoxy, (C₁-C₆)-Alkoxy -(C₁-C₆)-alkyl, Halogen-(C₁-C₆)-alkoxy-(C₁-C₆)-alkyl;
R¹⁰ bedeutet Wasserstoff oder durch n Halogenatome substituiertes (C₁-C₆)-Alkyl, (C₁-C₄)-Alkoxy, (C₁-C₄)-Alkylthio, (C₁-C₄)-Alkylsulfinyl oder (C₁-C₄)-Alkylsulfonyl;
R¹¹, R¹² und R¹³ bedeuten jeweils unabhängig voneinander Wasserstoff, Halogen oder durch n Halogenatome substituiertes (C₁-C₆)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C₄)-alkyl, Di-(C₁-C₄)-alkoxy-(C₁-C₆)-alkyl, (C₁-C₄)-Alkylthio, (C₁-C₄)-Alkylsulfinyl oder (C₁-C₄)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, und
R¹⁴ bedeutet Wasserstoff, durch n Halogenatome substituiertes (C₁-C₈)-Alkyl, (C₂-C₆)-Alkenyl, (C₂-C₆)-Alkinyl, (C₁-C₄)-Alkoxy-(C₁-C₆)-alkyl, (C₁-C₄)-Alkylthio-(C₁-C4)-alkyl oder Di-(C₁-C₄)-alkoxy-(C₁-C₆)-alkyl, (C₁-C₆)-Alkylcarbonyl, (C₁-C₆)-Alkoxycarbonyl, (C₁-C₆)-Alkylsulfonyl, oder
durch n Reste aus der Gruppe bestehend aus Halogen, (C₁-C₄)-Alkyl und (C₁-C₄)-Alkoxy substituiertes (C₃-C₆)-Cycloalkyl, (C₃-C₆)-Cycloalkyloarbonyl, (C₃-C₆)-Cycloalkoxycarbonyl, (C₃-C₆)-Cycloalkylsulfonyl.

7. Herbizide Mittel, **gekennzeichnet durch** einen herbizid wirksamen Gehalt an mindestens einer Verbindung der Formel (I) gemäß einem der Ansprüche 1 bis 6.

8. Herbizide Mittel nach Anspruch 7 in Mischung mit Formulierungshilfsmitteln.

9. Herbizide Mittel nach Anspruch 7 oder 8 enthaltend mindestens einen weiteren pestizid wirksame Stoffen aus der Gruppe Insektizide, Akarizide, Herbizide, Fungizide, Safenem und Wachstumsregulatoren.

10. Herbizide Mittel nach Anspruch 9 enthaltend einen Safener.

11. Herbizide Mittel nach Anspruch 9 oder 10 enthaltend ein weiteres Herbizid.

12. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 6 oder eines herbiziden Mittels nach einem der Ansprüche 7 bis 11 auf die Pflanzen oder auf den Ort des unerwünschten Pflanzenwachstums appliziert.

13. Verwendung von Verbindungen der Formel (I) gemäß einem der Ansprüche 1 bis 6 oder eines herbiziden Mittels nach einem der Ansprüche 7 bis 11 zur Bekämpfung unerwünschter Pflanzen.

14. Verwendung nach Anspruch 13, **dadurch gekennzeichnet, daß** die Verbindungen der Formel (I) zur Bekämpfung unerwünschter Pflanzen in Kulturen von Nutzpflanzen eingesetzt werden.

15. Verwendung nach Anspruch 14, **dadurch gekennzeichnet, daß** die Nutzpflanzen transgene Nutzpflanzen sind.

16. Verbindungen der Formel (II) 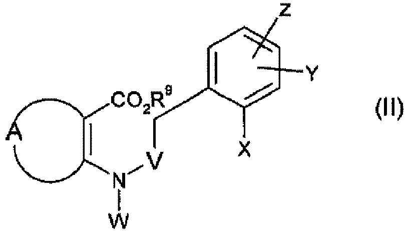 worin die Substituenten wie in einem der Ansprüche 1 bis 6 definiert sind und R9 für (C1-C6)-Alkyl steht.

## Claims

1. A ketosultam or diketopyridine of the formula (I) or salt thereof 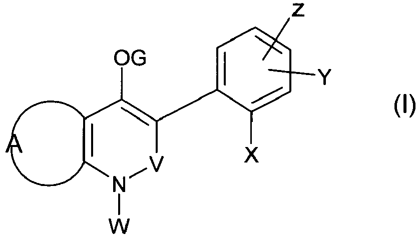 in which
A is a fused-on saturated or unsaturated five-membered heterocycle substituted by m radicals from the group consisting of R¹⁰, R¹¹, R¹², R¹³ and R¹⁴;
V is C(=O) or S(O)₂;
m is 0, 1 or 2;
n is 0, 1, 2 or 3;
G is hydrogen, C(=O)R¹, C(=L)MR², SO₂R³, P(=L)R⁴R⁵, C(=L)NR⁶R⁷, E or R⁸;
E is a metal ion equivalent or an ammonium ion;
L is oxygen or sulfur;
M is oxygen or sulfur;
R¹ is (C₁-C₆)-alkyl, (C₂-C₆)-alkenyl, (C₁-C₄)-alkoxy-(C₁-C₆)-alkyl, di-(C₁-C₄)-alkoxy-(C₁-C₆)-alkyl or (C₁-C₄)-alkylthio-(C₁-C₆)-alkyl each substituted by n halogen atoms,
a fully saturated 3- to 6-membered ring consisting of 3 to 5 carbon atoms and 1 to 3 heteroatoms from the group of oxygen, sulfur and nitrogen and substituted by n radicals from the group consisting of halogen, (C₁-C₄)-alkyl and (C₁-C₄)-alkoxy, (C₃-C₆)-cycloalkyl, phenyl, phenyl-(C₁-C₄)-alkyl, phenoxy-(C₁-C₄)-alkyl or heteroaryloxy-(C₁-C₄)-alkyl substituted by n radicals from the group consisting of halogen, (C₁-C₄)-alkyl and (C₁-C₄)-alkoxy;
R² is (C₁-C₆)-alkyl, (C₂-C₆)-alkenyl, (C₁-C₄)-alkoxy-(C₁-C₆)-alkyl or di-(C₁-C₄)-alkoxy-(C₁-C₆)-alkyl each substituted by n halogen atoms,
or (C₃-C₆)-cycloalkyl, phenyl or benzyl substituted by n radicals in each case from the group consisting of halogen, (C₁-C₄)-alkyl and (C₁-C₄)-alkoxy;
R³, R⁴ and R⁵ are each independently (C₁-C₆)-alkyl, (C₁-C₄)-alkoxy, N-(C₁-C₆)-alkylamino, N,N-di-(C₁-C₆)-alkylamino, (C₁-C₄)-alkylthio, (C₂-C₄)-alkenyl or (C₃-C₆)-cycloalkylthio each substituted by n halogen atoms,
or phenyl, benzyl, phenoxy or phenylthio substituted by n radicals from the group consisting of halogen, (C₁-C₄)-alkyl and (C₁-C₄)-alkoxy;
R⁶ and R⁷ are each independently hydrogen,
(C₁-C₆)-alkyl, (C₃-C₆)-cycloalkyl, (C₂-C₆)-alkenyl, (C₁-C₆)-alkoxy or (C₁-C₄)-alkoxy-(C₁-C₆)-alkyl substituted by n halogen atoms,
phenyl or benzyl substituted by in each case n radicals from the group consisting of halogen, (C₁-C₄)-alkyl and (C₁-C₄)-alkoxy,
or R⁶ and R⁷ form, together with the nitrogen atom to which they are bonded, a 3-to 6-membered ring containing 2 to 5 carbon atoms and 0 or 1 oxygen or sulfur atom;
R⁸ is (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms,
(C₃-C₆)-cycloalkyl substituted by n radicals from the group consisting of halogen, (C₁-C₄)-alkyl and (C₁-C₄)-alkoxy,
a fully saturated 3- to 6-membered ring consisting of 3 to 5 carbon atoms and 1 to 3 heteroatoms from the group of oxygen, sulfur and nitrogen and substituted by n radicals from the group consisting of halogen, (C₁-C₄)-alkyl and (C₁-C₄)-alkoxy, phenyl, phenyl-(C₁-C₄)-alkyl, heteroaryl, phenoxy-(C₁-C₄)-alkyl or heteroaryloxy-(C₁-C₄)-alkyl substituted by n radicals from the group consisting of halogen, (C₁-C₄)-alkyl and (C₁-C₄)-alkoxy;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹, R¹² and R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms, or (C₃-C₆)-cycloalkyl substituted by n radicals from the group consisting of halogen, (C₁-C₄)-alkyl and (C₁-C₄)-alkoxy;
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₃-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms, and
X, Y and Z are each independently hydrogen, (C₁-C₆)-alkyl, (C₃-C₆)-cycloalkyl, halogen, cyano, nitro, halo-(C₁-C₆)-alkyl, halo-(C₁-C₆)-alkoxy, (C₃-C₆)-cycloalkyl, (C₁-C₆)-alkoxy, (C₁-C₆)-alkoxy-(C₁-C₆)-alkyl, halo-(C₁-C₆)-alkoxy-(C₁-C₆)-alkyl.

2. A ketosultam or diketopyridine as claimed in claim 1, in which
A is one of the five-membered heterocycles A1 to A12 shown below, in which the broken lines mean the bond to the adjacent pyridine or ketosultam ring, 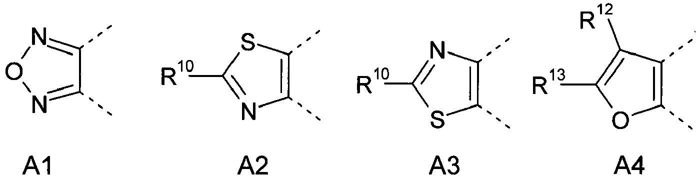 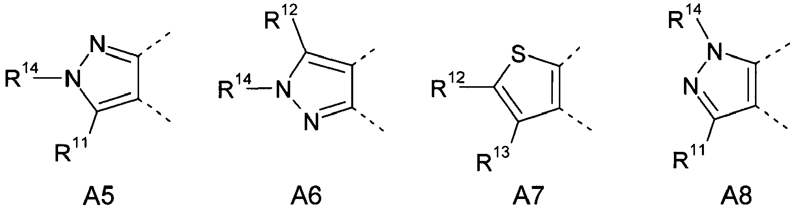 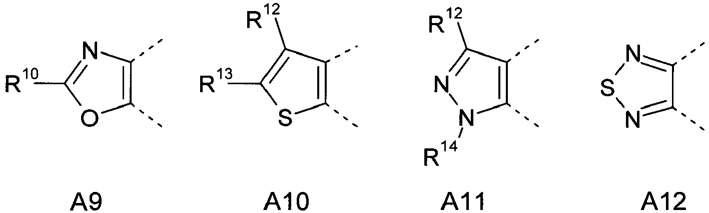
V is C(=O) or S(O)₂;
n is 0, 1, 2 for 3;
G is hydrogen, C(=O)R¹, C(=L)MR², SO₂R³, P(=L)R⁴R⁵, C(=L)NR⁶R⁷, E or R⁸;
E is a metal ion equivalent or an ammonium ion;
L is oxygen or sulfur;
M is oxygen or sulfur;
R¹ is (C₁-C₆)-alkyl, (C₂-C₆)-alkenyl, (C₁-C₄)-alkoxy-(C₁-C₆)-alkyl, di-(C₁-C₄)-alkoxy-(C₁-C₆)-alkyl or (C₁-C₄)-alkylthio-(C₁-C₆)-alkyl each substituted by n halogen atoms,
a fully saturated 3- to 6-membered ring consisting of 3 to 5 carbon atoms and 1 to 3 heteroatoms from the group of oxygen, sulfur and nitrogen and substituted by n radicals from the group consisting of halogen, (C₁-C₄)-alkyl and (C₁-C₄)-alkoxy, (C₃-C₆)-cycloalkyl, phenyl, phenyl-(C₁-C₄)-alkyl, heteroaryl, phenoxy-(C₁-C₄)-alkyl or heteroaryloxy-(C₁-C₄)-alkyl substituted by n radicals from the group consisting of halogen, (C₁-C₄)-alkyl and (C₁-C₄)-alkoxy;
R² is (C₁-C₆)-alkyl, (C₂-C₆)-alkenyl, (C₁-C₄)-alkoxy-(C₁-C₆)-alkyl or di-(C₁-C₄)-alkoxy-(C₁-C₆)-alkyl each substituted by n halogen atoms,
or (C₃-C₆)-cycloalkyl, phenyl or benzyl substituted by n radicals in each case from the group consisting of halogen, (C₁-C₄)-alkyl and (C₁-C₄)-alkoxy;
R³, R⁴ and R⁵ are each independently (C₁-C₆)-alkyl, (C₁-C₄)-alkoxy, N-(C₁-C₆)-alkylamino, N,N-di-(C₁-C₆)-alkylamino, (C₁-C₄)-alkylthio, (C₂-C₄)-alkenyl or (C₃-C₆)-cycloalkylthio each substituted by n halogen atoms,
or phenyl, benzyl, phenoxy or phenylthio substituted by n radicals from the group consisting of halogen, (C₁-C₄)-alkyl and (C₁-C₄)-alkoxy;
R⁶ and R⁷ are each independently hydrogen,
(C₁-C₆)-alkyl, (C₃-C₆)-cycloalkyl, (C₂-C₆)-alkenyl, (C₁-C₆)-alkoxy or (C₁-C₄)-alkoxy-(C₁-C₆)-alkyl substituted by n halogen atoms,
phenyl or benzyl substituted by in each case n radicals from the group consisting of halogen, (C₁-C₄)-alkyl and (C₁-C₄)-alkoxy,
or R⁶ and R⁷ form, together with the nitrogen atom to which they are bonded, a 3-to 6-membered ring containing 2 to 5 carbon atoms and 0 or 1 oxygen or sulfur atom;
R⁸ is (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms,
(C₃-C₆)-cycloalkyl substituted by n radicals from the group consisting of halogen, (C₁-C₄)-alkyl and (C₁-C₄)-alkoxy,
a fully saturated 3- to 6-membered ring consisting of 3 to 5 carbon atoms and 1 to 3 heteroatoms from the group of oxygen, sulfur and nitrogen and substituted by n radicals from the group consisting of halogen, (C₁-C₄)-alkyl and (C₁-C₄)-alkoxy, phenyl, phenyl-(C₁-C₄)-alkyl, heteroaryl, phenoxy-(C₁-C₄)-alkyl or heteroaryloxy-(C₁-C₄)-alkyl substituted by n radicals from the group consisting of halogen, (C₁-C₄)-alkyl and (C₁-C₄)-alkoxy;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms;
X, Y and Z are each independently hydrogen, (C₁-C₆)-alkyl, (C₃-C₆)-cycloalkyl, halogen, cyano, nitro, halo-(C₁-C₆)-alkyl, halo-(C₁-C₆)-alkoxy, (C₃-C₆)-cycloalkyl, (C₁-C₆)-alkoxy, (C₁-C₆)-alkoxy-(C₁-C₆)-alkyl, halo-(C₁-C₆)-alkoxy-(C₁-C₆)-alkyl;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹, R¹² and R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms, or (C₃-C₆)-cycloalkyl substituted by n radicals from the group consisting of halogen, (C₁-C₄)-alkyl and (C₁-C₄)-alkoxy; and
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₃-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy.

3. A ketosultam or diketopyridine as claimed in claim 1 or 2, in which
A is one of the five-membered heterocycles A1 to A12 shown below, in which the broken lines mean the bond to the adjacent pyridine or ketosultam ring, 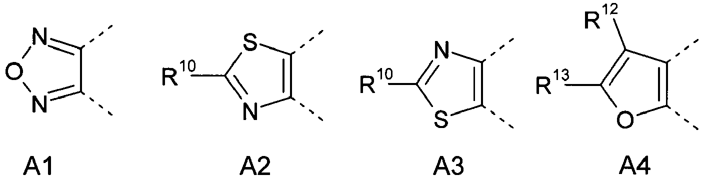 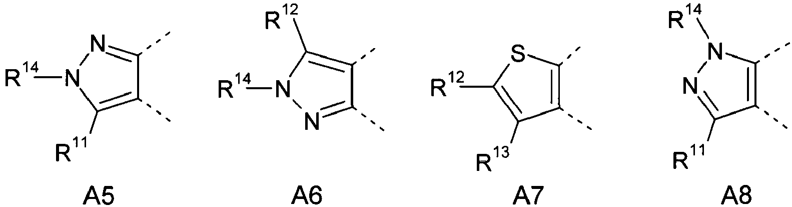 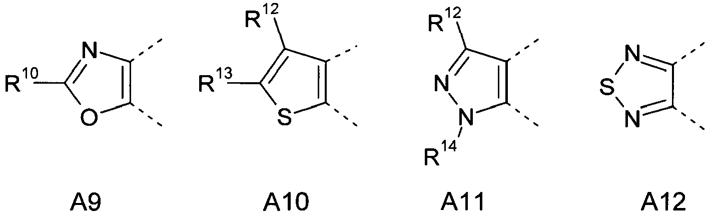
V is C(=O) or S(O)₂;
n is 0, 1, 2 or 3;
G is hydrogen;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms;
X, Y and Z are each independently hydrogen, (C₁-C₆)-alkyl, (C₃-C₆)-cycloalkyl, halogen, cyano, nitro, halo-(C₁-C₆)-alkyl, halo-(C₁-C₆)-alkoxy, (C₃-C₆)-cycloalkyl, (C₁-C₆)-alkoxy, (C₁-C₆)-alkoxy-(C₁-C₆)-alkyl, halo-(C₁-C₆)-alkoxy-(C₁-C₆)-alkyl;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹, R¹² and R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms or (C₃-C₆)-cycloalkyl substituted by n radicals from the group consisting of halogen, (C₁-C₄)-alkyl and (C₁-C₄)-alkoxy, and
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₃-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy.

4. A ketosultam or diketopyridine as claimed in claim 1 or 2, in which
A is one of the five-membered heterocycles A1 to A12 shown below, in which the broken lines mean the bond to the adjacent pyridine or ketosultam ring, 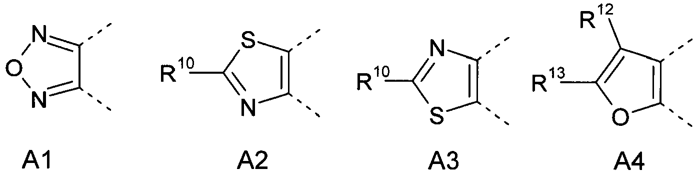 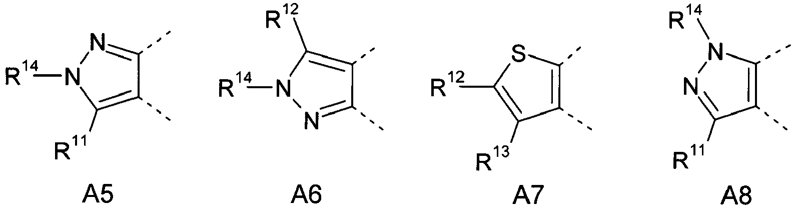 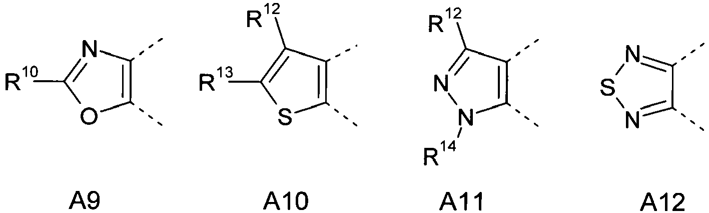
V is C(=O) or S(O)₂;
n is 0, 1, 2 or 3;
G is C(=O)R¹;
R¹ is (C₁-C₆)-alkyl each substituted by n halogen atoms;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹ R¹² R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms, or
(C₃-C₆)-cycloalkyl substituted by n radicals from the group consisting of halogen, (C₁-C₄)-alkyl and (C₁-C₄)-alkoxy, and
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₆-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy.

5. A ketosultam or diketopyridine as claimed in claim 1 or 2, in which
A is one of the five-membered heterocycles A1 to A12 shown below, in which the broken lines mean the bond to the adjacent pyridine or ketosultam ring, 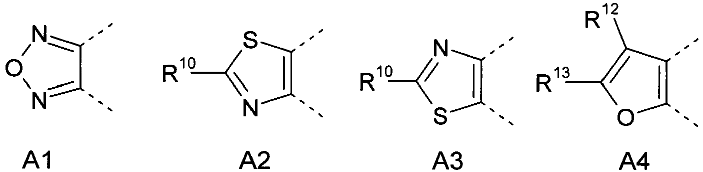 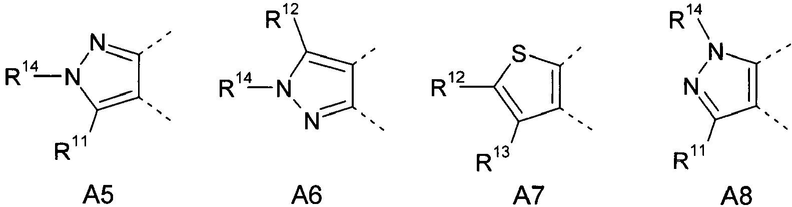 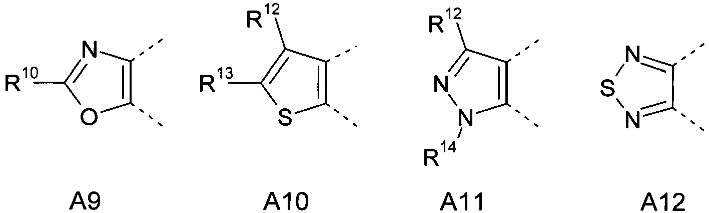
V is C(=O) or S(O)₂;
n is 0, 1, 2 or 3;
G is C(=L)MR²;
L is oxygen;
M is oxygen or sulfur;
R² is (C₁-C₆)-alkyl each substituted by n halogen atoms;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms and
X, Y and Z are each independently hydrogen, (C₁-C₆)-alkyl, (C₃-C₆)-cycloalkyl, halogen, cyano, nitro, halo-(C₁-C₆)-alkyl, halo-(C₁-C₆)-alkoxy, (C₃-C₆)-cycloalkyl, (C₁-C₆)-alkoxy, (C₁-C₆)-alkoxy-(C₁-C₆)-alkyl, halo-(C₁-C₆)-alkoxy-(C₁-C₆)-alkyl;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹, R¹² and R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms, or (C₃-C₆)-cycloalkyl substituted by n radicals from the group consisting of halogen, (C₁-C₄)-alkyl and (C₁-C₄)-alkoxy, and
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₃-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy.

6. A ketosultam or diketopyridine as claimed in claim 1 or 2, in which
A is one of the five-membered heterocycles A1 to A12 shown below, in which the broken lines mean the bond to the adjacent pyridine or ketosultam ring, 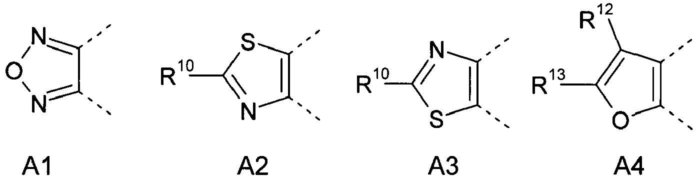 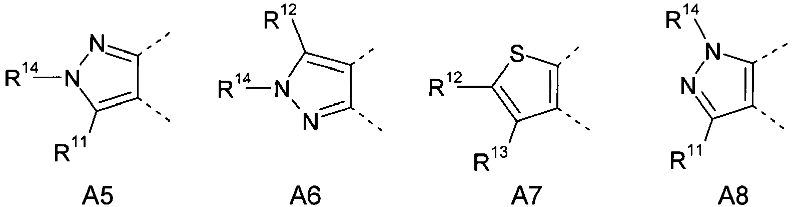 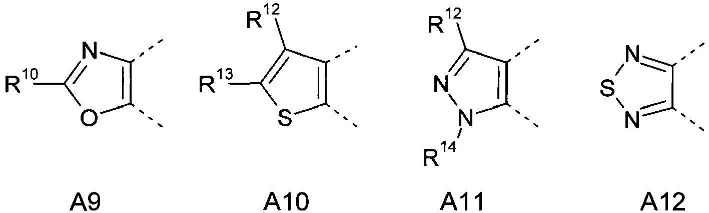
V is C(=O) or S(O)₂;
n is 0, 1, 2 or 3;
G is R⁸;
R⁸ is (C₁-C₆)-alkyl, (C₂-C₆)-alkenyl, (C₂-C₆)-alkynyl substituted by n halogen atoms;
W is (C₁-C₆)-alkyl, (C₂-C₆)-alkenyl, (C₃-C₆)-alkynyl, (C₃-C₆)-cycloalkyl, (C₁-C₄)-alkoxy-(C₁-C₆)-alkyl, di-(C₁-C₄)-alkoxy-(C₁-C₆)-alkyl, (C₁-C₄)-alkylthio-(C₁-C₆)-alkyl, (C₁-C₄)-alkylsulfinyl-(C₁-C₆)-alkyl, (C₁-C₄)-alkylsulfonyl-(C₁-C₆)-alkyl or (C₃-C₆)-cycloalkyl-(C₁-C₆)-alkyl each substituted by n halogen atoms;
X, Y and Z are each independently hydrogen, (C₁-C₆)-alkyl, (C₃-C₆)-cycloalkyl, halogen, cyano, nitro, halo-(C₁-C₆)-alkyl, halo-(C₁-C₆)-alkoxy, (C₃-C₆)-cycloalkyl, (C₁-C₆)-alkoxy, (C₁-C₆)-alkoxy-(C₁-C₆)-alkyl, halo-(C₁-C₆)-alkoxy-(C₁-C₆)-alkyl;
R¹⁰ is hydrogen, or (C₁-C₆)-alkyl, (C₁-C₄)-alkoxy, (C₁-C₄)-alkylthio, (C₁-C₄)-alkylsulfinyl or (C₁-C₄)-alkylsulfonyl substituted by n halogen atoms;
R¹¹, R¹² and R¹³ are each independently hydrogen, halogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl, di-(C₁-C₄)-alkoxy-(C₁-C₆)-alkyl, (C₁-C₄)-alkylthio, (C₁-C₄)-alkylsulfinyl or (C₁-C₄)-alkylsulfonyl substituted by n halogen atoms, or (C₃-C₆)-cycloalkyl substituted by n radicals from the group consisting of halogen, (C₁-C₄)-alkyl and (C₁-C₄)-alkoxy, and
R¹⁴ is hydrogen, or (C₁-C₆)-alkyl, (C₂-C₆)-alkenyl, (C₂-C₆)-alkynyl, (C₁-C₄)-alkoxy-(C₁-C₆)-alkyl, (C₁-C₄)-alkylthio-(C₁-C₄)-alkyl or di-(C₁-C₄)-alkoxy-(C₁-C₆)-alkyl substituted by n halogen atoms, or (C₁-C₆)-alkylcarbonyl, (C₁-C₆)-alkoxycarbonyl, (C₁-C₆)-alkylsulfonyl, or
(C₃-C₆)-cycloalkyl, (C₃-C₆)-cycloalkylcarbonyl, (C₃-C₆)-cycloalkoxycarbonyl, (C₃-C₆)-cycloalkylsulfonyl substituted by n radicals from the group consisting of halogen, (C₁-C₄)-alkyl and (C₁-C₄)-alkoxy.

7. A herbicidal composition, **characterized by** a herbicidally active content of at least one compound of the formula (I) as claimed in any of claims 1 to 6.

8. The herbicidal composition as claimed in claim 7 in a mixture with formulation assistants.

9. The herbicidal composition as claimed in claim 7 or 8 comprising at least one further pesticidally active substance from the group of insecticides, acaricides, herbicides, fungicides, safeners and growth regulators.

10. The herbicidal composition as claimed in claim 9 comprising a safener.

11. The herbicidal composition as claimed in claim 9 or 10 comprising a further herbicide.

12. A method for controlling unwanted plants, which comprises applying an effective amount of at least one compound of the formula (I) as claimed in any of claims 1 to 6 or of a herbicidal composition as claimed in any of claims 7 to 11 to the plants or to the site of unwanted plant growth.

13. The use of compounds of the formula (I) as claimed in any of claims 1 to 6 or of a herbicidal composition as claimed in any of claims 7 to 11 for control of unwanted plants.

14. The use as claimed in claim 13, wherein the compounds of the formula (I) are used for control of unwanted plants in crops of useful plants.

15. The use as claimed in claim 14, wherein the useful plants are transgenic useful plants.

16. A compound of the formula (II) 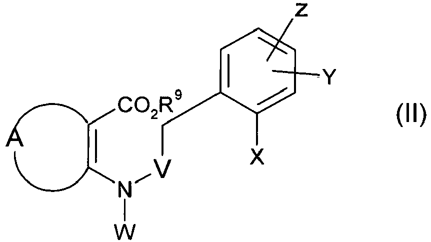 in which the substituents are as defined in any of claims 1 to 6 and R9 is (C1-C6)-alkyl.

## Revendications

1. Cétosultames et dicétopyridines de formule (I) ou leurs sels 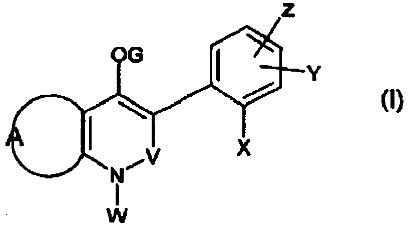 dans laquelle
A représente un hétérocycle condensé, saturé ou insaturé, à cinq chaînons, qui est substitué par m radicaux du groupe constitué par R¹⁰, R¹¹, R¹², R¹³ et R¹⁴ ;
V signifie C(=O) ou S(O)₂ ;
m signifie 0, 1 ou 2 ;
n signifie 0, 1, 2 ou 3 ;
G signifie hydrogène, C(=O)R¹, C(=L)MR², SO₂R³, P(=L)R⁴R⁵, C(=L)NR⁶R⁷, E ou R⁸ ;
E signifie un équivalent d'ion métallique ou un ion d'ammonium,
L signifie oxygène ou azote ;
M signifie oxygène ou azote ;
R¹ signifie (C₁-C₆)-alkyle, (C₂-C₆)-alcényle, (C₁-C₄)-alcoxy-(C₁-C₆)-alkyle, di-(C₁-C₄)-alcoxy-(C₁-C₆)-alkyle ou (C₁-C₄)-alkylthio-(C₁-C₆)-alkyle, à chaque fois substitué par n atomes d'halogène, un cycle de 3 à 6 chaînons, complètement saturé, constitué par 3 à 5 atomes de carbone et 1 à 3 hétéroatomes du groupe oxygène, soufre et azote, substitué par n radicaux du groupe constitué par halogène, (C₁-C₄)-alkyle et (C₁-C₄)-alcoxy ; (C₃-C₆)-cycloalkyle, phényle, phényl-(C₁-C₄)-alkyle, phénoxy-(C₁-C₄)-alkyle ou hétéroaryloxy-(C₁-C₄)-alkyle, substitués par n radicaux du groupe constitué par halogène, (C₁-C₄)-alkyle et (C₁-C₄)-alcoxy ;
R² signifie (C₁-C₆)-alkyle, (C₂-C₆)-alcényle, (C₁-C₄)-alcoxy-(C₁-C₆)-alkyle ou di-(C₁-C₄)-alcoxy-(C₁-C₆)-alkyle, à chaque fois substitué par n atomes d'halogène, ou (C₃-C₆)-cycloalkyle, phényle ou benzyle, à chaque fois substitué par n radicaux du groupe constitué par halogène, (C₁-C₄)-alkyle et (C₁-C₄)-alcoxy ;
R³, R⁴ et R⁵ signifient, indépendamment les uns des autres, (C₁-C₆)-alkyle, (C₁-C₄)-alcoxy, N-(C₁-C₆)-alkylamino, N,N-di-(C₁-C₆)-alkylamino, (C₁-C₄)-alkylthio, (C₂-C₄)-alcényle ou (C₃-C₆)-cycloalkylthio, à chaque fois substitué par n atomes d'halogène, ou phényle, benzyle, phénoxy ou phénylthio, substitués par n radicaux du groupe constitué par halogène, (C₁-C₄)-alkyle et (C₁-C₄)-alcoxy ;
R⁶ et R⁷ signifient, indépendamment l'un de l'autre, à chaque fois, hydrogène ; (C₁-C₆)-alkyle, (C₃-C₆)-cycloalkyle, (C₂-C₆)-alcényle, (C₁-C₆)-alcoxy ou (C₁-C₄)-alcoxy-(C₁-C₆)-alkyle, substitués par n atomes d'halogène, phényle ou benzyle, à chaque fois substitué par n radicaux du groupe constitué par halogène, (C₁-C₄)-alkyle et (C₁-C₄)-alcoxy ; ou ou R⁶ et R⁷ forment, ensemble avec l'atome de N auquel ils sont liés, un cycle de 3 à 6 chaînons, contenant 2 à 5 atomes de carbone et à chaque fois 0 ou 1 atome d'oxygène ou de soufre,
R⁸ signifie (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, substitués par n atomes d'halogène ; (C₃-C₆)-cycloalkyle substitué par n radicaux du groupe constitué par halogène, (C₁-C₄)-alkyle et (C₁-C₄)-alcoxy,
un cycle de 3 à 6 chaînons, complètement saturé, constitué par 3 à 5 atomes de carbone et 1 à 3 hétéroatomes du groupe oxygène, soufre et azote, substitué par n radicaux du groupe constitué par halogène, (C₁-C₄)-alkyle et (C₁-C₄)-alcoxy ; phényle, phényl-(C₁-C₄)-alkyle, hétéroaryle, phénoxy-(C₁-C₄)-alkyle ou hétéroaryloxy-(C₁-C₄)-alkyle, substitués par n radicaux du groupe constitué par halogène, (C₁-C₄)-alkyle et (C₁-C₄)-alcoxy ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹, R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy,
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou (C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄)-alkylthio-(C₁-C₆)-alkyle, (C₁-C₄)-alkylsulfïnyl-(C₁-C₆)-alkyle, (C₁-C₄)-alkylsulfonyl- (C₁-C₆)-alkyle ou (C₃-C₆)-cycloalkyl-(C₁-C₆)-alkyle, à chaque fois substitué par n atomes d'halogène et
X, Y et Z signifient, à chaque fois indépendamment les uns des autres, hydrogène, (C₁-C₆)alkyle, (C₃-C₆)-cycloalkyle, halogène, cyano, nitro, halogéno-(C₁-C₆)-alkyle, halogéno-(C₁-C₆)-alcoxy, (C₃-C₆)-cycloalkyle, (C₁-C₆)-alcoxy, (C₁-C₆)-alcoxy-(C₁-C₆)-alkyle, halogéno-(C₁-C₆)-alcoxy-(C₁-C₆)-alkyle.

2. Cétosultames et dicétopyridines selon la revendication 1, dans lesquels
A signifie un des hétérocycles A1 à A12 mentionnés ci-dessous, à cinq chaînons, les lignes en pointillés signifiant la liaison au cycle pyridine ou cétosultame adjacent, 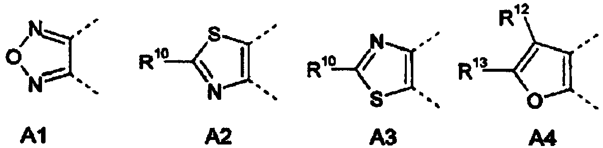 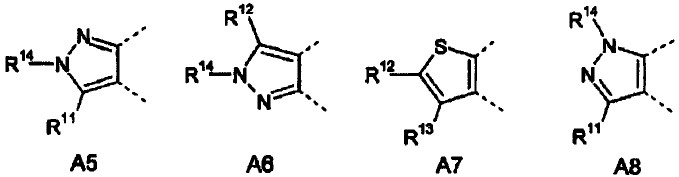 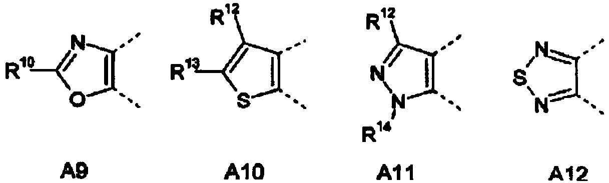
V signifie C(=O) ou S(O)₂ ;
n signifie 0, 1, 2 ou 3 ;
G signifie hydrogène, C(=O)R¹, C(=L)MR², SO₂R³, P(=L)R⁴R⁵, C(=L)NR⁶R⁷, E ou R⁸ ;
E signifie un équivalent d'ion métallique ou un ion d'ammonium,
L signifie oxygène ou azote ;
M signifie oxygène ou azote ;
R¹ signifie (C₁-C₆)-alkyle, (C₂-C₆)-alcényle, (C₁-C₄)-alcoxy-(C₁-C₆)-alkyle, di-(C₁-C₄)-alcoxy-(C₁-C₆)-alkyle ou (C₁-C₄)-alkylthio-(C₁-C₆)-alkyle, à chaque fois substitué par n atomes d'halogène, un cycle de 3 à 6 chaînons, complètement saturé, constitué par 3 à 5 atomes de carbone et 1 à 3 hétéroatomes du groupe oxygène, soufre et azote, substitué par n radicaux du groupe constitué par halogène, (C₁-C₄)-alkyle et (C₁-C₄)-alcoxy ; (C₃-C₆)-cycloalkyle, phényle, phényl-(C₁-C₄)-alkyle, hétéroaryle, phénoxy-(C₁-C₄)-alkyle ou hétéroaryloxy-(C₁-C₄)-alkyle, substitués par n radicaux du groupe constitué par halogène, (C₁-C₄)-alkyle et (C₁-C₄)-alcoxy ;
R² signifie (C₁-C₆)-alkyle, (C₂-C₆)-alcényle, (C₁-C₄)-alcoxy-(C₁-C₆)-alkyle ou di-(C₁-C₄)-alcoxy-(C₁-C₆)-alkyle, à chaque fois substitué par n atomes d'halogène, ou (C₃-C₆)-cycloalkyle, phényle ou benzyle, à chaque fois substitué par n radicaux du groupe constitué par halogène, (C₁-C₄)-alkyle et (C₁-C₄)-alcoxy ;
R³, R⁴ et R⁵ signifient, indépendamment les uns des autres, (C₁-C₆)-alkyle, (C₁-C₄)-alcoxy, N-(C₁-C₆)-alkylamino, N,N-di-(C₁-C₆)-alkylamino, (C₁-C₄)-alkylthio, (C₂-C₄)-alcényle ou (C₃-C₆)-cycloalkylthio, à chaque fois substitué par n atomes d'halogène, ou phényle, benzyle, phénoxy ou phénylthio, substitués par n radicaux du groupe constitué par halogène, (C₁-C₄)-alkyle et (C₁-C₄)-alcoxy ;
R⁶ et R⁷ signifient, indépendamment l'un de l'autre, à chaque fois hydrogène ; (C₁-C₆)-alkyle, (C₃-C₆)-cycloalkyle, (C₂-C₆)-alcényle, (C₁-C₆)-alcoxy ou (C₁-C₄)-alcoxy-(C₁-C₆)-alkyle, substitués par n atomes d'halogène, phényle ou benzyle, à chaque fois substitué par n radicaux du groupe constitué par halogène, (C₁-C₄)-alkyle et (C₁-C₄)-alcoxy ;
ou R⁶ et R⁷ forment, ensemble avec l'atome de N auquel ils sont liés, un cycle de 3 à 6 chaînons, contenant 2 à 5 atomes de carbone et 0 ou 1 atome d'oxygène ou de soufre,
R⁸ signifie (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, substitués par n atomes d'halogène ; (C₃-C₆)-cycloalkyle substitué par n radicaux du groupe constitué par halogène, (C₁-C₄)-alkyle et (C₁-C₄)-alcoxy,
un cycle de 3 à 6 chaînons, complètement saturé, constitué par 3 à 5 atomes de carbone et 1 à 3 hétéroatomes du groupe oxygène, soufre et azote, substitué par n radicaux du groupe constitué par halogène, (C₁-C₄)-alkyle et (C₁-C₄)-alcoxy ; phényle, phényl-(C₁-C₄)-alkyle, hétéroaryle, phénoxy-(C₁-C₄)-alkyle ou hétéroaryloxy-(C₁-C₄)-alkyle, substitués par n radicaux du groupe constitué par halogène, (C₁-C₄)-alkyle et (C₁-C₄)-alcoxy ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄) -alkylthio- (C₁-C₆)-alkyle, (C₁-C₄)-alkylsulfinyl-(C₁-C₆)-alkyle, (C₁-C₄)-alkylsulfonyl-(C₁-C₆)alkyle ou (C₃-C₆)-cycloalkyl-(C₁-C₆)-alkyle, à chaque fois substitué par n atomes d'halogène ;
X, Y et Z signifient, à chaque fois indépendamment les uns des autres, hydrogène, (C₁-C₆)-alkyle, (C₃-C₆)-cycloalkyle, halogène, cyano, nitro, halogéno-(C₁-C₆)-alkyle, halogéno-(C₁-C₆)-alcoxy, (C₃-C₆)-cycloalkyle, (C₁-C₆)-alcoxy, (C₁-C₆)-alcoxy-(C₁-C₆)-alkyle, halogéno-(C₁-C₆)-alcoxy-(C₁-C₆)-alkyle ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹, R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy, et
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou
(C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy.

3. Cétosultames et dicétopyridines selon la revendication 1 ou 2, dans lesquels
A signifie un des hétérocycles A1 à A12 mentionnés ci-dessous, à cinq chaînons, les lignes en pointillés signifiant la liaison au cycle pyridine ou cétosultame adjacent, 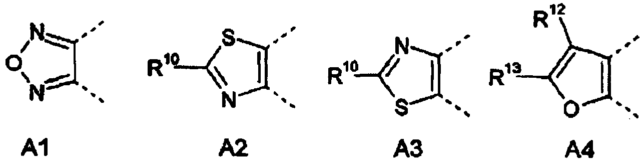 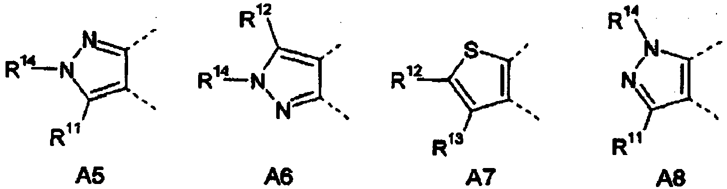 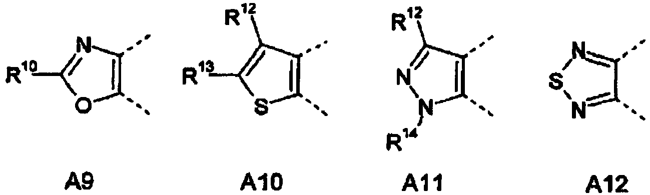
V signifie C(=O) ou S(O)₂ ;
n signifie 0, 1, 2 ou 3 ;
G signifie hydrogène ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄)-alkylthio- (C₁-C₆)-alkyle, (C₁-C₄)-alkylsulfinyl-(C₁-C₆)-alkyle, (C₁-C₄)-alkylsulfonyl-(C₁-C₆)-alkyle ou (C₃-C₆)-cycloalkyl-(C₁-C₆)-alkyle, à chaque fois substitué par n atomes d'halogène ;
X, Y et Z signifient, à chaque fois indépendamment les uns des autres, hydrogène, (C₁-C₆)-alkyle, (C₃-C₆)-cycloalkyle, halogène, cyano, nitro, halogéno-(C₁-C₆)-alkyle, halogéno-(C₁-C₆)-alcoxy, (C₃-C₆)-cycloalkyle, (C₁-C₆)-alcoxy, (C₁-C₆)-alcoxy-(C₁-C₆)-alkyle, halogéno-(C₁-C₆)-alcoxy-(C₁-C₆)-alkyle ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹, R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy, et
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou
(C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy.

4. Cétosultames et dicétopyridines selon la revendication 1 ou 2, dans lesquels
A signifie un des hétérocycles A1 à A12 mentionnés ci-dessous, à cinq chaînons, les lignes en pointillés signifiant la liaison au cycle pyridine ou cétosultame adjacent, 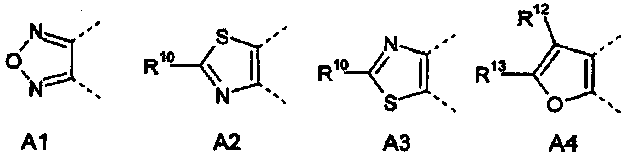 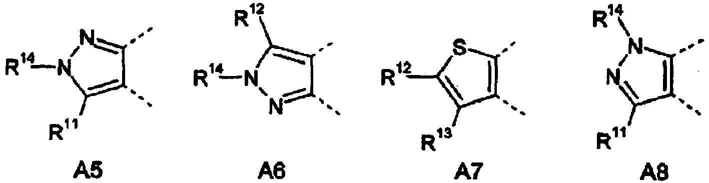 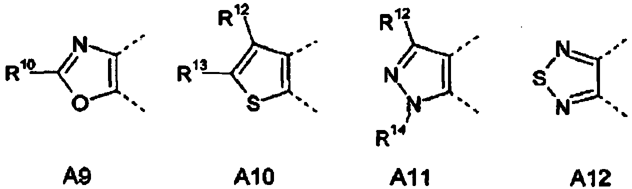
V signifie C(=O) ou S(O)₂ ;
n signifie 0, 1, 2 ou 3 ;
G signifie C(=O)R¹ ;
R¹ signifie (C₁-C₆)-alkyle à chaque fois substitué par n atomes d'halogène ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄)-alkylthio-(C₁-C₆)-alkyle, (C₁-C₄)-alkylsulfinyl-(C₁-C₆)-alkyle, (C₁-C₄)-alkylsulfonyl-(C₁-C₆)-alkyle ou (C₃-C₆)-cycloalkyl-(C₁-C₆)-alkyle, à chaque fois substitué par n atomes d'halogène ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹ R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy, et
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou
(C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy.

5. Cétosultames et dicétopyridines selon la revendication 1 ou 2, dans lesquels
A signifie un des hétérocycles A1 à A12 mentionnés ci-dessous, à cinq chaînons, les lignes en pointillés signifiant la liaison au cycle pyridine ou cétosultame adjacent, 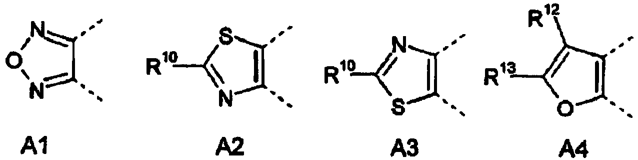 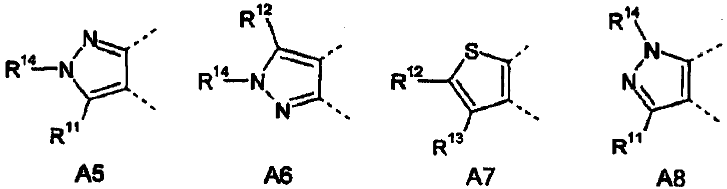 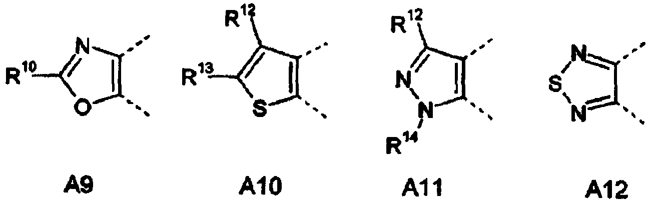
V signifie C(=O) ou S(O)₂ ;
n signifie 0, 1, 2 ou 3 ;
G signifie C(=L)MR² ;
L signifie oxygène ;
M signifie oxygène ou azote ;
R² signifie (C₁-C₆)-alkyle à chaque fois substitué par n atomes d'halogène ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄)-alkylthio-(C₁-C₆)-alkyle, (C₁-C₄)-alkylsulfinyl-(C₁-C₆)-alkyle, (C₁-C₄)-alkylsulfonyl- (C₁-C₆)-alkyle ou (C₃-C₆)-cycloalkyl-(C₁-C₆)-alkyle, à chaque fois substitué par n atomes d'halogène et
X, Y et Z signifient, à chaque fois indépendamment les uns des autres, hydrogène, (C₁-C₆)-alkyle, (C₃-C₆)-cycloalkyle, halogène, cyano, nitro, halogéno-(C₁-C₆)-alkyle, halogéno-(C₁-C₆)-alcoxy, (C₃-C₆)-cycloalkyle, (C₁-C₆)-alcoxy, (C₁-C₆)-alcoxy-(C₁-C₆)-alkyle, halogéno-(C₁-C₆)-alcoxy-(C₁-C₆)-alkyle ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹, R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy, et
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou (C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy.

6. Cétosultames et dicétopyridines selon la revendication 1 ou 2, dans lesquels
A signifie un des hétérocycles A1 à A12 mentionnés ci-dessous, à cinq chaînons, les lignes en pointillés signifiant la liaison au cycle pyridine ou cétosultame adjacent, 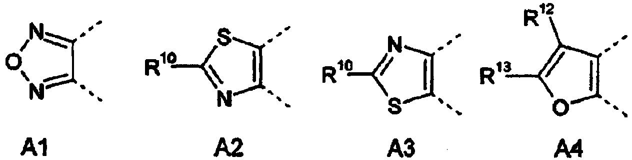 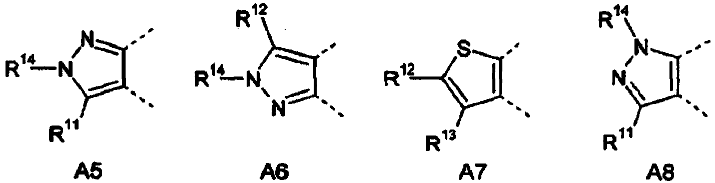 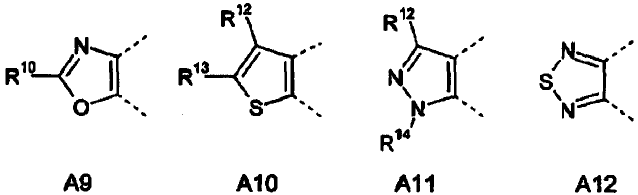
V signifie C(=O) ou S(O)₂ ;
n signifie 0, 1, 2 ou 3 ;
G signifie R⁸ ;
R⁸ signifie (C₁-C₆)-alkyle, (C₂-C₆)-alcényle, (C₂-C₆)-alcynyle, substitués par n atomes d'halogène ;
W signifie (C₁-C₆)-alkyle, (C₂-C₆)-alcényle, (C₃-C₆)-alcynyle, (C₃-C₆)-cycloalkyle, (C₁-C₄)-alcoxy-(C₁-C₆)-alkyle, di-(C₁-C₄)-alcoxy-(C₁-C₆)-alkyle, (C₁-C₄)-alkylthio-(C₁-C₆)-alkyle, (C₁-C₄)-alkylsulfinyl-(C₁-C₆)-alkyle, (C₁-C₄)-alkylsulfonyl-(C₁-C₆)-alkyle ou (C₃-C₆)-cycloalkyl-(C₁-C₆)-alkyle, à chaque fois substitué par n atomes d'halogène ;
X, Y et Z signifient, à chaque fois indépendamment les uns des autres, hydrogène, (C₁-C₆)-alkyle, (C₃-C₆)-cycloalkyle, halogène, cyano, nitro, halogéno-(C₁-C₆)-alkyle, halogéno-(C₁-C₆)-alcoxy, (C₃-C₆)-cycloalkyle, (C₁-C₆)-alcoxy, (C₁-C₆)-alcoxy-(C₁-C₆)-alkyle, halogéno-(C₁-C₆)-alcoxy-(C₁-C₆)-alkyle ;
R¹⁰ signifie hydrogène ; ou (C₁-C₆)-alkyle, (C₁-C₄)-alcoxy, (C₁-C₄)-alkylthio, (C₁-C₄)-alkylsulfinyle ou (C₁-C₄)-alkylsulfonyle, substitués par n atomes d'halogène ;
R¹¹, R¹² et R¹³ signifient, à chaque fois indépendamment les uns des autres, hydrogène ; halogène ; ou (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle, di-(C₁-C₄)-alcoxy-(C₁-C₆)-alkyle, (C₁-C₄)-alkylthio, (C₁-C₄)-alkylsulfinyle ou (C₁-C₄)-alkylsulfonyle, substitués par n atomes d'halogène, ou
(C₃-C₆)-cycloalkyle substitué par n radicaux du groupe constitué par halogène, (C₁-C₄)-alkyle et (C₁-C₄)-alcoxy, et
R¹⁴ signifie hydrogène ; (C₁-C₆)-alkyle, (C₂-C₆)-alcényle, (C₂-C₆)-alcynyle, (C₁-C₄)-alcoxy-(C₁-C₆)-alkyle, (C₁-C₄)-alkylthio-(C₁-C₄)-alkyle ou di-(C₁-C₄)-alcoxy-(C₁-C₆)-alkyle, (C₁-C₆)-alkylcarbonyle, (C₁-C₆)-alcoxycarbonyle, (C₁-C₆)-alkylsulfonyle, substitués par n atomes d'halogène ; ou
(C₃-C₆)-cycloalkyle, (C₃-C₆)-cycloalkylcarbonyle, (C₃-C₆)-cycloalcoxycarbonyle, (C₃-C₆)-cycloalkylsulfonyle, substitués par n radicaux du groupe constitué par halogène, (C₁-C₄)-alkyle et (C₁-C₄)-alcoxy.

7. Agents herbicides, **caractérisés par** une teneur active en tant qu'herbicide en au moins un composé de formule (I) selon l'une quelconque des revendications 1 à 6.

8. Agents herbicides selon la revendication 7 en mélange avec des adjuvants de formulation.

9. Agents herbicides selon la revendication 7 ou 8 contenant au moins une autre substance active en tant que pesticide du groupe formé par les insecticides, les acaricides, les herbicides, les fongicides, les phytoprotecteurs et les régulateurs de croissance.

10. Agents herbicides selon la revendication 9 contenant un antidote.

11. Agents herbicides selon la revendication 9 ou 10 contenant un autre herbicide.

12. Procédé pour lutter contre des plantes non souhaitées, **caractérisé en ce qu'**on applique une quantité active d'au moins un composé de formule (I) selon l'une quelconque des revendications 1 à 6 ou d'un agent herbicide selon l'une quelconque des revendications 7 à 11 sur les plantes ou sur le lieu de la croissance non souhaitée des plantes.

13. Utilisation de composés de formule (I) selon l'une quelconque des revendications 1 à 6 ou d'un agent herbicide selon l'une quelconque des revendications 7 à 11 pour lutter contre des plantes non souhaitées.

14. Utilisation selon la revendication 13, **caractérisée en ce que** les composés de formule (I) sont utilisés pour lutter contre des plantes non souhaitées dans les cultures de plantes utiles.

15. Utilisation selon la revendication 14, **caractérisée en ce que** les plantes utiles sont des plantes utiles transgéniques.

16. Composés de formule (II) 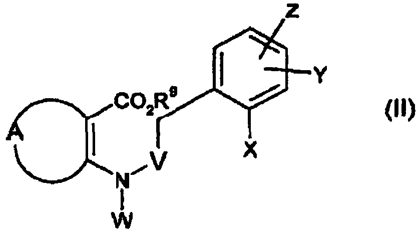 dans laquelle les substituants sont définis comme dans l'une quelconque des revendications 1 à 6 et R⁹ représente (C₁-C₆)-alkyle.
